# EUROPEAN PATENT APPLICATION

(11) **EP 2 762 475 A1**
(43) Date of publication of application: **06.08.2014**
(21) Application number: 14165975.5
(22) Date of filing: 05.11.2004
(51) Int. Cl.: C07D 401/04, A61K 31/4709, A61K 31/496, A61K 31/4184, A61P 35/00

(54) **Pharmaceutically acceptable salts of quinolinone compounds and their medical use**

(30) Priority: 07.11.2003 US 517915 P; 02.12.2003 US 526425 P; 02.12.2003 US 526426 P; 19.02.2004 US 546017 P
(62) Divisional of application: 04816941.1
(71) Applicant: Novartis Vaccines and Diagnostics, Inc., Emeryville, CA 94608 (US)
(72) Inventor: Machajewski, Timothy D., Emeryville, CA California 94608-2916 (US); Zhu, Shuguang, San Diego, CA California 92121 (US); Ryckman, David, San Diego, CA California 92130 (US); Harwood, Eric, Seattle, WA Washington 98115 (US); Cai, Shaopei, Emeryville, CA California 94608-2917 (US); Chou, Joyce, Novato, CA California 94949 (US); Shang, Xiao, Bellevue, WA Washington 98008 (US); Tesconi, Marc, Monroe, NY New York 10950 (US); Okhamafe, Augustus, Emeryville, CA California 94608-2917 (US)
(74) Representative: Rudge, Sewkian

(57) **Abstract**

A salt of a compound of Formula I or a tautomer of the compound, wherein Formula I has the following structure and R¹-R⁹ and R¹²-R¹⁴ are as defined herein

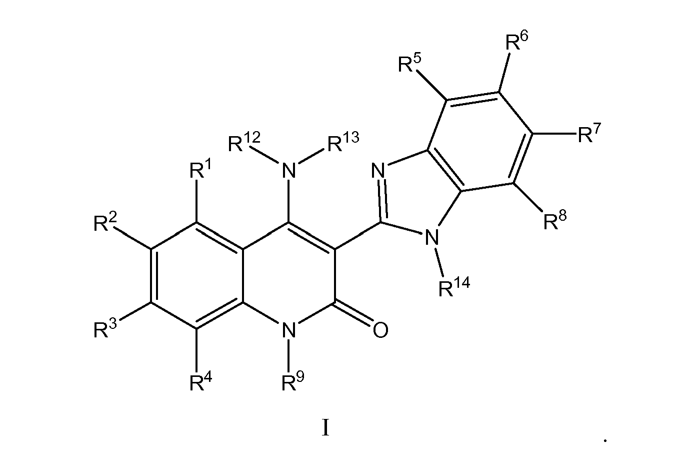

## Description

### FIELD OF THE INVENTION

This invention pertains generally to the preparation of pharmaceutically acceptable salts of specific protein kinase inhibiting quinolinone compounds having improved aqueous solubility and other desirable physicochemical properties (e.g., stability, hygroscopicity, crystallinity, and compactibility). The quinolinone compounds are useful in treating diseases characterized by angiogenesis including cancer. More specifically, the invention described herein pertains to pharmaceutically acceptable salts of specific protein kinase inhibiting quinolinone compounds which have improved aqueous solubility and desirable drug substance properties. The present invention provides these salts which are inhibitors of vascular endothelial growth factor receptor tyrosine kinase and can be used in methods of treating patients wherein inhibition of vascular endothelial growth factor receptor tyrosine kinase is indicated.

### BACKGROUND OF THE INVENTION

Changing a drug substance form from its free base or acid to a salt form is one technique that may be employed to improve its pharmacokinetics or physicochemical properties such as absorption, bioavailability, aqueous solubility, stability, hygroscopicity, crystallinity, and processability (Handbook of Pharmaceutical Salts Properties, Selection, and Use; P. H. Stahl, C.G. Wermuth (Eds.): Chapter 5 Biological effects of the drug salt form; F. Pfannkuch, H. Rettig, P.H. Stahl. Preservation of Pharmaceutical Products to Salt Forms of Drugs and Absorption, Encyclopedia of Pharmaceutical Technology; J. Swarbick, J.C. Boylan; Vol. 13, p. 453-476). To date however, there is no reliable method to predict exactly what effect changing the salt would have on its physicochemical or pharmacokinetic properties. There is also no reliable way to predict which salt-forming agent or counterion will produce the most desirable pharmaceutical properties in a drug compound or substance. Depending on the dose required, aqueous solubilities in the range of 0.1-1.0 mg/mL are typical for oral dosage formulations. Parenteral formulation may require higher solubilities, such as 10 mg/mL or greater (Handbook of Pharmaceutical Salts Properties, Selection, and Use; P. H. Stahl, C.G. Wermuth (Eds.): Chapter 7. A Procedure for Salt Selection and Optimization; M. J. Bowker).

Generally, a salt may be manufactured by mixing an acid and a base in a suitable media (solution or resin). Typical approaches to induce the salt to crystallize from the medium include cooling, evaporation, pH shift, and addition of anti-solvent among others. For example, a salt of a basic compound may be prepared by reacting this compound with an inorganic acid, an organic acid, an acidic amino acid. Salts formed by adding inorganic bases and organic bases, such as amine cation, ammonium, and quaternary ammonium compounds to a drug substance are also included in the definition of "pharmaceutically acceptable salts."

The selected salt ion can significantly influence the pharmacokinetics of a drug, especially the absorption or membrane-transfer process. The salt form of a drug substance is known to influence the factors that affect bioavailability. Salts differ in their solubility profiles and dissolution rates which affects the rate of absorption of the drug and its bioavailability.

The solubility of a drug substance can be improved by converting the free base or acid into a salt form. The solubility of a drug substance affects the pharmacokinetic profile, chemical stability, and final formulation of the ultimate dosage composition. The solubility of a compound depends upon the physical and chemical properties of the drug substance and other factors such as temperature, pressure and solvent properties (such as pH). The physical and chemical properties can vary from one salt form to another.

The particular salt form of a drug substance can affect its stability which can significantly affect the choice of the dosage form, the manufacturing process, packaging, and the ultimate therapeutic benefit of the finished drug product. Factors that influence stability include hygroscopicity and crystallinity. Non hygroscopic salts as well as crystalline, non-amorphous salts are generally preferred for developing formulations with optimal storage, handling, and processing properties.

Processability (i.e., crystal morphology and compactibility) is another characteristic to consider when selecting a salt form. Generally, plate-shaped crystals are preferred over needle-shaped crystals because of their better bulk powder flow properties. Compactibility is defined as the ability of the powdered material to be compressed into a tablet of specified tensile strength and is of particular importance in a high dose drug.

The ability to form a salt from the free acid or base of a drug substance provides a means for altering the chemical, physical, and/or biological characteristics of a drug product without modifying its chemical formula. Such changes allow formulations to be developed which have increased solubility, stability, processability, and bioavailability over the parent drug substance.

Capillaries reach into almost all tissues of the human body and supply tissues with oxygen and nutrients as well as removing waste products. Under typical conditions, the endothelial cells lining the capillaries do not divide, and capillaries, therefore, do not normally increase in number or size in a human adult. Under certain normal conditions, however, such as when a tissue is damaged, or during certain parts of the menstrual cycle, the capillaries begin to proliferate rapidly. This process of forming new capillaries from pre-existing blood vessels is known as angiogenesis or neovascularization. See Folkman, J. Scientific American 275, 150-154 (1996). Angiogenesis during wound healing is an example of pathophysiological neovascularization during adult life. During wound healing, the additional capillaries provide a supply of oxygen and nutrients, promote granulation tissue, and aid in waste removal. After termination of the healing process, the capillaries normally regress. Lymboussaki, A. "Vascular Endothelial Growth Factors and their Receptors in Embryos, Adults, and in Tumors" Academic Dissertation, University of Helsinki, Molecular/Cancer Biology Laboratory and Department of Pathology, Haartman Institute, (1999).

Angiogenesis also plays an important role in the growth of cancer cells. It is known that once a nest of cancer cells reaches a certain size, roughly 1 to 2 mm in diameter, the cancer cells must develop a blood supply in order for the tumor to grow larger as diffusion will not be sufficient to supply the cancer cells with enough oxygen and nutrients. Thus, inhibition of angiogenesis is expected to halt the growth of cancer cells.

Receptor tyrosine kinases (RTKs) are transmembrane polypeptides that regulate developmental cell growth and differentiation, remodeling and regeneration of adult tissues. Mustonen, T. et al., J. Cell Biology 129, 895-898 (1995); van der Geer, P. et al. Ann Rev. Cell Biol. 10, 251-337 (1994). Polypeptide ligands known as growth factors or cytokines, are known to activate RTKs. Signaling RTKs involves ligand binding and a shift in conformation in the external domain of the receptor resulting in its dimerization. Lymboussaki, A. "Vascular Endothelial Growth Factors and their Receptors in Embryos, Adults, and in Tumors" Academic Dissertation, University of Helsinki, Molecular/Cancer Biology Laboratory and Department of Pathology, Haartman Institute, (1999); Ullrich, A. et al., Cell 61, 203-212 (1990). Binding of the ligand to the RTK results in receptor trans-phosphorylation at specific tyrosine residues and subsequent activation of the catalytic domains for the phosphorylation of cytoplasmic substrates. Id.

FLT-3 is a receptor tyrosine kinase belonging to the PDGF Receptor family expressed on acute myelogenous leukemia (AML) cells in a majority of patients and can be present in wildtype form or have activating mutations that result in constitutively active kinase function. An internal tandem repeat (ITD) mutation is expressed in about 25% of AML patients and has been associated with poor prognosis in AML patients. Levis, M. et al., Blood 99, 11; 2002.

c-Kit is another receptor tyrosine kinase belonging to the PDGF Receptor family and is normally expressed in hematopoietic progenitor, mast and germ cells. C-kit expression has been implicated in a number of cancers including mast cell leukemia, germ cell tumors, small-cell lung carcinoma, gastroinstestinal stromal tumors, acute myelogenous leukemia (AML), neuroblastoma, melanoma, ovarian carcinoma, breast carcinoma. Heinrich, M. C. et al., J. Clin. Onc. 20, 6 1692-1703, 2002 (review article); Smolich, B. D. et al., Blood, 97, 5; 1413-1421.

c-ABL is a tyrosine kinase that was originally identified as an oncogene product from the genome of the Abelson murine leukemia virus. About 90% of chronic myelogenous leukemia (CML), 20-30% of acute lymphoblastic leukemia (ALL) and about 1% of acute myeloblastic leukemia (AML) have a reciprocal translocation between chromosome 9 and 22. The translocation results in the 'Philadelphia' chromosome and is the reason for the expression of a chimeric BCR/ABL transcript.

FGFR3 is a tyrosine kinase associated with various cancers. Fibroblast growth factor receptor 3 (FGFR3) is a class IV receptor tyrosine kinase. FGFR3 is deregulated due to a t(4,14) translocation in about 15-20% of multiple myeloma patients. This translocation causes the expression of a functional FGFR3 that can respond to FGF1 in e.g., the bone microenvironment. In some cases, activating mutations that make FGFR3 ligand independent have been identified. These activating FGFR3 mutations have been found to cause Ras-like tumor progression and evidence exists that similar signaling pathways are utilized (Chesi, et al., Blood 2001 97 729-736.).

CSF-1 (colony-stimulating factor-1) and its receptor Macrophage CSFR-1 (Fms) are required for macrophage proliferation and differentiation as well as placental development. It is expressed during pregnancy and lactation in the mammary gland. Abnormal expression of CSFR1 has been correlated with advanced stage and poor prognosis in breast cancer patients.

C-Met is a receptor tyrosine kinase that binds HGF (hepatocyte growth factor). C-Met is implicated in tumorigenesis, tumor progression and metastasis of multiple tumors including colon cancer, multiple myeloma, small and non small cell lung cancer and renal cell carcinoma. C-Met has been found mutated, amplified, and overexpressed in multiple cancers.

Two subfamilies of RTKs are specific to the vascular endothelium. These include the vascular endothelial growth factor (VEGF) subfamily and the Tie receptor subfamily. Class V RTKs include VEGF-1, VEGFR-2, and VEGFR-3. Shibuya, M. et al., Oncogene 5, 519-525 (1990); Terman, B. et al., Oncogene 6, 1677-1683 (1991); Aprelikova, O. et al., Cancer Res. 52, 746-748 (1992).

Members of the VEGF subfamily have been described as being able to induce vascular permeability and endothelial cell proliferation and further identified as a major inducer of angiogenesis and vasculogenesis. Ferrara, N. et al., Endocrinol. 1. Rev. 18, 4-25 (1997). VEGF is known to specifically bind to RTKs including VEGFR-1 and VEGFR-2. DeVries, C. et al., Science 255, 989-991 (1992); Quinn, T. et al., Proc. Natl. Acad. Sci. 90, 7533-7537 (1993). VEGF stimulates the migration and proliferation of endothelial cells and induces angiogenesis both *in vitro* and *in vivo.* Connolly, D. et al., J. Biol. Chem. 264, 20017-20024 (1989); Connolly, D. et al., J. Clin. Invest. 84, 1470-1478 (1989); Ferrara, N. et al., Endocrino. Rew. 18, 4-25 (1997); Leung, D. et al., Science 246, 1306-1309 (1989); Plouet, J. et al., EMBO J 8, 3801-3806 (1989).

Because angiogenesis is known to be critical to the growth of cancer and to be controlled by VEGF and VEGF-RTK, substantial efforts have been undertaken to develop therapeutics that are antagonists of VEGF-RTK to thereby inhibit or retard angiogenesis, and, hopefully, interfere or stop tumor proliferation.

Class III RTKs are characterized by an extracellular region composed of five immunoglobulin-like domains and by a split tyrosine kinase domain. Some of the Class III RTKs which are inhibited by the compounds of Formula I include, but are not limited to, KIT, FMS, FLT3, PDGFRα, and PDGFRβ.

Class IV RTKs contain three immunoglobulin-like domains in their extracellular regions. For example, FGFR is a class IV RTK which is inhibited by the compounds of Formula I.

Examples of Class V RTKs that are inhibited by the compound of Formula I include, but are not limited to, VEGF-1, VEGFR-2, and VEGFR-3.

A wide variety of chemical compounds and compositions have been reported as having activity against one of more the VEGF-RTKs. Examples include quinoline derivatives such as described in WO 98/13350, aminonicotinamide derivatives (see, *e.g.,* WO 01/55114), antisense compounds (see, *e.g.,* WO 01/52904), peptidomimetics (see, *e.g.,* WO 01/52875), quinazoline derivatives (see, *e.g.,* U.S. Patent No. 6,258,951) monoclonal antibodies (see, *e.g.,* EP 1 086 705 A1), various 5,10,15,20-tetraaryl-porphyrins and 5,10,15-triaryl-corroles (see, *e.g.,* WO 00/27379), heterocyclic alkanesulfonic and alkane carboxylic acid derivatives (see, *e.g.,* DE19841985), oxindolylquinazoline derivatives (see, *e.g.,* WO 99/10349), 1,4-diazaanthracine derivatives (see, *e.g.,* U.S. Patent No. 5,763,441), and cinnoline derivatives (see, *e.g.,* WO 97/34876), and various indazole compounds (see, *e.g.,* WO 01/02369 and WO 01/53268).

Various indolyl substituted compounds have recently been disclosed in WO 01/29025, WO 01/62251, and WO 01/62252, and various benzimidazolyl compounds have recently been disclosed in WO 01/28993. These compounds are reportedly capable of inhibiting, modulating, and/or regulating signal transduction of both receptor-type and non-receptor tyrosine kinases. Some of the disclosed compounds contain a quinolone fragment bonded to the indolyl or benzimidazolyl group.

The synthesis of 4-hydroxy quinolone and 4-hydroxy quinoline derivatives is disclosed in a number of references. For example, Ukrainets *et al.* have disclosed the synthesis of 3-(benzimidazol-2-yl)-4-hydroxy-2-oxo-1,2-dihydroquinoline. Ukrainets, I. et al., Tet. Lett. 42, 7747-7748 (1995); Ukrainets, I. et al., Khimiya Geterotsiklicheskikh Soedinii, 2, 239-241(1992). Ukrainets has also disclosed the synthesis, anticonvulsive and antithyroid activity of other 4-hydroxy quinolones and thio analogs such as 1H-2-oxo-3-(2-benzimidazolyl)-4-hydroxyquinolinine. Ukrainets, I. et al., Khimiya Geterotsiklicheskikh Soedinii, 1, 105-108 (1993); Ukrainets, I. et al., Khimiya Geterotsiklicheskikh Soedinii, 8, 1105-1108 (1993); Ukrainets, I. et al., Chem. Heterocyclic Comp. 33, 600-604, (1997).

The synthesis of various quinoline derivatives is disclosed in WO 97/48694. These compounds are disclosed as capable of binding to nuclear hormone receptors and being useful for stimulating osteoblast proliferation and bone growth. The compounds are also disclosed as being useful in the treatment or prevention of diseases associated with nuclear hormone receptor families.

Various quinoline derivatives in which the benzene ring of the quinolone is substituted with a sulfur group are disclosed in WO 92/18483. These compounds are disclosed as being useful in pharmaceutical formulations and as medicaments.

Quinolone and coumarin derivatives have been disclosed as having use in a variety of applications unrelated to medicine and pharmaceutical formulations. References that describe the preparation of quinolone derivatives for use in photopolymerizable compositions or for luminescent properties include: U.S. Patent No. 5,801,212 issued to Okamoto et al.*;* JP 8-29973; JP 7-43896; JP 6-9952; JP 63-258903; EP 797376; and DE 23 63 459.

Quinolinone derivatives have been disclosed in U.S. Patent Application Serial No. 09/951,265 published on August 8, 2002 as US 2002/0107392 and in a PCT Application published on March 21, 2002 as WO 02/22598A1, which are useful as inhibitors of VEGF-RTK. Both of the foregoing references are hereby incorporated by reference in their entirety and for all purposes. However, a continuing need exists for improving the bioavailability, stability, solubility, and hygroscopicity of compounds that inhibit the proliferation of capillaries, inhibit the growth of tumors, and/or inhibit vascular endothelial growth factor receptor tyrosine kinase and pharmaceutical formulations that contain such compounds by conversion of the free bases and acids into their corresponding salt forms.

As a result of inhibition of various RTKs, other ligand-stimulated cellular functions are blocked, including activation of downstream signaling molecules, cellular proliferation and survival. Agents which act as inhibitors of specific RTKs are useful in the treatment of disseminated disease and leukemia, as well as solid tumors, outside of the agent's antiangiogenic activity. That is, compounds such as those described in WO 01/60814, which have a broad range of activity at different RTKs and PTKs, are antiangiogenic agents as well as antitumor agents.

Multiple myeloma (MM), a disease of malignant B cells, is characterized by the accumulation of clonal plasma cells in the bone marrow (BM) and osteolytic bone lesions. Autologous stem cell transplant (ASCT) and advances in supportive care have had a significant impact on the disease and long-term survival. Attal, M. et al., N. Engl. J. Med., 1996; 335:91-97; and Barlogie, B. et al., Blood, 1997; 89:789-793. However, patients invariably relapse, and MM remains a universal fatal disease. The identification of nonrandom chromosomal translocations in MM has resulted in the development of powerful prognostic tools and the identification of novel molecular targets. Nearly half of patients with MM overexpress a putative oncogene, dysregulated by one of five recurrent immunoglobulin heavy (IgH) translocations: 11q13 (cyclin D1), 6p21 (cyclin D3), 4p16 (FGFR3 and MMSET), 16q23 (c-maf) and 20q11 (mafB). Kuehl, W. M. et al., Nat Rev Cancer, 2002; 2:175-187; and Avet-Loiseau, H. et al., Blood, 2002; 99:2185-2191. These translocations likely represent an early and possibly seminal event in the development of MM. More recently, it has become clear that these specific IgH translocations impart prognostic significance. Particularly, the t(4;14) translocation with occurs in approximately 20% of patients appears to confer a particularly poor prognosis for MM, with no apparent therapeutic benefit to ASCT. Fonseca, R. et al., Blood, 2003; 101:4569-4575; Keats, J. J. et al., Blood, 2003; 101:1520-1529; Moreau, P. et al., Blood, 2002; 100:1579-1583; and Chang, H. et al., Br. J. Haematol., 2004; 125:64-68. Clearly, novel treatment approaches are required for these patients.

The t(4;14) translocation is unusual in that it appears to dysregulate two potential oncogenes, MMSET on der(4) and FGFR3 on der(14). Chesi, M. et al., Nat. Genet., 1997; 16:260-265; and Chesi, M. et al., Blood, 1998; 92:3025-3034. Whether dysregulation of either or both of these genes is critical for MM pathogenesis is not known, however several lines of evidence support a role for FGFR3 in tumor initiation and progression. Activation of WT FGFR3, a RTK, promotes proliferation and survival in myeloma cells and is weakly transforming in a hematopoetic mouse model. Plowright, E. E. et al., Blood, 2000; 95:992-998; Chesi, M. et al., Blood, 2001; 97:729-736; and Pollett, J. B. et al., Blood, 2002; 100:3819-3821. Subsequent acquisition of activating mutations of FGFR3 in some MM are associated with progression to late stage myeloma and are strongly transforming in several experimental models. Chesi, M. et al., Blood, 2001; 97:729-736; and Li, Z. et al., Blood, 2001; 97:2413-2419. *In vitro* studies suggest that FGFR3 can impart chemoresistance, an observation supported by clinical data that demonstrate poor responses to conventional chemotherapy and shortened median survivaloft(4;14) MM patients. Fonseca, R. et al., Blood, 2003; 101:4569-4575; Keats, J. J. et al., Blood, 2003; 101:1520-1529; Moreau, P. et al., Blood, 2002; 100:1579-1583; and Chang, H. et al., Br. J. Haematol., 2004; 125:64-68. These findings suggest that ectopic expression of FGFR3 may play a significant, albeit not a singular, role in myeloma oncogenesis thus making this RTK a target for molecular based therapy.

Inhibition of FGFR3 in t(4;14) MM cell lines induces cytotoxic responses demonstrating that these cells remain dependent on FGFR3 signaling despite the complexity of genetic alterations in these cells derived from end stage patients. Trudel, S. et al., Blood, 2004; 103:3521-3528; Paterson, J. L. et al., Br. J. Haematol., 2004; 124:595-603; and Grand, E. K. et al., Leukemia, 2004; 18:962-966. These observations are congruent with the results of receptor tyrosine inactivation in a range of human malignancies where clinical successes have been documented and encourage the clinical development of FGFR3 inhibitors for the treatment of these poor-prognosis patients. Druker, B. J. et al., N. Engl. J. Med., 2001; 344:1031-1037; Demetri, G. D. et al., N. Engl. J. Med., 2002; 347:472-480; Slamon, D. J. et al., N. Engl. J. Med. 2001; 344:783-792; and Smith, B. D. et al., Blood, 2004; 103:3669-3676.

### SUMMARY OF THE INVENTION

The invention provides pharmaceutically acceptable salts of various compounds, methods for making such salts, pharmaceutical formulations and medicaments that include such salts, uses of the salts in preparing medicaments and pharmaceutical formulations for use in treating various conditions, and methods of treating that use the pharmaceutically acceptable salt or pharmaceutical formulations of the invention.

Therefore, in one aspect the invention provides a lactate salt of a compound of Formula I or a tautomer of the compound. The lactate may be present in various molar ratios such that in some embodiments, molar ratio of acid to free base includes any fractional ratio between 0.5-4.5. In some such embodiments, the salt includes mono-lactate or bis-lactate salts. Compounds of Formula I have the following structure: 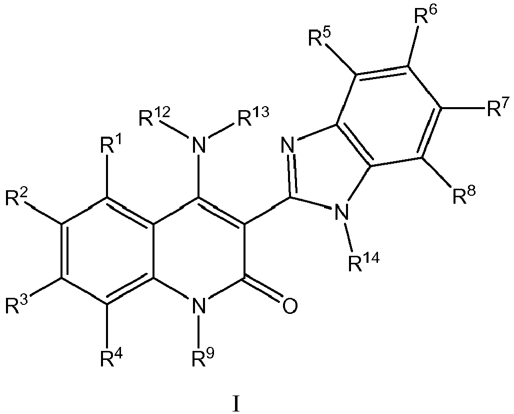 wherein,
R¹, R², R³, and R⁴ may be the same or different and are independently selected from the group consisting of H, Cl, Br, F, I, -CN, -NO₂, -OH, -OR¹⁵ groups, -NR¹⁶R¹⁷ groups, substituted and unsubstituted amidinyl groups, substituted and unsubstituted guanidinyl groups, substituted and unsubstituted primary, secondary, and tertiary alkyl groups, substituted and unsubstituted aryl groups, substituted and unsubstituted alkenyl groups, substituted and unsubstituted alkynyl groups, substituted and unsubstituted heterocyclyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, and -C(=O)R¹⁸ groups;
R⁵, R⁶, R⁷, and R⁸ may be the same or different and are independently selected from the group consisting of H, Cl, Br, F, I, -NO₂, -OH, -OR¹⁹ groups, -NR²⁰R²¹ groups, -SH, -SR²² groups, -S(=O)R²³ groups, -S(=O)₂R²⁴ groups, -CN, substituted and unsubstituted amidinyl groups, substituted and unsubstituted guanidinyl groups, substituted and unsubstituted primary, secondary, and tertiary alkyl groups, substituted and unsubstituted aryl groups, substituted and unsubstituted alkenyl groups, substituted and unsubstituted alkynyl groups, substituted and unsubstituted heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, -C(=O)R²⁵ groups, substituted and unsubstituted aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R⁹ is H;
R¹² is selected from the group consisting of H, substituted and unsubstituted alkyl groups, substituted and unsubstituted aryl groups, and substituted and unsubstituted heterocyclyl groups;
R¹³ is selected from the group consisting of H, substituted and unsubstituted alkyl groups, substituted and unsubstituted aryl groups, substituted and unsubstituted heterocyclyl groups, -OH, alkoxy groups, aryloxy groups, -NH₂, substituted and unsubstituted heterocyclylalkyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted alkylamino groups, substituted and unsubstituted arylamino groups, substituted and unsubstituted dialkylamino groups, substituted and unsubstituted diarylamino groups, substituted and unsubstituted (alkyl)(aryl)amino groups, -C(=O)H, -C(=O)-alkyl groups, -C(=O)-aryl groups, -C(=O)O-alkyl groups, -C(=O)O-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)-heterocyclyl groups, -C(=O)-O-heterocyclyl groups, - C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R¹⁴ is H;
R¹⁵ and R¹⁹ may be the same or different and are independently selected from the group consisting of substituted and unsubstituted alkyl groups, substituted and unsubstituted aryl groups, substituted and unsubstituted heterocyclyl groups, substituted and unsubstituted heterocyclylalkyl groups, -C(=O)H, -C(=O)-alkyl groups, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl, substituted and unsubstituted diheterocyclylaminoalkyl, substituted and unsubstituted (heterocyclyl)(alkyl)aminoalkyl, substituted and unsubstituted (heterocyclyl)(aryl)aminoalkyl, substituted and unsubstituted alkoxyalkyl groups, substituted and unsubstituted hydroxyalkyl groups, substituted and unsubstituted aryloxyalkyl groups, and substituted and unsubstituted heterocyclyloxyalkyl groups;
R¹⁶ and R²⁰ may be the same or different and are independently selected from the group consisting of H, substituted and unsubstituted alkyl groups, substituted and unsubstituted aryl groups, and substituted and unsubstituted heterocyclyl groups;
R¹⁷ and R²¹ may be the same or different and are independently selected from the group consisting of H, substituted and unsubstituted alkyl groups, substituted and unsubstituted aryl groups, substituted and unsubstituted heterocyclyl groups, -C(=O)H, -C(=O)-alkyl groups, -C(=O)-aryl groups, -C(=O)NH₂,-C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)O-alkyl groups, -C(=O)O-aryl groups, substituted and unsubstituted heterocyclylalkyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, -C(=O)-heterocyclyl groups, -C(=O)-O-heterocyclyl groups, -C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R¹⁸, R²³, R¹⁴, and R²⁵ may be the same or different and are independently selected from the group consisting of H, -NH₂, -NH(alkyl) groups, -NH(aryl) groups, -N(alkyl)₂ groups, -N(aryl)₂ groups, -N(alkyl)(aryl) groups, - NH(heterocyclyl) groups, -N(heterocyclyl)(alkyl) groups, -N(heterocyclyl)(aryl) groups, -N(heterocyclyl)₂ groups, substituted and unsubstituted alkyl groups, substituted and unsubstituted aryl groups, -OH, substituted and unsubstituted alkoxy groups, substituted and unsubstituted aryloxy groups, substituted and unsubstituted heterocyclyl groups, -NHOH, -N(alkyl)OH groups, -N(aryl)OH groups, -N(alkyl)O-alkyl groups, -N(aryl)O-alkyl groups, -N(alkyl)O-aryl groups, and -N(aryl)O-aryl groups; and
R²² is selected from the group consisting of substituted and unsubstituted alkyl groups, substituted and unsubstituted aryl groups, and substituted and unsubstituted heterocyclyl groups.

In some embodiments of the lactate salt of compounds of Formula I or tautomers thereof, at least one of R⁵, R⁶, R⁷, or R⁸ is selected from the group consisting of substituted and unsubstituted amidinyl groups, substituted and unsubstituted guanidinyl groups, substituted and unsubstituted saturated heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups; -OR¹⁹ groups wherein R¹⁹ is selected from the group consisting of substituted and unsubstituted aryl groups, substituted and unsubstituted heterocyclyl groups, substituted and unsubstituted heterocyclylalkyl groups, -C(=O)H, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted diheterocyclylaminoalkyl groups, substituted and unsubstituted (heterocyclyl)(alkyl)aminoalkyl groups, substituted and unsubstituted (heterocyclyl)(aryl)aminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups; -NR¹⁰R²¹ groups wherein R²⁰ is selected from the group consisting of substituted and unsubstituted heterocyclyl groups; -NR²⁰R²¹ groups wherein R²¹ is selected from the group consisting of substituted and unsubstituted heterocyclyl groups, -C(=O)H, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)O-alkyl groups, -C(=O)O-aryl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, substituted and unsubstituted heterocyclylalkyl groups, and substituted and unsubstituted heterocyclyloxyalkyl groups; and -C(=O)R²⁵ groups wherein R²⁵ is selected from the group consisting of H, -NH₂, -NH(alkyl) groups, -NH(aryl) groups, -N(alkyl)₂ groups, -N(aryl)₂ groups, -N(alkyl)(aryl) groups, -NH(heterocyclyl) groups, -N(heterocyclyl)(alkyl) groups, -N(heterocyclyl)(aryl) groups, -N(heterocyclyl)₂ groups, substituted and unsubstituted aryl groups, substituted and unsubstituted aryloxy groups, and substituted and unsubstituted heterocyclyl groups.

In one embodiment, the lactate salt of the compound of Formula I or the tautomer thereof has a water solubility at 22°C of from about 5 mg/mL to about 400 mg/mL. In some embodiments, the salt of the compound of Formula I or the tautomer thereof has a water solubility from about 100 mg/mL to about 400 mg/mL. In other embodiments, the salt of the compound of Formula I or the tautomer thereof has a water solubility from about 200 mg/mL to about 400 mg/mL. In some embodiments, the salt of the compound of Formula I or the tautomer thereof has a water solubility of greater than 30 mg/mL. In other embodiments, the salt of the compound of Formula I or the tautomer thereof has a water solubility from about 150 mg/mL to about 250 mg/mL. In another embodiment, the salt of the compound of Formula I or the tautomer thereof is capable of dissolution in an aqueous medium below about pH 7, such as from pH 1-7, from pH 3-7, or from pH 4-7. In some embodiments, the lactate salt is crystalline and in some such embodiments comprises plate-shaped crystals.

In some embodiments the lactate salt of the compound of Formula I or the tautomer thereof is a DL-lactate salt. In other embodiments the salt of the compound of Formula I is an L-lactate salt. In still other embodiments the salt of the compound of Formula I is a D-lactate salt. In still other embodiments, the salt of the compound of Formula I is a mixture of the D-lactate salt and the L-lactate salts. The salts can be present in more than one form. According to the present invention, the racemate or a specific enantiomer can be used to prepare the inventive salts.

In some embodiments the lactate salt of the compound of Formula I is a salt of 4-amino-5-fluoro-3-[6-(4-methylpiperazin-1-yl)-1H-benzimidazol-2-yl]quinolin-2(1H)-one or a tautomer thereof. In some such embodiments, the salt is a mono-lactate or bis-lactate salt of 4-amino-5-fluoro-3-[6-(4-methylpiperazin-1-yl)-1H-benzimidazol-2-yl]quinolin-2(1H)-one or a tautomer thereof. In more preferred embodiments, the salt is a DL-lactate salt of 4-amino-5-fluoro-3-[6-(4-methylpiperazin-1-yl)-1H-benzimidazol-2-yl]quinolin-2(1H)-one or a tautomer thereof. In other such embodiments, the salt is an L-lactate salt of 4-amino-5-fluoro-3-[6-(4-methylpiperazin-1-yl)-1H-benzimidazol-2-yl]quinolin-2(1H)-one or a tautomer thereof. In still other embodiments, the salt is a D-lactate salt of 4-amino-5-fluoro-3-[6-(4-methylpiperazin-1-yl)-1H-benzimidazol-2-yl]quinolin-2(1H)-one or a tautomer thereof.

In some embodiments of the lactate salt of the compound of Formula I, R¹² and R¹³ are both H.

In some embodiments of the lactate salt of the compound of Formula I, R¹ is selected from the group consisting of F, Cl, substituted and unsubstituted alkoxy groups, substituted and unsubstituted heterocyclylalkoxy groups, substituted and unsubstituted heterocyclyl groups, substituted and unsubstituted alkylaminoalkoxy groups, substituted and unsubstituted arylaminoalkoxy groups, substituted and unsubstituted dialkylaminoalkoxy groups, substituted and unsubstituted diarylaminoalkoxy groups, and substituted and unsubstituted (alkyl)(aryl)aminoalkoxy groups.

In some embodiments of the lactate salt of the compound of Formula I, R¹ is selected from the group consisting of H and F. In some such embodiments, R¹ is F. In some embodiments, R² is H.

In some embodiments of the lactate salt of the compound of Formula I, at least one R⁵, R⁶, R⁷, and R⁸ is a substituted or unsubstituted heterocyclyl group. In some such embodiments, at least one of R⁶ or R⁷ is a substituted or unsubstituted heterocyclyl group. In other such embodiments, at least one of R⁵, R⁶, R⁷, and R⁸ is a substituted or unsubstituted heterocyclyl group comprising at least one O or N atom. In some such embodiments, at least one of R⁶ or R⁷ is a substituted or unsubstituted heterocyclyl group comprising at least one O or N atom. In some such embodiments, the substituted or unsubstituted heterocyclyl group or the heterocyclyl group is selected from morpholine, piperazine, piperidine, pyrrolidine, thiomorpholine, homopiperazine, tetrahydrothiophene, tetrahydrofuran, or tetrahydropyran. In other such embodiments, at least one of R⁵, R⁶, R⁷, or R⁸, and in some such embodiments one of R⁶ or R⁷ is selected from substituted or unsubstituted morpholine groups, or substituted and unsubstituted piperazine groups.

In some embodiments of the lactate salt of the compound of Formula I, at least one of R⁶ or R⁷ is selected from substituted or unsubstituted morpholine groups, or substituted or unsubstituted piperazine groups. In some such embodiments, R¹ is F. In some such embodiments, R² is H. In some such embodiments, R¹² and R¹³ are both H. In other such embodiments, R⁵ is H and R⁸ is H. In other such embodiments, R³ is H, and R⁴ is H.

In some embodiments of the lactate salt of the compound of Formula I, R⁵ and R⁸ are both H.

In some embodiments of the lactate salt of the compound of Formula I, at least one of R⁶ or R⁷ is selected from the group consisting of -NR²⁰R²¹ groups wherein R²⁰ is selected from the group consisting of substituted and unsubstituted heterocyclyl groups; and -NR¹⁰R²¹ groups wherein R²¹ is selected from the group consisting of substituted and unsubstituted heterocyclyl groups, groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, substituted and unsubstituted heterocyclylalkyl groups, and substituted and unsubstituted heterocyclyloxyalkyl groups. In some such embodiments, R¹ is selected from the group consisting of H and F. In some such embodiments, R² is H. In some such embodiments, R¹² and R¹³ are both H. In some such embodiments, R⁵ is H and R⁸ is H. In some such embodiments, R³ is H and R⁴ is H.

The invention also provides lactate salts of a compound having Formula II or a tautomer of the compound. In some such embodiments, the salt includes the mono-lactate or bis-lactate salt. Compounds of Formula II have the following formula: 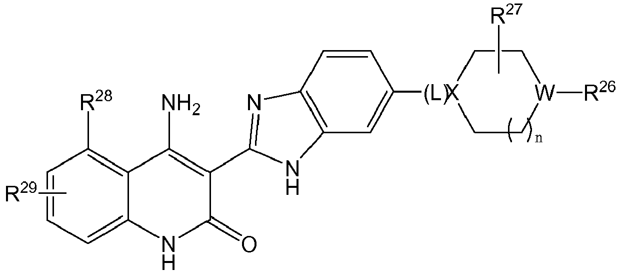 wherein:
L is a covalent bond, -(CH₂)ₘ-, -CHR³⁰-, or -N(R³¹)-;
X is CH or N;
W is CH, O, or N;
R²⁶ is selected from the group consisting of substituted or unsubstituted alkyl groups, substituted or unsubstituted alkylamino groups, substituted or unsubstituted dialkylamino, -OH, substituted or unsubstituted alkoxy groups, substituted or unsubstituted alkylaminoalkyl groups, substituted or unsubstituted dialkylaminoalkyl groups, substituted or unsubstituted heterocyclyl groups, substituted or unsubstituted heterocyclylalkyl groups; provided that when W is O, R²⁶ is absent,
R²⁷ is absent or selected from the group consisting of -NO₂, -OH, F, Cl, Br, I, -NH₂, substituted or unsubstituted alkyl groups, substituted or unsubstituted alkylamino groups, substituted or unsubstituted dialkylamino groups, substituted or unsubstituted alkylaminoalkyl groups, substituted or unsubstituted dialkylaminoalkyl groups, and substituted or unsubstituted alkoxy groups;
R²⁸ is selected from the group consisting of F, Cl, Br, I, -OH, -NH₂, and substituted or unsubstituted alkyl groups;
R^{29,} R³⁰, and R³¹ are independently selected from the group consisting of H, F, Cl, Br, I, substituted and unsubstituted alkyl groups, -OH, alkoxy groups, substituted and unsubstituted aryloxy groups, -NH₂, substituted and unsubstituted aminoalkyl groups, substituted and unsubstituted aryl groups, substituted and unsubstituted heteroaryl groups, substituted and unsubstituted heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted alkylamino groups, substituted and unsubstituted dialkylamino groups, substituted and unsubstituted diarylamino groups, substituted and unsubstituted (alkyl)(aryl)amino groups, -C(=O)H, -C(=O)-alkyl groups, -C(=O)-aryl groups, -C(=O)O-alkyl groups, -C(=O)O-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)-heterocyclyl groups, -C(=O)-O-heterocyclyl groups, -C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
n is 0, 1, or 2; and
m is 1, 2, 3, or 4.

In one embodiment, the lactate salt of the compound of Formula II or the tautomer thereof has a water solubility at 22°C of from about 5 mg/mL to about 400 mg/mL. In some embodiments, the salt of the compound of Formula II or the tautomer thereof has a water solubility from about 100 mg/mL to about 400 mg/mL. In other embodiments, the salt of the compound of Formula II or the tautomer has a water solubility from about 200 mg/mL to about 400 mg/mL. In some embodiments, the salt of the compound of Formula II or the tautomer thereof has a water solubility of greater than 30 mg/mL. In other embodiments, the salt of the compound of Formula II or the tautomer thereof has a water solubility from about 150 mg/mL to about 250 mg/mL. In another embodiment, the salt of the compound of Formula II or the tautomer thereof is capable of dissolution in an aqueous medium below about pH 7, such as from pH 1-7, from pH 3-7, or from pH 4-7.

In some embodiments, the lactate salt of the compound of Formula II or the tautomer thereof is a DL-lactate salt. In other embodiments, the lactate salt of the compound of Formula II is an L-lactate salt. In still other embodiments, the lactate salt of the compound of Formula II is a D-lactate salt.

In some embodiments of the lactate salt of the compound of Formula II, R²⁸ is F and R²⁹ is H. In some such embodiments, n is 1.

In some embodiments of the lactate salt of the compound of Formula II, n is 1.

In some embodiments, the invention provides a method for preparing a lactate salt of a compound or tautomer of the compound of Formula I or the compound of Formula II. Such methods typically include:
(a) suspending the free base of the compound of Formula I or Formula II or the tautomers thereof in a solvent or a mixture of solvents;
(b) contacting lactic acid, acetic with the compound of Formula I or Formula II or the tautomers thereof to provide a mixture;
(c) heating the mixture;
(d) cooling the mixture;
(e) and isolating the salt.

In some methods for preparing a lactate salt of the compound of Formula I or Formula II or the tautomers thereof, the mixture is cooled and the salt is precipitated out of the solution.

In some methods for preparing a lactate salt of the compound of Formula I or Formula II, the mixture is heated and refluxed prior to cooling.

In some methods for preparing a lactate salt of the compound of Formula I or Formula II, the solvent is a protic solvent.

In some embodiments of the method for preparing a lactate salt of the compound of Formula I or Formula II, the solvent is selected from methanol, ethanol, propanol, isopropanol, butanol, 2-butanol, acetone, butanone, dioxanes, water, tetrahydrofuran, or combinations of these.

In a preferred embodiment of the method for preparing a lactate salt of a compound or tautomer of the compound of Formula I or the compound of Formula II, the isolating step includes filtering the mixture.

The invention further provides a composition comprising a tablet of the lactate salt of the compound of Formula I or the tautomer thereof or of the compound of Formula II or the tautomer thereof.

The invention further provides pharmaceutical formulations and medicaments. Such formulations and medicaments include the lactate salt of Formula I or Formula II in combination with a pharmaceutically acceptable carrier.

The invention also provides methods of treating a patient in need of an inhibitor of vascular endothelial growth factor receptor tyrosine kinase. Such methods include administering an effective amount of a lactate salt or a pharmaceutical formulation or medicament that includes the lactate salt to a patient in need thereof.

Further objects, features and advantages of the invention will be apparent from the following detailed description.

### BRIEF DESCRIPTION OF THE DRAWINGS

FIG. 1 is a graph showing that 4-amino-5-fluoro-3-[6-(4-methylpiperazin-1-yl)-1H-benzimidazol-2-yl]quinolin-2(1H)-one inhibits proliferation of multiple myeloma cell lines including KMS 11, OPM-2, and H929.
FIG. 2 is a western blot showing that 4-amino-5-fluoro-3-[6-(4-methylpiperazin-1-yl)-1H-benzimidazol-2-yl]quinolin-2(1H)-one inhibits FGFR3 phosphorylation at 0.5 µM in KMS11 cells.
FIGS. 3A, 3B, and 3C are western blots showing that 4-amino-5-fluoro-3-[6-(4-methylpiperazin-1-yl)-1H-benzimidazol-2-yl]quinolin-2(1H)-one inhibits ERK phosphorylation at 0.5 µM in KMS11 cells (FIG. 3A), at 0.1 µM in OPM-2 cells (FIG. 3B), and has no effect on ERK phosphorylation up to 5 µM in H929 cells (FIG. 3C).
FIG. 4 is a graph showing apoptosis of KMS11 cells, as measured by AnnexinVPE staining, when such cells were incubated with 4-amino-5-fluoro-3-[6-(4-methylpiperazin-1-yl)-1H-benzimidazol-2-yl]quinolin-2(1H)-one at various concentrations.
FIG. 5 is a graph showing that 4-amino-5-fluoro-3-[6-(4-methylpiperazin-1-yl)-1H-benzimidazol-2-yl]quinolin-2(1H)-one, at various concentrations, has minor effects on the cell cycle of KMS11 cells when it is incubated with the cell for 72 hours but induces apoptosis.
FIG. 6 is a graph showing apoptosis of OPM-2 cells, as measured by AnnexinVPE staining, when such cells were incubated with 4-amino-5-fluoro-3-[6-(4-methylpiperazin-1-yl)-1H-benzimidazol-2-yl]quinolin-2(1H)-one at various concentrations.
FIG. 7 is a graph showing that 4-amino-5-fluoro-3-[6-(4-methylpiperazin-1-yl)-1H-benzimidazol-2-yl]quinolin-2(1H)-one, at various concentrations, has minor effects on the cell cycle of OPM-2 cells when it is incubated with the cells for 72 hours but induces apoptosis.
FIG. 8 is a graph showing that 4-amino-5-fluoro-3-[6-(4-methylpiperazin-1-yl)-1H-benzimidazol-2-yl]quinolin-2(1H)-one, at various concentrations, has minor to no effect on the cell cycle of H929 cells when it is incubated with the cells.
FIG. 9 is a graph showing that M-CSF mediated proliferation of a mouse myeloblastic cell line M-NFS-60 was inhibited when the cells were incubated with 4-amino-5-fluoro-3-[6-(4-methylpiperazin-1-yl)-1H-benzimidazol-2-yl]quinolin-2(1H)-one (EC₅₀ of 220 nM).
FIG. 10 is a graph showing that 4-amino-5-fluoro-3-[6-(4-methylpiperazin-1-yl)-1H-benzimidazol-2-yl]quinolin-2(1H)-one inhibits the viability of FGFR3 expressing B9 cells, but not parental interleukin-6 (IL6) stimulated cells. The values represent the mean +/- the standard deviation of four independent experiments.
FIG. 11 is a graph showing apoptosis in various human myeloma cell lines as assessed with a flow cytometric assay of annexin V binding and propidium iodide exclusion. KMS11, KMS18, OPM2, H929, and 8226 cells were incubated with vehicle (unshaded bar); with 100 nM (shaded bar) 4-amino-5-fluoro-3-[6-(4-methylpiperazin-1-yl)-1H-benzimidazol-2-yl]quinolin-2(1H)-one; and with 500 nM (hatched bar) 4-amino-5-fluoro-3-[6-(4-methylpiperazin-1-yl)-1H-benzimidazol-2-yl]quinolin-2(1H)-one. The values represent the mean +/- the standard deviation of four independent experiments.

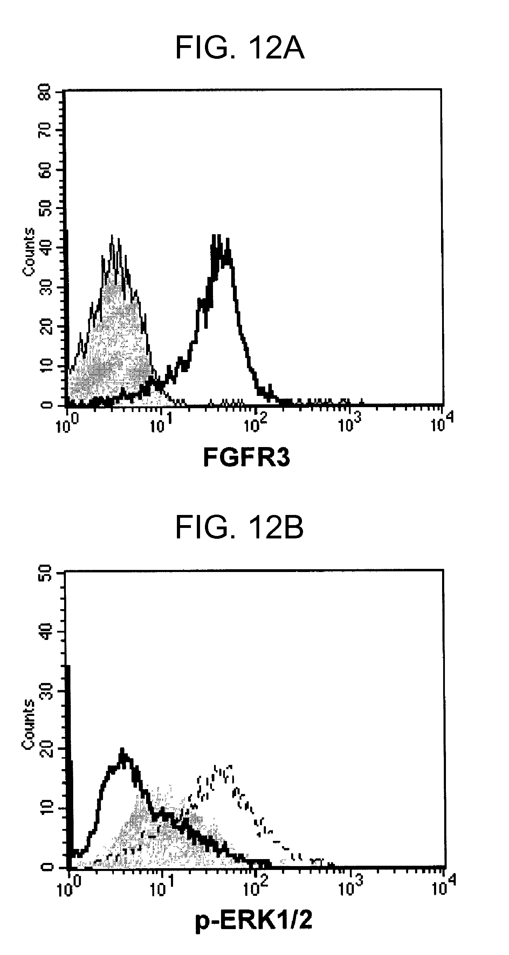

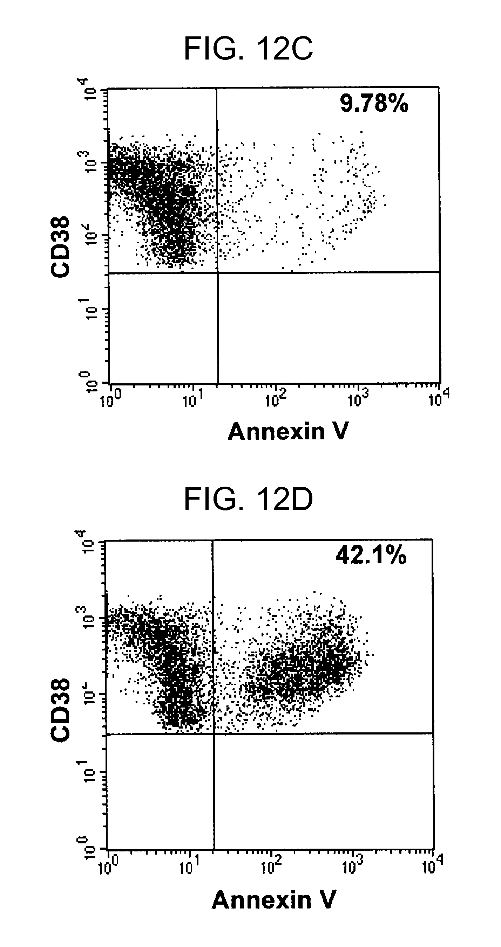

FIGS 12A-12D are graphs showing that 4-amino-5-fluoro-3-[6-(4-methylpiperazin-1-yl)-1H-benzimidazol-2-yl]quinolin-2(1H)-one inhibits FGF-mediated ERK1/2 phosphorylation and induces cytotoxicity in FGFR3 expressing primary multiple myeloma cells. FIG. 12A shows a graph obtained using flow cytometry of cells stained with FGFR3 antibody (open) or rabbit pre-immune serum (filled) and then stained with goat anti-rabbit FITC. Myeloma cells were identified by CD138 labeling. FIG. 12B shows a graph obtained using flow cytometry of primary myeloma cells incubated in the absence (filled) or presence of aFGF (- -) or preincubated with 500 nM 4-amino-5-fluoro-3-[6-(4-methylpiperazin-1-yl)-1H-benzimidazol-2-yl]quinolin-2(1H)-one for 2 hours and then stimulated with aFGF. ERK1/2 phosphorylation was assessed using flow cytometry. FIGS. 12C and 12D are graphs obtained using flow cytometry of primary myeloma cells cultured in growth medium in the presence of DMSO (FIG. 12C) or 500 nM 4-amino-5-fluoro-3-[6-(4-methylpiperazin-1-yl)-1H-benzimidazol-2-yl]quinolin-2(1H)-one (FIG. 12D). Cells were harvested after 7 days and stained with annexin V-FITC and analyzed by flow cytometry. Myeloma cells were identified by CD38⁺⁺/CD45⁻ labeling. The total percentage of CD38⁺⁺/CD45⁻/annexin V⁺ cells is shown in upper right quadrant.

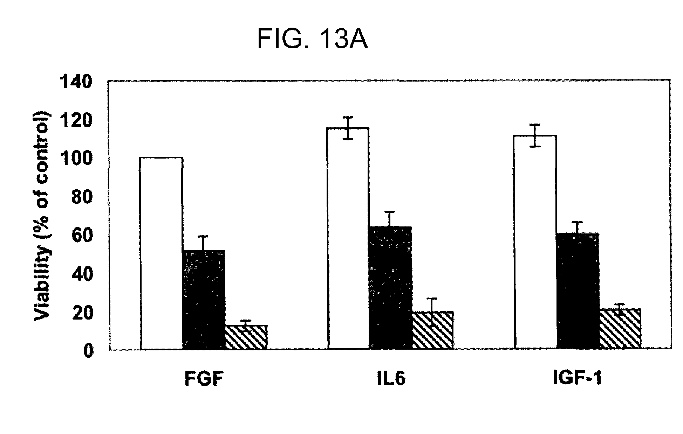

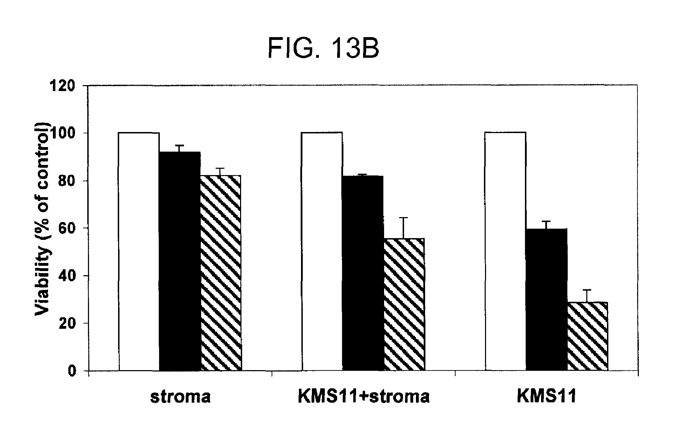

FIGS. 13A and 13B are graphs showing that 4-amino-5-fluoro-3-[6-(4-methylpiperazin-1-yl)-1H-benzimidazol-2-yl]quinolin-2(1H)-one inhibits the viability of KMS11 cells in the presence of interleukin-6 (IL6), insulin growth factor (IGF-1), and bone marrow stroma cells (BMSCs). FIG 13A is a graph in which KMS11 cells were cultured with DMSO (unshaded bar); with 100 nM (shaded bar) 4-amino-5-fluoro-3-[6-(4-methylpiperazin-1-yl)-1H-benzimidazol-2-yl]quinolin-2(1H)-one; and with 500 nM (hatched bar) 4-amino-5-fluoro-3-[6-(4-methylpiperazin-1-yl)-1H-benzimidazol-2-yl]quinolin-2(1H)-one in the presence or absence of 50 ng/mL IL6 or 50 ng/mL IGF-1. Cell viability was assessed by MTT assay after 48 hours. FIG. 13B is a graph in which BMSCs alone or together with KMS11 were cultured with DMSO (unshaded bar); with 100 nM (shaded bar) 4-amino-5-fluoro-3-[6-(4-methylpiperazin-1-yl)-1H-benzimidazol-2-yl]quinolin-2(1H)-one; and with 500 nM (hatched bar) 4-amino-5-fluoro-3-[6-(4-methylpiperazin-1-yl)-1H-benzimidazol-2-yl]quinolin-2(1H)-one. Viability was assessed after 96 hours by MTT assay. The data represent means of quadruplicate cultures +/- standard deviations.
FIG. 14 is a graph showing that 4-amino-5-fluoro-3-[6-(4-methylpiperazin-1-yl)-1H-benzimidazol-2-yl]quinolin-2(1H)-one inhibits proliferation of M-NFS-60, a M-CSF growth driven mouse myeloblastic cell line with an EC₅₀ of 220 nM. M-NSF-60 cells were incubated with serial dilutions of 4-amino-5-fluoro-3-[6-(4-methylpiperazin-1-yl)-1H-benzimidazol-2-yl]quinolin-2(1H)-one in the presence of M-CSF and without GM-CSF. The number of viable cells was assessed after 72 hours using the Cell Titer-Glo™ assay.
FIG. 15 is a graph showing that 4-amino-5-fluoro-3-[6-(4-methylpiperazin-1-yl)-1H-benzimidazol-2-yl]quinolin-2(1H)-one inhibits FGFR3 phosphorylation and demonstrates anti-tumor effects *in vivo.* When tumor size reached 200 mm³, mice were randomly assigned (8-10/group) to receive vehicle alone or varying doses of 4-amino-5-fluoro-3-[6-(4-methylpiperazin-1-yl)-1H-benzimidazol-2-yl]quinolin-2(1H)-one by oral gavage for 21 days. The graph shows tumor volume (mean +/- standard deviation) as a function of the days of treatment.

### DETAILED DESCRIPTION OF THE INVENTION

The present invention provides novel pharmaceutical salts of quinolinone compounds of Formula I and Formula II that act as antagonists of receptor tyrosine kinases, and, more particularly, as inhibitors of FGFR1 and FGFR3, PDGFRα and PDGFRβ, macrophage CSFR-1, FLT-3, c-KIT and/or VEGF-RTK function. Such kinases may also include IGFR1, EphA2, FGFR2, and FGFR4. The salts provided herein can be formulated into pharmaceutical formulations that are useful in treating patients with a need for an inhibitor of VEGF-RTK, especially, in particular embodiments, to provide compositions and methods for reducing capillary proliferation and in the treatment of cancer.

Pharmaceutically acceptable salts include a salt with an inorganic acid, an organic acid, a basic amino acid, or an acidic amino acid. As salts of inorganic acids, the instant invention includes, for example, hydrochloric acid, hydroboric acid, nitric acid, sulfuric acid, and phosphoric acid. As salts of organic acids, the instant invention includes, for example, lactic acid, formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Acidic amino acids include, for example, glycine, aspartic acid and glutamic acid.

Certain salts are preferred among the list above because of the properties that they impart to the compounds of Formula (I). Therefore, in some embodiments the salts are tartrate, malate, lactate, bishydrochloride, citrate, acetate, bismesylate, bis-acetate, and mesylate salts. Some of the improved properties that these salts impart include solubility, hygroscopicity, crystallinity, compactibility, and morphology.

The following abbreviations and definitions are used throughout this application:
"bFGF" is an abbreviation that stands for basic fibroblast growth factor;
"bFGFR", also referred to as FGFR1, is an abbreviation that stands for a receptor tyrosine kinase that interacts with the fibroblast growth factor FGF;
"C-MET", also referred to as 'HGF receptor", is an abbreviation that stands for cellular product of the met gene or Hepatocyte growth factor receptor; a receptor tyrosine kinase that interacts with the Hepatocyte growth factor (HGF) also referred to as Scatter factor;
"CSF-1" is an abbreviation that stands for colony stimulating factor-1 and its receptor. Macrophage CSFR-1 (Fms) is a receptor for CSF-1;
"EGFR1" is an abbreviation that stands for Epidermal growth factor receptor 1 and binds Epidermal growth factor "EGF";
"EphA2" is an abbreviation that stands for Ephrin receptor A2, also referred to as epithelial cell receptor protein-tyrosine kinase or ECK;
"ERK" is an abbreviation that stands for extracellular regulated kinase;
"FACS" is an abbreviation that stands for fluorescence activated cell sorting;
"FBS" is an abbreviation that stands for fetal bovine serum;
"Flk-1" is an abbreviation that stands for fetal liver tyrosine kinase 1, also known as kinase-insert domain tyrosine kinase or KDR (human), also known as vascular endothelial growth factor receptor-2 or VEGFR2 (KDR (human), Flk-1 (mouse));
"FLT-1" is an abbreviation that stands for fms-like tyrosine kinase-1, also known as vascular endothelial growth factor receptor-1 or VEGF1;
"FLT-3" is an abbreviation that stands for fms-like tyrosine kinase-3, also known as stem cell tyrosine kinase I (STK I);
"FLT-4" is an abbreviation that stands for fms-like tyrosine kinase-4, also known as VEGFR3;
"FGFR2" and "FGFR4" are abbreviations that stands for Fibroblast Growth Factor Receptor 2 and Fibroblast Growth Factor Receptor 4, respectively. FGFR2 and FGFR4 are Class IV receptor tyrosine kinases;
"FGFR3" is an abbreviation that stands for the tyrosine kinase fibroblast growth factor receptor 3 that is expressed in 15-20% of multiple myeloma-type cancers;
"G₁" is an abbreviation that stands for a phase of the standard eukaryotic cell cycle. In the standard cell cycle, G₁ phase is the gap before S phase and after M phase and G₂ is the gap after S phase and before M phase;
"GM-CSF" is an abbreviation that stands for granulocyte macrophage colony stimulating factor;
"IGFR1" is an abbreviation that stands for Insulin-like Growth Factor Receptor-1. IFGR1 is a Class II receptor tyrosine kinase;
"HER2" is an abbreviation that stands for human epidermal growth factor receptor 2;
"IL6" is an abbreviation that stands for interleukin 6;
"KDR" is an abbreviation that stands for kinase- insert domain- containing receptor, also known as vascular endothelial growth factor receptor-2 or "VEGFR2";
"MAPK" is an abbreviation that stands for mitogen activated protein kinase;
"M-CSF" is an abbreviation that stands for macrophage colony stimulating factor;
"PDGF" is an abbreviation that stands for platelet derived growth factor. PDGF interacts with tyrosine kinases PDGFRα and PDGFRβ;
"RTK" is an abbreviation that stands for receptor tyrosine kinase;
"Tie-2" is an abbreviation that stands for tyrosine kinase with Ig and EGF homology domains;
"VEGF" is an abbreviation that stands for vascular endothelial growth factor;
"VEGF-RTK" is an abbreviation that stands for vascular endothelial growth factor receptor tyrosine kinase.

Generally, reference to a certain element such as hydrogen or H is meant to include all isotopes of that element. For example, if an R group is defined to include hydrogen or H, it also includes deuterium and tritium.

The phrase "unsubstituted alkyl" refers to alkyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: -CH(CH₃)₂, -CH(CH₃)(CH₂CH₃), -CH(CH₂CH₃)₂,-C(CH₃)₃, -C(CH₂CH₃)₃, -CH₂CH(CH₃)₂, -CH₂CH(CH₃)(CH₂CH₃),-CH₂CH(CH₂CH₃)₂, -CH₂C(CH₃)₃, -CH₂C(CH₂CH₃)₃,-CH(CH₃)CH(CH₃)(CH₂CH₃), -CH₂CH₂CH(CH₃)₂, -CH₂CH₂CH(CH₃)(CH₂CH₃),-CH₂CH₂CH(CH₂CH₃)₂, -CH₂CH₂C(CH₃)₃, -CH₂CH₂C(CH₂CH₃)₃,-CH(CH₃)CH₂CH(CH₃)₂, -CH(CH₃)CH(CH₃)CH(CH₃)₂, -CH(CH₂CH₃)CH(CH₃)CH(C H₃)(CH₂CH₃), and others. The phrase also includes cyclic alkyl groups such as cyclopropyl, cyclobutyl, cyclopentyl, cyclohexyl, cycloheptyl, and cyclooctyl and such rings substituted with straight and branched chain alkyl groups as defined above. The phrase also includes polycyclic alkyl groups such as, but not limited to, adamantyl norbornyl, and bicyclo[2.2.2]octyl and such rings substituted with straight and branched chain alkyl groups as defined above. Thus, the phrase unsubstituted alkyl groups includes primary alkyl groups, secondary alkyl groups, and tertiary alkyl groups. Unsubstituted alkyl groups may be bonded to one or more carbon atom(s), oxygen atom(s), nitrogen atom(s), and/or sulfur atom(s) in the parent compound. Preferred unsubstituted alkyl groups include straight and branched chain alkyl groups and cyclic alkyl groups having 1 to 20 carbon atoms. More preferred such unsubstituted alkyl groups have from 1 to 10 carbon atoms while even more preferred such groups have from 1 to 5 or 1 to 6 carbon atoms. Most preferred unsubstituted alkyl groups include straight and branched chain alkyl groups having from 1 to 3 carbon atoms and include methyl, ethyl, propyl, and -CH(CH₃)₂.

The phrase "substituted alkyl" refers to an unsubstituted alkyl group as defined above in which one or more bonds to a carbon(s) or hydrogen(s) are replaced by a bond to non-hydrogen and non-carbon atoms such as, but not limited to, a halogen atom in halides such as F, Cl, Br, and I; and oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups. Substituted alkyl groups also include groups in which one or more bonds to a carbon(s) or hydrogen(s) atom is replaced by a bond to a heteroatom such as oxygen in carbonyl, carboxyl, and ester groups; nitrogen in groups such as imines, oximes, hydrazones, and nitriles. Preferred substituted alkyl groups include, among others, alkyl groups in which one or more bonds to a carbon or hydrogen atom is/are replaced by one or more bonds to fluorine atoms. One example of a substituted alkyl group is the trifluoromethyl group and other alkyl groups that contain the trifluoromethyl group. Other alkyl groups include those in which one or more bonds to a carbon or hydrogen atom is replaced by a bond to an oxygen atom such that the substituted alkyl group contains a hydroxyl, alkoxy, aryloxy group, or heterocyclyloxy group. Still other alkyl groups include alkyl groups that have an amine, alkylamine, dialkylamine, arylamine, (alkyl)(aryl)amine, diarylamine, heterocyclylamine, (alkyl)(heterocyclyl)amine, (aryl)(heterocyclyl)amine, or diheterocyclylamine group.

The phrase "unsubstituted aryl" refers to aryl groups that do not contain heteroatoms. Thus the phrase includes, but is not limited to, groups such as phenyl, biphenyl, anthracenyl, naphthenyl by way of example. Although the phrase "unsubstituted aryl" includes groups containing condensed rings such as naphthalene, it does not include aryl groups that have other groups such as alkyl or halo groups bonded to one of the ring members, as aryl groups such as tolyl are considered herein to be substituted aryl groups as described below. In some embodiments, aryl groups have from 6 to 14 carbon atoms. A preferred unsubstituted aryl group is phenyl. Unsubstituted aryl groups may be bonded to one or more carbon atom(s), oxygen atom(s), nitrogen atom(s), and/or sulfur atom(s) in the parent compound, however.

The phrase "substituted aryl group" has the same meaning with respect to unsubstituted aryl groups that substituted alkyl groups had with respect to unsubstituted alkyl groups. However, a substituted aryl group also includes aryl groups in which one of the aromatic carbons is bonded to one of the non-carbon or non-hydrogen atoms described above and also includes aryl groups in which one or more aromatic carbons of the aryl group is bonded to a substituted and/or unsubstituted alkyl, alkenyl, or alkynyl group as defined herein. This includes bonding arrangements in which two carbon atoms of an aryl group are bonded to two atoms of an alkyl, alkenyl, or alkynyl group to define a fused ring system (e.g., dihydronaphthyl or tetrahydronaphthyl). Thus, the phrase "substituted aryl" includes, but is not limited to tolyl, and hydroxyphenyl among others.

The phrase "unsubstituted alkenyl" refers to straight and branched chain and cyclic groups such as those described with respect to unsubstituted alkyl groups as defined above, except that at least one double bond exists between two carbon atoms. Examples include, but are not limited to vinyl,-CH=C(H)(CH₃), -CH=C(CH₃)₂, -C(CH₃)=C(H)₂, -C(CH₃)=C(H)(CH₃),-C(CH₂CH₃)=CH₂, cyclohexenyl, cyclopentenyl, cyclohexadienyl, butadienyl, pentadienyl, and hexadienyl among others. In some embodiments, alkenyl groups have from 2 to 8 carbon atoms.

The phrase "substituted alkenyl" has the same meaning with respect to unsubstituted alkenyl groups that substituted alkyl groups had with respect to unsubstituted alkyl groups. A substituted alkenyl group includes alkenyl groups in which a non-carbon or non-hydrogen atom is bonded to a carbon double bonded to another carbon and those in which one of the non-carbon or non-hydrogen atoms is bonded to a carbon not involved in a double bond to another carbon.

The phrase "unsubstituted alkynyl" refers to straight and branched chain groups such as those described with respect to unsubstituted alkyl groups as defined above, except that at least one triple bond exists between two carbon atoms. Examples include, but are not limited to -C≡C(H), -C≡C(CH₃),-C≡C(CH₂CH₃), -C(H₂)C≡C(H), -C(H)₂C≡C(CH₃), and -C(H)₂C≡C(CH₂CH₃) among others. In some embodiments, alkynyl groups have from 2 to 8 carbon atoms.

The phrase "substituted alkynyl" has the same meaning with respect to unsubstituted alkynyl groups that substituted alkyl groups had with respect to unsubstituted alkyl groups. A substituted alkynyl group includes alkynyl groups in which a non-carbon or non-hydrogen atom is bonded to a carbon triple bonded to another carbon and those in which a non-carbon or non-hydrogen atom is bonded to a carbon not involved in a triple bond to another carbon.

The phrase "unsubstituted aralkyl" refers to unsubstituted alkyl groups as defined above in which a hydrogen or carbon bond of the unsubstituted alkyl group is replaced with a bond to an aryl group as defined above. For example, methyl (-CH₃) is an unsubstituted alkyl group. If a hydrogen atom of the methyl group is replaced by a bond to a phenyl group, such as if the carbon of the methyl were bonded to a carbon of benzene, then the compound is an unsubstituted aralkyl group (i.e., a benzyl group). Thus the phrase includes, but is not limited to, groups such as benzyl, diphenylmethyl, and 1-phenylethyl (-CH(C₆H₅)(CH₃)) among others.

The phrase "substituted aralkyl" has the same meaning with respect to unsubstituted aralkyl groups that substituted aryl groups had with respect to unsubstituted aryl groups. However, a substituted aralkyl group also includes groups in which a carbon or hydrogen bond of the alkyl part of the group is replaced by a bond to a non-carbon or a non-hydrogen atom. Examples of substituted aralkyl groups include, but are not limited to, -CH₂C(=O)(C₆H₅), and -CH₂(2-methylphenyl) among others.

The phrase "unsubstituted heterocyclyl" refers to both aromatic and nonaromatic ring compounds including monocyclic, bicyclic, and polycyclic ring compounds such as, but not limited to, quinuclidyl, containing 3 or more ring members of which one or more is a heteroatom such as, but not limited to, N, O, and S. Although the phrase "unsubstituted heterocyclyl" includes condensed heterocyclic rings such as benzimidazolyl, it does not include heterocyclyl groups that have other groups such as alkyl or halo groups bonded to one of the ring members as compounds such as 2-methylbenzimidazolyl are substituted heterocyclyl groups. Examples of heterocyclyl groups include, but are not limited to: unsaturated 3 to 8 membered rings containing 1 to 4 nitrogen atoms such as, but not limited to pyrrolyl, pyrrolinyl, imidazolyl, pyrazolyl, pyridyl, dihydropyridyl, pyrimidyl, pyrazinyl, pyridazinyl, triazolyl (e.g., 4H-1,2,4-triazolyl, 1H-1,2,3-triazolyl, 2H-1,2,3-triazolyl etc.), tetrazolyl, (e.g., 1H-tetrazolyl, 2H tetrazolyl, etc.); saturated 3 to 8 membered rings containing 1 to 4 nitrogen atoms such as, but not limited to, pyrrolidinyl, imidazolidinyl, piperidinyl, piperazinyl; condensed unsaturated heterocyclic groups containing 1 to 4 nitrogen atoms such as, but not limited to, indolyl, isoindolyl, indolinyl, indolizinyl, benzimidazolyl, quinolyl, isoquinolyl, indazolyl, benzotriazolyl; unsaturated 3 to 8 membered rings containing 1 to 2 oxygen atoms and 1 to 3 nitrogen atoms such as, but not limited to, oxazolyl, isoxazolyl, oxadiazolyl (e.g., 1,2,4-oxadiazolyl, 1,3,4-oxadiazolyl, 1,2,5-oxadiazolyl, etc.); saturated 3 to 8 membered rings containing 1 to 2 oxygen atoms and 1 to 3 nitrogen atoms such as, but not limited to, morpholinyl; unsaturated condensed heterocyclic groups containing 1 to 2 oxygen atoms and 1 to 3 nitrogen atoms, for example, benzoxazolyl, benzoxadiazolyl, benzoxazinyl (e.g., 2H-1,4-benzoxazinyl, etc.); unsaturated 3 to 8 membered rings containing 1 to 3 sulfur atoms and 1 to 3 nitrogen atoms such as, but not limited to, thiazolyl, isothiazolyl, thiadiazolyl (e.g., 1,2,3-thiadiazolyl, 1,2,4-thiadiazolyl, 1,3,4-thiadiazolyl, 1,2,5-thiadiazolyl, etc.); saturated 3 to 8 membered rings containing 1 to 2 sulfur atoms and 1 to 3 nitrogen atoms such as, but not limited to, thiazolodinyl; saturated and unsaturated 3 to 8 membered rings containing 1 to 2 sulfur atoms such as, but not limited to, thienyl, dihydrodithiinyl, dihydrodithionyl, tetrahydrothiophene, tetrahydrothiopyran; unsaturated condensed heterocyclic rings containing 1 to 2 sulfur atoms and 1 to 3 nitrogen atoms such as, but not limited to, benzothiazolyl, benzothiadiazolyl, benzothiazinyl (e.g., 2H-1,4-benzothiazinyl, etc.), dihydrobenzothiazinyl (e.g., 2H-3,4-dihydrobenzothiazinyl, etc.), unsaturated 3 to 8 membered rings containing oxygen atoms such as, but not limited to furyl; unsaturated condensed heterocyclic rings containing 1 to 2 oxygen atoms such as benzodioxolyl (e.g., 1,3-benzodioxoyl, etc.); unsaturated 3 to 8 membered rings containing an oxygen atom and 1 to 2 sulfur atoms such as, but not limited to, dihydrooxathiinyl; saturated 3 to 8 membered rings containing 1 to 2 oxygen atoms and 1 to 2 sulfur atoms such as 1,4-oxathiane; unsaturated condensed rings containing 1 to 2 sulfur atoms such as benzothienyl, benzodithiinyl; and unsaturated condensed heterocyclic rings containing an oxygen atom and 1 to 2 oxygen atoms such as benzoxathiinyl. Heterocyclyl group also include those described above in which one or more S atoms in the ring is double-bonded to one or two oxygen atoms (sulfoxides and sulfones). For example, heterocyclyl groups include tetrahydrothiophene, tetrahydrothiophene oxide, and tetrahydrothiophene 1,1-dioxide. Preferred heterocyclyl groups contain 5 or 6 ring members. More preferred heterocyclyl groups include morpholine, piperazine, piperidine, pyrrolidine, imidazole, pyrazole, 1,2,3-triazole, 1,2,4-triazole, tetrazole, thiomorpholine, thiomorpholine in which the S atom of the thiomorpholine is bonded to one or more O atoms, pyrrole, homopiperazine, oxazolidin-2-one, pyrrolidin-2-one, oxazole, quinuclidine, thiazole, isoxazole, furan, and tetrahydrofuran.

The phrase "substituted heterocyclyl" refers to an unsubstituted heterocyclyl group as defined above in which one of the ring members is bonded to a non-hydrogen atom such as described above with respect to substituted alkyl groups and substituted aryl groups. Examples, include, but are not limited to, 2-methylbenzimidazolyl, 5-methylbenzimidazolyl, 5-chlorobenzthiazolyl, 1-methyl piperazinyl, and 2-chloropyridyl among others.

The phrase "unsubstituted heterocyclylalkyl" refers to unsubstituted alkyl groups as defined above in which a hydrogen or carbon bond of the unsubstituted alkyl group is replaced with a bond to a heterocyclyl group as defined above. For example, methyl (-CH₃) is an unsubstituted alkyl group. If a hydrogen atom of the methyl group is replaced by a bond to a heterocyclyl group, such as if the carbon of the methyl were bonded to carbon 2 of pyridine (one of the carbons bonded to the N of the pyridine) or carbons 3 or 4 of the pyridine, then the compound is an unsubstituted heterocyclylalkyl group.

The phrase "substituted heterocyclylalkyl" has the same meaning with respect to unsubstituted heterocyclylalkyl groups that substituted aralkyl groups had with respect to unsubstituted aralkyl groups. However, a substituted heterocyclylalkyl group also includes groups in which a non-hydrogen atom is bonded to a heteroatom in the heterocyclyl group of the heterocyclylalkyl group such as, but not limited to, a nitrogen atom in the piperidine ring of a piperidinylalkyl group.

The phrase "unsubstituted alkylaminoalkyl" refers to an unsubstituted alkyl group as defined above in which a carbon or hydrogen bond is replaced by a bond to a nitrogen atom that is bonded to a hydrogen atom and an unsubstituted alkyl group as defined above. For example, methyl (-CH₃) is an unsubstituted alkyl group. If a hydrogen atom of the methyl group is replaced by a bond to a nitrogen atom that is bonded to a hydrogen atom and an ethyl group, then the resulting compound is -CH₂-N(H)(CH₂CH₃) which is an unsubstituted alkylaminoalkyl group.

The phrase "substituted alkylaminoalkyl" refers to an unsubstituted alkylaminoalkyl group as defined above except where one or more bonds to a carbon or hydrogen atom in one or both of the alkyl groups is replaced by a bond to a non-carbon or non-hydrogen atom as described above with respect to substituted alkyl groups except that the bond to the nitrogen atom in all alkylaminoalkyl groups does not by itself qualify all alkylaminoalkyl groups as being substituted. However, substituted alkylaminoalkyl groups does include groups in which the hydrogen bonded to the nitrogen atom of the group is replaced with a non-carbon and non-hydrogen atom.

The phrase "unsubstituted dialkylaminoalkyl" refers to an unsubstituted alkyl group as defined above in which a carbon bond or hydrogen bond is replaced by a bond to a nitrogen atom which is bonded to two other similar or different unsubstituted alkyl groups as defined above.

The phrase "substituted dialkylaminoalkyl" refers to an unsubstituted dialkylaminoalkyl group as defined above in which one or more bonds to a carbon or hydrogen atom in one or more of the alkyl groups is replaced by a bond to a non-carbon and non-hydrogen atom as described with respect to substituted alkyl groups. The bond to the nitrogen atom in all dialkylaminoalkyl groups does not by itself qualify all dialkylaminoalkyl groups as being substituted.

The phrase "unsubstituted heterocyclyloxyalkyl" refers to an unsubstituted alkyl group as defined above in which a carbon bond or hydrogen bond is replaced by a bond to an oxygen atom which is bonded to an unsubstituted heterocyclyl group as defined above.

The phrase "substituted heterocyclyloxyalkyl" refers to an unsubstituted heterocyclyloxyalkyl group as defined above in which a bond to a carbon or hydrogen group of the alkyl group of the heterocyclyloxyalkyl group is bonded to a non-carbon and non-hydrogen atom as described above with respect to substituted alkyl groups or in which the heterocyclyl group of the heterocyclyloxyalkyl group is a substituted heterocyclyl group as defined above.

The phrase "unsubstituted arylaminoalkyl" refers to an unsubstituted alkyl group as defined above in which a carbon bond or hydrogen bond is replaced by a bond to a nitrogen atom which is bonded to at least one unsubstituted aryl group as defined above.

The phrase "substituted arylaminoalkyl" refers to an unsubstituted arylaminoalkyl group as defined above except where either the alkyl group of the arylaminoalkyl group is a substituted alkyl group as defined above or the aryl group of the arylaminoalkyl group is a substituted aryl group except that the bonds to the nitrogen atom in all arylaminoalkyl groups does not by itself qualify all arylaminoalkyl groups as being substituted. However, substituted arylaminoalkyl groups does include groups in which the hydrogen bonded to the nitrogen atom of the group is replaced with a non-carbon and non-hydrogen atom.

The phrase "unsubstituted heterocyclylaminoalkyl" refers to an unsubstituted alkyl group as defined above in which a carbon or hydrogen bond is replaced by a bond to a nitrogen atom which is bonded to at least one unsubstituted heterocyclyl group as defined above.

The phrase "substituted heterocyclylaminoalkyl" refers to unsubstituted heterocyclylaminoalkyl groups as defined above in which the heterocyclyl group is a substituted heterocyclyl group as defined above and/or the alkyl group is a substituted alkyl group as defined above. The bonds to the nitrogen atom in all heterocyclylaminoalkyl groups does not by itself qualify all heterocyclylaminoalkyl groups as being substituted. However, substituted heterocyclylaminoalkyl groups do include groups in which the hydrogen bonded to the nitrogen atom of the group is replaced with a non-carbon and non-hydrogen atom.

The phrase "unsubstituted alkylaminoalkoxy" refers to an unsubstituted alkyl group as defined above in which a carbon or hydrogen bond is replaced by a bond to an oxygen atom which is bonded to the parent compound and in which another carbon or hydrogen bond of the unsubstituted alkyl group is bonded to a nitrogen atom which is bonded to a hydrogen atom and an unsubstituted alkyl group as defined above.

The phrase "substituted alkylaminoalkoxy" refers to unsubstituted alkylaminoalkoxy groups as defined above in which a bond to a carbon or hydrogen atom of the alkyl group bonded to the oxygen atom which is bonded to the parent compound is replaced by one or more bonds to a non-carbon and non-hydrogen atoms as discussed above with respect to substituted alkyl groups and/or if the hydrogen bonded to the amino group is bonded to a non-carbon and non-hydrogen atom and/or if the alkyl group bonded to the nitrogen of the amine is bonded to a non-carbon and non-hydrogen atom as described above with respect to substituted alkyl groups. The presence of the amine and alkoxy functionality in all alkylaminoalkoxy groups does not by itself qualify all such groups as substituted alkylaminoalkoxy groups.

The phrase "unsubstituted dialkylaminoalkoxy" refers to an unsubstituted alkyl group as defined above in which a carbon or hydrogen bond is replaced by a bond to an oxygen atom which is bonded to the parent compound and in which another carbon or hydrogen bond of the unsubstituted alkyl group is bonded to a nitrogen atom which is bonded to two other similar or different unsubstituted alkyl groups as defined above.

The phrase "substituted dialkylaminoalkoxy" refers to an unsubstituted dialkylaminoalkoxy group as defined above in which a bond to a carbon or hydrogen atom of the alkyl group bonded to the oxygen atom which is bonded to the parent compound is replaced by one or more bonds to a non-carbon and non-hydrogen atoms as discussed above with respect to substituted alkyl groups and/or if one or more of the alkyl groups bonded to the nitrogen of the amine is bonded to a non-carbon and non-hydrogen atom as described above with respect to substituted alkyl groups. The presence of the amine and alkoxy functionality in all dialkylaminoalkoxy groups does not by itself qualify all such groups as substituted dialkylaminoalkoxy groups.

The phrase "unsubstituted heterocyclyloxy" refers to a hydroxyl group (-OH) in which the bond to the hydrogen atom is replaced by a bond to a ring atom of an otherwise unsubstituted heterocyclyl group as defined above.

The phrase "substituted heterocyclyloxy" refers to a hydroxyl group (-OH) in which the bond to the hydrogen atom is replaced by a bond to a ring atom of an otherwise substituted heterocyclyl group as defined above.

The term "protected" with respect to hydroxyl groups, amine groups, and sulfhydryl groups refers to forms of these functionalities which are protected from undesirable reaction with a protecting group known to those skilled in the art such as those set forth in Protective Groups in Organic Synthesis, Greene, T.W.; Wuts, P. G. M., John Wiley & Sons, New York, NY, (3rd Edition, 1999) which can be added or removed using the procedures set forth therein. Examples of protected hydroxyl groups include, but are not limited to, silyl ethers such as those obtained by reaction of a hydroxyl group with a reagent such as, but not limited to, t-butyldimethyl-chlorosilane, trimethylchlorosilane, triisopropylchlorosilane, triethylchlorosilane; substituted methyl and ethyl ethers such as, but not limited to methoxymethyl ether, methythiomethyl ether, benzyloxymethyl ether, t-butoxymethyl ether, 2-methoxyethoxymethyl ether, tetrahydropyranyl ethers, 1-ethoxyethyl ether, allyl ether, benzyl ether; esters such as, but not limited to, benzoylformate, formate, acetate, trichloroacetate, and trifluoracetate. Examples of protected amine groups include, but are not limited to, amides such as, formamide, acetamide, trifluoroacetamide, and benzamide; imides, such as phthalimide, and dithiosuccinimide; and others. Examples of protected sulfhydryl groups include, but are not limited to, thioethers such as S-benzyl thioether, and S-4-picolyl thioether; substituted S-methyl derivatives such as hemithio, dithio and aminothio acetals; and others.

The salts of compounds of Formula I and Formula II with lactic acid, acetic acid, tartaric acid, malic acid, methanesulfonic acid, hydrochloric acid, and citric acid can be prepared by dissolving a base of a compound of Formula I or Formula II in a suitable organic solvent or a mixture of solvents together with one, two, or more equivalents, of lactic acid, acetic acid, tartaric acid, malic acid, citric acid, hydrochloric acid, or methanesulfonic acid. The mixture is heated, usually refluxed, and then cooled. The formed salt is typically recovered by filtering or by evaporating to dryness. Suitable organic solvents include, but are not limited to, lower alcohols and ethers, preferably methanol, ethanol, diethyl ether, and combinations of these. The salts can be formulated into any one of a number of known dosage forms or delivery systems by means known in the art e.g., for oral, parenteral, transdermal or topical use. In some embodiments, the salt is crystalline and in some embodiments, the crystals are plate-shaped or needles. The salts may be compressed to form tablets. Preferably, the salts of the invention are used for preparing aqueous formulations of the compounds of Formula I or Formula II. The salts of the invention have generally improved water solubility over the free base or acid of the compounds of Formula I or Formula II. For example, the solubility of the lactate salt of 4-amino-5-fluoro-3-[6-(4-methylpiperazin-1-yl)-1H-benzimidazol-2-yl]-1H-quinolin-2-one in distilled water is about 330 mg/mL.

The present invention is directed to a pharmaceutically acceptable salt of a compound having Formula I or a tautomer of the compound. In some such embodiments, the salt is selected from lactate, malate, mesylate, acetate, tartrate, phosphate, sulfate, nitrate, HCl, citrate, or maleate. In some such embodiments, the salt is selected from lactate, malate, mesylate, acetate, or tartrate salts. In some such embodiments, the salt is selected from lactate, bis-lactate, malate, mesylate, bismesylate, bis-acetate, or tartrate salts. In other embodiments the salt is selected from lactate, malate, or mesylate salts. Compounds of Formula I have the following formula: 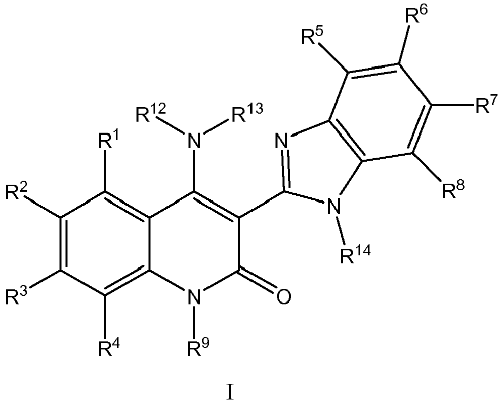 wherein,
R¹, R², R³, and R⁴ may be the same or different and are independently selected from the group consisting of H, Cl, Br, F, I, -CN, -NO₂, -OH, -OR¹⁵ groups, -NR¹⁶R¹⁷ groups, substituted and unsubstituted amidinyl groups, substituted and unsubstituted guanidinyl groups, substituted and unsubstituted primary, secondary, and tertiary alkyl groups, substituted and unsubstituted aryl groups, substituted and unsubstituted alkenyl groups, substituted and unsubstituted alkynyl groups, substituted and unsubstituted heterocyclyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, and -C(=O)R¹⁸ groups;
R⁵, R⁶, R⁷, and R⁸ may be the same or different and are independently selected from the group consisting of H, Cl, Br, F, I, -NO₂, -OH, -OR¹⁹ groups, -NR¹⁰R²¹ groups, -SH, -SR²² groups, -S(=O)R²³ groups, -S(=O)₂R²⁴ groups, -CN, substituted and unsubstituted amidinyl groups, substituted and unsubstituted guanidinyl groups, substituted and unsubstituted primary, secondary, and tertiary alkyl groups, substituted and unsubstituted aryl groups, substituted and unsubstituted alkenyl groups, substituted and unsubstituted alkynyl groups, substituted and unsubstituted heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, -C(=O)R²⁵ groups, substituted and unsubstituted aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R⁹ is H;
R¹² is selected from the group consisting of H, substituted and unsubstituted alkyl groups, substituted and unsubstituted aryl groups, and substituted and unsubstituted heterocyclyl groups;
R¹³ is selected from the group consisting of H, substituted and unsubstituted alkyl groups, substituted and unsubstituted aryl groups, substituted and unsubstituted heterocyclyl groups, -OH, alkoxy groups, aryloxy groups, -NH₂, substituted and unsubstituted heterocyclylalkyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted alkylamino groups, substituted and unsubstituted arylamino groups, substituted and unsubstituted dialkylamino groups, substituted and unsubstituted diarylamino groups, substituted and unsubstituted (alkyl)(aryl)amino groups, -C(=O)H, -C(=O)-alkyl groups, -C(=O)-aryl groups, -C(=O)O-alkyl groups, -C(=O)O-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)-heterocyclyl groups, -C(=O)-O-heterocyclyl groups, - C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R¹⁴ is H;
R¹⁵ and R¹⁹ may be the same or different and are independently selected from the group consisting of substituted and unsubstituted alkyl groups, substituted and unsubstituted aryl groups, substituted and unsubstituted heterocyclyl groups, substituted and unsubstituted heterocyclylalkyl groups, -C(=O)H, -C(=O)-alkyl groups, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl, substituted and unsubstituted diheterocyclylaminoalkyl, substituted and unsubstituted (heterocyclyl)(alkyl)aminoalkyl, substituted and unsubstituted (heterocyclyl)(aryl)aminoalkyl, substituted and unsubstituted alkoxyalkyl groups, substituted and unsubstituted hydroxyalkyl groups, substituted and unsubstituted aryloxyalkyl groups, and substituted and unsubstituted heterocyclyloxyalkyl groups;
R¹⁶ and R²⁰ may be the same or different and are independently selected from the group consisting of H, substituted and unsubstituted alkyl groups, substituted and unsubstituted aryl groups, and substituted and unsubstituted heterocyclyl groups;
R¹⁷ and R²¹ may be the same or different and are independently selected from the group consisting of H, substituted and unsubstituted alkyl groups, substituted and unsubstituted aryl groups, substituted and unsubstituted heterocyclyl groups, -C(=O)H, -C(=O)-alkyl groups, -C(=O)-aryl groups, -C(=O)NH₂,-C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)O-alkyl groups, -C(=O)O-aryl groups, substituted and unsubstituted heterocyclylalkyl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, -C(=O)-heterocyclyl groups, -C(=O)-O-heterocyclyl groups, -C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
R¹⁸, R²³, R²⁴, and R²⁵ may be the same or different and are independently selected from the group consisting of H, -NH₂, -NH(alkyl) groups, -NH(aryl) groups, -N(alkyl)₂ groups, -N(aryl)₂ groups, -N(alkyl)(aryl) groups, -NH(heterocyclyl) groups, -N(heterocyclyl)(alkyl) groups, -N(heterocyclyl)(aryl) groups, -N(heterocyclyl)₂ groups, substituted and unsubstituted alkyl groups, substituted and unsubstituted aryl groups, -OH, substituted and unsubstituted alkoxy groups, substituted and unsubstituted aryloxy groups, substituted and unsubstituted heterocyclyl groups, -NHOH, -N(alkyl)OH groups, -N(aryl)OH groups, -N(alkyl)O-alkyl groups, -N(aryl)O-alkyl groups, -N(alkyl)O-aryl groups, and -N(aryl)O-aryl groups; and
R²² is selected from the group consisting of substituted and unsubstituted alkyl groups, substituted and unsubstituted aryl groups, and substituted and unsubstituted heterocyclyl groups.

In some embodiments of the pharmaceutically acceptable salts of the compounds or the tautomers of the compounds of Formula I, at least one of R⁵, R⁶, R⁷, or R⁸ is selected from the group consisting of substituted and unsubstituted amidinyl groups, substituted and unsubstituted guanidinyl groups, substituted and unsubstituted saturated heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups; -OR¹⁹ groups wherein R¹⁹ is selected from the group consisting of substituted and unsubstituted aryl groups, substituted and unsubstituted heterocyclyl groups, substituted and unsubstituted heterocyclylalkyl groups, -C(=O)H, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted diheterocyclylaminoalkyl groups, substituted and unsubstituted (heterocyclyl)(alkyl)aminoalkyl groups, substituted and unsubstituted (heterocyclyl)(aryl)aminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups; -NR¹⁰R²¹ groups wherein R²⁰ is selected from the group consisting of substituted and unsubstituted heterocyclyl groups; -NR¹⁰R²¹ groups wherein R²¹ is selected from the group consisting of substituted and unsubstituted heterocyclyl groups, -C(=O)H, -C(=O)-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)O-alkyl groups, -C(=O)O-aryl groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, substituted and unsubstituted heterocyclylalkyl groups, and substituted and unsubstituted heterocyclyloxyalkyl groups; and -C(=O)R²⁵ groups wherein R²⁵ is selected from the group consisting of H, -NH₂, -NH(alkyl) groups, -NH(aryl) groups, -N(alkyl)₂ groups, -N(aryl)₂ groups, -N(alkyl)(aryl) groups, -NH(heterocyclyl) groups, -N(heterocyclyl)(alkyl) groups, -N(heterocyclyl)(aryl) groups, -N(heterocyclyl)₂ groups, substituted and unsubstituted aryl groups, substituted and unsubstituted aryloxy groups, and substituted and unsubstituted heterocyclyl groups.

In one embodiment, the invention relates to a pharmaceutically acceptable salt of 4-amino-5-fluoro-3-[6-(4-methylpiperazin-1-yl)-1H-benzimidazol-2-yl]quinolin-2(1H)-one. In some such embodiments, the salt is selected from tartrate, malate, lactate, acetate, bis-acetate, citrate, mesylate, bismesylate and bishydrochloride. In some such embodiments, the salt is selected from the group consisting of tartrate, malate, lactate, bis-lactate, bis-acetate, citrate, mesylate, bismesylate and bishydrochloride.

In some specific embodiments, the compound of structure I is a lactate salt of 4-amino-5-fluoro-3-[6-(4-methylpiperazin-1-yl)-1H-benzimidazol-2-yl]quinolin-2(1H)-one or a tautomer thereof.

In a some embodiments of the compounds or the tautomers of the compounds of Formula I, R¹ is selected from the group consisting of F, Cl, substituted and unsubstituted alkoxy groups, substituted and unsubstituted heterocyclylalkoxy groups, substituted and unsubstituted heterocyclyl groups, substituted and unsubstituted alkylaminoalkoxy groups, substituted and unsubstituted arylaminoalkoxy groups, substituted and unsubstituted dialkylaminoalkoxy groups, substituted and unsubstituted diarylaminoalkoxy groups, and substituted and unsubstituted (alkyl)(aryl)aminoalkoxy groups.

In some other embodiments of the compounds or the tautomers of the compounds of Formula I, at least one of R⁵, R⁶, R⁷, and R⁸ is a substituted or unsubstituted heterocyclyl group.

In still other embodiments of the compounds or the tautomers of the compounds of Formula I, at least one of R⁵, R⁶, R⁷, and R⁸ is a substituted or unsubstituted heterocyclyl group comprising at least one O or N atom.

In yet other embodiments of the compounds or the tautomers of the compounds of Formula I, at least one of R⁵, R⁶, R⁷, and R⁸ is a substituted or unsubstituted heterocyclyl group and the heterocyclyl group is selected from the group consisting of morpholine, piperazine, piperidine, pyrrolidine, thiomorpholine, homopiperazine, tetrahydrothiophene, tetrahydrofuran, and tetrahydropyran.

In yet other embodiments of the compounds or the tautomers of the compounds of Formula I, at least one of R⁶ or R⁷ is a substituted or unsubstituted heterocyclyl group.

In yet other embodiments of the compounds or the tautomers of the compounds of Formula I, at least one of R⁶ or R⁷ is a substituted or unsubstituted heterocyclyl group comprising at least one O or N atom.

In yet other embodiments of the compounds or the tautomers of the compounds of Formula I, one of R⁶ or R⁷ is a substituted or unsubstituted heterocyclyl group and the heterocyclyl group is selected from the group consisting of morpholine, piperazine, piperidine, pyrrolidine, thiomorpholine, homopiperazine, tetrahydrothiophene, tetrahydrofuran, and tetrahydropyran.

In still other particular embodiments of the compounds or the tautomers of the compounds of Formula I, one of R⁶ or R⁷ is selected from the group consisting of substituted and unsubstituted morpholine groups, and substituted and unsubstituted piperazine groups.

In yet other embodiments of the compounds or the tautomers of the compounds of Formula I, at least one of and in some embodiments one of R⁶ or R⁷ is selected from the group consisting of -NR²⁰R²¹ groups wherein R²⁰ is selected from the group consisting of substituted and unsubstituted heterocyclyl groups; and -NR²⁰R²¹ groups wherein R²¹ is selected from the group consisting of substituted and unsubstituted heterocyclyl groups, groups, substituted and unsubstituted aminoalkyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, substituted and unsubstituted heterocyclylalkyl groups, and substituted and unsubstituted heterocyclyloxyalkyl groups.

In yet another embodiments of the compounds or the tautomers of the compounds of Formula I, R¹ is selected from the group consisting of H and F. In some such embodiments, R¹ is F, In some such embodiments, R² is H.

In one embodiment, the pharmaceutically acceptable salt of the compound of Formula I or the tautomer thereof has a water solubility at 22°C of from about 5 mg/mL to about 400 mg/mL. In some embodiments, the salt has a water solubility from about 100 mg/mL to about 400 mg/mL. In other embodiments, the salt has a water solubility from about 200 mg/mL to about 400 mg/mL. In some embodiments, the salt of the compound of Formula I or the tautomer thereof has a water solubility of greater than 30 mg/mL. In other embodiments, the salt of the compound of Formula I or the tautomer thereof has a water solubility from about 150 mg/mL to about 250 mg/mL. In another embodiment, the pharmaceutically acceptable salt of the compound of Formula I or the tautomer thereof is capable of dissolution in an aqueous medium below about pH 7, such as from pH 1-7, from pH 3-7, or from pH 4-7.

The invention also provides pharmaceutically acceptable salts of a compound having Formula II or a tautomer of the compound. In some such embodiments, the salt is selected from lactate, malate, mesylate, acetate, tartrate, phosphate, sulfate, nitrate, HCl, citrate, or maleate. In some such embodiments, the salt is selected from lactate, malate, mesylate, acetate, or tartrate salts. In other embodiments the salt is selected from lactate, malate, or mesylate salts. Compounds of Formula II have the following formula: 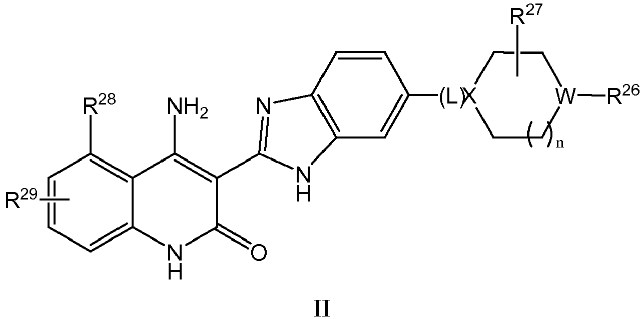 wherein:
L is a covalent bond, -(CH₂)ₘ, -CHR³⁰-, or -N(R³¹)-;
X is CH or N;
W is CH, O, or N;
R²⁶ is selected from the group consisting of substituted or unsubstituted alkyl groups, substituted or unsubstituted alkylamino groups, substituted or unsubstituted dialkylamino, -OH, substituted or unsubstituted alkoxy groups, substituted or unsubstituted alkylaminoalkyl groups, substituted or unsubstituted dialkylaminoalkyl groups, substituted or unsubstituted heterocyclyl groups, substituted or unsubstituted heterocyclylalkyl groups; provided that when W is O, R²⁶ is absent;
R²⁷ is absent or selected from the group consisting of -NO₂, -OH, F, Cl, Br, I, -NH₂, substituted or unsubstituted alkyl groups, substituted or unsubstituted alkylamino groups, substituted or unsubstituted dialkylamino groups, substituted or unsubstituted alkylaminoalkyl groups, substituted or unsubstituted dialkylaminoalkyl groups, and substituted or unsubstituted alkoxy groups;
R²⁸ is selected from the group consisting of H, F, Cl, Br, I, -OH, -NH₂, and substituted or unsubstituted alkyl groups;
R²⁹, R³⁰, and R³¹ are independently selected from the group consisting of H, F, Cl, Br, I, substituted and unsubstituted alkyl groups, -OH, alkoxy groups, substituted and unsubstituted aryloxy groups, -NH₂, substituted and unsubstituted aminoalkyl groups, substituted and unsubstituted aryl groups, substituted and unsubstituted heteroaryl groups, substituted and unsubstituted heterocyclyl groups, substituted and unsubstituted alkylaminoalkyl groups, substituted and unsubstituted dialkylaminoalkyl groups, substituted and unsubstituted arylaminoalkyl groups, substituted and unsubstituted diarylaminoalkyl groups, substituted and unsubstituted (alkyl)(aryl)aminoalkyl groups, substituted and unsubstituted alkylamino groups, substituted and unsubstituted dialkylamino groups, substituted and unsubstituted diarylamino groups, substituted and unsubstituted (alkyl)(aryl)amino groups, -C(=O)H, -C(=O)-alkyl groups, -C(=O)-aryl groups, -C(=O)O-alkyl groups, -C(=O)O-aryl groups, -C(=O)NH₂, -C(=O)NH(alkyl) groups, -C(=O)NH(aryl) groups, -C(=O)N(alkyl)₂ groups, -C(=O)N(aryl)₂ groups, -C(=O)N(alkyl)(aryl) groups, -C(=O)-heterocyclyl groups, -C(=O)-O-heterocyclyl groups, -C(=O)NH(heterocyclyl) groups, -C(=O)-N(heterocyclyl)₂ groups, -C(=O)-N(alkyl)(heterocyclyl) groups, -C(=O)-N(aryl)(heterocyclyl) groups, substituted and unsubstituted heterocyclylaminoalkyl groups, substituted and unsubstituted hydroxyalkyl groups, substituted and unsubstituted alkoxyalkyl groups, substituted and unsubstituted aryloxyalkyl groups, and substituted and unsubstituted heterocyclyloxyalkyl groups;
n is 0, 1, or 2; and
m is 1, 2, 3, or 4.

In some embodiments of the pharmaceutically acceptable salt of the compound of Formula II, the salt is selected from lactate, malate, mesylate, acetate, tartrate, phosphate, sulfate, nitrate, HCl, citrate, or maleate. In some such embodiments, the salt is selected from lactate, malate, mesylate, acetate, or tartrate salts. In some such embodiments, the salt is selected from lactate, malate, mesylate, bis-acetate, or tartrate salts. In some embodiments of the pharmaceutically acceptable salt of the compound of Formula II, the salt is provided as the lactate salt. In other embodiments, the salt is provided as the tartrate salt. In other embodiments, the salt is provided as the malate salt. In other embodiments, the salt is provided as the bis-acetate salt. In other embodiments, the salt is provided as the tartrate, mesylate, bishydrochloride, citrate or bismesylate salt.

In some embodiments of the pharmaceutically acceptable salt of the compound of Formula II, R²⁸ is F and R²⁹ is H. In some such embodiments, n is 1.

In some embodiments of the pharmaceutically acceptable salt of the compound of Formula II, n is 1.

In one embodiment, the pharmaceutically acceptable salt of the compound of Formula II or the tautomer thereof has a water solubility from about 20 mg/mL to about 100 mg/mL. In some embodiments, the salt of the compound of Formula II or the tautomer thereof has a water solubility of greater than 30 mg/mL. In other embodiments, the salt of the compound of Formula I or the tautomer thereof has a water solubility from about 150 mg/mL to about 250 mg/mL. In a more preferred embodiment the pharmaceutically acceptable salt of the compound of Formula II or the tautomer thereof is capable of dissolution in an aqueous medium below about pH 7.

The invention further provides pharmaceutical formulations and medicaments. Such formulations and medicaments include the pharmaceutically acceptable salt of Formula I or Formula II in combination with a pharmaceutically acceptable carrier.

The invention also provides methods of treating a patient in need of an inhibitor of vascular endothelial growth factor receptor tyrosine kinase. Such methods include administering an effective amount of a pharmaceutically acceptable salt or a pharmaceutical formulation or medicament that includes the pharmaceutically acceptable salt to a patient in need thereof.

The invention also relates to a method of preparing a pharmaceutically acceptable salt of a compound of Formula I or Formula II. The method includes:
(a) suspending the free base of the compound of Formula I or Formula II or the tautomers thereof in a solvent or mixture of solvents;
(b) contacting an acid selected from tartaric acid, malic acid, lactic acid, acetic acid, citric acid, hydrochloric acid, or methanesulfonic acid with the compound of Formula I or Formula II or the tautomers thereof to provide a mixture;
(c) heating the mixture;
(d) cooling the mixture;
(e) and isolating the salt.

In some methods for preparing a pharmaceutically acceptable salt of the compound of Formula I or Formula II, the mixture is cooled and the salt is precipitated out of the solution.

In some methods for preparing a pharmaceutically acceptable salt of the compound of Formula I or Formula II, the mixture is heated and refluxed prior to cooling.

In some embodiments of the method of preparing a pharmaceutically acceptable salt of a compound of Formula I or Formula II, the isolating step includes filtering the mixture.

In some embodiments, the acid is lactic acid and may be a mixture of the D and L forms of lactic acid or may be the D lactic acid or the L lactic acid.

In some embodiments, the solvent used in the method of preparing the salt is a protic solvent.

In other embodiments of the invention, the solvent used in the method of preparing the salt is selected from the group consisting of methanol, ethanol, propanol, isopropanol, butanol, 2-butanol, acetone, butanone, dioxanes, water, tetrahydrofuran, and combinations of these.

Compounds of Formula I and Formula II are readily synthesized from simple starting molecules as shown in the following Examples. Compounds of Formula I and Formula II may generally be prepared using benzene substituted with nitrile or carboxylic acid groups in addition to other optional groups.

Compounds of Formula I and Formula II and analogs of such compounds may be synthesized from simple starting molecules as shown in Schemes 1-4 and exemplified in the Examples. As shown in Scheme 1, 4-hydroxy analogs of compounds of Formula I and Formula II may generally be prepared using aromatic compounds substituted with amines and carboxylic acid groups.

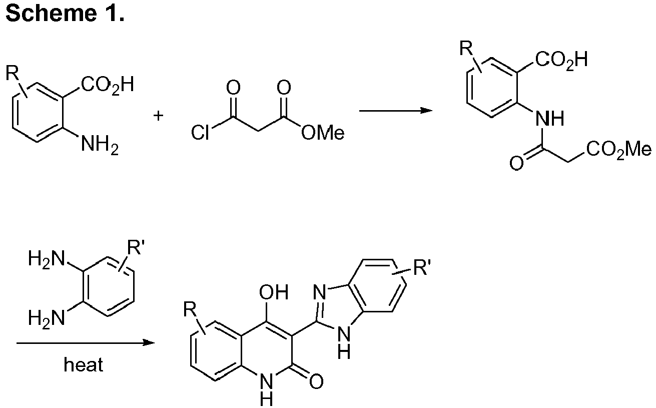

As shown in Scheme 1, a substituted aromatic compound such as a substituted or unsubstituted 2-aminobenzoic acid may be reacted with an acyl halide such as methyl 2-(chlorocarbonyl)acetate to produce an amide that will react with a substituted or unsubstituted 1,2-diaminobenzene. The resulting product is a 4-hydroxy-substituted analog of a compound of Formula I or Formula II. One skilled in the art will recognize that the procedure set forth in Scheme 1 may be modified to produce various compounds.

A method for preparing 4-amino substituted compounds of Formula I and Formula II is shown in Scheme 2. As shown in Scheme 2, aromatic compounds substituted with amine and nitrile groups may be used to synthesize 4-amino substituted compounds of Formula I or Formula II. A compound such as ethyl 2-cyanoacetate may be reacted with ethanol to produce ethyl 3-ethoxy-3-iminopropanoate hydrochloride. Subsequent reaction with a substituted or unsubstituted 1,2-phenylenediamine provides substituted or unsubstituted ethyl 2-benzimidazol-2-ylacetate. Reaction of a substituted or unsubstituted ethyl 2-benzimidazol-2-ylacetate with an aromatic compound having an amine and nitrile group such as substituted or unsubstituted 2-aminobenzonitrile with a base such as lithium bis(trimethylsilyl)amide or a Lewis acid such as tin tetrachloride provides the substituted or unsubstituted 4-amino substituted compound of Formula I and Formula II.

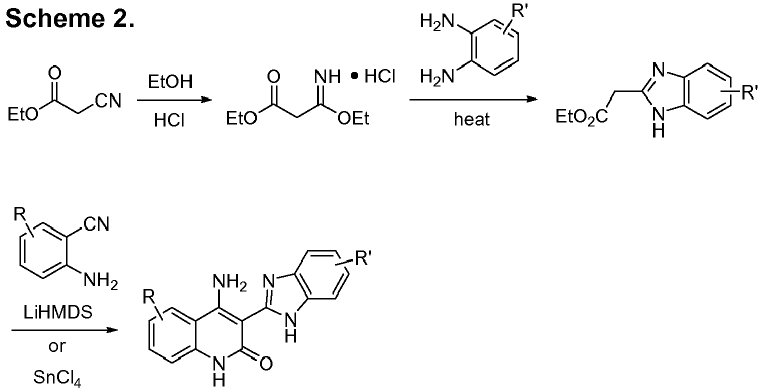

Scheme 3 illustrates a general synthetic route that allows for the synthesis of 4-dialkylamino and 4-alkylamino compounds of Formula I and Formula II. An inspection of Scheme 3 shows that 4-hydroxy substituted analogs of compounds of Formula I or Formula II may be converted into the 4-chloro derivative by reaction with phosphorous oxychloride or thionyl chloride. The 4-chloro derivative may then be reacted with an alkylamine or dialkylamine to produce the corresponding 4-alkylamino or 4-dialkylamino derivative. Deprotection affords the final 4-alkylamino or 4-dialkylamino compounds of Formula I or Formula II. Other groups that may be reacted with the 4-chloro derivative in this manner include, but are not limited to, ROH, RSH, and CuCN.

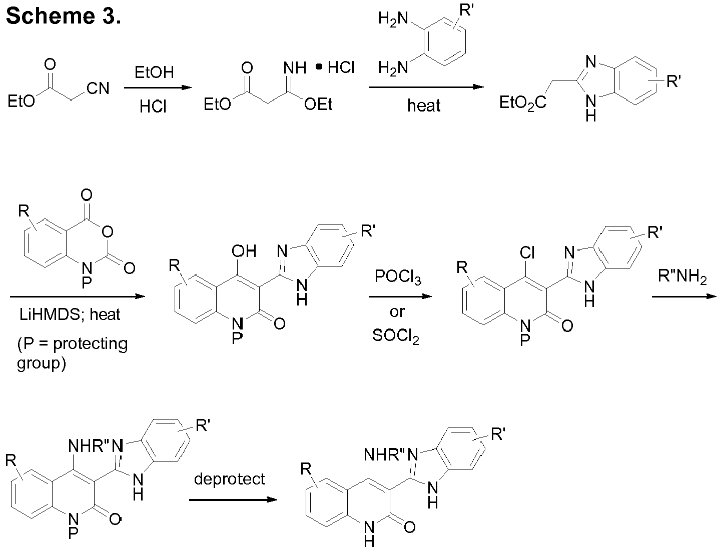

As shown in Scheme 4, the synthesis of analogs of compounds of Formula I or Formula II having a H, alkyl group, aryl group, or heterocyclyl group in the 4-position may be accomplished using a substituted or unsubstituted 2-benzimidazol-2-ylacetate prepared as shown in Schemes 2 and 3.

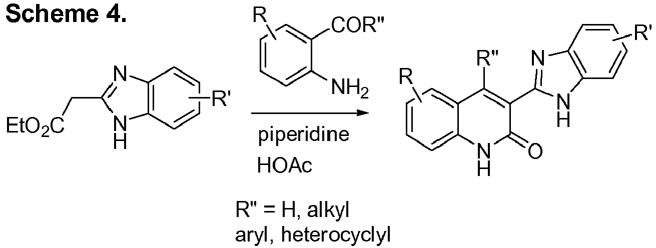

Heteroaromatic diamines may be used as precursors to produce heterocyclic analogs compounds of Formula I and Formula II. The synthesis of such analog compounds of Formula I and Formula II where NR¹²R¹³ = NH₂ is depicted in Scheme 5.

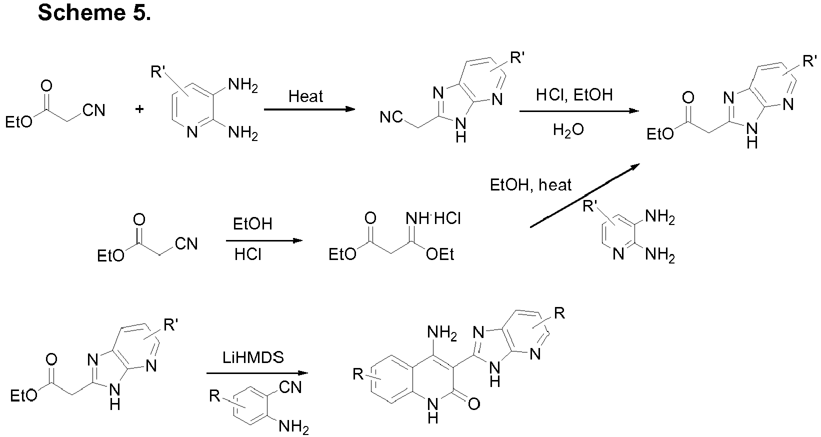

A compound such as ethyl cyanoacetate may be condensed with a substituted or unsubstituted heterocycle containing two ortho amino groups such as substituted or unsubstituted 1,2-diaminopyridine to obtain a substituted or unsubstituted 2-imidazolo[5,4-b]pyridin-2-ylethanenitrile, which may subsequently be hydrolyzed in acidic medium to provide a substituted or unsubstituted ethyl 2-imidazolo[5,4-b]pyridin-2-ylacetate. As an alternate route, a substituted or unsubstituted ethyl 2-imidazolo[5,4-b]pyridin-2-ylacetate may be obtained from a compound such as the hydrochloride salt of 3-ethoxy-3-iminopropanoate and a substituted or unsubstituted 1,2-diaminopyridine. Reaction of a substituted or unsubstituted ethyl 2-imidazolo[5,4-b]pyridin-2-ylacetates with an aromatic compound having an amine and nitrile group such as substituted or unsubstituted 2-aminobenzonitrile with a base such as lithium bis(trimethylsilyl)amide provides the substituted or unsubstituted analog of compounds of Formula I and Formula II.

The instant invention also provides for compositions which may be prepared by mixing one or more salts of the compounds of Formula I or Formula II, with pharmaceutically acceptable carriers, excipients, binders, diluents or the like, to treat or ameliorate a variety of disorders related to the activity of VEGF-RTK, more particularly angiogenesis associated with cancer.

Excipients, diluents, binders, carriers and the like include, but are not limited to, microcrystalline cellulose, lactose, dibasic calcium phosphate, tribasic calcium phosphate, sodium starch glycolate (NaSG), crospovidone, crosscarmellose (CC), sodium lauryl sulfate (SLS), Tween, polyethylene glycol (PEG), povidone, hydroxypropyl cellulose (HPMC), Mg stearate, Ca stearate, stearic acid, sodium stearate fumarate, and silicon dioxide.

A therapeutically effective dose further refers to that amount of one or more salts of the compounds of Formula I and/or Formula II sufficient to result in amelioration of symptoms of the disorder. The pharmaceutical compositions of the instant invention can be manufactured by methods well known in the art such as conventional granulating, mixing, dissolving, encapsulating, lyophilizing, emulsifying or levigating processes, among others. The compositions can be in the form of, for example, granules, powders, tablets, capsules, syrup, suppositories, injections, emulsions, elixirs, suspensions or solutions. The instant compositions can be formulated for various routes of administration, for example, by oral administration, by transmucosal administration, by rectal administration, or subcutaneous administration as well as intrathecal, intravenous, intramuscular, intraperitoneal, intranasal, intraocular or intraventricular injection. The salts of the compound or compounds of Formula I and Formula II can also be administered in a local rather than a systemic fashion, such as injection as a sustained release formulation. The following dosage forms are given by way of example and should not be construed as limiting the instant invention.

In order to determine the amount of compound in a patient following administration, certain manipulative steps can be taken. Such a method is described in the U.S. Provisional Application Serial No. 60/517,915, titled, "Methods of Treating Cancer and Related Methods" filed on November 7, 2003, by Vora *et al.* incorporated by reference in its entirety herein.

Oral, buccal, and sublingual administration, powders, suspensions, granules, tablets, pills, capsules, gelcaps, and caplets are acceptable as solid dosage forms. These can be prepared, for example, by mixing one or more salts of the compounds of Formula I and/or Formula II, with at least one additive or excipient such as a starch or other additive. Suitable additives or excipients are sucrose, lactose, cellulose sugar, mannitol, maltitol, dextran, sorbitol, starch, agar, alginates, chitins, chitosans, pectins, tragacanth gum, gum arabic, gelatins, collagens, casein, albumin, synthetic or semi-synthetic polymers or glycerides, methyl cellulose, hydroxypropylmethyl-cellulose, and/or polyvinylpyrrolidone . Optionally, oral dosage forms can contain other ingredients to aid in administration, such as an inactive diluent, or lubricants such as magnesium stearate, or preservatives such as paraben or sorbic acid, or anti-oxidants such as ascorbic acid, tocopherol or cysteine, a disintegrating agent, or chelating agents such as EDTA, binders, thickeners, buffers, sweeteners, flavoring agents or perfuming agents. Additionally, dyestuffs or pigments may be added for identification. Tablets and pills may be further treated with suitable coating materials known in the art, such as moisture protective, enteric, or sustained release coatings.

Liquid dosage forms for oral administration may be in the form of pharmaceutically acceptable emulsions, syrups, elixirs, suspensions, slurries and solutions, which may contain an inactive diluent, such as water. Pharmaceutical formulations may be prepared as liquid suspensions or solutions using a sterile liquid, such as, but not limited to, an oil, water, an alcohol, and combinations of these. Pharmaceutically suitable surfactants, suspending agents, emulsifying agents, sweeteners, flavoring agents, chelating agents, preservatives, antioxidants, solubilizers such as propylene glycol and glycerin and sorbitol may be added for oral or parenteral administration.

As noted above, suspensions may include oils. Such oil include, but are not limited to, peanut oil, sesame oil, cottonseed oil, corn oil and olive oil. Suspension preparation may also contain esters of fatty acids such as ethyl oleate, isopropyl myristate, fatty acid glycerides and acetylated fatty acid glycerides. Suspension formulations may include alcohols, such as, but not limited to, ethanol, isopropyl alcohol, hexadecyl alcohol, glycerol and propylene glycol. Ethers, such as but not limited to, poly(ethyleneglycol), petroleum hydrocarbons such as mineral oil and petrolatum; and water may also be used in suspension formulations. Furthermore suspension formulations may also include stabilizers, preservatives, antioxidants, surfactants, dyes, sweeteners, flavoring agents, solubilizers, thickeners, and emulsifying agents.

For nasal administration, the pharmaceutical formulations may be a spray or aerosol containing and appropriate solvents and optionally other compounds such as, but not limited to, stabilizers, antimicrobial agents, antioxidants, pH modifiers, surfactants, bioavailability modifiers and combinations of these. A propellant for an aerosol formulation may include compressed air, nitrogen, carbon dioxide, or a hydrocarbon based low boiling solvent. The salts of the compound or compounds of Formula I and/or Formula II are conveniently delivered in the form of an aerosol spray presentation from a nebulizer or the like.

Injectable dosage forms generally include aqueous suspensions or oil suspensions which may be prepared using a suitable dispersant or wetting agent and a suspending agent. Injectable forms may be in solution phase or in the form of a suspension, which is prepared with a solvent or diluent. Acceptable solvents or vehicles include sterilized water, Ringer's solution, or an isotonic aqueous saline solution. Alternatively, sterile oils may be employed as solvents or suspending agents. Preferably, the oil or fatty acid is non-volatile, including natural or synthetic oils, fatty acids, mono-, di- or tri-glycerides.

For injection, the pharmaceutical formulation may be a powder suitable for reconstitution with an appropriate solution as described above. Examples of these include, but are not limited to, freeze dried, rotary dried or spray dried powders, amorphous powders, granules, precipitates, or particulates. For injection, the formulations may optionally contain stabilizers, pH modifiers, surfactants, bioavailability modifiers and combinations of these. The salts of the compounds of Formula I and Formula II may be formulated for parenteral administration by injection such as by bolus injection or continuous infusion. A unit dosage form for injection may be in ampoules or in multi-dose containers.

For rectal administration, the pharmaceutical formulations may be in the form of a suppository, an ointment, an enema, a tablet or a cream for release of compound in the intestines, sigmoid flexure and/or rectum. Rectal suppositories are prepared by mixing one or more salts of the compounds of Formula I or Formula II, with acceptable vehicles, for example, cocoa butter or polyethylene glycol, which is present in a solid phase at normal storing temperatures, and present in a liquid phase at those temperatures suitable to release a drug inside the body, such as in the rectum. Oils may also be employed in the preparation of formulations of the soft gelatin type and suppositories. Water, saline, aqueous dextrose and related sugar solutions, and glycerols may be employed in the preparation of suspension formulations which may also contain suspending agents such as pectins, carbomers, methyl cellulose, hydroxypropyl cellulose or carboxymethyl cellulose, as well as buffers and preservatives.

In some embodiment, the salt is supplied in a powder form in a storage container such as a vial In some embodiments, the vial is closed and in other embodiments the vial can be evacuated with an inert gas and stoppered.

Besides those representative dosage forms described above, pharmaceutically acceptable excipients and carriers are generally known to those skilled in the art and are thus included in the instant invention. Such excipients and carriers are described, for example, in "Remingtons Pharmaceutical Sciences" Mack Pub. Co., New Jersey (1991), which is incorporated herein by reference.

The formulations of the invention may be designed for to be short-acting, fast-releasing, long-acting, and sustained-releasing as described below. Thus, the pharmaceutical formulations may also be formulated for controlled release or for slow release.

The instant compositions may also comprise, for example, micelles or liposomes, or some other encapsulated form, or may be administered in an extended release form to provide a prolonged storage and/or delivery effect. Therefore, the pharmaceutical formulations may be compressed into pellets or cylinders and implanted intramuscularly or subcutaneously as depot injections or as implants such as stents. Such implants may employ known inert materials such as silicones and biodegradable polymers.

Specific dosages may be adjusted depending on conditions of disease, the age, body weight, general health conditions, sex, and diet of the subject, dose intervals, administration routes, excretion rate, and combinations of drugs. Any of the above dosage forms containing effective amounts are well within the bounds of routine experimentation and therefore, well within the scope of the instant invention.

A therapeutically effective dose may vary depending upon the route of administration and dosage form. The preferred salts of compound or compounds of Formula I or Formula II are in a formulation that exhibits a high therapeutic index. The therapeutic index is the dose ratio between toxic and therapeutic effects which can be expressed as the ratio between LD₅₀ and ED₅₀. The LD₅₀ is the dose lethal to 50% of the population and the ED₅₀ is the dose therapeutically effective in 50% of the population. The LD₅₀ and ED₅₀ are determined by standard pharmaceutical procedures in animal cell cultures or experimental animals.

"Treating" within the context of the instant invention, means an alleviation of symptoms associated with a disorder or disease, or halt of further progression or worsening of those symptoms, or prevention or prophylaxis of the disease or disorder. For example, within the context of treating patients in need of an inhibitor of VEGF-RTK, successful treatment may include a reduction in the proliferation of capillaries feeding a tumor or diseased tissue, an alleviation of symptoms related to a cancerous growth or tumor, proliferation of capillaries, or diseased tissue, a halting in capillary proliferation, or a halting in the progression of a disease such as cancer or in the growth of cancerous cells. Treatment may also include administering the pharmaceutical formulations of the present invention in combination with other therapies. For example, the compounds and pharmaceutical formulations of the present invention may be administered before, during, or after surgical procedure and/or radiation therapy. The compounds of the invention can also be administered in conjunction with other anti-cancer drugs including those used in antisense and gene therapy.

In one embodiment of the invention is a method of treating a patient in need of an inhibitor of vascular endothelial growth factor receptor tyrosine kinase includes administering an effective amount of a pharmaceutical formulation according to the invention to a patient in need thereof.

In one embodiment of the invention is a method for inhibiting tumor growth in a patient includes administering an effective amount of a salt of the compound Formula I or Formula II to a patient having a tumor.

In one embodiment of the invention is a method for inhibiting the proliferation of capillaries in a patient includes administering an effective amount of a salt of the compound of Formula I or Formula II according to a patient in need.

In one embodiment of the invention is a method of preparing pharmaceutical formulations includes mixing any of the above-described salts of the compounds of Formula I or Formula II with a pharmaceutically acceptable carrier and water or an aqueous solution.

The present invention, thus generally described, will be understood more readily by reference to the following examples, which are provided by way of illustration and are not intended to be limiting of the present invention.

### EXAMPLES

The following abbreviations are used in the Examples:
- ATP:: Adenosine triphosphate
- BSA:: Bovine Serum Albumin
- DMA:: *N,N*-Dimethylacetamide
- DMF:: *N,N*-Dimethylformamide
- dppf:: 1,1' (diphenylphosphino)ferrocene
- DTT:: DL-Dithiothreitol
- EDTA:: Ethylene diamine tetraacetic acid
- EtOAc:: Ethyl acetate
- EtOH:: Ethanol
- HBTU:: O-Benzotriazol-1-yl-N,N,N',N'-tetramethyluronium hexafluorophosphate
- IC₅₀ value:: The concentration of an inhibitor that causes a 50% reduction in a measured activity.
- LiHMDS:: Lithium bis(trimethylsilyl)amide
- MeOH:: Methanol
- NMP:: N-methylpyrrolidone
- THF:: Tetrahydrofuran

The compounds were named using Nomenclator (v. 3.0 & v. 5.0) from CmemInovation Software, Inc. and ACD/Name v. 4.53.

### SYNTHETIC METHODOLOGY

The various aryl diamine starting materials used to synthesize benzimidazole acetates may be obtained from commercial sources, prepared by methods know to one of skill in the art, or prepared by the following general Methods 1-15.

### Method 1

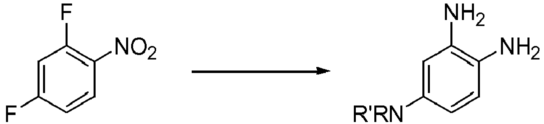

2,4-Difluoronitrobenzene (1.0 equivalent) was placed in a dry roundbottomed flask equipped with a dry ice condenser charged with acetone and dry ice. Ammonia was condensed into the flask and the resulting solution was stirred at reflux for 7 hours. A yellow precipitate formed within 1 hour. After 7 hours, the condenser was removed and the liquid ammonia was allowed to evaporate over several hours. The crude product was purified by flash chromatography on silica gel (85:15 hexanes:ethyl acetate, product at R_{f} = 0.32, contaminant at R_{f} = 0.51); GC/MS m/z 156.1 (M+), Rₜ 11.16 minutes.

The resulting 5-fluoro-2-nitrophenylamine (1.0 equivalent) and an amine (1.1 equivalents) e.g., N-methyl piperazine, were dissolved in NMP and triethylamine (2.0 equivalents) was added. The reaction mixture was heated at 100°C for 3 hours. The solution was then cooled to room temperature and diluted with water. The resulting precipitate was filtered and dried under vacuum to provide the 2-nitro-diamino product. Alternatively, the same product may be obtained from commercially available 5-chloro-2-nitrophenylamine under identical conditions except heating at 130°C for 1-2 days. In some examples, the displacement on either 5-fluoro-2-nitrophenylamine or 5-chloro-2-nitrophenylamine can be conducted in neat amine (5 equivalents) at 100°C or 130 °C, respectively. The product is isolated in an identical manner. LC/MS m/z 237.1 (MH+), Rₜ 1.304 minutes.

The nitroamine (1.0 equivalent) and 10% Pd/C (0.1 equivalents) was suspended in anhydrous ethanol at room temperature. The reaction flask was evacuated and subsequently filled with H₂. The resulting mixture was then stirred under a hydrogen atmosphere overnight. The resulting solution was filtered through Celite and concentrated under vacuum to provide the crude product which was used without further purification.

### Method 2

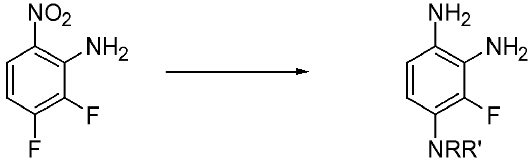

A round-bottom flask was charged with 2,3-difluoro-6-nitrophenylamine (1 equivalent) and enough NMP to make a viscous slurry. An amine (5 equivalents), e.g., N-methyl piperazine, was added and the solution was heated at 100°C. After 2 hours, the solution was cooled and poured into water. A bright yellow solid formed which was filtered and dried. The nitroamine was reduced as in Method 1 to provide the crude product which was used without further purification. LC/MS *m*/*z* 225.1 (MH+), R*ₜ* 0.335 minutes.

### Method 3

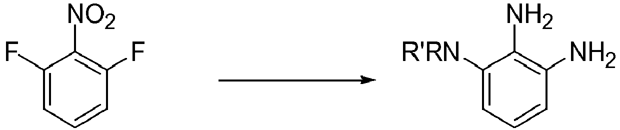

To a 0.1 M DMF solution of 1,3-difluoro-2-nitrobenzene was added Et₃N (2 equivalents) followed by an amine (1 equivalent), e.g., morpholine. The mixture was stirred for 18 hours and then diluted with water and extracted with ethyl acetate. LC/MS m/z 227.2 (MH+), R*ₜ* 2.522 minutes. The combined organic layers were dried over MgSO₄, filtered, and concentrated. Ammonia was condensed into a bomb containing the crude product. The bomb was sealed and heated to 100°C (over 400 psi). After 72 hours the bomb was allowed to cool and the ammonia was evaporated to provide a reddish solid. The nitroamine was reduced as in Method 1 to provide the crude product which was used without further purification. LC/MS m/z 194.1 (MH+), *Rₜ* 1.199 minutes.

### Method 4

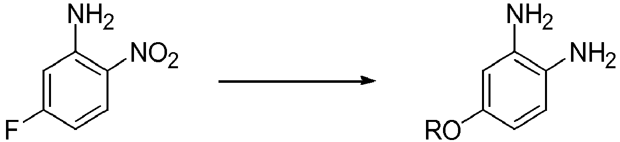

To a stirred NMP solution containing NaH (1.3 equivalents) was added an alcohol (1.0 equivalent), e.g., 2-methyloxyethanol. The resulting mixture was then stirred for 30 minutes. A slurry of 5-fluoro-2-nitrophenylamine in NMP was then added slowly. The mixture was then heated to 100°C. After 2 hours, the reaction mixture was cooled and water was added. The mixture was then filtered and the captured solid was washed with water and purified by silica gel chromatography (1:1 ethyl acetate:hexane). LC/MS m/z 213.2 (MH+), R*ₜ* 2.24 minutes. The nitroamine was reduced as in Method 1 to provide the crude product which was used without further purification. LC/MS *m*/*z* 183.1 (MH+), R*ₜ* 0.984 minutes.

### Method 5

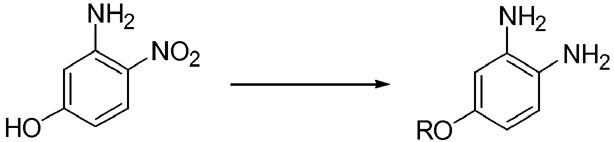

Diisopropyl azodicarboxylate (1.1 equivalents) was added dropwise to a stirred solution of 4-amino-3-nitrophenol (1.0 equivalent), triphenylphosphine (1.1 equivalents), and an alcohol, e.g., N-(2-hydroxyethyl)morpholine (1.0 equivalent), in tetrahydrofuran at 0°C. The mixture was allowed to warm to room temperature and stirred for 18 hours. The solvent was evaporated, and the product was purified by silica gel chromatography (98:2 CH₂Cl₂:methanol) to yield 4-(2-morpholin-4-ylethoxy)-2-nitrophenylamine as a dark reddish-brown oil. LC/MS m/z 268.0 (MH+), R*ₜ* 1.01 minutes. The nitroamine was reduced as in Method 1 to give the crude product which was used without further purification. LC/MS m/z 238.3 (MH+), *Rₜ* 0.295 minutes.

### Method 6

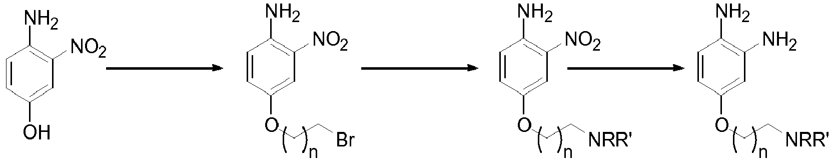

To a flask charged with 4-amino-3-nitrophenol (1 equivalent), K₂CO₃ (2 equivalents), and 2-butanone was added an alkyl dibromide, e.g., 1,3-dibromopropane (1.5 equivalents). The resulting mixture was then heated at 80°C for 18 hours. After cooling, the mixture was filtered, concentrated, and diluted with water. The solution was then extracted with CH₂Cl₂ (3 x) and the combined organic layers were concentrated to give a solid that was then washed with pentane. LCMS *m*/*z* 275.1 (MH+), R*ₜ* 2.74 minutes.

An acetonitrile solution of the bromide prepared above, an amine, e.g., pyrrolidine (5 equivalents), Cs₂CO₃ (2 eq) and Bu₄NI (0.1 equivalents) was heated at 70°C for 48 hours. The reaction mixture was cooled, filtered, and concentrated. The residue was dissolved in CH₂Cl₂, washed with water, and concentrated to give the desired nitroamine, 2-nitro-4-(3-pyrrolidin-1-ylpropoxy)phenylamine. LCMS m/z 266.2 (MH+), R*ₜ* 1.51 minutes. The nitroamine was reduced as in Method 1 to provide the crude product which was used without further purification.

### Method 7

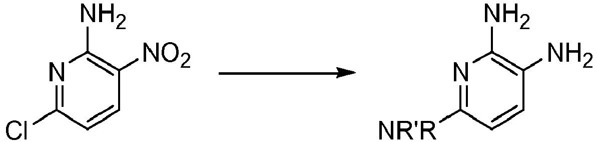

To a suspension of 6-chloro-3-nitropyridin-2-amine (1 equivalent) in acetonitrile was added an amine, e.g., morpholine (4 equivalent). The resulting reaction mixture was stirred at 70°C for 5 hours. The solvent was evaporated under reduced pressure, and the residue triturated with ether to provide the desired compound as a bright yellow powder. LC/MS m/z 225.0 (MH+), R*ₜ* 1.79 minutes. The nitroamine was reduced as in Method 1 to provide the crude product which was used without further purification.

### Method 8

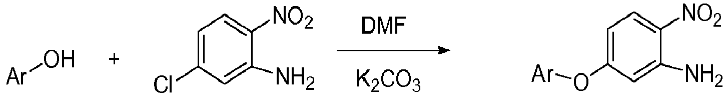

A phenol (1 equivalent) and 5-chloro-2-nitro aniline (1 equivalent) were dissolved in DMF, and solid K₂CO₃ (2 equivalents) was added in one portion. The reaction mixture was heated at 120°C overnight. The reaction mixture was cooled to room temperature, most of the DMF was distilled off, and water was added to the residue to obtain a precipitate. The solid was dried and purified by chromatography on silicagel (2-10% MeOH/CH₂Cl₂) to afford the desired product. The nitroamine was reduced as in method 1 to give the crude product that was used without further purification.

### Method 9

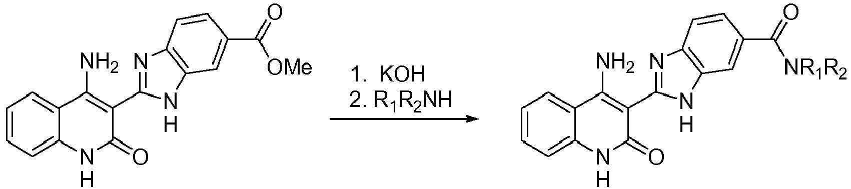

The introduction of substituents on the benzimidazole ring need not be limited to the early stages of the synthesis and may arise after formation of the quinolinone ring. For example, the crude methyl ester shown in the figure above was dissolved in a 1:1 mixture of EtOH and 30% aqueous KOH and stirred overnight at 70°C. The reaction mixture was then cooled and acidified with 1N HCl to give a precipitate. The solid was filtered, washed with water and dried to obtain 2-(4-amino-2-oxo-1,2-dihydroquinolin-3-yl)-1*H*-benzimidazole-6-carboxylic acid 2-(4-amino-2-oxo-3-hydroquinolyl)benzimidazole-6-carboxylic acid as a brown solid. LC/MS *m*/*z:* 321.1 (MH+), R*ₜ* 2.26 minutes.

A mixture of 2-(4-amino-2-oxo-1,2-dihydroquinolin-3-yl)-1*H-*benzimidazole-6-carboxylic acid (1 equivalent) the amine (1 equivalent), EDC (1-(3-dimethylaminopropyl)-3-ethylcarbodiimide hydrochloride, 1.2 equivalents), HOAT (1-hydroxy-7-azabenzotriazole, 1.2 eq) and triethylamine (2.5 equivalents) in DMF, was stirred at 23 °C for 20 hours. The reaction mixture was partitioned between water and ethyl acetate. The combined organic layers were dried (Na₂SO₄) and concentrated. Water was added and the precipitate thus formed was filtered off and dried to afford the desired product.

The various 2-amino benzoic acid starting materials used to synthesize isatoic anhydrides may be obtained from commercial sources, prepared by methods known to one of skill in the art, or prepared by the following general Methods 10-11. General isatoic anhydride synthesis methods are described in J. Med. Chem. 1981, 24 (6), 735 and J. Heterocycl. Chem. 1975, 12(3), 565.

### Method 10

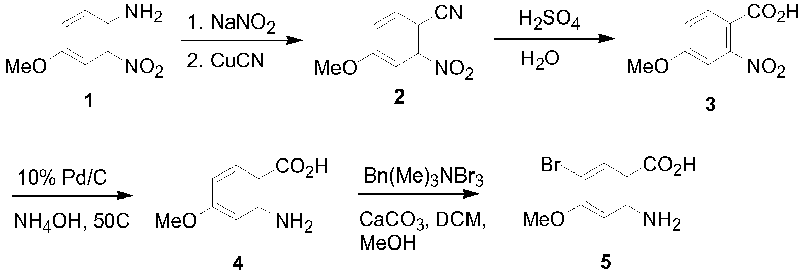

Compounds 1-3 were made using similar procedures as found in U.S. Patent No. 4,287,341. Compound 3 was reduced using standard hydrogenation conditions of 10% Pd/C in NH₄OH at 50°C over 48 hours. The product was precipitated by neutralizing with glacial acetic acid, filtering, and washing with water and ether. Yields were about 50%. Compound 5 was prepared in a manner similar to that disclosed in U.S. Patent No. 5,716,993.

### Method 11

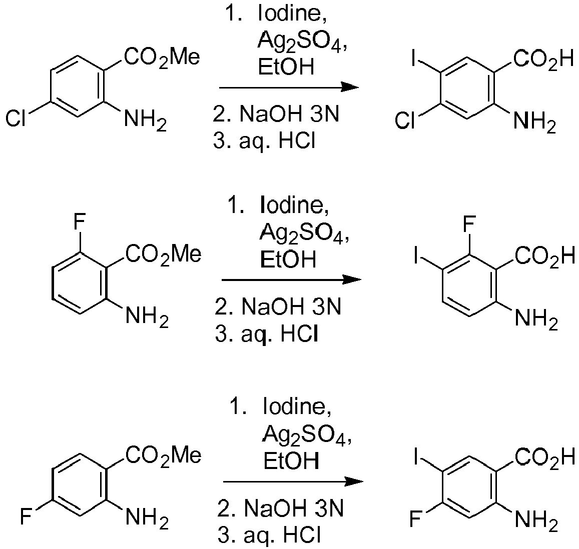

Iodination of aniline containing compounds was accomplished using various procedures. Iodination was accomplished using a procedure similar to that described in J. Med. Chem. 2001, 44, 6, 917-922. The anthranilic ester in EtOH was added to a mixture of silver sulfate (1 equivalent) and I₂ (1 equivalent). The reaction was typically done after 3 hours at room temperature. The reaction was filtered through celite and concentrated. The residue was taken up in EtOAc and washed with aqueous saturated NaHCO₃ (3x), water (3x), brine (1x), dried (MgSO₄), filtered, and concentrated. The crude product (∼5 g) was dissolved in MeOH (60-100 mL), NaOH 6N (25 mL), and water (250 mL). The reactions were typically done after heating at 70-80°C for 4 hours. The reaction mixture was extracted with EtOAc (2x), neutralized with aqueous HCl, filtered to collect the solids, and the solid products were washed with water. The products were dried *in vacuo.*

In various instances, substitutions on the quinolinone ring may also be introduced after coupling as shown in the general methods 12-15.

### Method 12

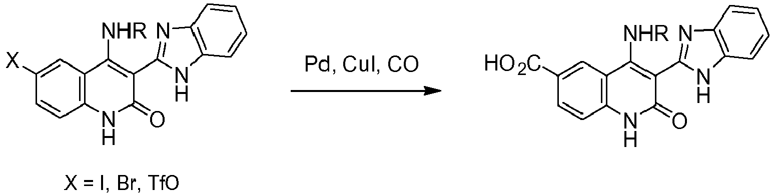

Conversion of the C-6 or C-7 halides to an acid group was accomplished using procedures in the following references: Koga, H. et al., Tet. Let., 1995, 36, 1, 87-90; and Fukuyama, T. et al., J. Am. Chem. Soc., 1994, 116, 3125-3126.

### Method 13

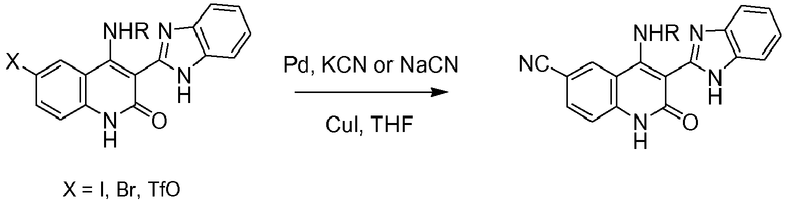

Conversion of the C-6 or C-7 halides to a cyano group was accomplished using procedures in the following reference. Anderson, B.A. et al., J. Org. Chem., 1998, 63, 8224-828.

### Method 14

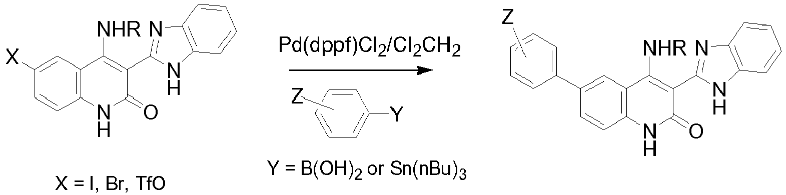

Conversion of the C-6 or C-7 halides to an aryl group was accomplished using standard Suzuki or Stille procedures such as described below.

Suzuki Method: To a 1 dram (4 mL) vial was added sequentially the quinolone (1 equivalent), boronic acid (1.2-1.5 equivalents), Pd(dppf)Cl₂, Cl₂CH₂ (0.2 equivalents), DMF (0.5 - 1 mL) and TEA (4 equivalents). The reaction was flushed with argon, capped and heated at 85°C for 12 hours. Once done, the reaction was cooled to room temperature, and filtered with a syringe filter disk. The clear solution was then neutralized with TFA (a couple of drops) and injected directly onto a preparative HPLC. The products were lyophilized to dryness.

Stille Method: To a 1 dram (4 mL) vial was added sequentially the quinolone (1 equivalent), tin reagent (1.8 equivalent), Pd(dppf)Cl₂. Cl₂CH₂ (0.2 equivalents), and DMF (0.5 - 1 mL). The reaction was flushed with argon, capped and heated at 60-85°C for 4 hours. Once done, the reaction was cooled to room temperature, and filtered with a syringe filter disk. The clear solution was then neutralized with TFA (a couple of drops) and injected directly onto a preparative HPLC. The products were lyophilized to dryness.

### Method 15

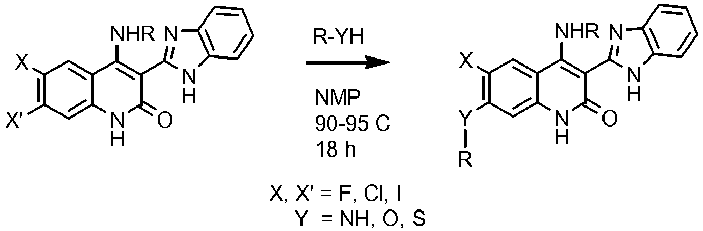

A dihaloquinolone such as a difluoroquinolone (12-15 mg) was placed in a 1 dram (2 mL) vial. NMP (dry and pre-purged with argon for 5 minutes) was added to the vial (0.5 mL). The amine reagent (40-50 mg) was added next. If the amine was an HCl salt, the reaction was neutralized with TEA (∼1.2-1.5 equivalents). The reaction was purged again with argon for about 5 seconds, and immediately capped. The reaction was typically heated in a heating block at 90-95°C for 18 hours. The reaction was followed by HPLC or LCMS. After taking samples for HPLC, the vial was purged with argon again and capped. Some coupling partners took 24 or 48 hours to reach completion. Less nucleophilic amines like pyrrole required the addition of a strong base to reach completion. In these cases, cesium carbonate (2 equivalents based on the amine used) was added to the reaction. Once done, the reaction was cooled to room temperature, and filtered with a syringe filter disk. The clear solution was then neutralized with TFA (a couple of drops) and injected directly onto a preparative HPLC. The products were lyophilized to dryness.

### Method 16

### General synthesis of compounds of Formula I and Formula II such as 4-amino-5-fluoro-3-[6-(4-methylpiperazin-1-yl)-1H-benzimidazol-2-yl]-1H-quinolin-2-one

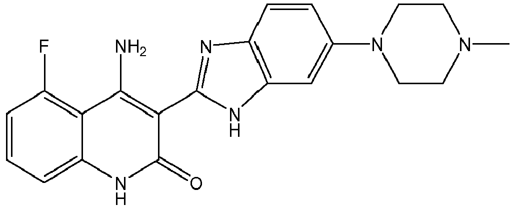

### A. Synthesis of 5-(4-Methyl-piperazin-1-yl)-2-nitroaniline

### Procedure A

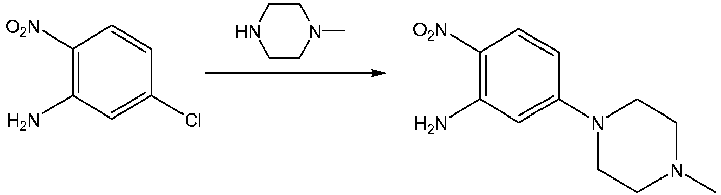

5-Chloro-2-nitroaniline (500 g, 2.898 mol) and 1-methyl piperazine (871 g, 8.693 mol) were placed in a 2000 mL flask fitted with a condenser and purged with N₂. The flask was placed in an oil bath at 100°C and heated until the 5-chloro-2-nitroaniline was completely reacted (typically overnight) as determined by HPLC. After HPLC confirmed the disappearance of the 5-chloro-2-nitroaniline, the reaction mixture was poured directly (still warm) into 2500 mL of room temperature water with mechanical stirring. The resulting mixture was stirred until it reached room temperature and then it was filtered. The yellow solid thus obtained was added to 1000 mL of water and stirred for 30 minutes. The resulting mixture was filtered, and the resulting solid was washed with TBME (500 mL, 2X) and then was dried under vacuum for one hour using a rubber dam. The resulting solid was transferred to a drying tray and dried in a vacuum oven at 50°C to a constant weight to yield 670 g (97.8%) of the title compound as a yellow powder.

### Procedure B

5-Chloro-2-nitroaniline (308.2 g, 1.79 mol) was added to a 4-neck 5000 mL round bottom flask fitted with an overhead stirrer, condenser, gas inlet, addition funnel, and thermometer probe. The flask was then purged with N₂. 1-Methylpiperazine (758.1 g, 840 mL, 7.57 mol) and 200 proof ethanol (508 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 20°C to 25°C with stirring, and the reaction was stirred for 2 to 3 hours. Seed crystals (0.20 g, 0.85 mmol) of 5-(4-methyl-piperazin-1-yl)-2-nitroaniline were added to the reaction mixture unless precipitation had already occurred. Water (2,450 mL) was added to the stirred reaction mixture over a period of about one hour while the internal temperature was maintained at a temperature ranging from about 20°C to 30°C. After the addition of water was complete, the resulting mixture was stirred for about one hour at a temperature of 20°C to 30°C. The resulting mixture was then filtered, and the flask and filter cake were washed with water (3 x 2.56 L). The golden yellow solid product was dried to a constant weight of 416 g (98.6% yield) under vacuum at about 50°C in a vacuum oven.

### Procedure C

5-Chloro-2-nitroaniline (401 g, 2.32 mol) was added to a 4-neck 12 L round bottom flask fitted with an overhead stirrer, condenser, gas inlet, addition funnel, and thermometer probe. The flask was then purged with N₂. 1-Methylpiperazine (977 g, 1.08 L, 9.75 mol) and 100% ethanol (650 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 80°C with stirring, and water (3.15 L) was added to the mixture via an addition funnel over the period of 1 hour while the internal temperature was maintained at 82°C (+/- 3°C). After water addition was complete, heating was discontinued and the reaction mixture was allowed to cool over a period of no less than 4 hours to an internal temperature of 20-25°C. The reaction mixture was then stirred for an additional hour at an internal temperature of 20-30°C. The resulting mixture was then filtered, and the flask and filter cake were washed with water (1 x 1 L), 50% ethanol (1 x 1L), and 95% ethanol (1 x 1L). The golden yellow solid product was placed in a drying pan and dried to a constant weight of 546 g (99% yield) under vacuum at about 50°C in a vacuum oven.

### B. Synthesis of [6-(4-Methyl-piperazin-1-yl)-1H-benzimidazol-2-yl]-acetic acid ethyl ester

### Procedure A

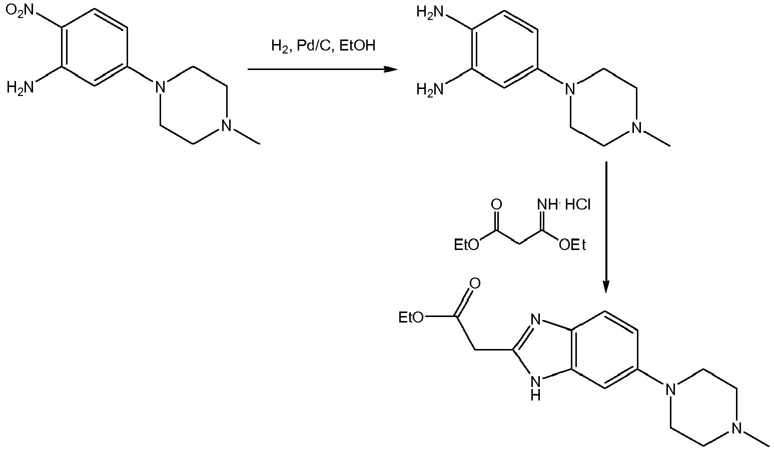

A 5000 mL, 4-neck flask was fitted with a stirrer, thermometer, condenser, and gas inlet/outlet. The equipped flask was charged with 265.7 g (1.12 mol. 1.0 eq) of 5-(4-methyl-piperazin-1-yl)-2-nitroaniline and 2125 mL of 200 proof EtOH. The resulting solution was purged with N₂ for 15 minutes. Next, 20.0 g of 5% Pd/C (50% H₂O w/w) was added. The reaction was vigorously stirred at 40-50°C (internal temperature) while H₂ was bubbled through the mixture. The reaction was monitored hourly for the disappearance of 5-(4-methyl-piperazin-1-yl)-2-nitroaniline by HPLC. The typical reaction time was 6 hours.

After all the 5-(4-methyl-piperazin-1-yl)-2-nitroaniline had disappeared from the reaction, the solution was purged with N₂ for 15 minutes. Next, 440.0 g (2.25 mol) of ethyl 3-ethoxy-3-iminopropanoate hydrochloride was added as a solid. The reaction was stirred at 40-50°C (internal temperature) until the reaction was complete. The reaction was monitored by following the disappearance of the diamino compound by HPLC. The typical reaction time was 1-2 hours. After the reaction was complete, it was cooled to room temperature and filtered through a pad of Celite filtering material. The Celite filtering material was washed with absolute EtOH (2 x 250 mL), and the filtrate was concentrated under reduced pressure providing a thick brown/orange oil. The resulting oil was taken up in 850 mL of a 0.37% HCl solution. Solid NaOH (25 g) was then added in one portion, and a precipitate formed. The resulting mixture was stirred for 1 hour and then filtered. The solid was washed with H₂O (2 x 400 mL) and dried at 50°C in a vacuum oven providing 251.7 g (74.1%) of [6-(4-methyl-piperazin-1-yl)-1H-benzoimidazol-2-yl]-acetic acid ethyl ester as a pale yellow powder.

### Procedure B

A 5000 mL, 4-neck jacketed flask was fitted with a mechanical stirrer, condenser, temperature probe, gas inlet, and oil bubbler. The equipped flask was charged with 300 g (1.27 mol) of 5-(4-methyl-piperazin-1-yl)-2-nitroaniline and 2400 mL of 200 proof EtOH (the reaction may be and has been conducted with 95% ethanol and it is not necessary to use 200 proof ethanol for this reaction). The resulting solution was stirred and purged with N₂ for 15 minutes. Next, 22.7 g of 5% Pd/C (50% H₂O w/w) was added to the reaction flask. The reaction vessel was purged with N₂ for 15 minutes. After purging with N₂, the reaction vessel was purged with H₂ by maintaining a slow, but constant flow of H₂ through the flask. The reaction was stirred at 45-55°C (internal temperature) while H₂ was bubbled through the mixture until the 5-(4-methyl-piperazin-1-yl)-2-nitroaniline was completely consumed as determined by HPLC. The typical reaction time was 6 hours.

After all the 5-(4-methyl-piperazin-1-yl)-2-nitroaniline had disappeared from the reaction, the solution was purged with N₂ for 15 minutes. The diamine intermediate is air sensitive so care was taken to avoid exposure to air. 500 g (2.56 mol) of ethyl 3-ethoxy-3-iminopropanoate hydrochloride was added to the reaction mixture over a period of about 30 minutes. The reaction was stirred at 45-55°C (internal temperature) under N₂ until the diamine was completely consumed as determined by HPLC. The typical reaction time was about 2 hours. After the reaction was complete, the reaction was filtered while warm through a pad of Celite. The reaction flask and Celite were then washed with 200 proof EtOH (3 x 285 mL). The filtrates were combined in a 5000 mL flask, and about 3300 mL of ethanol was removed under vacuum producing an orange oil. Water (530 mL) and then 1M HCL (350 mL) were added to the resulting oil, and the resulting mixture was stirred. The resulting solution was vigorously stirred while 30% NaOH (200 mL) was added over a period of about 20 minutes maintaining the internal temperature at about 25-30°C while the pH was brought to between 9 and 10. The resulting suspension was stirred for about 4 hours while maintaining the internal temperature at about 20-25°C. The resulting mixture was filtered, and the filter cake was washed with H₂O (3 x 300 mL). The collected solid was dried to a constant weight at 50°C under vacuum in a vacuum oven providing 345.9 g (90.1%) of [6-(4-methyl-piperazin-1-yl)-1H-benzoimidazol-2-yl]-acetic acid ethyl ester as a pale yellow powder. In an alternative work up procedure, the filtrates were combined and the ethanol was removed under vacuum until at least about 90% had been removed. Water at a neutral pH was then added to the resulting oil, and the solution was cooled to about 0°C. An aqueous 20% NaOH solution was then added slowly with rapid stirring to bring the pH up to 9.2 (read with pH meter). The resulting mixture was then filtered and dried as described above. The alternative work up procedure provided the light tan to light yellow product in yields as high as 97%.

### Method for Reducing Water Content of [6-(4-Methyl-piperazin-1-yl)-1H-benzoimidazol-2-yl]-acetic acid ethyl ester

[6-(4-Methyl-piperazin-1-yl)-1H-benzimidazol-2-yl]-acetic acid ethyl ester (120.7 grams) that had been previously worked up and dried to a water content of about 8-9% H₂O was placed in a 2000 mL round bottom flask and dissolved in absolute ethanol (500 mL). The amber solution was concentrated to a thick oil using a rotary evaporator with heating until all solvent was removed. The procedure was repeated two more times. The thick oil thus obtained was left in the flask and placed in a vacuum oven heated at 50°C overnight. Karl Fisher analysis results indicated a water content of 5.25%. The lowered water content obtained by this method provided increased yields in the procedure of the following Example. Other solvents such as toluene and THF may be used in place of the ethanol for this drying process.

### C. Synthesis of 4-Amino-5-fluoro-3-[6-(4-methyl-piperazin-1-yl)-1H-benzimidazol-2-yl]-1H-quinolin-2-one

### Procedure A

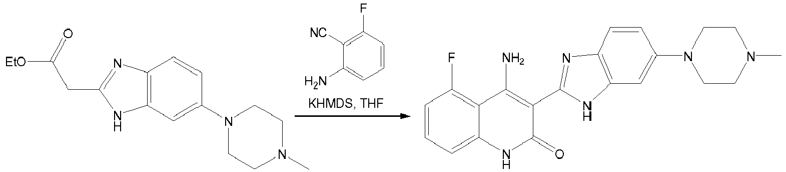

[6-(4-Methyl-piperazin-1-yl)-1H-benzimidazol-2-yl]-acetic acid ethyl ester (250 g, 820 mmol) (dried with ethanol as described above) was dissolved in THF (3800 mL) in a 5000 mL flask fitted with a condenser, mechanical stirrer, temperature probe, and purged with argon. 2-Amino-6-fluoro-benzonitrile (95.3 g, 700 mmol) was added to the solution, and the internal temperature was raised to 40°C. When all the solids had dissolved and the solution temperature had reached 40°C, solid KHMDS (376.2 g, 1890 mmol) was added over a period of 5 minutes. When addition of the potassium base was complete, a heterogeneous yellow solution was obtained, and the internal temperature had risen to 62°C. After a period of 60 minutes, the internal temperature decreased back to 40°C, and the reaction was determined to be complete by HPLC (no starting material or uncyclized intermediate was present). The thick reaction mixture was then quenched by pouring it into H₂O (6000 mL) and stirring the resulting mixture until it had reached room temperature. The mixture was then filtered, and the filter pad was washed with water (1000 mL 2X). The bright yellow solid was placed in a drying tray and dried in a vacuum oven at 50°C overnight providing 155.3 g (47.9%) of the desired 4-amino-5-fluoro-3-[6-(4-methyl-piperazin-1-yl)-1H-benzimidazol-2-yl]-1H-quinolin-2-one.

### Procedure B

A 5000 mL 4-neck jacketed flask was equipped with a distillation apparatus, a temperature probe, a N₂ gas inlet, an addition funnel, and a mechanical stirrer. [6-(4-Methyl-piperazin-1-yl)-1H-benzimidazol-2-yl]-acetic acid ethyl ester (173.0 g, 570 mmol) was charged into the reactor, and the reactor was purged with N₂ for 15 minutes. Dry THF (2600 mL) was then charged into the flask with stirring. After all the solid had dissolved, solvent was removed by distillation (vacuum or atmospheric (the higher temperature helps to remove the water) using heat as necessary. After 1000 mL of solvent had been removed, distillation was stopped and the reaction was purged with N₂. 1000 mL of dry THF was then added to the reaction vessel, and when all solid was dissolved, distillation (vacuum or atmospheric) was again conducted until another 1000 mL of solvent had been removed. This process of adding dry THF and solvent removal was repeated at least 4 times (on the 4^{th} distillation, 60% of the solvent is removed instead of just 40% as in the first 3 distillations) after which a 1 mL sample was removed for Karl Fischer analysis to determine water content. If the analysis showed that the sample contained less than 0.20% water, then reaction was continued as described in the next paragraph. However, if the analysis showed more than 0.20% water, then the drying process described above was continued until a water content of less than 0.20% was achieved.

After a water content of less than or about 0.20% was achieved using the procedure described in the previous paragraph, the distillation apparatus was replaced with a reflux condenser, and the reaction was charged with 2-amino-6-fluoro-benzonitrile (66.2 g, 470 mmol) (in some procedures 0.95 equivalents is used). The reaction was then heated to an internal temperature of 38-42°C. When the internal temperature had reached 38-42°C, KHMDS solution (1313 g, 1.32 mol, 20% KHMDS in THF) was added to the reaction via the addition funnel over a period of 5 minutes maintaining the internal temperature at about 38-50°C during the addition. When addition of the potassium base was complete, the reaction was stirred for 3.5 to 4.5 hours (in some examples it was stirred for 30 to 60 minutes and the reaction may be complete within that time) while maintaining the internal temperature at from 38-42°C. A sample of the reaction was then removed and analyzed by HPLC. If the reaction was not complete, additional KHMDS solution was added to the flask over a period of 5 minutes and the reaction was stirred at 38-42°C for 45-60 minutes (the amount of KHMDS solution added was determined by the following: If the IPC ratio is < 3.50, then 125 mL was added; if 10.0 ≥ IPC ratio ≥ 3.50, then 56 mL was added; if 20.0 ≥ IPC ratio ≥ 10, then 30 mL was added. The IPC ratio is equal to the area corresponding to 4-amino-5-fluoro-3-[6-(4-methyl-piperazin-1-yl)-1H-benzimidazol-2-yl]-1H-quinolin-2-one) divided by the area corresponding to the uncyclized intermediate). Once the reaction was complete (IPC ratio > 20), the reactor was cooled to an internal temperature of 25-30°C, and water (350 mL) was charged into the reactor over a period of 15 minutes while maintaining the internal temperature at 25-35°C (in one alternative, the reaction is conducted at 40°C and water is added within 5 minutes. The quicker quench reduces the amount of impurity that forms over time). The reflux condenser was then replaced with a distillation apparatus and solvent was removed by distillation (vacuum or atmospheric) using heat as required. After 1500 mL of solvent had been removed, distillation was discontinued and the reaction was purged with N₂. Water (1660 mL) was then added to the reaction flask while maintaining the internal temperature at 20-30°C. The reaction mixture was then stirred at 20-30°C for 30 minutes before cooling it to an internal temperature of 5-10°C and then stirring for 1 hour. The resulting suspension was filtered, and the flask and filter cake were washed with water (3 x 650 mL). The solid thus obtained was dried to a constant weight under vacuum at 50°C in a vacuum oven to provide 103.9 g (42.6% yield) of 4-amino-5-fluoro-3-[6-(4-methyl-piperazin-1-yl)-1H-benzimidazol-2-yl]-1H-quinolin-2-one as a yellow powder.

### Procedure C

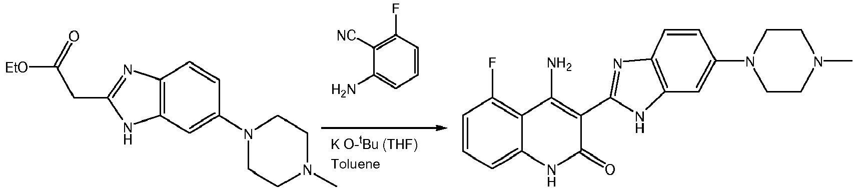

[6-(4-Methyl-piperazin-1-yl)-1H-benzimidazol-2-yl]-acetic acid ethyl ester (608 g, 2.01 mol) (dried) and 2-amino-6-fluoro-benzonitrile (274 g, 2.01 mol) were charged into a 4-neck 12 L flask seated on a heating mantle and fitted with a condenser, mechanical stirrer, gas inlet, and temperature probe. The reaction vessel was purged with N₂, and toluene (7.7 L) was charged into the reaction mixture while it was stirred. The reaction vessel was again purged with N₂ and maintained under N₂. The internal temperature of the mixture was raised until a temperature of 63°C (+/- 3°C) was achieved. The internal temperature of the mixture was maintained at 63°C (+/- 3°C) while approximately 2.6 L of toluene was distilled from the flask under reduced pressure (380 +/- 10 torr, distilling head t = 40°C (+/- 10°C) (Karl Fischer analysis was used to check the water content in the mixture. If the water content was greater than 0.03%, then another 2.6 L of toluene was added and distillation was repeated. This process was repeated until a water content of less than 0.03% was achieved). After a water content of less than 0.03% was reached, heating was discontinued, and the reaction was cooled under N₂ to an internal temperature of 17-19°C. Potassium t-butoxide in THF (20% in THF; 3.39 kg, 6.04 moles potassium t-butoxide) was then added to the reaction under N₂ at a rate such that the internal temperature of the reaction was kept below 20°C. After addition of the potassium t-butoxide was complete, the reaction was stirred at an internal temperature of less than 20°C for 30 minutes. The temperature was then raised to 25°C, and the reaction was stirred for at least 1 hour. The temperature was then raised to 30°C, and the reaction was stirred for at least 30 minutes. The reaction was then monitored for completion using HPLC to check for consumption of the starting materials (typically in 2-3 hours, both starting materials were consumed (less than 0.5% by area % HPLC)). If the reaction was not complete after 2 hours, another 0.05 equivalents of potassium t-butoxide was added at a time, and the process was completed until HPLC showed that the reaction was complete. After the reaction was complete, 650 mL of water was added to the stirred reaction mixture. The reaction was then warmed to an internal temperature of 50°C and the THF was distilled away (about 3 L by volume) under reduced pressure from the reaction mixture. Water (2.6 L) was then added dropwise to the reaction mixture using an addition funnel. The mixture was then cooled to room temperature and stirred for at least 1 hour. The mixture was then filtered, and the filter cake was washed with water (1.2 L), with 70% ethanol (1.2 L), and with 95% ethanol (1.2 L). The bright yellow solid was placed in a drying tray and dried in a vacuum oven at 50°C until a constant weight was obtained providing 674 g (85.4%) of the desired 4-amino-5-fluoro-3-[6-(4-methyl-piperazin-1-yl)-1H-benzimidazol-2-yl]-1H-quinolin-2-one.

### Purification of 4-Amino-5-fluoro-3-[6-(4-methyl-piperazin-1-yl)-1H-benzimidazol-2-yl]-1H-quinolin-2-one

A 3000 mL 4-neck flask equipped with a condenser, temperature probe, N₂ gas inlet, and mechanical stirrer was placed in a heating mantle. The flask was then charged with 4-amino-5-fluoro-3-[6-(4-methyl-piperazin-1-yl)-1H-benzimidazol-2-yl]-1H-quinolin-2-one (101.0 g, 0.26 mol), and the yellow solid was suspended in 95% ethanol (1000 mL) and stirred. In some cases an 8:1 solvent ratio is used The suspension was then heated to a gentle reflux (temperature of about 76°C) with stirring over a period of about 1 hour. The reaction was then stirred for 45-75 minutes while refluxed. At this point, the heat was removed from the flask and the suspension was allowed to cool to a temperature of 25-30°C. The suspension was then filtered, and the filter pad was washed with water (2 x 500 mL). The yellow solid was then placed in a drying tray and dried in a vacuum oven at 50°C until a constant weight was obtained (typically 16 hours) to obtain 97.2 g (96.2%) of the purified product as a yellow powder.

### D. Preparation of Lactic Acid Salt of 4-Amino-5-fluoro-3-[6-(4-methyl-piperazin-1-yl)-1H-benzimidazol-2-yl]-1H-quinolin-2-one

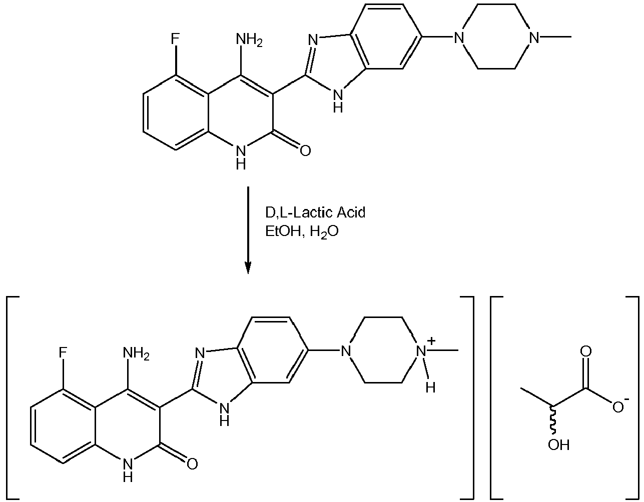

A 3000 mL 4-necked jacketed flask was fitted with a condenser, a temperature probe, a N₂ gas inlet, and a mechanical stirrer. The reaction vessel was purged with N₂ for at least 15 minutes and then charged with 4-amino-5-fluoro-3-[6-(4-methyl-piperazin-1-yl)-1H-benzimidazol-2-yl]-1H-quinolin-2-one (484 g, 1.23 mol). A solution of D,L-Lactic acid (243.3 g, 1.72 mol of monomer-see the following paragraph), water (339 mL), and ethanol (1211 mL) was prepared and then charged to the reaction flask. Stirring was initiated at a medium rate, and the reaction was heated to an internal temperature of 68-72°C. The internal temperature of the reaction was maintained at 68-72°C for 15-45 minutes and then heating was discontinued. The resulting mixture was filtered through a 10-20 micron frit collecting the filtrate in a 12 L flask. The 12 L flask was equipped with an internal temperature probe, a reflux condenser, an addition funnel, a gas inlet an outlet, and an overhead stirrer. The filtrate was then stirred at a medium rate and heated to reflux (internal temperature of about 78°C). While maintaining a gentle reflux, ethanol (3,596 mL) was charged to the flask over a period of about 20 minutes. The reaction flask was then cooled to an internal temperature ranging from about 64-70°C within 15-25 minutes and this temperature was maintained for a period of about 30 minutes. The reactor was inspected for crystals. If no crystals were present, then crystals of the lactic acid salt of 4-amino-5-fluoro-3-[6-(4-methyl-piperazin-1-yl)-1H-benzimidazol-2-yl]-1H-quinolin-2-one (484 mg, 0.1 mole %) were added to the flask, and the reaction was stirred at 64-70°C for 30 minutes before again inspecting the flask for crystals. Once crystals were present, stirring was reduced to a low rate and the reaction was stirred at 64-70°C for an additional 90 minutes. The reaction was then cooled to about 0°C over a period of about 2 hours, and the resulting mixture was filtered through a 25-50 micron fritted filter. The reactor was washed with ethanol (484 mL) and stirred until the internal temperature was about 0°C. The cold ethanol was used to wash the filter cake, and this procedure was repeated 2 more times. The collected solid was dried to a constant weight at 50°C under vacuum in a vacuum oven yielding 510.7 g (85.7%) of the crystalline yellow lactic acid salt of 4-amino-5-fluoro-3-[6-(4-methyl-piperazin-1-yl)-1H-benzimidazol-2-yl]-1H-quinolin-2-one. A rubber dam or inert conditions were typically used during the filtration process. While the dry solid did not appear to be very hygroscopic, the wet filter cake tends to pick up water and become sticky. Precautions were taken to avoid prolonged exposure of the wet filter cake to the atmosphere.

Commercial lactic acid generally contains about 8-12% w/w water, and contains dimers and trimers in addition to the monomeric lactic acid. The mole ratio of lactic acid dimer to monomer is generally about 1.0:4.7. Commercial grade lactic acid may be used in the process described in the preceding paragraph as the monolactate salt preferentially precipitates from the reaction mixture.

### SCREENING PROCEDURE FOR SALT SELECTION

In order to determine the improvements in characteristics of a salt of a compound of Formula I, certain baseline criteria were set and observed. These include:
1. Aqueous solubility > 10 mg/mL
2. Highly crystalline material as determined by X-Ray Powder Diffraction (XRPD), i.e., more chemically stable, less hygroscopic, preference for mono-salt to bis-salt as determined by NMR or ion chromatography, and high chemical yield.

### SCREENING TECHNIQUES

Physiochemical property screening techniques including equilibrium solubility, XRPD, hygroscopicity, compactibility, morphology, and solid-state stability were utilized to evaluate the free base and salts.

### Solubility

The aqueous solubility of compounds of Formula I and Formula II and their salts was determined by equilibrating excess solid with 1 mL of water for 24 hours at 22°C. A 200 uL aliquot was centrifuged at 15,000 rpm for 15 minutes. The supernatant was analyzed by HPLC and the solubility is expressed as its free base equivalent (mg FB/mL). For example, salts of 4-amino-5-fluoro-3-[6-(4-methylpiperazin-1-yl)-1H-benzimidazol-2-yl]-1H-quinolin-2-one were prepared and the solubility and solution pH was measured. A table comparing the aqueous solubility and pH at saturation for the various salts is shown below.

**Table 1. Solubility of 4-Amino-5-fluoro-3-[6-(4-methylpiperazin-1-yl)-1H-benzimidazol-2-yl]-1H-quinolin-2-one Free Base and Salts**

| **Form** | **Lot** | **pH at saturation** | **Solubility (mg FB/mL)** | **Crystallinity** |
|---|---|---|---|---|
| Mono-lactate | A | 5.78 | 216 | Crystalline |
| Mono-acetate | B | 5.38 | 84.4 | Amorphous |
| Mono-malate | C | 3.99 | 46.3 | Crystalline |
| Hemi-malate | D | 5.37 | 6.39 | Crystalline |
| Mono-tartrate | E | 4.33 | 2.18 | Crystalline |
| Mono-tartrate | F | 4.14 | 0.910 | Crystalline |
| Mono-tartrate | G | 3.92 | 0.889 | Crystalline |
| Mono-tartrate | H | 4.11 | 0.886 | Crystalline |
| Mono-mesylate | I | 5.33 | 185 | Crystalline |
| Mono-mesylate | J | 5.11 | 180 | Crystalline |
| Free Base | K | 6.03 | 0.0703 | Mixture of crystalline and amorphous |
| Free Base | L | 8.65 | 0.00748 | Crystalline |
| Mono-malate | M | 4.60 | 3.13 | Crystalline |
| Mono-malate | N | 3.93 | 8.14 | Crystalline |
| Mono-malate | O | 4.08 | 3.66 | Crystalline |
| Mono-lactate | P | 6.14 | 182 | Crystalline |
| Mono-lactate | Q | 5.65 | 202 | Crystalline |
| Mono-lactate | R | 5.60 | 196 | Crystalline |
| Bis-mesylate | S | 1.21 | >234 | Crystalline |
| Mono-citrate | T | 4.56 | 0.456 | Crystalline |
| Mono-lactate | U | 5.50 | 330 | Crystalline |
| Free base | AA | 9.64 | 0.011 | mixture of crystalline & amorphous |
| Lactate | BB | 5.05 | 34.2 | mixture of crystalline & amorphous |
| Bis-Lactate | CC | 3.89 | 304 (gelled) | mixture of crystalline & amorphous |
| Mesylate | DD | 5.77 | 22.3 | mixture of crystalline & amorphous |
| Bis-mesylate | EE | 2.51 | >475 (metaphasic liquid crystal gel) | mixture of crystalline & amorphous |
| Phosphate | FF | 3.98 | 13.8 | mixture of crystalline & amorphous |
| Sulfate | GG | 2.68 | 19.7 | mixture of crystalline & amorphous |
| L-Tartrate | HH | 3.44 | 103.4 | mixture of crystalline & amorphous |
| Acetate | II | 5.72 | 15.4 | mixture of crystalline & amorphous |
| Bis-Acetate | JJ | 5.46 | 31.9 | mixture of crystalline & amorphous |
| Nitrate | KK | 3.27 | 6.7 | ND |
| Hydrochloride | LL | 3.70 | 26.3 (gelled) | ND |
| BisHCl | MM | 1.39 | 42 (severe gelling) | ND |
| Citrate | NN | 3.48 | 27.2 | ND |
| Maleate | OO | 2.82 | 0.866 | ND |
| L-Malate | PP | 3.82 | 69 | mixture of crystalline & amorphous |
| Glycinate | QQ | 7.68 | 0.0638 | ND |
| Bis-Glycinate | RR | 7.40 | 0.11 | ND |

| | | | | |
|---|---|---|---|---|
| ND = Not Determined | | | | |

### Crystallinity

XRPD analyses were carried out on a Shimadzu XRD-6000 X-ray powder diffractometer using Cu K*α* radiation. The instrument is equipped with a fine focus X-ray tube. The tube voltage and amperage were set to 40 kV and 40 mA, respectively. The divergence and scattering slits were set at 1° and the receiving slit was set at 0.15 mm. Diffracted radiation was detected by a NaI scintillation detector. A theta-two theta continuous scan at 3 °/minute (0.4 seconds/0.02° step) from 2.5 to 40 °C was used. For example, salts of 4-amino-5-fluoro-3-[6-(4-methylpiperazin-1-yl)-1H-benzimidazol-2-yl]-1H-quinolin-2-one were prepared and the degree of crystallinity observed. Several of the salt forms in the above table were found to exhibit a high degree of crystallinity and have distinct powder X-ray diffraction patterns.

### Hygroscopicity

Moisture sorption/desorption data were collected on a VTI SGA-100 moisture balance system or equivalent. For sorption isotherms, a sorption range of 5 to 95% relative humidity (RH) and a desorption range of 95 to 5% RH in 10% RH increments at 25°C were used for each analysis. For example, salts of 4-amino-5-fluoro-3-[6-(4-methylpiperazin-1-yl)-1H-benzimidazol-2-yl]-1H-quinolin-2-one were prepared and the moisture induced weight change was measured.

**Table 2. Moisture Induced Weight Change in Salts of 4-Amino-5-fluoro-3-[6-(4-methylpiperazin-1-yl)-1H-benzimidazol-2-yl]-1H-quinolin-2-one**

| **Salt Form** | **Moisture induced % weight change** | | |
|---|---|---|---|
| | 55% RH | 85% RH | 95% RH |
| Lactate-trial 1 | 0.61 | 1.39 | 12.84 |
| Lactate-trial 2 | 0.13 | 0.42 | 2.76 |
| Lactate-trial 3 | 0.08 | 0.15 | 0.24 |
| Mesylate-trial 1 | 1.88 | 2.38 | 4.12 |
| Mesylate-trial 2 | 6.32 | 7.65 | 22.63 |
| Malate-trial 1 | 0.64 | 1.49 | 2.71 |
| Malate-trial 2 | 0.16 | 0.34 | 0.56 |
| Malate-trial 3 | 0.08 | 0.18 | 0.30 |

### Chemical Stability

Dry powder samples of free base and salts were maintained in open flasks under stress conditions at 30°C/60% relative humidity and 40°C/70% relative humidity. Solution samples of the free base and salts were stored in sealed vials under ambient temperature. Samples were pulled at pre-determined time-points and analyzed for chemical stability. Samples were pulled at pre-determined time-points and assayed by HPLC with UV-visible multiple wavelength detector Two tables comparing the solid state and solution state chemical stability of the various salts are given below.

**Table 3. Solid State Stability/HPLC Analysis of 4-amino-5-fluoro-3-[6-(4-methylpiperazin-1-yl)-1H-benzimidazol-2-yl]-1H-quinolin-2-one Free Base and Salts**

| **Salt** | **Area % at Time = 0** | **Area % at Time = 6 weeks** | **Storage condition** |
|---|---|---|---|
| Free base | 97.38 | 97.83 | 30°C/60% Relative humidity |
| Mesylate | 98.80 | 98.68 | 30°C/60% Relative humidity |
| Sulphate | 98.75 | 99.39 | 30°C/60% Relative humidity |
| Phosphate | 98.47 | 99.28 | 30°C/60% Relative humidity |
| Lactate | 98.41 | 99.04 | 30°C/60% Relative humidity |
| Bis-Mesylate | 99.01 | 98.12 | 30°C/60% Relative humidity |
| Bis-Lactate | 98.71 | 98.64 | 30°C/60% Relative humidity |
| Free base | 97.77 | 98.23 | 40°C/70% Relative humidity |
| Mesylate | 98.89 | 98.7 | 40°C/70% Relative humidity |
| Sulphate | 98.96 | 98.9 | 40°C/70% Relative humidity |
| Phosphate | 98.84 | 98.86 | 40°C/70% Relative humidity |
| Lactate | 98.46 | 98.55 | 40°C/70% Relative humidity |
| Bis-Mesylate | 98.78 | 98.35 | 40°C/70% Relative humidity |
| Bis-lactate | 98.99 | 97.92 | 40°C/70% Relative humidity |

**Table 4. Solution State Stability/HPLC Analysis of 4-Amino-5-fluoro-3-[6-(4-methylpiperazin-1-yl)-1H-benzimidazol-2-yl]-1H-quinolin-2-one Salts**

| **Salt** | **Area % at Time = 0** | **Area % at Time = 7 days** | **Storage condition** |
|---|---|---|---|
| Malate | 98.70 | 98.69 | 5°C |
| Mesylate | 98.70 | 98.76 | 5°C |
| Lactate | 98.80 | 98.60 | 5°C |
| Malate | 98.70 | 98.65 | ambient temperature |
| Mesylate | 98.70 | 98.77 | ambient temperature |
| Lactate | 98.80 | 98.71 | ambient temperature |

### Compaction studies

200 mg of powdered 4-amino-5-fluoro-3-[6-(4-methylpiperazin-1-yl)-1H-benzimidazol-2-yl]-1H-quinolin-2-one salt was preweighed and filled into a 0.8 cm diameter die and compressed at 5000 psi using a Carver Press (hold for 1 minute). The resulting tensile strength and thickness of the compacts were measured using a VK 200 Tablet Hardness Tester and Mitutoyo thickness gauge. For example, the lactate and mesylate salts of 4-amino-5-fluoro-3-[6-(4-methylpiperazin-1-yl)-1H-benzimidazol-2-yl]-1H-quinolin-2-one was prepared and compaction studies were performed. When compressed under the same applied pressure, lactate, malate, and mesylate salts form compacts; the lactate salt generally forms stronger compacts without a tendency to cap or chip.

**Table 5. Compaction of 4-amino-5-fluoro-3-[6-(4-methylpiperazin-1-yl)-1H-benzimidazol-2-yl]-1H-quinolin-2-one Salts**

| **Salt Form** | **Compact (200 mg at 5000 psi)** | | |
|---|---|---|---|
| | **Forms Compact** | **Thickness (mm)** | **Tensile strength (SC-Strong Cobb)** |
| Lactate-trial 1 | Yes | 3.06 | 13.8 |
| Lactate-trial 2 | Yes | 2.92 | 17.9 |
| Lactate-trial 3 | Yes | 2.97 | 16.1 |
| Mesylate-trial 1 | Yes | 2.77 | 6.9 |
| Mesylate-trial 2 | Yes | 2.87 | 17.9 |

### Morphology

The crystal morphology of 4-amino-5-fluoro-3-[6-(4-methylpiperazin-1-yl)-1H-benzimidazol-2-yl]-1H-quinolin-2-one salt was determined using a Nikon Eclipse 6600 POL polarized light microscope at 10x and 40x magnification.

**Table 6. Morphology of 4-amino-5-fluoro-3-[6-(4-methylpiperazin-1-yl)-1H-benzimidazol-2-yl]-1H-quinolin-2-one Salts**

| **Salt Form** | **Morphology** |
|---|---|
| Lactate-trial 1 | Plate |
| Lactate-trial 2 | Plate |
| Lactate-trial 3 | Plate |
| Mesylate-trial 1 | Plate |
| Mesylate-trial 2 | Needle |

As described above various physicochemical property screening techniques including solubility, XRPD, hygroscopicity, compactibility, morphology, and solid-state stability were utilized to evaluate salt forms of 4-amino-5-fluoro-3-[6-(4-methylpiperazin-1-yl)-1H-benzimidazol-2-yl]quinolin-2(1H)-one.

In the case of 4-amino-5-fluoro-3-[6-(4-methylpiperazin-1-yl)-1H-benzimidazol-2-yl]quinolin-2(1H)-one, the salt forms of the compound which exhibited solubilities in water generally between about 20 mg/mL and 330 mg/mL yielding a final water pH approximately between pH 3 to 6 without gelling were acetate, tartrate, malate, lactate, bis-acetate, mesylate, citrate, and bismesylate. Thus, changing 4-amino-5-fluoro-3-[6-(4-methylpiperazin-1-yl)-1H-benzimidazol-2-yl]-1H-quinolin-2-one free base (aqueous solubility about 0.01 mg/mL) to a salt form can significantly increase its solubility and rate of dissolution.

The XRPD pattern of the L-tartrate, citrate, L-malate, DL-lactate, mesylate, and bis-mesylate salts of 4-amino-5-fluoro-3-[6-(4-methylpiperazin-1-yl)-1H-benzimidazol-2-yl]-1H-quinolin-2-one (Lots A-U) display resolution of reflections, demonstrating that the samples are crystalline in nature. The XRPD pattern of acetate salt suggests that the sample is amorphous in nature.

The following salts of 4-amino-5-fluoro-3-[6-(4-methylpiperazin-1-yl)-1H-benzimidazol-2-yl]-1H-quinolin-2-one were analyzed by XRPD, mono-mesylate, sulfate, phosphate, mono-DL-lactate, bis-mesylate, bis-lactate, L-tartrate, bis-acetate, bis-acetate, and L-malate (Lots BB-PP). The XRPD patterns of all the samples (except bis-mesylate salt) display several broad reflections on an offset baseline, suggesting that the samples are composed of a mixture of crystalline and amorphous material or have a low degree of order (low crystallinity). The XRPD pattern of bis-mesylate salt displays resolution of reflections, demonstrating that the sample is crystalline in nature.

Of the crystalline salts, the malate, lactate, mesylate, and bis-mesylate salts exhibited solubilities in water generally between about 50 mg/mL and 330 mg/mL yielding a final water pH near pH 4 to 6. The degree of crystallinity of the free base or salt form can significantly impact its solubility and rate of dissolution. The following crystalline salts of 4-amino-5-fluoro-3-[6-(4-methylpiperazin-1-yl)-1H-benzimidazol-2-yl]-1H-quinolin-2-one were analyzed for hygroscopicity. The affinity of water sorption at 85% RH in general were mesylate > lactate = malate. The lactate and malate salts are considered non-hygroscopic. Stability data demonstrated that 4-amino-5-fluoro-3-[6-(4-methylpiperazin-1-yl)-1H-benzimidazol-2-yl]-1H-quinolin-2-one free base and selected salts exhibited adequate chemical stability.

The lactate salts have plate shape crystal morphology, whereas the mesylate varies from plate to needle, and the malate shows needle shape crystal morphology. Plates are preferred to needle shape crystals because of their better flow properties, which are critical for efficient formulation blending, filling, and tableting.

When compressed under the same applied pressure, lactate, malate, and mesylate salts form compacts; the lactate salt generally forms stronger compacts without tendency to cap or chip. The data generated from this example indicates that the lactate, acetate, malate, tartrate, mesylate, and citrate salts have improved aqueous solubility over the corresponding free base form. The lactate, malate, tartrate, mesylate, and citrate, bis-mesylate salts are crystalline in nature.

The malate and lactate salts can be considered non-hygroscopic thus minimizing the risk of chemical instability. The lactate salt shows good processability characteristics and is suitable for the development of tablets.

### Assay Procedures

### In vitro kinase assays for receptor tyrosine kinases

The kinase activity of various protein tyrosine kinases can be measured by providing ATP and a suitable peptide or protein tyrosine-containing substrate, and assaying the transfer of phosphate moiety to the tyrosine residue. Recombinant proteins corresponding to the cytoplasmic domains of the flt-1 (VEGFR1), KDR (VEGFR2), PDGF, and bFGF receptors were expressed in Sf9 insect cells using a Baculovirus expression system (In Vitrogen) and purified via Glu antibody interaction (for Glu-epitope tagged constructs) or by Metal Ion Chromatography (for His₆ (SEQ ID NO: 1) tagged constructs). For each assay, test compounds were serially diluted in DMSO then mixed with an appropriate kinase reaction buffer plus ATP. Kinase protein and an appropriate biotinylated peptide substrate were added to give a final volume of 100 µL, reactions were incubated for 1-2 hours at room temperature and stopped by the addition of 50 µL of 45 mM EDTA, 50 mM Hepes pH 7.5. Stopped reaction mix (75 µL) was transferred to a streptavidin coated microtiter plate (Boehringer Mannheim) and incubated for 1 hour. Phosphorylated peptide product was measured with the DELFIA time-resolved fluorescence system (Wallac), using a Eu-labeled anti-phosphotyrosine antibody PT66 with the modification that the DELFIA assay buffer was supplemented with 1 mM MgCl₂ for the antibody dilution. Time resolved fluorescence was read on a Wallac 1232 DELFIA fluorometer. The concentration of each compound for 50% inhibition (IC₅₀) was calculated by non-linear regression using XL Fit data analysis software.

Flt-1, KDR, PDGF, c-KIT, FLT-3 and bFGFR kinases were assayed in 50 mM Hepes pH 7.0, 2 mM MgCl₂, 10 mM MnCl₂, 1 mM NaF, 1 mM DTT, 1 mg/mL BSA, 2 µM ATP, and 0.42 µM biotin-GGGGQDGKDYIVLPI-NH₂ (SEQ ID NO: 2). Flt-1, KDR, and bFGFR kinases were added at 0.1 µg/mL, 0.05 µg/mL, or 0.1 µg/mL respectively.

### Example 1-Inhibition of CSF-1 mediated growth by 4-amino-5-fluoro-3-[6-(4-methylpiperazin-1-yl)-1H-benzimidazol-2-yl]-1H-quinolin-2-one

The antiproliferative activity of 4-amino-5-fluoro-3-[6-(4-methylpiperazin-1-yl)-1H-benzimidazol-2-yl]-1H-quinolin-2-one was shown to inhibit CSF-1 (Colony Stimulating Factor-1) mediated proliferation of M-NFS-60 cells (mouse myeloblast cell line) with an EC₅₀ of 300 nM. The assay was run by plating 5000 cells/well in 50 uL assay media (growth media without 67.1 ng/ml GM-CSF: RPMI-1640+10% FBS+0.044 mM beta Mercaptoethanol+2 mM L-Glut+Pen/Strep) in a 96 well plate. Serially-diluted 4-amino-5-fluoro-3-[6-(4-methylpiperazin-1-yl)-1H-benzimidazol-2-yl]-1H-quinolin-2-one in a DMSO stock solution starting at 20 µM was added to the plate in 50 µL assay media containing CSF-1 to make a final concentration of 10 ng/ml and then incubated for 72 hours at 37°C and 5% CO₂. The final DMSO concentration was 0.2%. After 72 hours of incubation, 100 µL of Cell Titer Glo (Promega #G755B) was added to the plate and, after shaking and a 10 minute incubation time, the luminescence was measured. The EC₅₀ was calculated using nonlinear regression.

Autophosphorylation of CSFR1 is inhibited by 4-amino-5-fluoro-3-[6-(4-methylpiperazin-1-yl)-1H-benzimidazol-2-yl]-1H-quinolin-2-one with concentrations < 1 µM. Treatment of M-NFS-60 cells with 4-amino-5-fluoro-3-[6-(4-methylpiperazin-1-yl)-1H-benzimidazol-2-yl]-1H-quinolin-2-one and treatment of the cells with CSF-1 for 5 minutes at the end of the incubation time, resulted in inhibition of receptor tyrosine phosphorylation detected by immunoprecipitation of CSFR1 and western blotting with an anti-phosphotyrosine antibody.

### Example 2-Inhibition of FGFR3 by 4-amino-5-fluoro-3-[6-(4-methylpiperazin-1-yl)-1H-benzimidazol-2-yl]-1H-quinolin-2-one

The t(4;14) translocation that occurs uniquely in a subset (15-20%) of multiple myeloma (MM) patients results in the ectopic expression of the receptor tyrosine kinase (RTK), FGFR3. The subsequent acquisition of FGFR3 activating mutations in some MM is associated with disease progression and is strongly transforming in experimental models.

4-Amino-5-fluoro-3-[6-(4-methylpiperazin-1-yl)-1H-benzimidazol-2-yl]-1H-quinolin-2-one inhibited proliferation of OPM-2 cells that express constitutively activated FGFR3 due to a K650E mutation with an EC₅₀ of 100 nM. The assay was run by plating 8000 cells/well in 50 µL assay media (RPMI-1640+10%FBS+Pen/Strep) in a 96 well plate. Serially-diluted 4-amino-5-fluoro-3-[6-(4-methylpiperazin-1-yl)-1H-benzimidazol-2-yl]-1H-quinolin-2-one in a DMSO stock solution starting at 20 µM was added to the plate in 50 µL assay media and then incubated for 72 hours at 37C and 5% CO₂. The final DMSO concentration was 0.2%. After 72 hours of incubation, 100 µL of Cell Titer Glo (Promega #G755B) was added to the plate and, after shaking and a 10 minute incubation time, the luminescence was read. The EC₅₀ was calculated using nonlinear regression. The EC₅₀ for 4-amino-5-fluoro-3-[6-(4-methylpiperazin-1-yl)-1H-benzimidazol-2-yl]-1H-quinolin-2-one in the H929 cell line (IMDM+10%FBS+Pen/Strep) that expresses WT FGFR3 receptor was 0.63 µM. The EC₅₀ was determined as described above using assay media that contained 50 ng/ml aFGF, 10 µg/ml Heparin and 1% FBS). The EC₅₀ was calculated using nonlinear regression from the ODs at 490 nm which were determined after adding MTS tetrazolium reagent (Promega) for 4 hours.

Significant apoptosis was seen after 6 days of treatment of OPM-2 cells with 4-amino-5-fluoro-3-[6-(4-methylpiperazin-1-yl)-1H-benzimidazol-2-yl]-1H-quinolin-2-one (>60% of the cells were AnnexinV positive using the protocol and instrument from Guava Technologies for detection of Annexin V positive cells).

The phosphorylation of downstream signaling component ERK was completely inhibited after incubation of OPM-2 cells with 0.1 µM of 4-amino-5-fluoro-3-[6-(4-methylpiperazin-1-yl)-1H-benzimidazol-2-yl]-1H-quinolin-2-one. Western blotting was used to show inhibition of ERK phosphorylation.

### Example 3-Inhibition of C-Met by 4-amino-5-fluoro-3-[6-(4-methylpiperazin-1-yl)-1H-benzimidazol-2-yl]-1H-quinolin-2-one

4-Amino-5-fluoro-3-[6-(4-methylpiperazin-1-yl)-1H-benzimidazol-2-yl]-1H-quinolin-2-one inhibited c-MET with an IC₅₀>3 µM. The kinase activity of c-MET was measured by providing ATP at a final concentration of 25 µM and 10 nM of the c-MET enzyme (Upstate#14-526) in the presence of 1 µM biotinylated substrate (KKKSPGEYVNIEFG (SEQ ID NO: 3)). Substrate bound to Streptavidin plates was detected with Europium labeled antiphosphotyrosine Antibody PT66. Phosphorylated peptide substrate was measured with the DELPHIA time resolved fluorescence system, and the IC₅₀ was calculated employing non-linear regression using XL Fit data analysis software. C-MET was constitutively activated in KM12L4A cells which is one of the most sensitive cell lines with respect to inhibition of proliferation by 4-amino-5-fluoro-3-[6-(4-methylpiperazin-1-yl)-1H-benzimidazol-2-yl]-1H-quinolin-2-one (EC₅₀ 20 nM). This suggests that 4-amino-5-fluoro-3-[6-(4-methylpiperazin-1-yl)-1H-benzimidazol-2-yl]-1H-quinolin-2-one either inhibits mutated c-MET or a kinase in the downstream signaling pathway of c-MET.

Each of the following compounds was synthesized and assayed using the procedures described above, or those described in U.S. Patent No. 6,605,617 which is hereby incorporated by reference in its entirety as if fully set forth herein:

**Table 7. Example Compounds**

| **Example** | **Name** | **LC/MS m/z (MH+)** |
|---|---|---|
| 1 | 4-amino-3-{5-[(3S)-3-(dimethylamino)pyrrolidin-1-yl]-1H-benzimidazol-2-yl}quinolin-2(1H)-one | 389.4 |
| 2 | 4-[(3S)-1-azabicyclo[2.2.2]oct-3-ylamino]-3-(1H-benzimidazol-2-yl)-6-chloroquinolin-2(1H)-one | 420 |
| 3 | 4-[(3R)-1-azabicyclo[2.2.2]oct-3-ylamino]-3-(1H-benzimidazol-2-yl)-6-chloroquinolin-2(1H)-one | 420 |
| 4 | 3-(1H-benzimidazol-2-yl)-4-[(3R)-3-(dimethylamino)pyrrolidin-1-yl]quinolin-2(1H)-one | 374.2 |
| 5 | 3-(1H-benzimidazol-2-yl)-6-chloro-4-[(3R)-3-(dimethylamino)pyrrolidin-1-yl]quinolin-2(1H)-one | 408.1 |
| 6 | 4-amino-3-[5-(4-ethylpiperazin-1-yl)-1H-benzimidazol-2-yl]-1-methylquinolin-2(1H)-one | 403.2 |
| 7 | 4-amino-3-(6-piperazin-1-yl-1H-benzimidazol-2-yl)quinolin-2(1 H)-one | 361.2 |
| 8 | 4-amino-3-[6-(pyridin-4-ylmethyl)-1H-benzimidazol-2-yl]quinolin-2(1H)-one | 368.2 |
| 9 | 4-amino-3-{5-[(3R,5S)-3,5-dimethylpiperazin-1-yl]-1H-benzimidazol-2-yl}quinolin-2(1H)-one | 389.4 |
| 10 | 4-amino-3-[5-(4-methylpiperazin-1-yl)-1H-benzimidazol-2-yl]quinolin-2(1H)-one | 375.2 |
| 11 | 4-amino-3-(6-methyl-5-morpholin-4-yl-1H-benzimidazol-2-yl)quinolin-2(1H)-one | 376 |
| 12 | 4-amino-3-{5-[(1-methylpiperidin-3-yl)oxy]-1H-benzimidazol-2-yl}quinolin-2(1H)-one | 390.1 |
| 13 | 4-amino-3-{5-[(2R,6S)-2,6-dimethylmorpholin-4-yl]-6-fluoro-1H-benzimidazol-2-yl}quinolin-2(1H)-one | 408.2 |
| 14 | 4-amino-3-{5-[(1-methylpyrrolidin-3-yl)oxy]-1H-benzimidazol-2-yl}quinolin-2(1H)-one | 376.2 |
| 15 | 4-amino-3-[5-(4-methyl-1,4-diazepan-1-yl)-1H-benzimidazol-2-yl]quinolin-2(1H)-one | 389.2 |
| 16 | 4-amino-3-{5-[(3R)-3-(dimethylamino)pyrrolidin-1-yl]-1H-benzimidazol-2-yl}quinolin-2(1H)-one | 389.2 |
| 17 | 4-amino-6-chloro-3-{5-[(3R)-3-(dimethylamino)pyrrolidin-1-yl]-1H-benzimidazol-2-yl}quinolin-2(1H)-one | 423 |
| 18 | ethyl {4-[2-(4-amino-2-oxo-1,2-dihydroquinolin-3-yl)-1H-benzimidazol-6-yl]piperazin-1-yl}acetate | 447.2 |
| 19 | 4-amino-3-{6-[methyl(1-methylpiperidin-4-yl)amino]-1H-benzimidazol-2-yl}quinolin-2(1H)-one | 403.1 |
| 20 | 3-[6-(4-acetylpiperazin-1-yl)-1H-benzimidazol-2-yl]-4-aminoquinolin-2(1H)-one | 403.3 |
| 21 | 4-amino-3-[6-(1,4'-bipiperidin-1'-yl)-1H-benzimidazol-2-yl]quinolin-2(1H)-one | 443.3 |
| 22 | 2-(4-amino-2-oxo-1,2-dihydroquinolin-3-yl)-1H-benzimidazole-6-carboxylic acid | 321.2 |
| 23 | 4-amino-5-(methyloxy)-3-[6-(4-methylpiperazin-1-yl)-1H-benzimidazol-2-yl]quinolin-2(1H)-one | 405.3 |
| 24 | 4-amino-3-{6-[4-(1-methylethyl)piperazin-1-yl]-1H-benzimidazol-2-yl}quinolin-2(1H)-one | 403.3 |
| 25 | {4-[2-(4-amino-2-oxo-1,2-dihydroquinolin-3-yl)-1H-benzimidazol-6-yl]piperazin-1-yl}acetic acid | 419.2 |
| 26 | 4-[(3S)-1-azabicyclo[2.2.2]oct-3-ylamino]-3-(1H-benzimidazol-2-yl)quinolin-2(1H)-one | 386.1 |
| 27 | 4-[(3R)-1-azabicyclo[2.2.2]oct-3-ylamino]-3-(1H-benzimidazol-2-yl)quinolin-2(1H)-one | 386.1 |
| 28 | 4-amino-3-[5-(4-ethylpiperazin-1-yl)-1H-benzimidazol-2-yl]quinolin-2(1H)-one | 389.1 |
| 29 | 4-amino-3-(5-{(2S,5S)-2-[(dimethylamino)methyl]-5-methylmorpholin-4-yl}-1H-benzimidazol-2-yl)quinolin-2(1 H)-one | 433.3 |
| 30 | 4-amino-6-chloro-3-[5-(4-methylpiperazin-1-yl)-1H-benzimidazol-2-yl]quinolin-2(1H)-one | 409.2 |
| 31 | 4-amino-6-chloro-3-{5-[(3S)-3-(dimethylamino)pyrrolidin-1-yl]-1H-benzimidazol-2-yl}quinolin-2(1H)-one | 423.1 |
| 32 | 4-amino-5,6-dichloro-3-{5-[(3S)-3-(dimethylamino)pyrrolidin-1-yl]-1H-benzimidazol-2-yl}quinolin-2(1H)-one | 457.2 |
| 33 | 4-amino-5,6-dichloro-3-[5-(4-methylpiperazin-1-yl)-1H-benzimidazol-2-yl]quinolin-2(1H)-one | 443.2 |
| 34 | 4-amino-3-(1H-benzimidazol-2-yl)-6-[(pyridin-2-ylmethyl)oxy]quinolin-2(1H)-one | 384.2 |
| 35 | 4-amino-3-(1H-benzimidazol-2-yl)-6-[(2R,6S)-2,6-dimethylmorpholin-4-yl]quinolin-2(1H)-one | 390.1 |
| 36 | 4-amino-3-(1H-benzimidazol-2-yl)-6-morpholin-4-ylquinolin-2(1H)-one | 362.2 |
| 37 | 4-amino-3-(1H-benzimidazol-2-yl)-5-[(1-methylpiperidin-3-yl)oxy]quinolin-2(1H)-one | 390.2 |
| 38 | 4-amino-3-(1H-benzimidazol-2-yl)-5-[(pyridin-2-ylmethyl)oxy]quinolin-2(1H)-one | 384.1 |
| 39 | 4-amino-3-(5-morpholin-4-yl-1H-benzimidazol-2-yl)-5-[(pyridin-4-ylmethyl)oxy]quinolin-2(1H)-one | 469.2 |
| 40 | 4-amino-3-(1H-benzimidazol-2-yl)-5-(methyloxy)quinolin-2(1H)-one | 307.1 |
| 41 | 4-amino-3-(5-methyl-1H-benzimidazol-2-yl)-5-(methyloxy)quinolin-2(1H)-one | 321.1 |
| 42 | 4-amino-3-{5-[(2R,6S)-2,6-dimethylmorpholin-4-yl]-1H-benzimidazol-2-yl}-5-(methyloxy)quinolin-2(1H)-one | 420.2 |
| 43 | 4-amino-3-(1H-benzimidazol-2-yl)-5-morpholin-4-ylquinolin-2(1H)-one | 362.2 |
| 44 | 4-amino-3-(1H-benzimidazol-2-yl)-5-[(2R,6S)-2,6-dimethylmorpholin-4-yl]quinolin-2(1H)-one | 390.2 |
| 45 | 4-amino-3-(1H-benzimidazol-2-yl)-5-(4-methylpiperazin-1-yl)quinolin-2(1H)-one | 375.1 |
| 46 | 4-amino-5,6-dichloro-3-(5-morpholin-4-yl-1H-benzimidazol-2-yl)quinolin-2(1H)-one | 430 |
| 47 | 3-{5-[(2-morpholin-4-ylethyl)oxy]-1H-benzimidazol-2-yl}quinolin-2(1H)-one | 391.3 |
| 48 | 4-amino-3-{5-[(3-pyrrolidin-1-ylpropyl)oxy]-1H-benzimidazol-2-yl}quinolin-2(1H)-one | 404 |
| 49 | 4-amino-3-{5-[(3-morpholin-4-ylpropyl)oxy]-1H-benzimidazol-2-yl}quinolin-2(1H)-one | 420.4 |
| 50 | 4-amino-6-fluoro-3-(5-morpholin-4-yl-1H-benzimidazol-2-yl)quinolin-2(1H)-one | 380 |
| 51 | 4-amino-3-{5-[3-(dimethylamino)pyrrolidin-1-yl]-1H-benzimidazol-2-yl}-6-fluoroquinolin-2(1H)-one | 407 |
| 52 | 4-amino-3-(1H-benzimidazol-2-yl)-6-fluoroquinolin-2(1H)-one | 295 |
| 53 | 4-amino-3-(6-fluoro-5-morpholin-4-yl-1H-benzimidazol-2-yl)quinolin-2(1H)-one | 380 |
| 54 | 4-amino-3-{5-[(tetrahydrofuran-2-ylmethyl)oxy]-1H-benzimidazol-2-yl}quinolin-2(1H)-one | 377 |
| 55 | 4-amino-6-fluoro-3-(6-fluoro-5-morpholin-4-yl-1H-benzimidazol-2-yl)quinolin-2(1H)-one | 398 |
| 56 | 4-amino-3-[6-fluoro-5-(4-methylpiperazin-1-yl)-1H-benzimidazol-2-yl]quinolin-2(1H)-one | 393 |
| 57 | 4-amino-3-(5-{[2-(methyloxy)ethyl]oxy}-1H-benzimidazol-2-yl)quinolin-2(1H)-one | 351 |
| 58 | 4-amino-3-[4,6-difluoro-5-(4-methylpiperazin-1-yl)-1H-benzimidazol-2-yl]quinolin-2(1H)-one | 411 |
| 59 | 4-amino-3-{5-[3-(dimethylamino)pyrrolidin-1-yl]-1H-benzimidazol-2-yl}-5-fluoroquinolin-2(1H)-one | 407.1 |
| 60 | 4-amino-5-fluoro-3-[6-(4-methylpiperazin-1-yl)-1H-benzimidazol-2-yl]quinolin-2(1H)-one | 393.1 |
| 61 | 4-amino-5-chloro-3-[5-(4-methylpiperazin-1-yl)-1H-benzimidazol-2-yl]quinolin-2(1H)-one | 409.1 |
| 62 | 4-amino-3-{5-[3-(dimethylamino)pyrrolidin-1-yl]-6-fluoro-1H-benzimidazol-2-yl}quinolin-2(1H)-one | 407.1 |
| 63 | 4-amino-5-chloro-3-{5-[3-(dimethylamino)pyrrolidin-1-yl]-1H-benzimidazol-2-yl}quinolin-2(1H)-one | 423.1 |
| 64 | 4-amino-6-chloro-3-{5-[3-(dimethylamino)pyrrolidin-1-yl]-6-fluoro-1H-benzimidazol-2-yl}quinolin-2(1H)-one | 441 |
| 65 | 4-amino-5-[(2R,6S)-2,6-dimethylmorpholin-4-yl]-3-(3H-imidazo[4,5-b]pyridin-2-yl)quinolin-2(1H)-one | 391.2 |
| 66 | 4-amino-3-(6-thiomorpholin-4-yl-1H-benzimidazol-2-yl)quinolin-2(1H)-one | 378.4 |
| 67 | 4-amino-3-[5-(4-cyclohexylpiperazin-1-yl)-1H-benzimidazol-2-yl]quinolin-2(1H)-one | 443.1 |
| 68 | 4-amino-3-{6-[3-(diethylamino)pyrrolidin-1-yl]-1 H-benzimidazol-2-yl}quinolin-2(1H)-one | 417.1 |
| 69 | 4-amino-3-[6-(4-pyridin-2-ylpiperazin-1-yl)-1H-benzimidazol-2-yl]quinolin-2(1H)-one | 438.3 |
| 70 | 4-amino-3-[5-(4-methylpiperazin-1-yl)-3H-imidazo[4,5-b]pyridin-2-yl]quinolin-2(1H)-one | 376.3 |
| 71 | 4-amino-6-chloro-3-[5-(4-methylpiperazin-1-yl)-1 H-imidazo[4,5-b]pyridin-2-yl]quinolin-2(1H)-one | 410.2 |
| 72 | 2-(4-amino-2-oxo-1,2-dihydroquinolin-3-yl)-N-methyl-N-(1-methylpiperidin-4-yl)-1H-benzimidazole-5-carboxamide | 431.3 |
| 73 | 4-amino-3-(5-{[4-(1-methylethyl)piperazin-1-yl]carbonyl}-1H-benzimidazol-2-yl)quinolin-2(1H)-one | 431.3 |
| 74 | 4-amino-3-[5-(4-methylpiperazin-1-yl)-1H-benzimidazol-2-yl]-6-nitroquinolin-2(1H)-one | 420.2 |
| 75 | 4-amino-3-[5-(1,4'-bipiperidin-1'-ylcarbonyl)-1H-benzimidazol-2-yl]quinolin-2(1H)-one | 471.1 |
| 76 | 4-amino-3-{5-[(4-methylpiperazin-1-yl)carbonyl]-1 H-benzimidazol-2-yl}quinolin-2(1H)-one | 403.3 |
| 77 | 4-amino-3-[5-(1-oxidothiomorpholin-4-yl)-1H-benzimidazol-2-yl]quinolin-2(1H)-one | 394.5 |
| 78 | 3-{5-[(4-acetylpiperazin-1-yl)carbonyl]-1H-benzimidazol-2-yl}-4-aminoquinolin-2(1H)-one | 431.3 |
| 79 | 4-amino-3-(5-{[(3R)-3-(dimethylamino)pyrrolidin-1-yl]carbonyl}-1H-benzimidazol-2-yl)quinolin-2(1H)-one | 417.4 |
| 80 | 4-amino-3-(5-{[(3S)-3-(dimethylamino)pyrrolidin-1-yl]carbonyl}-1H-benzimidazol-2-yl)quinolin-2(1H)-one | 417.4 |
| 81 | 4-amino-3-(5-{[4-(dimethylamino)piperidin-1-yl]carbonyl}-1H-benzimidazol-2-yl)quinolin-2(1H)-one | 431.4 |
| 82 | methyl 2-(4-amino-5-fluoro-2-oxo-1,2-dihydroquinolin-3-yl)-1H-benzimidazole-6-carboxylate | 353.2 |
| 83 | 4-amino-3-[5-(1,3'-bipyrrolidin-1'-yl)-1H-benzimidazol-2-yl]quinolin-2(1H)-one | 415.5 |
| 84 | 4-amino-3-[5-(pyridin-3-yloxy)-1H-benzimidazol-2-yl]quinolin-2(1H)-one | 370.2 |
| 85 | 4-amino-5,6-bis(methyloxy)-3-[5-(4-methylpiperazin-1-yl)-1H-benzimidazol-2-yl]quinolin-2(1H)-one | 435.5 |
| 86 | 2-(4-amino-2-oxo-1,2-dihydroquinolin-3-yl)-N-[2-(dimethylamino)ethyl]-N-methyl-1H-benzimidazole-5-carboxamide | 405.3 |
| 87 | 2-(4-amino-2-oxo-1,2-dihydroquinolin-3-yl)-N-methyl-N-(1-methylpyrrolidin-3-yl)-1H-benzimidazole-5-carboxamide | 417.2 |
| 88 | 4-amino-3-{5-[(5-methyl-2,5-diazabicyclo[2.2.1]hept-2-yl)carbonyl]-1H-benzimidazol-2-yl}quinolin-2(1H)-one | 415.2 |
| 89 | 4-amino-3-{5-[(4-cyclohexylpiperazin-1-yl)carbonyl]-1H-benzimidazol-2-yl}quinolin-2(1H)-one | 471.6 |
| 90 | 4-amino-3-{5-[(2-piperidin-1-ylethyl)amino]-1H-benzimidazol-2-yl}quinolin-2(11H)-one | 403.2 |
| 91 | ethyl 4-{[2-(4-amino-2-oxo-1,2-dihydroquinolin-3-yl)-1H-benzimidazol-5-yl]amino}piperidine-1-carboxylate | 447.3 |
| 92 | 4-amino-3-[5-({(5R)-5-[(methyloxy)methyl]pyrrolidin-3-yl}amino)-1H-benzimidazol-2-yl]quinolin-2(1H)-one | 405.2 |
| 93 | 4-amino-3-{5-[(pyridin-2-ylmethyl)amino]-1H-benzimidazol-2-yl}quinolin-2(1H)-one | 383.3 |
| 94 | 4-amino-3-[5-(piperidin-3-ylamino)-1H-benzimidazol-2-yl]quinolin-2(1H)-one | 375.2 |
| 95 | 4-amino-5-fluoro-3-{5-[(pyridin-2-ylmethyl)amino]-1H-benzimidazol-2-yl}quinolin-2(1H)-one | 401.3 |
| 96 | ethyl 4-{[2-(4-amino-5-fluoro-2-oxo-1,2-dihydroquinolin-3-yl)-1H-benzimidazol-5-yl]amino}piperidine-1-carboxylate | 465.5 |
| 97 | 4-amino-5-fluoro-3-[5-(piperidin-3-ylamino)-1H-benzimidazol-2-yl]quinolin-2(1H)-one | 393.3 |
| 98 | 4-amino-3-(1H-benzimidazol-2-yl)-6-bromoquinolin-2(1H)-one | 357.1 |
| 99 | 4-amino-3-(1H-benzimidazol-2-yl)-7-bromoquinolin-2(1H)-one | 357.1 |
| 100 | 4-amino-3-(5-bromo-1H-benzimidazol-2-yl)quinolin-2(1H)-one | 357.1 |
| 101 | N,N-dimethyl-2-(2-oxo-1,2-dihydroquinolin-3-yl)-1 H-benzimidazole-5-carboxamide | 333.1 |
| 102 | 4-amino-3-(5-thien-2-yl-1H-benzimidazol-2-yl)quinolin-2(1H)-one | 359.2 |
| 103 | 2-(4-amino-2-oxo-1,2-dihydroquinolin-3-yl)-N,N-dimethyl-1H-benzimidazole-5-sulfonamide | 384.1 |
| 104 | 4-amino-6-iodo-3-[5-(4-methylpiperazin-1-yl)-1H-benzimidazol-2-yl]quinolin-2(1H)-one | 501.1 |
| 105 | 4-amino-3-(5-{2-[(dimethylamino)methyl]-morpholin-4-yl}-1H-benzimidazol-2-yl)quinolin-2(1H)-one | 419.2 |
| 106 | 4-[(3R)-1-azabicyclo[2.2.2]oct-3-ylamino]-3-(1H-benzimidazol-2-yl)-7-chloro-6-iodoquinolin-2(1H)-one | 547 |
| 107 | 4-[(3R)-1-azabicyclo[2.2.2]oct-3-ylamino]-3-(1H-benzimidazol-2-yl)-6-nitroquinolin-2(1H)-one | 431 |
| 108 | 4-[(3R)-1-azabicyclo[2.2.2]oct-3-ylamino]-3-(1H-benzimidazol-2-yl)-6-methylquinolin-2(1H)-one | 401 |
| 109 | 4-[(3R)-1-azabicyclo[2.2.2]oct-3-ylamino]-3-(1H-benzimidazol-2-yl)-6,7-difluoroquinolin-2(1H)-one | 422 |
| 110 | 4-[(3S)-1-azabicyclo[2.2.2]oct-3-ylamino]-3-(1H-benzimidazol-2-yl)-7-chloroquinolin-2(1H)-one | 421 |
| 111 | 4-[(3R)-1-azabicyclo[2.2.2]oct-3-ylamino]-3-(1H-benzimidazol-2-yl)-6-bromoquinolin-2(1H)-one | 465 |
| 112 | 4-[(3R)-1-azabicyclo[2.2.2]oct-3-ylamino]-3-(1H-benzimidazol-2-yl)-2-oxo-1,2-dihydroquinoline-6-carbonitrile | 411 |
| 113 | 4-[(3R)-1-azabicyclo[2.2.2]oct-3-ylamino]-3-(1H-benzimidazol-2-yl)-6-fluoroquinolin-2(1H)-one | 404 |
| 114 | 4-[(3S)-1-azabicyclo[2.2.2]oct-3-ylamino]-3-(1H-benzimidazol-2-yl)-6,7-bis(methyloxy)quinolin-2(1H)-one | 447 |
| 115 | 4-[(3R)-1-azabicyclo[2.2.2]oct-3-ylamino]-3-(1H-benzimidazol-2-yl)-6,7-dichloroquinolin-2(1H)-one | 455 |
| 116 | 1-[4-[(3S)-1-azabicyclo[2.2.2]oct-3-ylamino]-3-(1H-benzimidazol-2-yl)-6-fluoro-2-oxo-1,2-dihydroquinolin-7-yl]piperidine-4-carboxamide | 531 |
| 117 | 4-[(3S)-1-azabicyclo[2.2.2]oct-3-ylamino]-3-(1H-benzimidazol-2-yl)-6-fluoro-7-[(3-hydroxypropyl)amino]quinolin-2(1H)-one | 478 |
| 118 | 4-[(3S)-1-azabicyclo[2.2.2]oct-3-ylamino]-3-(1H-benzimidazol-2-yl)-7-(dimethylamino)-6-fluoroquinolin-2(1H)-one | 448 |
| 119 | 4-[(3R)-1-azabicyclo[2.2.2]oct-3-ylamino]-3-(1H-benzimidazol-2-yl)-5-fluoroquinolin-2(1H)-one | 404 |
| 120 | 4-[(3R)-1-azabicyclo[2.2.2]oct-3-ylamino]-3-(1H-benzimidazol-2-yl)-6-(4-nitrophenyl)quinolin-2(1H)-one | 508 |
| 121 | 4-[(3S)-1-azabicyclo[2.2.2]oct-3-ylamino]-3-(1H-benzimidazol-2-yl)-7-{[2-(dimethylamino)ethyl]amino}-6-fluoroquinolin-2(1H)-one | 491 |
| 122 | 4-[(3S)-1-azabicyclo[2.2.2]oct-3-ylamino]-3-(1H-benzimidazol-2-yl)-6-fluoro-7-(1H-imidazol-1-yl)quinolin-2(1H)-one | 471 |
| 123 | 4-[(3R)-1-azabicyclo[2.2.2]oct-3-ylamino]-3-(1H-benzimidazol-2-yl)-6-[4-(methyloxy)phenyl]quinolin-2(1H)-one | 493 |
| 124 | 4-[(3S)-1-azabicyclo[2.2.2]oct-3-ylamino]-3-(1H-benzimidazol-2-yl)-6-fluoro-7-morpholin-4-ylquinolin-2(1H)-one | 490 |
| 125 | 4-[(3R)-1-azabicyclo[2.2.2]oct-3-ylamino]-6,7-difluoro-3-(3H-imidazo[4,5-b]pyridin-2-yl)quinolin-2(1H)-one | 423 |
| 126 | 4-[(3R)-1-azabicyclo[2.2.2]oct-3-ylamino]-3-(1H-benzimidazol-2-yl)-6-(3-nitrophenyl)quinolin-2(1H)-one | 508 |
| 127 | 1-[4-[(3S)-1-azabicyclo[2.2.2]oct-3-ylamino]-3-(1H-benzimidazol-2-yl)-6-fluoro-2-oxo-1,2-dihydroquinolin-7-yl]piperidine-3-carboxamide | 531 |
| 128 | 4-[(3S)-1-azabicyclo[2.2.2]oct-3-ylamino]-3-(1H-benzimidazol-2-yl)-5-methylquinolin-2(1H)-one | 401 |
| 129 | 6-(3-acetylphenyl)-4-[(3R)-1-azabicyclo[2.2.2]oct-3-ylamino]-3-(3H-imidazo[4,5-b]pyridin-2-yl)quinolin-2(1H)-one | 506 |
| 130 | 4-[(3S)-1-azabicyclo[2.2.2]oct-3-ylamino]-3-(1H-benzimidazol-2-yl)-5-chloroquinolin-2(1H)-one | 421 |
| 131 | 4-[(3R)-1-azabicyclo[2.2.2]oct-3-ylamino]-6-fluoro-3-(3H-imidazo[4,5-b]pyridin-2-yl)-7-morpholin-4-ylquinolin-2(1H)-one | 491 |
| 132 | 4-[(3S)-1-azabicyclo[2.2.2]oct-3-ylamino]-3-(1H-benzimidazol-2-yl)-7-(cyclopropylamino)-6-fluoroquinolin-2(1H)-one | 460 |
| 133 | N-{3-[4-[(3R)-1-azabicyclo[2.2.2]oct-3-ylamino]-3-(3H-imidazo[4,5-b]pyridin-2-yl)-2-oxo-1,2-dihydroquinolin-6-yl]phenyl}acetamide | 521 |
| 134 | 4-[(3S)-1-azabicyclo[2.2.2]oct-3-ylamino]-3-(1H-benzimidazol-2-yl)-6-fluoro-7-(4-methylpiperazin-1-yl)quinolin-2(1H)-one | 503 |
| 135 | 4-[(3R)-1-azabicyclo[2.2.2]oct-3-ylamino]-6-fluoro-7-(1H-imidazol-1-yl)-3-(3H-imidazo[4,5-b]pyridin-2-yl)quinolin-2(1H)-one | 472 |
| 136 | 4-[(3S)-1-azabicyclo[2.2.2]oct-3-ylamino]-3-(1H-benzimidazol-2-yl)-6-fluoro-7-[(2-pyridin-2-ylethyl)amino]quinolin-2(1H)-one | 525 |
| 137 | 4-[(3S)-1-azabicyclo[2.2.2]oct-3-ylamino]-3-(1H-benzimidazol-2-yl)-6-fluoro-7-piperidin-1-ylquinolin-2(1H)-one | 488 |
| 138 | 6-chloro-3-(3H-imidazo[4,5-b]pyridin-2-yl)quinolin-2(1H)-one | 298 |
| 139 | ethyl 1-[4-[(35)-1-azabicyclo[2.2.2]oct-3-ylamino]-3-(1H-benzimidazol-2-yl)-6-fluoro-2-oxo-1,2-dihydroquinolin-7-yl]piperidine-4-carboxylate | 560 |
| 140 | 4-[(3R)-1-azabicyclo[2.2.2]oct-3-ylamino]-3-(1H-benzimidazol-2-yl)-6-(1-benzothien-2-yl)quinolin-2(1H)-one | 519 |
| 141 | 4-[(3S)-1-azabicyclo[2.2.2]oct-3-ylamino]-3-(1H-benzimidazol-2-yl)-6-fluoro-7-pyrrolidin-1-ylquinolin-2(1H)-one | 474 |
| 142 | 4-[(3R)-1-azabicyclo[2.2.2]oct-3-ylamino]-3-(3H-imidazo[4,5-b]pyridin-2-yl)-6-[2-(trifluoromethyl)phenyl]quinolin-2(1H)-one | 532 |
| 143 | 4-[(3R)-1-azabicyclo[2.2.2]oct-3-ylamino]-3-(3H-imidazo[4,5-b]pyridin-2-yl)-6-[2-(methyloxy)phenyl]quinolin-2(1H)-one | 494 |
| 144 | ethyl 1-[4-[(3S)-1-azabicyclo[2.2.2]oct-3-ylamino]-3-(1H-benzimidazol-2-yl)-6-fluoro-2-oxo-1,2-dihydroquinolin-7-yl]piperidine-3-carboxylate | 560 |
| 145 | 4-[(3R)-1-azabicyclo[2.2.2]oct-3-ylamino]-3-(1H-benzimidazol-2-yl)-6-(4-ethylphenyl)quinolin-2(1H)-one | 491 |
| 146 | 4-[(3S)-1-azabicyclo[2.2.2]oct-3-ylamino]-3-(1H-benzimidazol-2-yl)-6-fluoro-7-[(2-methylpropyl)amino]quinolin-2(1H)-one | 476 |
| 147 | 4-[(3R)-1-azabicyclo[2.2.2]oct-3-ylamino]-3-(1H-benzimidazol-2-yl)-5-methylquinolin-2(1H)-one | 401 |
| 148 | 4-[(3R)-1-azabicyclo[2.2.2]oct-3-ylamino]-6-(2,4-dichlorophenyl)-3-(3H-imidazo[4,5-b]pyridin-2-yl)quinolin-2(1H)-one | 532 |
| 149 | 4-[(3R)-1-azabicyclo[2.2.2]oct-3-ylamino]-3-(1H-benzimidazol-2-yl)-6-[3-(trifluoromethyl)phenyl]quinolin-2(1H)-one | 531 |
| 150 | 3-(1H-benzimidazol-2-yl)-4-(dimethylamino)quinolin-2(1H)-one | 305 |
| 151 | 4-hydroxy-3-(1H-imidazo[4,5-f]quinolin-2-yl)quinolin-2(1H)-one | 329 |
| 152 | 4-hydroxy-3-(1H-imidazo[4,5-b]pyridin-2-yl)quinolin-2(1H)-one | 279 |
| 153 | 4-[4-[(3R)-1-azabicyclo[2.2.2]oct-3-ylamino]-3-(1H-benzimidazol-2-yl)-5-fluoro-2-oxo-1,2-dihydroquinolin-6-yl]benzoic acid | 525 |
| 154 | 4-[4-[(3R)-1-azabicyclo[2.2.2]oct-3-ylamino]-3-(1H-benzimidazol-2-yl)-5-fluoro-2-oxo-1,2-dihydroquinolin-6-yl]benzamide | 524 |
| 155 | N-{3-[4-[(3R)-1-azabicyclo[2.2.2]oct-3-ylamino]-3-(1H-benzimidazol-2-yl)-5-fluoro-2-oxo-1,2-dihydroquinolin-6-yl]phenyl}acetamide | 538 |
| 156 | 3-[4-[(3R)-1-azabicyclo[2.2.2]oct-3-ylamino]-3-(1H-benzimidazol-2-yl)-5-fluoro-2-oxo-1,2-dihydroquinolin-6-yl]benzoic acid | 525 |
| 157 | 4-[4-[(3R)-1-azabicyclo[2.2.2]oct-3-ylamino]-3-(1H-benzimidazol-2-yl)-7-fluoro-2-oxo-1,2-dihydroquinolin-6-yl]benzoic acid | 525 |
| 158 | N-{3-[4-[(3R)-1-azabicyclo[2.2.2]oct-3-ylamino]-3-(1H-benzimidazol-2-yl)-7-fluoro-2-oxo-1,2-dihydroquinolin-6-yl]phenyl}acetamide | 538 |
| 159 | 4-[(3R)-1-azabicyclo[2.2.2]oct-3-ylamino]-3-(1H-benzimidazol-2-yl)-7-chloro-6-(2-methylphenyl)quinolin-2(1H)-one | 511 |
| 160 | 4-[(3R)-1-azabicyclo[2.2.2]oct-3-ylamino]-3-(1H-benzimidazol-2-yl)-2-oxo-1,2-dihydroquinoline-7-carbonitrile | 411 |
| 161 | 4-[(3R)-1-azabicyclo[2.2.2]oct-3-ylamino]-3-(1H-benzimidazol-2-yl)-7-(methyloxy)quinolin-2(1 H)-one | 417 |
| 162 | 4-[4-[(3R)-1-azabicyclo[2.2.2]oct-3-ylamino]-3-(1H-benzimidazol-2-yl)-2-oxo-1,2-dihydroquinolin-7-yl]benzamide | 506 |
| 163 | 4-[(3R)-1-azabicyclo[2.2.2]oct-3-ylamino]-3-(1H-benzimidazol-2-yl)-6-fluoro-7-(methyloxy)quinolin-2(1H)-one | 434 |
| 164 | 4-[(3R)-1-azabicyclo[2.2.2]oct-3-ylamino]-3-(1H-benzimidazol-2-yl)-6-chloro-7-(dimethylamino)quinolin-2(1H)-one | 464 |
| 165 | 4-[(3R)-1-azabicyclo[2.2.2]oct-3-ylamino]-3-(1H-benzimidazol-2-yl)-7-(dimethylamino)-6-iodoquinolin-2(1H)-one | 555 |
| 166 | 3-[4-[(3R)-1-azabicyclo[2.2.2]oct-3-ylamino]-3-(1H-benzimidazol-2-yl)-7-(1H-imidazol-1-yl)-2-oxo-1,2-dihydroquinolin-6-yl]benzoic acid | 573 |
| 167 | 4-[4-[(3R)-1-azabicyclo[2.2.2]oct-3-ylamino]-3-(1H-benzimidazol-2-yl)-2-oxo-7-piperidin-1-yl-1,2-dihydroquinolin-6-yl]benzoic acid | 590 |
| 168 | 4-[(3R)-1-azabicyclo[2.2.2]oct-3-ylamino]-3-(1H-benzimidazol-2-yl)-7-(methyloxy)-6-[4-(methylsulfonyl)phenyl]quinolin-2(1H)-one | 571 |
| 169 | 4-[(3R)-1-azabicyclo[2.2.2]oct-3-ylamino]-3-(1H-benzimidazol-2-yl)-8-methylquinolin-2(1H)-one | 401 |
| 170 | 4-[(3S)-1-azabicyclo[2.2.2]oct-3-ylamino]-3-(1H-benzimidazol-2-yl)-6,7-difluoroquinolin-2(1H)-one | 422 |
| 171 | 3-(1H-benzimidazol-2-yl)-6-methyl-4-(piperidin-3-ylamino)quinolin-2(1H)-one | 374 |
| 172 | 4-[(3S)-1-azabicyclo[2.2.2]oct-3-ylamino]-3-(1H-benzimidazol-2-yl)-6-[2-(methyloxy)phenyl]quinolin-2(1H)-one | 493 |
| 173 | 4-[(3S)-1-azabicyclo[2.2.2]oct-3-ylamino]-3-(1H-benzimidazol-2-yl)-6-[3-(methyloxy)phenyl]quinolin-2(1H)-one | 493 |
| 174 | 3-(1H-benzimidazol-2-yl)-6,7-difluoro-4-(piperidin-4-ylamino)quinolin-2(1H)-one | 396 |
| 175 | 3-(1H-benzimidazol-2-yl)-6,7-difluoro-4-(pyrrolidin-3-ylamino)quinolin-2(1H)-one | 382 |
| 176 | 3-(1H-benzimidazol-2-yl)-6-chloro-4-[(3-morpholin-4-ylpropyl)amino]quinolin-2(1H)-one | 439 |
| 177 | 6-chloro-3-(5-morpholin-4-yl-1H-benzimidazol-2-yl)-4-(piperidin-4-ylamino)quinolin-2(1H)-one | 480 |
| 178 | 6-chloro-3-(5-morpholin-4-yl-1H-benzimidazol-2-yl)-4-[(piperidin-2-ylmethyl)amino]quinolin-2(1H)-one | 494 |
| 179 | 4-[(3S)-1-azabicyclo[2.2.2]oct-3-ylamino]-6-chloro-3-(5-morpholin-4-yl-1H-benzimidazol-2-yl)quinolin-2(1H)-one | 506 |
| 180 | 6-chloro-3-(5-morpholin-4-yl-1H-benzimidazol-2-yl)-4-(piperidin-3-ylamino)quinolin-2(1H)-one | 480 |
| 181 | 6-chloro-4-{[2-(dimethylamino)ethyl]amino}-3-(5-morpholin-4-yl-1H-benzimidazol-2-yl)quinolin-2(1H)-one | 468 |
| 182 | 4-[(3R)-1-azabicyclo[2.2.2]oct-3-ylamino]-6-chloro-3-(5-morpholin-4-yl-1H-benzimidazol-2-yl)quinolin-2(1H)-one | 506 |
| 183 | 6-chloro-3-(5-morpholin-4-yl-1H-benzimidazol-2-yl)-4-[(piperidin-3-ylmethyl)amino]quinolin-2(1H)-one | 494 |
| 184 | 6-chloro-3-(5-morpholin-4-yl-1H-benzimidazol-2-yl)-4-[(piperidin-4-ylmethyl)amino]quinolin-2(1H)-one | 494 |
| 185 | 4-{[(1R,2R)-2-aminocyclohexyl]amino}-6-chloro-3-(5-morpholin-4-yl-1H-benzimidazol-2-yl)quinolin-2(1H)-one | 494 |
| 186 | 4-[(4-aminocyclohexyl)amino]-6-chloro-3-(5-morpholin-4-yl-1H-benzimidazol-2-yl)quinolin-2(1H)-one | 494 |
| 187 | 4- {[(2S)-2-amino-3-methylbutyl]amino}-6-chloro-3-(5-morpholin-4-yl-1H-benzimidazol-2-yl)quinolin-2(1 H)-one | 482 |
| 188 | 4-({[4-(aminomethyl)phenyl]methyl}amino)-6-chloro-3-(5-morpholin-4-yl-1H-benzimidazol-2-yl)quinolin-2(1H)-one | 516 |
| 189 | 6-chloro-3-(5-morpholin-4-yl-1H-benzimidazol-2-yl)-4-[(pyrrolidin-2-ylmethyl)amino]quinolin-2(1H)-one | 480 |
| 190 | 4- {[(1R)-1-(aminomethyl)propyl]amino}-6-chloro-3-(5-morpholin-4-yl-1H-benzimidazol-2-yl)quinolin-2(1 H)-one | 468 |
| 191 | 4- {[(1S)-2-amino-1-(phenylmethyl)ethyl]amino}-6-chloro-3 -(5-morpholin-4-yl-1H-benzimidazol-2-yl)quinolin-2(1 H)-one | 530 |
| 192 | 6-chloro-4-{[3-(4-methylpiperazin-1-yl)propyl]amino}-3-(5-morpholin-4-yl-1H-benzimidazol-2-yl)quinolin-2(1H)-one | 537 |
| 193 | 6-chloro-3-(5-morpholin-4-yl-1H-benzimidazol-2-yl)-4- {[1-(phenylmethyl)piperidin-4-yl]amino}quinolin-2(1H)-one | 570 |
| 194 | 6-chloro-3-(5-morpholin-4-yl-1H-benzimidazol-2-yl)-4-[(3-morpholin-4-ylpropyl)amino]quinolin-2(1H)-one | 524 |
| 195 | 6-chloro-3-(5-morpholin-4-yl-1H-benzimidazol-2-yl)-4- [(2-piperidin-1-ylethyl)amino]quinolin-2(1H)-one | 508 |
| 196 | 6-chloro-3-(5-morpholin-4-yl-1H-benzimidazol-2-yl)-4-[(pyridin-3-ylmethyl)amino]quinolin-2(1H)-one | 488 |
| 197 | 6-chloro-4- {[3-(1H-imidazol-1-yl)propyl]amino}-3-(5-morpholin-4-yl-1H-benzimidazol-2-yl)quinolin-2(1H)-one | 505 |
| 198 | 6-chloro-3-(5-morpholin-4-yl-1H-benzimidazol-2-yl)-4-[(pyridin-4-ylmethyl)amino]quinolin-2(1H)-one | 488 |
| 199 | 6-chloro-4-{[2-(methylamino)ethyl]amino}-3-(5-morpholin-4-yl-1H-benzimidazol-2-yl)quinolin-2(1H)-one | 454 |
| 200 | 6-chloro-4- {[(2-methyl-1-piperidin-4-yl-1H-benzimidazol-5-yl)methyl] amino}-3-(5-morpholin-4-yl-1H-benzimidazol-2-yl)quinolin-2(1H)-one | 624 |
| 201 | 6-chloro-3-(5-morpholin-4-yl-1H-benzimidazol-2-yl)-4-[(2-pyrrolidin-1-ylethyl)amino]quinolin-2(1H)-one | 494 |
| 202 | 6-chloro-3-(5-morpholin-4-yl-1H-benzimidazol-2-yl)-4-(pyrrolidin-3-ylamino)quinolin-2(1H)-one | 466 |
| 203 | 4-{[(1R,2R)-2-aminocyclohexyl]amino}-6-chloro-3-[5-(4-methylpiperazin-1-yl)-1H-benzimidazol-2-yl]quinolin-2(1H)-one | 507 |
| 204 | 4-[(4-aminocyclohexyl)amino]-6-chloro-3-[5-(4-methylpiperazin-1-yl)-1H-benzimidazol-2-yl]quinolin-2(1H)-one | 507 |
| 205 | 4-({[4-(aminomethyl)phenyl]methyl}amino)-6-chloro-3-[5-(4-methylpiperazin-1-yl)-1H-benzimidazol-2-yl]quinolin-2(1H)-one | 529 |
| 206 | 6-chloro-4-{[2-(methylamino)ethyl]amino}-3-[5-(4-methylpiperazin-1-yl)-1H-benzimidazol-2-yl]quinolin-2(1H)-one | 467 |
| 207 | 6-chloro-3-[5-(4-methylpiperazin-1-yl)-1H-benzimidazol-2-yl]-4- {[3-(4-methylpiperazin-1-yl)propyl]amino}quinolin-2(1H)-one | 550 |
| 208 | 6-chloro-3-[5-(4-methylpiperazin-1-yl)-1H-benzimidazol-2-yl]-4-{[1-(phenylmethyl)piperidin-4-yl]ammo}quinolm-2(1H)-one | 583 |
| 209 | 6-chloro-3-[5-(4-methylpiperazin-1-yl)-1H-benzimidazol-2-yl]-4-[(2-pyrrolidin-1-ylethyl)amino]quinolin-2(1H)-one | 507 |
| 210 | 6-chloro-3-[5-(4-methylpiperazin-1-yl)-1H-benzimidazol-2-yl]-4-(pyrrolidin-3-ylamino)quinolin-2(1H)-one | 479 |
| 211 | 6-chloro-3-[5-(4-methylpiperazin-1-yl)-1H-benzimidazol-2-yl]-4-(piperidin-4-ylamino)quinolin-2(1H)-one | 493 |
| 212 | 6-chloro-3-(5-morpholin-4-yl-1H-benzimidazol-2-yl)-4- [(2-piperidin-2-ylethyl)amino]quinolin-2(1H)-one | 508 |
| 213 | 4-[(3S)-1-azabicyclo[2.2.2]oct-3-ylamino]-7-chloro-3-(5-morpholin-4-yl-1H-benzimidazol-2-yl)quinolin-2(1H)-one | 506 |
| 214 | 7-chloro-3-(5-morpholin-4-yl-1H-benzimidazol-2-yl)-4-(piperidin-3-ylamino)quinolin-2(1H)-one | 480 |
| 215 | 6-chloro-3-[5-(4-methylpiperazin-1-yl)-1H-benzimidazol-2-yl]-4-[(piperidin-2-ylmethyl)amino]quinolin-2(1H)-one | 507 |
| 216 | 6-chloro-3-[5-(4-methylpiperazin-1-yl)-1H-benzimidazol-2-yl]-4-{[(2S)-pyrrolidin-2-ylmethyl]amino}quinolin-2(1H)-one | 493 |
| 217 | 6-chloro-3-[5-(4-methylpiperazin-1-yl)-1H-benzimidazol-2-yl]-4-{[(2R)-pyrrolidin-2-ylmethyl]amino}quinolin-2(1H)-one | 493 |
| 218 | 6-chloro-4-({[(2S)-1-ethylpyrrolidin-2-yl]methyl}amino)-3-[5-(4-methylpiperazin-1-yl)-1 H-benzimidazol-2-yl]quinolin-2(1H)-one | 521 |
| 219 | 6-chloro-4-({[(2R)-1-ethylpyrrolidin-2-yl]methyl}amino)-3-[5-(4-methylpiperazin-1-yl)-1 H-benzimidazol-2-yl]quinolin-2(1H)-one | 521 |
| 220 | 4-[(3S)-1-azabicyclo[2.2.2]oct-3-ylamino]-3-(1H-benzimidazol-2-yl)-6-[4-(methyloxy)phenyl]quinolin-2(1H)-one | 493 |
| 221 | 6-(3-aminophenyl)-4-[(3S)-1-azabicyclo[2.2.2]oct-3-ylamino]-3-(1H-benzimidazol-2-yl)quinolin-2(1H)-one | 478 |

**Table 8. Additional Example Compounds**

| **Example** | **Name** | **LC/MS m/z (MH⁺)** |
|---|---|---|
| 222 | 4-amino-3-(1H-benzimidazol-2-yl)quinolin-2(1H)-one | 277.3 |
| 223 | 4-amino-3-(1H-benzimidazol-2-yl)-6,7-dimethoxyquinolin-2(1H)-one | 337.3 |
| 224 | 3-(1H-benzimidazol-2-yl)-4-(dimethylamino)-1-methylquinolin-2(1H)-one | 319.4 |
| 225 | 3-(1H-benzimidazol-2-yl)-4-{[2-(dimethylamino)ethyl]amino}-1-methylquinolin-2(1H)-one | 362.4 |
| 226 | 4-amino-3-(1H-benzimidazol-2-yl)-1-methylquinolin-2(1H)-one | 291.3 |
| 227 | 4-amino-3-(6-methyl-1H-benzimidazol-2-yl)quinolin-2(1H)-one | 291.3 |
| 228 | 3-(1H-benzimidazol-2-yl)-4-{[3-(1H-imidazol-1-yl)propyl] amino}quinolin-2(1H)-one | 385.4 |
| 229 | 3-(1H-benzimidazol-2-yl)-4-[(pyridin-3-ylmethyl)amino]quinolin-2(1H)-one | 368.4 |
| 230 | 4-amino-3-(1H-benzimidazol-2-yl)-5-fluoroquinolin-2(1H)-one | 295.3 |
| 231 | 3-(1H-benzimidazol-2-yl)-4-pyrrolidin-1-ylquinolin-2(1H)-one | 331.4 |
| 232 | 3-(1H-benzimidazol-2-yl)-4-[(pyridin-4-ylmethyl)amino]quinolin-2(1H)-one | 368.4 |
| 233 | 3-(1H-benzimidazol-2-yl)-4-{[2-(1-methylpyrrolidin-2-yl)ethyl]amino}quinolin-2(1H)-one | 388.5 |
| 234 | 4-amino-3-(1H-benzimidazol-2-yl)-7-methylquinolin-2(1H)-one | 291.3 |
| 235 | 4-amino-3-(1H-benzimidazol-2-yl)-7-chloroquinolin-2(1H)-one | 311.7 |
| 236 | 4-amino-3-(1H-benzimidazol-2-yl)-6-chloroquinolin-2(1H)-one | 311.7 |
| 237 | 4-amino-3-[6-(3-aminopyrrolidin-1-yl)-1H-benzimidazol-2-yl]quinolin-2(1H)-one | 361.4 |
| 238 | 3-(1H-benzimidazol-2-yl)-4-(diethylamino)quinolin-2(1H)-one | 333.4 |
| 239 | 3-(1H-benzimidazol-2-yl)-4-(1,2-dimethylhydrazino)quinolin-2(1H)-one | 320.4 |
| 240 | 4-amino-3-[5-(trifluoromethyl)-1H-benzimidazol-2-yl]quinolin-2(1H)-one | 345.3 |
| 241 | 4-amino-3-(5,6-dichloro-1H-benzimidazol-2-yl)quinolin-2(1H)-one | 346.2 |
| 242 | 4-(3-aminopyrrolidin-1-yl)-3-(5-morpholin-4-yl-1H-benzimidazol-2-yl)quinolin-2(1H)-one | 431.5 |
| 243 | 4-amino-5-fluoro-3-(5-methyl-1H-benzimidazol-2-yl)quinolin-2(1H)-one | 309.3 |
| 244 | 4-amino-3-(1H-benzimidazol-2-yl)-6-nitroquinolin-2(1H)-one | 322.3 |
| 245 | 4-amino-3-(4-methyl-1H-benzimidazol-2-yl)quinolin-2(1H)-one | 291.3 |
| 246 | 4-amino-3-(6-ethoxy-1H-benzimidazol-2-yl)quinolin-2(1H)-one | 321.4 |
| 247 | 4-amino-3-(7-hydroxy-1H-benzimidazol-2-yl)quinolin-2(1H)-one | 293.3 |
| 248 | 4-amino-3-(6-tert-butyl-1H-benzimidazol-2-yl)quinolin-2(1H)-one | 333.4 |
| 249 | 2-(4-amino-2-oxo-1,2-dihydroquinolin-3-yl)-1H-benzimidazole-5-carbonitrile | 302.3 |
| 250 | 4-amino-3-(5,6-dimethyl-1H-benzimidazol-2-yl)quinolin-2(1H)-one | 305.4 |
| 251 | 4-amino-3-(4,5-dimethyl-1H-benzimidazol-2-yl)quinolin-2(1H)-one | 305.4 |
| 252 | 4-amino-6-chloro-3 -(5-methyl-1H-benzimidazol-2-yl)quinolin-2(1H)-one | 325.8 |
| 253 | 4-amino-3-(1H-benzimidazol-2-yl)-6,8-dichloroquinolin-2(1H)-one | 346.2 |
| 254 | 4-amino-3-(1H-benzimidazol-2-yl)-5-chloroquinolin-2(1H)-one | 311.7 |
| 255 | 2-(4-amino-2-oxo-1,2-dihydroquinolin-3-yl)-N,N-dimethyl-1H-benzimidazole-5-carboxamide | 348.4 |
| 256 | 4-amino-3-{5-[3-(dimethylamino)pyrrolidin-1-yl]-1H-benzimidazol-2-yl}quinolin-2(1H)-one | 389.5 |
| 257 | 4-amino-3-(6-methoxy-5-methyl-1H-benzimidazol-2-yl)quinolin-2(1H)-one | 321.4 |
| 258 | 2-(4-amino-2-oxo-1,2-dihydroquinolin-3-yl)-1H-benzimidazole-6-carboximidamide | 319.3 |
| 259 | 4-amino-7-(3-aminophenyl)-3-(1H-benzimidazol-2-yl)quinolin-2(1H)-one | 368.4 |
| 260 | 4-amino-3-(1H-benzimidazol-2-yl)-7-thien-2-ylquinolin-2(1H)-one | 359.4 |
| 261 | 4-amino-3-(5-thien-3-yl-1H-benzimidazol-2-yl)quinolin-2(1H)-one | 359.4 |
| 262 | 4-amino-3-(1H-benzimidazol-2-yl)-7-thien-3-ylquinolin-2(1H)-one | 359.4 |
| 263 | 4-{[(1S,2R)-2-aminocyclohexyl]amino}-3-(5-morpholin-4-yl-1H-benzimidazol-2-yl)quinolin-2(1H)-one | 459.6 |
| 264 | 4- {[(1R,2R)-2-aminocyclohexyl]amino}-3-(5-morpholin-4-yl-1H-benzimidazol-2-yl)quinolin-2(1H)-one | 459.6 |
| 265 | 4-{[(1S,2S)-2-aminocyclohexyl]amino}-3-(5-morpholin-4-yl-1H-benzimidazol-2-yl)quinolin-2(1H)-one | 459.6 |
| 266 | 4-amino-3-{5-[(2R,6S)-2,6-dimethylmorpholin-4-yl]-1H-benzimidazol-2-yl}quinolin-2(1H)-one | 390.5 |
| 267 | 3-(1H-benzimidazol-2-yl)-4-morpholin-4-ylquinolin-2(1H)-one | 347.4 |
| 268 | 3-(1H-benzimidazol-2-yl)-4-(piperidin-3-ylamino)quinolin-2(1H)-one | 360.4 |
| 269 | 4-(1-azabicyclo[2.2.2]oct-3-ylamino)-3-(5-chloro-1H-benzimidazol-2-yl)quinolin-2(1H)-one | 420.9 |
| 270 | 4-[(3R)-1-azabicyclo[2.2.2]oct-3-ylamino]-6-chloro-3-(5-methyl-1H-benzimidazol-2-yl)quinolin-2(1H)-one | 434.9 |
| 271 | 6-chloro-3-(5-methyl-1H-benzimidazol-2-yl)-4-(piperidin-3-ylamino)quinolin-2(1H)-one | 408.9 |
| 272 | 3-(1H-benzimidazol-2-yl)-4-[(2-hydroxyethyl)amino]quinolin-2(1H)-one | 321.4 |
| 273 | 3-(1H-benzimidazol-2-yl)-6-chloro-4-(piperidin-3-ylamino)quinolin-2(1H)-one | 394.9 |
| 274 | 3-(1H-benzimidazol-2-yl)-6-chloro-4-{[(1S)-1-cyclohexylethyl]amino}quinolin-2(1H)-one | 421.9 |
| 275 | 3-(1H-benzimidazol-2-yl)-6-chloro-4-[(piperidin-3-ylmethyl)amino]quinolin-2(1H)-one | 408.9 |
| 276 | 3-(1H-benzimidazol-2-yl)-6-chloro-4-(pyridin-4-ylamino)quinolin-2(1H)-one | 388.8 |
| 277 | 3-(1H-benzimidazol-2-yl)-6-chloro-4-[(piperidin-4-ylmethyl)amino]quinolin-2(1H)-one | 408.9 |
| 278 | 3-(1H-benzimidazol-2-yl)-6-chloro-4-[(2-morpholin-4-ylethyl)amino]quinolin-2(1H)-one | 424.9 |
| 279 | 3-(1 H-benzimidazol-2-yl)-6-chloro-4-(cyclohexylamino)quinolin-2(1H)-one | 393.9 |
| 280 | 3-(1H-benzimidazol-2-yl)-6-chloro-4-{[3-(1H-imidazol-1-yl)propyl] amino}quinolin-2(1H)-one | 419.9 |
| 281 | 3-(1H-benzimidazol-2-yl)-6-chloro-4-{[2-(dimethylamino)ethyl]amino}quinolin-2(1H)-one | 382.9 |
| 282 | 3-(1H-benzimidazol-2-yl)-6-chloro-4-[(cyclohexylmethyl)amino]quinolin-2(1H)-one | 407.9 |
| 283 | 3-(1H-benzimidazol-2-yl)-6-chloro-4-[(tetrahydrofuran-2-ylmethyl)amino]quinolin-2(1H)-one | 395.9 |
| 284 | 3-(1H-benzimidazol-2-yl)-6-chloro-4-[(pyridin-4-ylmethyl)amino]quinolin-2(1H)-one | 402.9 |
| 285 | 3-(1 H-benzimidazol-2-yl)-6,7-difluoro-4-(piperidin-3-ylamino)quinolin-2(1H)-one | 396.4 |
| 286 | 4-[(3S)-1-azabicyclo[2.2.2]oct-3-ylamino]-3-(1H-benzimidazol-2-yl)-6-bromoquinolin-2(1H)-one | 465.4 |
| 287 | 3-(1H-benzimidazol-2-yl)-6-fluoro-4-(piperidin-3-ylamino)quinolin-2(1H)-one | 378.4 |
| 288 | 4-[(3S)-1-azabicyclo[2.2.2]oct-3-ylamino]-3-(1H-benzimidazol-2-yl)-6-methylquinolin-2(1H)-one | 400.5 |
| 289 | 4-[(3S)-1-azabicyclo[2.2.2]oct-3-ylamino]-3-(1H-benzimidazol-2-yl)-6-fluoroquinolin-2(1H)-one | 404.5 |
| 290 | 4-amino-3-[6-(4-methylpiperazin-1-yl)-1H-benzimidazol-2-yl]-1-propylquinolin-2(1H)-one | 417.5 |
| 291 | 3-(1H-benzimidazol-2-yl)-6-chloro-4-{[(1-ethylpyrrolidin-2-yl)methyl]amino}quinolin-2(1H)-one | 422.9 |
| 292 | 3-(1H-benzimidazol-2-yl)-6-chloro-4-{[3-(2-oxopyrrolidin-1-yl)propyl] amino}quinolin-2(1H)-one | 436.9 |
| 293 | 3-(1H-benzimidazol-2-yl)-6-chloro-4-[(piperidin-2-ylmethyl)amino]quinolin-2(1H)-one | 408.9 |
| 294 | 3-(1H-benzimidazol-2-yl)-6-chloro-4-(4-methyl-1,4-diazepan-1-yl)quinolin-2(1 H)-one | 408.9 |
| 295 | 3-(1H-benzimidazol-2-yl)-6-chloro-4-[(pyridin-3-ylmethyl)amino]quinolin-2(1H)-one | 402.9 |
| 296 | 4-anilino-3-(1H-benzimidazol-2-yl)-6-chloroquinolin-2(1H)-one | 387.8 |
| 297 | 3-(1H-benzimidazol-2-yl)-6-chloro-4-{[(5-methylpyrazin-2-yl)methyl]amino}quinolin-2(1H)-one | 417.9 |
| 298 | 3-(1H-benzimidazol-2-yl)-6-chloro-4-(piperidin-4-ylamino)quinolin-2(1H)-one | 402.9 |
| 299 | 3-(1H-benzimidazol-2-yl)-6-chloro-4-{[2-(1-methylpyrrolidin-2-yl)ethyl]amino}quinolin-2(1H)-one | 422.9 |
| 300 | 3-(1H-benzimidazol-2-yl)-4-[(1H-benzimidazol-5-ylmethyl)amino]-6-chloroquinolin-2(1H)-one | 441.9 |
| 301 | 3-(1H-benzimidazol-2-yl)-6-chloro-4-(piperidin-4-ylamino)quinolin-2(1H)-one | 394.9 |
| 302 | 3-(1H-benzimidazol-2-yl)-6-chloro-4-[(4-hydroxycyclohexyl)amino]quinolin-2(1H)-one | 409.9 |
| 303 | 4-[(3S)-1-azabicyclo[2.2.2]oct-3-ylamino]-3-(1H-benzimidazol-2-yl)-5-fluoroquinolin-2(1H)-one | 404.5 |
| 304 | 3-(1 H-benzimidazol-2-yl)-6, 8-dimethyl-4-(piperidin-3-ylamino)quinolin-2(1H)-one | 388.5 |
| 305 | 3-(1H-benzimidazol-2-yl)-5-fluoro-4-(piperidin-3-ylamino)quinolin-2(1H)-one | 378.4 |
| 306 | 4-[(3R)-1-azabicyclo[2.2.2]oct-3-ylamino]-3-(1H-benzimidazol-2-yl)-6,8-dimethylquinolin-2(1H)-one | 414.5 |
| 307 | 4-[(3S)-1-azabicyclo[2.2.2]oct-3-ylamino]-3-(1H-benzimidazol-2-yl)-6,8-dimethylquinolin-2(1H)-one | 414.5 |
| 308 | 4-[(3R)-1-azabicyclo[2.2.2]oct-3-ylamino]-3-(1H-benzimidazol-2-yl)-7-chloroquinolin-2(1H)-one | 420.9 |
| 309 | 3-(1H-benzimidazol-2-yl)-6-chloro-4-[(2-piperidin-1-ylethyl)amino]quinolin-2(1H)-one | 422.9 |
| 310 | 4-({2-[(4-amino-5-nitropyridin-2-yl)amino]ethyl}amino)-3-(1H-benzimidazol-2-yl)-6-chloroquinolin-2(1H)-one | 491.9 |
| 311 | 3-(1H-benzimidazol-2-yl)-6-chloro-4-({2-[(5-nitropyridin-2-yl)amino] ethyl}amino)quinolin-2(1H)-one | 476.9 |
| 312 | 3-(1H-benzimidazol-2-yl)-4-[(1H-benzimidazol-2-ylmethyl)amino]-6-chloroquinolin-2(1H)-one | 441.9 |
| 313 | 3-(1H-benzimidazol-2-yl)-6-chloro-4-(2,5-diazabicyclo[2.2.1]hept-2-yl)quinolin-2(1H)-one | 392.9 |
| 314 | 3-(1H-benzimidazol-2-yl)-6-chloro-4-[(2-{[5-(trifluoromethyl)pyridin-2-yl]amino}ethyl)amino]quinolin-2(1H)-one | 499.9 |
| 315 | 4-[(3S)-1-azabicyclo[2.2.2]oct-3-ylamino]-3-(1H-benzimidazol-2-yl)-7-methylquinolin-2(1H)-one | 400.5 |
| 316 | 4-[(3R)-1-azabicyclo[2.2.2]oct-3-ylamino]-3-(1H-benzimidazol-2-yl)-7-methylquinolin-2(1H)-one | 400.5 |
| 317 | 3-(1H-benzimidazol-2-yl)-7-chloro-4-{[(2R)-pyrrolidin-2-ylmethyl]amino}quinolin-2(1H)-one | 394.9 |
| 318 | 3-(1H-benzimidazol-2-yl)-6-chloro-4-[(pyrrolidin-2-ylmethyl)amino]quinolin-2(1H)-one | 394.9 |
| 319 | 6-[(2- {[3-(1H-benzimidazol-2-yl)-6-chloro-2-oxo-1,2-dihydroquinolin-4-yl]amino}ethyl)amino]nicotinamide | 474.9 |
| 320 | 3-(1H-benzimidazol-2-yl)-6-chloro-4-(pyrrolidin-3-ylamino)quinolin-2(1H)-one | 380.8 |
| 321 | 4- {[(2R)-2-aminobutyl]amino}-3-(1H-benzimidazol-2-yl)-6-chloroquinolin-2(1H)-one | 382.9 |
| 322 | 4-{[(2S)-2-amino-3-phenylpropyl]amino}-3-(1H-benzimidazol-2-yl)-6-chloroquinolin-2(1H)-one | 444.9 |
| 323 | 4-[(4-aminocyclohexyl)amino]-3-(1H-benzimidazol-2-yl)-6-chloroquinolin-2(1H)-one | 408.9 |
| 324 | 4-[(3R)-1-azabicyclo[2.2.2]oct-3-ylamino]-3-(1H-benzimidazol-2-yl)-6-iodoquinolin-2(1H)-one | 512.4 |
| 325 | 4-[(3S)-1-azabicyclo[2.2.2]oct-3-ylamino]-3-(1H-benzimidazol-2-yl)-6-iodoquinolin-2(1H)-one | 512.4 |
| 326 | 3-(1 H-benzimidazol-2-yl)-6,7-dimethoxy-4-(piperidin-3-ylamino)quinolin-2(1H)-one | 420.5 |
| 327 | 4-[(3R)-1-azabicyclo[2.2.2]oct-3-ylamino]-3-(1H-benzimidazol-2-yl)-6,7-dimethoxyquinolin-2(1H)-one | 446.5 |
| 328 | 4-[(3S)-1-azabicyclo[2.2.2]oct-3-ylamino]-3-(1H-benzimidazol-2-yl)-6-nitroquinolin-2(1H)-one | 431.5 |
| 329 | 3-(1 H-benzimidazol-2-yl)-6-iodo-4-(piperidin-3-ylamino)quinolin-2(1H)-one | 486.3 |
| 330 | 4-[(3R)-1-azabicyclo[2.2.2]oct-3-ylamino]-3-(1H-benzimidazol-2-yl)-5-chloroquinolin-2(1H)-one | 420.9 |
| 331 | 3-(1H-benzimidazol-2-yl)-6-chloro-4-{[(1-piperidin-4-yl-1H-benzimidazol-6-yl)methyl]amino}quinolin-2(1H)-one | 525.0 |
| 332 | 3-(1H-benzimidazol-2-yl)-6-methyl-4-[(piperidin-3-ylmethyl)amino]quinolin-2(1H)-one | 388.5 |
| 333 | 3-(1H-benzimidazol-2-yl)-6-methyl-4-(piperidin-4-ylamino)quinolin-2(1H)-one | 374.5 |
| 334 | 3-(1H-benzimidazol-2-yl)-6-methyl-4-[(piperidin-4-ylmethyl)amino]quinolin-2(1H)-one | 388.5 |
| 335 | 3-(1H-benzimidazol-2-yl)-6-methyl-4-[(piperidin-2-ylmethyl)amino]quinolin-2(1H)-one | 388.5 |
| 336 | 4-{[4-(2-aminoethoxy)benzyl]amino}-3-(1H-benzimidazol-2-yl)-6-chloroquinolin-2(1H)-one | 460.9 |
| 337 | 4-{[2-(2-aminoethoxy)benzyl]amino}-3-(1H-benzimidazol-2-yl)-6-chloroquinolin-2(1H)-one | 460.9 |
| 338 | 4-(1-azabicyclo[2.2.2]oct-3-ylamino)-3-(5-hydroxy-1H-benzimidazol-2-yl)quinolin-2(1H)-one | 402.5 |
| 339 | 4-[(3S)-1-azabicyclo[2.2.2]oct-3-ylamino]-3-(1H-benzimidazol-2-yl)-2-oxo-1,2-dihydroquinoline-6-carbonitrile | 411.5 |
| 340 | 4-[(3R)-1-azabicyclo[2.2.2]oct-3-ylamino]-3-(1H-benzimidazol-2-yl)-6,7-dihydroxyquinolin-2(1H)-one | 418.5 |
| 341 | 4-[(3S)-1-azabicyclo[2.2.2]oct-3-ylamino]-3-(1H-benzimidazol-2-yl)-6,7-dihydroxyquinolin-2(1H)-one | 418.5 |
| 342 | 4-[(3R)-1-azabicyclo[2.2.2]oct-3-ylamino]-3-(1H-benzimidazol-2-yl)-2-oxo-1,2-dihydroquinoline-6-carboxylic acid | 430.5 |
| 343 | 4-[(3R)-1-azabicyclo[2.2.2]oct-3-ylamino]-3-(1H-benzimidazol-2-yl)-7-fluoroquinolin-2(1H)-one | 404.5 |
| 344 | 4-[(3S)-1-azabicyclo[2.2.2]oct-3-ylamino]-3-(1H-benzimidazol-2-yl)-7-fluoroquinolin-2(1H)-one | 404.5 |
| 345 | 2-(4-amino-2-oxo-1-propyl-1,2-dihydroquinolin-3-yl)-1H-benzimidazole-6-carbonitrile | 344.4 |
| 346 | tert-butyl 4-[4-[(3R)-1-azabicyclo[2.2.2]oct-3-ylamino]-3-(1H-benzimidazol-2-yl)-2-oxo-1,2-dihydroquinolin-6-yl]-3,6-dihydropyridine-1(2H)-carboxylate | 567.7 |
| 347 | tert-butyl 4-[4-[(3S)-1-azabicyclo[2.2.2] oct-3-ylamino]-3-(1H-benzimidazol-2-yl)-2-oxo-1,2-dihydroquinolin-6-yl]-3,6-dihydropyridine-1(2H)-carboxylate | 567.7 |
| 348 | 4-[(3R)-1-azabicyclo[2.2.2]oct-3-ylamino]-3-(1H-benzimidazol-2-yl)-6-(1,2,3,6-tetrahydropyridin-4-yl)quinolin-2(1H)-one | 467.6 |
| 349 | 4-[(3S)-1-azabicyclo[2.2.2]oct-3-ylamino]-3-(1H-benzimidazol-2-yl)-6-thien-2-ylquinolin-2(1H)-one | 468.6 |
| 350 | 4-[(3S)-1-azabicyclo[2.2.2]oct-3-ylamino]-3-(1H-benzimidazol-2-yl)-6-(1,2,3,6-tetrahydropyridin-4-yl)quinolin-2(1H)-one | 467.6 |
| 351 | 4-[(3S)-1-azabicyclo[2.2.2]oct-3-ylamino]-3-(1H-benzimidazol-2-yl)-6-(2,4-difluorophenyl)quinolin-2(1H)-one | 498.5 |
| 352 | tert-butyl 2-[4-[(3S)-1-azabicyclo[2.2.2] oct-3-ylamino]-3-(1H-benzimidazol-2-yl)-2-oxo-1,2-dihydroquinolin-6-yl]-1H-pyrrole-1-carboxylate | 551.7 |
| 353 | tert-butyl 2-[4-[(3R)-1-azabicyclo[2.2.2]oct-3-ylamino]-3-(1H-benzimidazol-2-yl)-2-oxo-1,2-dihydroquinolin-6-yl]-1H-pyrrole-1-carboxylate | 551.7 |
| 354 | 4-[(3S)-1-azabicyclo[2.2.2]oct-3-ylamino]-3-(1H-benzimidazol-2-yl)-6-pyridin-2-ylquinolin-2(1H)-one | 463.6 |
| 355 | 4-[(3R)-1-azabicyclo[2.2.2]oct-3-ylamino]-3-(1H-benzimidazol-2-yl)-6-thien-2-ylquinolin-2(1H)-one | 468.6 |
| 356 | 4-[(3R)-1-azabicyclo[2.2.2]oct-3-ylamino]-3-(1H-benzimidazol-2-yl)-6-(2,4-difluorophenyl)quinolin-2(1H)-one | 498.5 |
| 357 | 4-[(3R)-1-azabicyclo[2.2.2]oct-3-ylamino]-3-(1H-benzimidazol-2-yl)-6-thien-3-ylquinolin-2(1H)-one | 468.6 |
| 358 | 4-[4-[(3S)-1-azabicyclo[2.2.2]oct-3-ylamino]-3-(1H-benzimidazol-2-yl)-2-oxo-1,2-dihydroquinolin-6-yl]benzonitrile | 487.6 |
| 359 | 4-[(3R)-1-azabicyclo[2.2.2]oct-3-ylamino]-3-(1H-benzimidazol-2-yl)-6-(2-chlorophenyl)quinolin-2(1H)-one | 497.0 |
| 360 | 4-[(3R)-1-azabicyclo[2.2.2]oct-3-ylamino]-3-(1H-benzimidazol-2-yl)-6- [2-(trifluoromethyl)phenyl]quinolin-2(1H)-one | 530.6 |
| 361 | 4-[(3R)-1-azabicyclo[2.2.2]oct-3-ylamino]-3-(1H-benzimidazol-2-yl)-6-(3-methoxyphenyl)quinolin-2(1H)-one | 492.6 |
| 362 | 4-[(3S)-1-azabicyclo[2.2.2]oct-3-ylamino]-3-(1H-benzimidazol-2-yl)-6-pyridin-3-ylquinolin-2(1H)-one | 463.6 |
| 363 | 4-[(3S)-1-azabicyclo[2.2.2]oct-3-ylamino]-3-(1H-benzimidazol-2-yl)-6-pyridin-4-ylquinolin-2(1H)-one | 463.6 |
| 364 | 4-[(3S)-1-azabicyclo[2.2.2]oct-3-ylamino]-3-(1H-benzimidazol-2-yl)-2-oxo-1,2-dihydroquinoline-6-carboxylic acid | 430.5 |
| 365 | 3-(5-hydroxy-1H-benzimidazol-2-yl)-4-(piperidin-3-ylamino)quinolin-2(1H)-one | 376.4 |
| 366 | 4-[(3S)-1-azabicyclo[2.2.2]oct-3-ylamino]-3-(1H-benzimidazol-2-yl)-8-methylquinolin-2(1H)-one | 400.5 |
| 367 | 4-[(3S)-1-azabicyclo[2.2.2]oct-3-ylamino]-3-(1H-benzimidazol-2-yl)-6-(2-chlorophenyl)quinolin-2(1H)-one | 497.0 |
| 368 | 4-[(3S)-1-azabicyclo[2.2.2]oct-3-ylamino]-3-(1H-benzimidazol-2-yl)-6-[2-(trifluoromethyl)phenyl]quinolin-2(1H)-one | 530.6 |
| 369 | 4-[4-[(3S)-1-azabicyclo[2.2.2]oct-3-ylamino]-3-(1H-benzimidazol-2-yl)-2-oxo-1,2-dihydroquinolin-6-yl]benzonitrile | 487.6 |
| 370 | 4-[(3S)-1-azabicyclo[2.2.2]oct-3-ylamino]-3-(1H-benzimidazol-2-yl)-6-thien-3-ylquinolin-2(1H)-one | 468.6 |
| 371 | 4-[(3R)-1-azabicyclo[2.2.2]oct-3-ylamino]-3-(1H-benzimidazol-2-yl)-6-pyridin-4-ylquinolin-2(1H)-one | 463.6 |
| 372 | 4-[(3R)-1-azabicyclo[2.2.2]oct-3-ylamino]-3-(1H-benzimidazol-2-yl)-6-(2-methoxyphenyl)quinolin-2(1H)-one | 492.6 |
| 373 | 4-[(3R)-1-azabicyclo[2.2.2]oct-3-ylamino]-3-(1H-benzimidazol-2-yl)-6-(2-methylphenyl)quinolin-2(1H)-one | 476.6 |
| 374 | 6-(3-acetylphenyl)-4-[(3R)-1-azabicyclo[2.2.2]oct-3-ylamino]-3-(1H-benzimidazol-2-yl)quinolin-2(1H)-one | 504.6 |
| 375 | 6-(4-acetylphenyl)-4-[(3R)-1-azabicyclo[2.2.2]oct-3-ylamino]-3-(1H-benzimidazol-2-yl)quinolin-2(1H)-one | 504.6 |
| 376 | 4-[4-[(3R)-1-azabicyclo[2.2.2]oct-3-ylamino]-3-(1H-benzimidazol-2-yl)-2-oxo-1,2-dihydroquinolin-6-yl]benzoic acid | 506.6 |
| 377 | N-{3-[4-[(3R)-1-azabicyclo[2.2.2]oct-3-ylamino]-3-(1H-benzimidazol-2-yl)-2-oxo-1,2-dihydroquinolin-6-yl]phenyl}acetamide | 519.6 |
| 378 | 4-[(3R)-1-azabicyclo[2.2.2]oct-3-ylamino]-3-(1H-benzimidazol-2-yl)-6-(2,6-difluorophenyl)quinolin-2(1H)-one | 498.5 |
| 379 | 4-[(3R)-1-azabicyclo[2.2.2]oct-3-ylamino]-3-(1H-benzimidazol-2-yl)-6-(1,3-benzodioxol-5-yl)quinolin-2(1H)-one | 506.6 |
| 380 | 4-[(3R)-1-azabicyclo[2.2.2]oct-3-ylamino]-3-(1H-benzimidazol-2-yl)-6-(4-chlorophenyl)quinolin-2(1H)-one | 497.0 |
| 381 | 4-[4-[(3R)-1-azabicyclo[2.2.2]oct-3-ylamino]-3-(1H-benzimidazol-2-yl)-2-oxo-1,2-dihydroquinolin-6-yl]benzaldehyde | 490.6 |
| 382 | 4-[(3R)-1-azabicyclo[2.2.2]oct-3-ylamino]-3-(1H-benzimidazol-2-yl)-6-[4-(methylthio)phenyl]quinolin-2(1H)-one | 508.7 |
| 383 | 4-[(3R)-1-azabicyclo[2.2.2]oct-3-ylamino]-3-(1H-benzimidazol-2-yl)-6- [4-(dimethylamino)phenyl]quinolin-2(1H)-one | 505.6 |
| 384 | 4-[(3R)-1-azabicyclo[2.2.2]oct-3-ylamino]-3-(1H-benzimidazol-2-yl)-6-(4-chloro-2-fluorophenyl)quinolin-2(1H)-one | 515.0 |
| 385 | 4-[(3R)-1-azabicyclo[2.2.2]oct-3-ylamino]-3-(1H-benzimidazol-2-yl)-6-(2,4-dichlorophenyl)quinolin-2(1H)-one | 531.5 |
| 386 | 4-[(3R)-1-azabicyclo[2.2.2]oct-3-ylamino]-3-(1H-benzimidazol-2-yl)-6-phenylquinolin-2(1H)-one | 462.6 |
| 387 | 3-(1H-benzimidazol-2-yl)-6-chloro-4-[(1-ethylpiperidin-3-yl)amino]quinolin-2(1H)-one | 422.9 |
| 388 | 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-4-carboxamide | 530.6 |
| 389 | ethyl 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-4-carboxylate | 559.7 |
| 390 | 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-3-carboxamide | 530.6 |
| 391 | ethyl 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-3-carboxylate | 559.7 |
| 392 | 4-[(3R)-1-azabicyclo[2.2.2]oct-3-ylamino]-3-(1H-benzimidazol-2-yl)-6-fluoro-7-(1H-imidazol-1-yl)quinolin-2(1H)-one | 470.5 |
| 393 | 4-[(3R)-1-azabicyclo[2.2.2]oct-3-ylamino]-3-(1H-benzimidazol-2-yl)-7- {[2-(dimethylamino)ethyl]amino}-6-fluoroquinolin-2(1H)-one | 490.6 |
| 394 | 4-[(3R)-1-azabicyclo[2.2.2]oct-3-ylamino]-3-(1H-benzimidazol-2-yl)-6-fluoro-7-morpholin-4-ylquinolin-2(1H)-one | 489.6 |
| 395 | 4-[(3R)-1-azabicyclo[2.2.2]oct-3-ylamino]-3-(1H-benzimidazol-2-yl)-7-(dimethylamino)-6-fluoroquinolin-2(1H)-one | 447.5 |
| 396 | 4-[(3R)-1-azabicyclo[2.2.2]oct-3-ylamino]-3-(1H-benzimidazol-2-yl)-7-bromoquinolin-2(1H)-one | 465.4 |
| 397 | 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-4-carboxylic acid | 531.6 |
| 398 | 1-[4-[(3R)-1-azabicyclo[2.2.2]oct-3-ylamino]-3-(1H-benzimidazol-2-yl)-6-fluoro-2-oxo-1,2-dihydroquinolin-7-yl]piperidine-3-carboxylic acid | 531.6 |
| 399 | methyl 4-[4-[(3R)-1-azabicyclo[2.2.2]oct-3-ylamino]-3-(1H-benzimidazol-2-yl)-2-oxo-1,2-dihydroquinolin-6-yl]benzoate | 520.6 |
| 400 | 4-[4-[(3R)-1-azabicyclo[2.2.2]oct-3-ylamino]-3-(1H-benzimidazol-2-yl)-7-chloro-2-oxo-1,2-dihydroquinolin-6-yl]benzamide | 505.6 |
| 401 | 4-[(3R)-1-azabicyclo[2.2.2]oct-3-ylamino]-3-(1H-benzimidazol-2-yl)-6- [4-(methylsulfonyl)phenyl]quinolin-2(H)-one | 540.7 |
| 402 | methyl 3-amino-4-[4-[(3R)-1-azabicyclo[2.2.2]oct-3-ylamino]-3-(1H-benzimidazol-2-yl)-2-oxo-1,2-dihydroquinolin-6-yl]benzoate | 535.6 |
| 403 | 4-[4-[(3R)-1-azabicyclo[2.2.2]oct-3-ylamino]-3-(1H-benzimidazol-2-yl)-7-chloro-2-oxo-1,2-dihydroquinolin-6-yl]benzoic acid | 541.0 |
| 404 | N-{3-[4-[(3R)-1-azabicyclo[2.2.2]oct-3-ylamino]-3-(1H-benzimidazol-2-yl)-7-chloro-2-oxo-1,2-dihydroquinolin-6-yl]phenyl}acetamide | 554.1 |
| 405 | 6-(3-acetylphenyl)-4-[(3R)-1-azabicyclo[2.2.2]oct-3-ylamino]-3-(1H-benzimidazol-2-yl)-7-chloroquinolin-2(1H)-one | 539.0 |
| 406 | 4-[(3R)-1-azabicyclo[2.2.2]oct-3-ylamino]-3-(1H-benzimidazol-2-yl)-7-chloro-6-(2-methoxyphenyl)quinolin-2(1H)-one | 527.0 |
| 407 | 4-[(3R)-1-azabicyclo[2.2.2]oct-3-ylamino]-3-(1H-benzimidazol-2-yl)-7-chloro-6-(2,4-dichlorophenyl)quinolin-2(1H)-one | 565.9 |
| 408 | 6-(4-acetylphenyl)-4-[(3R)-1-azabicyclo[2.2.2]oct-3-ylamino]-3-(1H-benzimidazol-2-yl)-7-chloroquinolin-2(1H)-one | 539.0 |
| 409 | 4-[4-[(3R)-1-azabicyclo[2.2.2]oct-3-ylamino]-3-(1H-benzimidazol-2-yl)-7-chloro-2-oxo-1,2-dihydroquinolin-6-yl]benzamide | 540.0 |
| 410 | methyl 4-[4-[(3R)-1-azabicyclo[2.2.2]oct-3-ylamino]-3-(1H-benzimidazol-2-yl)-7-chloro-2-oxo-1,2-dihydroquinolin-6-yl]benzoate | 555.0 |
| 411 | 4-[(3R)-1-azabicyclo[2.2.2]oct-3-ylamino]-3-(1H-benzimidazol-2-yl)-7-[[2-(dimethylamino)ethyl](methyl)amino]-6-fluoroquinolin-2(1H)-one | 504.6 |
| 412 | 4-[(3R)-1-azabicyclo[2.2.2]oct-3-ylamino]-3-(1H-benzimidazol-2-yl)-6-fluoro-7-[(3-methoxypropyl)amino]quinolin-2(1H)-one | 491.6 |
| 413 | N-{(3R)-1-[4-[(3R)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}acetamide | 530.6 |
| 414 | 4-[(3R)-1-azabicyclo[2.2.2]oct-3-ylamino]-3-(1H-benzimidazol-2-yl)-6-fluoro-7- {[3-(2-oxopyrrolidin-1-yl)propyl]amino}quinolin-2(1H)-one | 544.6 |
| 415 | 4-[(3R)-1-azabicyclo[2.2.2]oct-3-ylamino]-7-azepan-1-yl-3-(1H-benzimidazol-2-yl)-6-fluoroquinolin-2(1H)-one | 501.6 |
| 416 | 4-[(3R)-1-azabicyclo[2.2.2]oct-3-ylamino]-3-(1H-benzimidazol-2-yl)-6-fluoro-7-(1H-pyrrol-1-yl)quinolin-2(1H)-one | 469.5 |
| 417 | 4-[(3R)-1-azabicyclo[2.2.2]oct-3-ylamino]-3-(1H-benzimidazol-2-yl)-6-fluoro-7-(2-methyl-1H-imidazol-1-yl)quinolin-2(1H)-one | 484.5 |
| 418 | 4-[(3R)-1-azabicyclo[2.2.2]oct-3-ylamino]-3-(1H-benzimidazol-2-yl)-6-fluoro-7-pyrrolidin-1-ylquinolin-2(1H)-one | 473.6 |
| 419 | 4-[(3R)-1-azabicyclo[2.2.2]oct-3-ylamino]-3-(1H-benzimidazol-2-yl)-6-fluoro-7-piperidin-1-ylquinolin-2(1H)-one | 487.6 |
| 420 | 4-[(3R)-1-azabicyclo[2.2.2]oct-3-ylamino]-3-(1H-benzimidazol-2-yl)-6-fluoro-7-(4-methylpiperazin-1-yl)quinolin-2(1H)-one | 502.6 |
| 421 | 4-[(3R)-1-azabicyclo[2.2.2]oct-3-ylamino]-3-(1H-benzimidazol-2-yl)-6-fluoro-7-[(3-hydroxypropyl)amino]quinolin-2(1H)-one | 477.6 |
| 422 | 4-[(3R)-1-azabicyclo[2.2.2]oct-3-ylamino]-3-(1H-benzimidazol-2-yl)-6-chloro-7-morpholin-4-ylquinolin-2(1H)-one | 506.0 |
| 423 | 4-[(3R)-1-azabicyclo[2.2.2]oct-3-ylamino]-3-(1H-benzimidazol-2-yl)-6-chloro-7-(4-methylpiperazin-1-yl)quinolin-2(1H)-one | 519.1 |
| 424 | 4-[(3R)-1-azabicyclo[2.2.2]oct-3-ylamino]-3-(1H-benzimidazol-2-yl)-6-chloro-7-piperidin-1-ylquinolin-2(1H)-one | 504.0 |
| 425 | 4-[4-[(3R)-1-azabicyclo[2.2.2]oct-3-ylamino]-3-(1H-benzimidazol-2-yl)-2-oxo-1,2-dihydroquinolin-7-yl]benzoic acid | 506.6 |
| 426 | 4-[(3R)-1-azabicyclo[2.2.2]oct-3-ylamino]-3-(1H-benzimidazol-2-yl)-7-(2,4-dichlorophenyl)quinolin-2(1H)-one | 531.5 |
| 427 | 4-[(3R)-1-azabicyclo[2.2.2]oct-3-ylamino]-3-(1H-benzimidazol-2-yl)-7-(dimethylamino)quinolin-2(1H)-one | 429.5 |
| 428 | 7-(4-acetylphenyl)-4-[(3R)-1-azabicyclo[2.2.2]oct-3-ylamino]-3-(1H-benzimidazol-2-yl)quinolin-2(1H)-one | 504.6 |
| 429 | 4-[(3R)-1-azabicyclo[2.2.2]oct-3-ylamino]-3-(1H-benzimidazol-2-yl)-7-(2-methylphenyl)quinolin-2(1H)-one | 476.6 |
| 430 | 7-(3-acetylphenyl)-4-[(3R)-1-azabicyclo[2.2.2]oct-3-ylamino]-3-(1H-benzimidazol-2-yl)quinolin-2(1H)-one | 504.6 |
| 431 | 4-[(3R)-1-azabicyclo[2.2.2]oct-3-ylamino]-3-(1H-benzimidazol-2-yl)-7-(2-methoxyphenyl)quinolin-2(1H)-one | 492.6 |
| 432 | 3-(1H-benzimidazol-2-yl)-6,7-difluoro-4-[(piperidin-2-ylmethyl)amino]quinolin-2(1H)-one | 410.4 |
| 433 | N-[3-(1H-benzimidazol-2-yl)-6,7-difluoro-2-oxo-1,2-dihydroquinolin-4-yl]glycine | 371.3 |
| 434 | N-[3-(1H-benzimidazol-2-yl)-6,7-difluoro-2-oxo-1,2-dihydroquinolin-4-yl]-beta-alanine | 385.3 |
| 435 | 4-[(3S)-1-azabicyclo[2.2.2]oct-3-ylamino]-3-(6-fluoro-1H-benzimidazol-2-yl)-6,7-dimethoxyquinolin-2(1H)-one | 464.5 |
| 436 | 3-(6-fluoro-1H-benzimidazol-2-yl)-6,7-dimethoxy-4-(piperidin-3-ylamino)quinolin-2(1H)-one | 438.5 |
| 437 | 3-(6-fluoro-1H-benzimidazol-2-yl)-6,7-dimethoxy-4-(pyrrolidin-3-ylamino)quinolin-2(1H)-one | 424.4 |
| 438 | 4-[(4-aminocyclohexyl)amino]-3-(6-fluoro-1H-benzimidazol-2-yl)-6,7-dimethoxyquinolin-2(1H)-one | 452.5 |
| 439 | 4-[(3R)-1-azabicyclo[2.2.2]oct-3-ylamino]-3-(6-fluoro-1H-benzimidazol-2-yl)-6,7-dimethoxyquinolin-2(1H)-one | 464.5 |
| 440 | 4-[(3R)-1-azabicyclo[2.2.2]oct-3-ylamino]-3-(1H-benzimidazol-2-yl)-7- [ethyl(methyl)amino]-6-fluoroquinolin-2(1H)-one | 461.6 |
| 441 | 4-[(3R)-1-azabicyclo[2.2.2]oct-3-ylamino]-3-(1H-benzimidazol-2-yl)-7-(diethylamino)-6-fluoroquinolin-2(1H)-one | 475.6 |
| 442 | 4-[(3R)-1-azabicyclo[2.2.2]oct-3-ylamino]-3-(1H-benzimidazol-2-yl)-7-[(3R)-3-(dimethylamino)pyrrolidin-1-yl]-6-fluoroquinolin-2(1H)-one | 516.6 |
| 443 | 7-(3-acetyl-1H-pyrrol-1-yl)-4-[(3R)-1-azabicyclo[2.2.2]oct-3-ylamino]-3-(1H-benzimidazol-2-yl)-6-fluoroquinolin-2(1H)-one | 511.6 |
| 444 | ethyl 4-[4-[(3R)-1-azabicyclo[2.2.2]oct-3-ylamino]-3-(1H-benzimidazol-2-yl)-2-oxo-1,2-dihydroquinolin-6-yl]benzoate | 534.6 |
| 445 | methyl 3-[4-[(3R)-1-azabicyclo[2.2.2]oct-3-ylamino]-3-(1H-benzimidazol-2-yl)-2-oxo-1,2-dihydroquinolin-6-yl]benzoate | 520.6 |
| 446 | 4-[(3R)-1-azabicyclo[2.2.2]oct-3-ylamino]-3-(1H-benzimidazol-2-yl)-7-{[2-(diethylamino)ethyl]amino}-6-fluoroquinolin-2(1H)-one | 518.6 |
| 447 | 4-[(3R)-1-azabicyclo[2.2.2]oct-3-ylamino]-3-(1H-benzimidazol-2-yl)-6-fluoro-7-[(2-pyrrolidin-1-ylethyl)amino]quinolin-2(1H)-one | 516.6 |
| 448 | 4-[(3R)-1-azabicyclo[2.2.2]oct-3-ylamino]-3-(1H-benzimidazol-2-yl)-6-fluoro-7-[(2-piperidin-1-ylethyl)amino]quinolin-2(1H)-one | 530.7 |
| 449 | 4-[(3R)-1-azabicyclo[2.2.2]oct-3-ylamino]-3-(1H-benzimidazol-2-yl)-7-{[3-(dimethylamino)propyl]amino}-6-fluoroquinolin-2(1H)-one | 504.6 |
| 450 | N-(2-{[4-[(3R)-1-azabicyclo[2.2.2]oct-3-ylamino]-3-(1H-benzimidazol-2-yl)-6-fluoro-2-oxo-1,2-dihydroquinolin-7-yl]amino}ethyl) acetamide | 504.6 |
| 451 | N-{1-[4-[(3R)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}-2,2,2-trifluoroacetamide | 584.6 |
| 452 | 3- {[4-[(3R)-1-azabicyclo[2.2.2]oct-3-ylamino]-3-(1H-benzimidazol-2-yl)-6-fluoro-2-oxo-1,2-dihydroquinolin-7-yl]amino}propanenitrile | 472.5 |
| 453 | 4-[(3R)-1-azabicyclo[2.2.2]oct-3-ylamino]-3-(1H-benzimidazol-2-yl)-6-fluoro-7-[(2-hydroxyethyl)amino]quinolin-2(1H)-one | 463.5 |
| 454 | 4-[(3R)-1-azabicyclo[2.2.2]oct-3-ylamino]-3-(1H-benzimidazol-2-yl)-6-fluoro-7-[(2-methoxyethyl)amino]quinolin-2(1H)-one | 477.6 |
| 455 | 4-[(3R)-1-azabicyclo[2.2.2]oct-3-ylamino]-3-(1H-benzimidazol-2-yl)-6-fluoro-7-(3-hydroxypiperidin-1-yl)quinolin-2(1H)-one | 503.6 |
| 456 | 4-[(3S)-1-azabicyclo[2.2.2]oct-3-ylamino]-3-(1H-benzimidazol-2-yl)-7-[[2-(dimethylamino)ethyl](methyl)amino]-6-fluoroquinolin-2(1 H)-one | 504.6 |
| 457 | 4-[(3S)-1-azabicyclo[2.2.2]oct-3-ylamino]-3-(1H-benzimidazol-2-yl)-7-{[3-(dimethylamino)propyl]amino}-6-fluoroquinolin-2(1H)-one | 504.6 |
| 458 | 4-[(3S)-1-azabicyclo[2.2.2]oct-3-ylamino]-3-(1H-benzimidazol-2-yl)-7- {[2-(diethylamino)ethyl]amino}-6-fluoroquinolin-2(1H)-one | 518.6 |
| 459 | 4-[(3S)-1-azabicyclo[2.2.2]oct-3-ylamino]-3-(1H-benzimidazol-2-yl)-6-fluoro-7-[(2-pyrrolidin-1-ylethyl)amino]quinolin-2(1H)-one | 516.6 |
| 460 | 4-[(3S)-1-azabicyclo[2.2.2]oct-3-ylamino]-3-(1H-benzimidazol-2-yl)-6-fluoro-7-(3-hydroxypiperidin-1-yl)quinolin-2(1H)-one | 530.7 |
| 461 | 4-[(3S)-1-azabicyclo[2.2.2]oct-3-ylamino]-3-(1H-benzimidazol-2-yl)-6-fluoro-7-{[3-(2-oxopyrrolidin-1-yl)propyl]amino}quinolin-2(1H)-one | 544.6 |
| 462 | N-(2-{[4-[(3S)-1-azabicyclo[2.2.2]oct-3-ylamino]-3-(1H-benzimidazol-2-yl)-6-fluoro-2-oxo-1,2-dihydroquinolin-7-yl]amino}ethyl)acetamide | 504.6 |
| 463 | 4-[(3S)-1-azabicyclo[2.2.2]oct-3-ylamino]-3-(1H-benzimidazol-2-yl)-6-fluoro-7-[(3-methoxypropyl)amino]quinolin-2(1H)-one | 491.6 |
| 464 | 4-[(3S)-1-azabicyclo[2.2.2]oct-3-ylamino]-3-(1H-benzimidazol-2-yl)-6-fluoro-7-[(2-methoxyethyl)amino]quinolin-2(1H)-one | 477.6 |
| 465 | 4-[(3S)-1-azabicyclo[2.2.2]oct-3-ylamino]-3-(1H-benzimidazol-2-yl)-6-fluoro-7-[(2-hydroxyethyl)amino]quinolin-2(1H)-one | 463.5 |
| 466 | 4-[(3S)-1-azabicyclo[2.2.2]oct-3-ylamino]-3-(1H-benzimidazol-2-yl)-7-[ethyl(methyl)amino]-6-fluoroquinolin-2(1H)-one | 461.6 |
| 467 | 4-[(3S)-1-azabicyclo[2.2.2]oct-3-ylamino]-3-(1H-benzimidazol-2-yl)-7-(diethylamino)-6-fluoroquinolin-2(1H)-one | 475.6 |
| 468 | N-{(3R)-1-[4-[(3S)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}acetamide | 530.6 |
| 469 | N-{(3S)-1-[4-[(3S)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}acetamide | 530.6 |
| 470 | 4-[(3S)-1-azabicyclo[2.2.2]oct-3-ylamino]-3-(1H-benzimidazol-2-yl)-7-[(3R)-3-(dimethylamino)pyrrolidin-1-yl]-6-fluoroquinolin-2(1H)-one | 516.6 |
| 471 | N-{1-[4-[(3S)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}-2,2,2-trifluoroacetamide | 584.6 |
| 472 | 4-[(3S)-1-azabicyclo[2.2.2]oct-3-ylamino]-7-azepan-1-yl-3-(1H-benzimidazol-2-yl)-6-fluoroquinolin-2(1H)-one | 501.6 |
| 473 | 4-[(3S)-1-azabicyclo[2.2.2]oct-3-ylamino]-3-(1H-benzimidazol-2-yl)-6-fluoro-7-(3-hydroxypiperidin-1-yl)quinolin-2(1H)-one | 503.6 |
| 474 | 3- {[4-[(3S)-1-azabicyclo[2.2.2]oct-3-ylamino]-3-(1H-benzimidazol-2-yl)-6-fluoro-2-oxo-1,2-dihydroquinolin-7-yl]amino}propanenitrile | 472.5 |
| 475 | 4-[(3S)-1-azabicyclo[2.2.2]oct-3-ylamino]-3-(1H-benzimidazol-2-yl)-6-fluoro-7-(1H-pyrrol-1-yl)quinolin-2(1H)-one | 469.5 |
| 476 | 7-(3-acetyl-1H-pyrrol-1-yl)-4-[(3S)-1-azabicyclo[2.2.2]oct-3-ylamino]-3-(1H-benzimidazol-2-yl)-6-fluoroquinolin-2(1H)-one | 511.6 |
| 477 | 4-[(3S)-1-azabicyclo[2.2.2]oct-3-ylamino]-3-(1H-benzimidazol-2-yl)-6-fluoro-7-(2-methyl-1H-imidazol-1-yl)quinolin-2(1H)-one | 484.5 |
| 478 | 4-[(3S)-1-azabicyclo[2.2.2]oct-3-ylamino]-3-(1H-benzimidazol-2-yl)-7-[(3S)-3-(dimethylamino)pyrrolidin-1-yl]-6-fluoroquinolin-2(1H)-one | 516.6 |
| 479 | 4-[(3S)-1-azabicyclo[2.2.2]oct-3-ylamino]-3-(1H-benzimidazol-2-yl)-6-fluoro-7-methoxyquinolin-2(1H)-one | 434.5 |
| 480 | 4-[(3R)-1-azabicyclo[2.2.2]oct-3-ylamino]-3-(1H-benzimidazol-2-yl)-7-[(3S)-3-(dimethylamino)pyrrolidin-1-yl]-6-fluoroquinolin-2(1H)-one | 516.6 |
| 481 | N-{(3S)-1-[4-[(3R)-1-azabicyclo[2.2.2]oct-3-ylamino]-3-(1H-benzimidazol-2-yl)-6-fluoro-2-oxo-1,2-dihydroquinolin-7-yl]pyrrolidin-3-yl}acetamide | 530.6 |
| 482 | 4-[(3R)-1-azabicyclo[2.2.2]oct-3-ylamino]-3-(1H-benzimidazol-2-yl)-6-fluoro-7-[(2-pyridin-2-ylethyl)amino]quinolin-2(1H)-one | 524.6 |
| 483 | 4-[(3R)-1-azabicyclo[2.2.2]oct-3-ylamino]-3-(1H-benzimidazol-2-yl)-6-fluoro-7-(isobutylamino)quinolin-2(1H)-one | 475.6 |
| 484 | methyl 3-amino-4-[4-[(3R)-1-azabicyclo[2.2.2]oct-3-ylamino]-3-(1H-benzimidazol-2-yl)-7-chloro-2-oxo-1,2-dihydroquinolin-6-yl]benzoate | 570.1 |
| 485 | 4-[(3R)-1-azabicyclo[2.2.2]oct-3-ylamino]-3-(1H-benzimidazol-2-yl)-7-chloro-6-[4-(methylsulfonyl)phenyl]quinolin-2(1H)-one | 575.1 |
| 486 | methyl 3-[4-[(3R)-1-azabicyclo[2.2.2]oct-3-ylamino]-3-(1H-benzimidazol-2-yl)-7-chloro-2-oxo-1,2-dihydroquinolin-6-yl]benzoate | 555.0 |
| 487 | 1-[4-[(3S)-1-azabicyclo[2.2.2]oct-3-ylamino]-3-(1H-benzimidazol-2-yl)-6-fluoro-2-oxo-1,2-dihydroquinolin-7-yl]piperidine-4-carboxylic acid | 531.6 |
| 488 | 1-[4-[(3S)-1-azabicyclo[2.2.2]oct-3-ylamino]-3-(1H-benzimidazol-2-yl)-6-fluoro-2-oxo-1,2-dihydroquinolin-7-yl]piperidine-3-carboxylic acid | 531.6 |
| 489 | 4-[(4-aminobenzyl)amino]-3-(1H-benzimidazol-2-yl)-6,7-dimethoxyquinolin-2(1H)-one | 442.5 |
| 490 | 4-(2-{[3-(1H-benzimidazol-2-yl)-6,7-dimethoxy-2-oxo-1,2-dihydroquinolin-4-yl]amino}ethyl)benzenesulfonamide | 520.6 |
| 491 | 4-[(3-aminopropyl)amino]-3-(1H-benzimidazol-2-yl)-6,7-dimethoxyquinolin-2(1H)-one | 394.4 |
| 492 | 4-[(2-aminoethyl)amino]-3-(1H-benzimidazol-2-yl)-6,7-dimethoxyquinolin-2(1H)-one | 380.4 |
| 493 | 3-(1H-benzimidazol-2-yl)-4-{[2-(1H-imidazol-5-yl)ethyl]amino}-6,7-dimethoxyquinolin-2(1H)-one | 431.5 |
| 494 | 3-(1H-benzimidazol-2-yl)-4-{[2-(1H-benzimidazol-2-yl)ethyl]amino}-6,7-dimethoxyquinolin-2(1H)-one | 481.5 |
| 495 | 4-{[(4-amino-2-methylpyrimidin-5-yl)methyl]amino}-3-(1H-benzimidazol-2-yl)-6,7-dimethoxyquinolin-2(1H)-one | 458.5 |
| 496 | 3-(1H-benzimidazol-2-yl)-4-{[2-(5-fluoro-1H-indol-3-yl)ethyl]amino}-6,7-dimethoxyquinolin-2(1H)-one | 498.5 |
| 497 | 4-{[2-(4-aminophenyl)ethyl]amino}-3-(1H-benzimidazol-2-yl)-6,7-dimethoxyquinolin-2(1H)-one | 456.5 |
| 498 | 4-[(3R)-1-azabicyclo[2.2.2]oct-3-ylamino]-3-(1H-benzimidazol-2-yl)-7-morpholin-4-ylquinolin-2(1H)-one | 471.6 |
| 499 | 4-[(3R)-1-azabicyclo[2.2.2]oct-3-ylamino]-3-(5,6-difluoro-1H-benzimidazol-2-yl)-6,7-dimethoxyquinolin-2(1H)-one | 430.5 |
| 500 | methyl 3-amino-4-[4-[(3R)-1-azabicyclo[2.2.2]oct-3-ylamino]-3-(1H-benzimidazol-2-yl)-2-oxo-1,2-dihydroquinolin-7-yl]benzoate | 535.6 |
| 501 | 4-[(3R)-1-azabicyclo[2.2.2]oct-3-ylamino]-3-(1H-benzimidazol-2-yl)-7-[4-(methylsulfonyl)phenyl]quinolin-2(1H)-one | 540.7 |
| 502 | methyl 4-[4-[(3R)-1-azabicyclo[2.2.2]oct-3-ylamino]-3-(1H-benzimidazol-2-yl)-2-oxo-1,2-dihydroquinolin-7-yl]benzoate | 520.6 |
| 503 | methyl 3-[4-[(3R)-1-azabicyclo[2.2.2]oct-3-ylamino]-3-(1H-benzimidazol-2-yl)-2-oxo-1,2-dihydroquinolin-7-yl]benzoate | 520.6 |
| 504 | N-{3-[4-[(3R)-1-azabicyclo[2.2.2]oct-3-ylamino]-3-(1H-benzimidazol-2-yl)-2-oxo-1,2-dihydroquinolin-7-yl]phenyl}acetamide | 519.6 |
| 505 | 4-[(3R)-1-azabicyclo[2.2.2]oct-3-ylamino]-3-(5,6-difluoro-1H-benzimidazol-2-yl)-6,7-dimethoxyquinolin-2(1H)-one | 482.5 |
| 506 | 3-(5,6-difluoro-1H-benzimidazol-2-yl)-6,7-dimethoxy-4-(piperidin-3-ylamino)quinolin-2(1H)-one | 456.5 |
| 507 | 4-[(4-aminocyclohexyl)amino]-3-(5,6-difluoro-1H-benzimidazol-2-yl)-6,7-dimethoxyquinolin-2(1H)-one | 470.5 |
| 508 | 3-(5,6-difluoro-1H-benzimidazol-2-yl)-6,7-dimethoxy-4-(pyrrolidin-3-ylamino)quinolin-2(1H)-one | 442.4 |
| 509 | 4-[(3R)-1-azabicyclo[2.2.2]oct-3-ylamino]-3-(1H-benzimidazol-2-yl)-6-chloro-7-(1H-imidazol-1-yl)quinolin-2(1H)-one | 487.0 |
| 510 | 4-[(3R)-1-azabicyclo[2.2.2]oct-3-ylamino]-3-(1H-benzimidazol-2-yl)-7-[(3-hydroxypropyl)amino]quinolin-2(1H)-one | 459.6 |
| 511 | 4-[(3R)-1-azabicyclo[2.2.2]oct-3-ylamino]-3-(1H-benzimidazol-2-yl)-7-{[3-(2-oxopyrrolidin-1-yl)propyl]amino}quinolin-2(1H)-one | 526.7 |
| 512 | 4-[(3R)-1-azabicyclo[2.2.2]oct-3-ylamino]-3-(1H-benzimidazol-2-yl)-7-(4-methylpiperazin-1-yl)quinolin-2(1H)-one | 484.6 |
| 513 | 4-[4-[(3R)-1-azabicyclo[2.2.2]oct-3-ylamino]-3-(1H-benzimidazol-2-yl)-2-oxo-1,2-dihydroquinolin-7-yl]benzonitrile | 487.6 |
| 514 | 4-[(3R)-1-azabicyclo[2.2.2]oct-3-ylamino]-3-(1H-benzimidazol-2-yl)-7-[2-(trifluoromethyl)phenyl]quinolin-2(1H)-one | 530.6 |
| 515 | 4-[(3R)-1-azabicyclo[2.2.2]oct-3-ylamino]-3-(1H-benzimidazol-2-yl)-7-(1,3-benzodioxol-5-yl)quinolin-2(1H)-one | 506.6 |
| 516 | 4-[(3R)-1-azabicyclo[2.2.2]oct-3-ylamino]-3-(1H-benzimidazol-2-yl)-7-(morpholin-4-ylcarbonyl)quinolin-2(1H)-one | 499.6 |
| 517 | 4-[(3R)-1-azabicyclo[2.2.2]oct-3-ylamino]-3-(1H-benzimidazol-2-yl)-N,N-dimethyl-2-oxo-1,2-dihydroquinoline-7-carboxamide | 457.5 |
| 518 | 4-[(3R)-1-azabicyclo[2.2.2]oct-3-ylamino]-3-(1H-benzimidazol-2-yl)-2-oxo-1,2-dihydroquinoline-7-carboxamide | 429.5 |
| 519 | 3-[4-[(3R)-1-azabicyclo[2.2.2]oct-3-ylamino]-3-(1H-benzimidazol-2-yl)-2-oxo-1,2-dihydroquinolin-7-yl]benzoic acid | 506.6 |
| 520 | 4-[(3S)-1-azabicyclo[2.2.2]oct-3-ylamino]-3-(1H-benzimidazol-2-yl)-7-bromoquinolin-2(1H)-one | 465.4 |
| 521 | 4-{4-[(3R)-1-azabicyclo[2.2.2]oct-3-ylamino]-3-(1H-benzimidazol-2-yl)-7-[4-(ethoxycarbonyl)piperidin-1-yl]-2-oxo-1,2-dihydroquinolin-6-yl}benzoic acid | 661.8 |
| 522 | 4-[7-(3-acetyl-1H-pyrrol-1-yl)-4-[(3R)-1-azabicyclo[2.2.2]oct-3-ylamino]-3-(1H-benzimidazol-2-yl)-2-oxo-1,2-dihydroquinolin-6-yl]benzoic acid | 613.7 |
| 523 | 4-[4-[(3R)-1-azabicyclo[2.2.2]oct-3-ylamino]-3-(1H-benzimidazol-2-yl)-7-(dimethylamino)-2-oxo-1,2-dihydroquinolin-6-yl]benzoic acid | 549.6 |
| 524 | 4-[4-[(3R)-1-azabicyclo[2.2.2]oct-3-ylamino]-3-(1H-benzimidazol-2-yl)-7-(1H-imidazol-1-yl)-2-oxo-1,2-dihydroquinolin-6-yl]benzoic acid | 572.6 |
| 525 | 4-[(3R)-1-azabicyclo[2.2.2]oct-3-ylamino]-3-(1H-benzimidazol-2-yl)-7-fluoro-6-iodoquinolin-2(1H)-one | 530.4 |
| 526 | 4-[(3R)-1-azabicyclo[2.2.2]oct-3-ylamino]-3-(1H-benzimidazol-2-yl)-7-fluoro-6-[4-(methylsulfonyl)phenyl]quinolin-2(1H)-one | 558.6 |
| 527 | 4-[4-[(3R)-1-azabicyclo[2.2.2]oct-3-ylamino]-3-(1H-benzimidazol-2-yl)-7-fluoro-2-oxo-1,2-dihydroquinolin-6-yl]benzamide | 523.6 |
| 528 | 6-(4-acetylphenyl)-4-[(3R)-1-azabicyclo[2.2.2]oct-3-ylamino]-3-(1H-benzimidazol-2-yl)-7-fluoroquinolin-2(1H)-one | 522.6 |
| 529 | methyl 4-[4-[(3R)-1-azabicyclo[2.2.2]oct-3-ylamino]-3-(1H-benzimidazol-2-yl)-7-fluoro-2-oxo-1,2-dihydroquinolin-6-yl]benzoate | 538.6 |
| 530 | methyl 3-amino-4-[4-[(3R)-1-azabicyclo[2.2.2]oct-3-ylamino]-3-(1H-benzimidazol-2-yl)-7-fluoro-2-oxo-1,2-dihydroquinolin-6-yl]benzoate | 553.6 |
| 531 | 6-(3-acetylphenyl)-4-[(3R)-1-azabicyclo[2.2.2]oct-3-ylamino]-3-(1H-benzimidazol-2-yl)-7-fluoroquinolin-2(1H)-one | 522.6 |
| 532 | methyl 3-[4-[(3R)-1-azabicyclo[2.2.2]oct-3-ylamino]-3-(1H-benzimidazol-2-yl)-7-fluoro-2-oxo-1,2-dihydroquinolin-6-yl]benzoate | 538.6 |
| 533 | 4-[(3R)-1-azabicyclo[2.2.2]oct-3-ylamino]-3-(1H-benzimidazol-2-yl)-7-fluoro-6-(2-methylphenyl)quinolin-2(1H)-one | 494.6 |
| 534 | 4-[(3R)-1-azabicyclo[2.2.2]oct-3-ylamino]-3-(1H-benzimidazol-2-yl)-7-fluoro-6-(2-methoxyphenyl)quinolin-2(1H)-one | 510.6 |
| 535 | 4-[(3R)-1-azabicyclo[2.2.2]oct-3-ylamino]-3-(1H-benzimidazol-2-yl)-6-(2,4-dichlorophenyl)-7-fluoroquinolin-2(1H)-one | 549.4 |
| 536 | ethyl 1-[4-[(3R)-1-azabicyclo[2.2.2]oct-3-ylamino]-3-(1H-benzimidazol-2-yl)-6-iodo-2-oxo-1,2-dihydroquinolin-7-yl]piperidine-4-carboxylate | 667.6 |
| 537 | 4-[(3R)-1-azabicyclo[2.2.2]oct-3-ylamino]-3-(1H-benzimidazol-2-yl)-7-(1H-imidazol-1-yl)-6-iodoquinolin-2(1H)-one | 578.4 |
| 538 | 4-[(3R)-1-azabicyclo[2.2.2]oct-3-ylamino]-3-(1H-benzimidazol-2-yl)-6-(2-ethylphenyl)-7-(1H-imidazol-1-yl)quinolin-2(1H)-one | 556.7 |
| 539 | 4-[4-[(3R)-1-azabicyclo[2.2.2]oct-3-ylamino]-3-(1H-benzimidazol-2-yl)-7-(1H-imidazol-1-yl)-2-oxo-1,2-dihydroquinolin-6-yl]benzamide | 571.7 |
| 540 | 6-(4-acetylphenyl)-4-[(3R)-1-azabicyclo[2.2.2]oct-3-ylamino]-3-(1H-benzimidazol-2-yl)-7-(1H-imidazol-1-yl)quinolin-2(1H)-one | 570.7 |
| 541 | 6-(3-acetylphenyl)-4-[(3R)-1-azabicyclo[2.2.2]oct-3-ylamino]-3-(1H-benzimidazol-2-yl)-7-(1H-imidazol-1-yl)quinolin-2(1H)-one | 587.7 |
| 542 | N-{3-[4-[(3R)-1-azabicyclo[2.2.2]oct-3-ylamino]-3-(1H-benzimidazol-2-yl)-7-(1H-imidazol-1-yl)-2-oxo-1,2-dihydroquinolin-6-yl]phenyl}acetamide | 585.7 |
| 543 | 6-(3-acetylphenyl)-4-[(3R)-1-azabicyclo[2.2.2]oct-3-ylamino]-3-(1H-benzimidazol-2-yl)-7-(1H-imidazol-1-yl)quinolin-2(1H)-one | 570.7 |
| 544 | 4-[(3R)-1-azabicyclo[2.2.2]oct-3-ylamino]-3-(1H-benzimidazol-2-yl)-7-(1H-imidazol-1-yl)-6-(2-methylphenyl)quinolin-2(1H)-one | 542.7 |
| 545 | 4-[(3R)-1-azabicyclo[2.2.2]oct-3-ylamino]-3-(1H-benzimidazol-2-yl)-7-(1H-imidazol-1-yl)-6-(2-methoxyphenyl)quinolin-2(1H)-one | 558.7 |
| 546 | 4-[(3R)-1-azabicyclo[2.2.2]oct-3-ylamino]-3-(1H-benzimidazol-2-yl)-6-(2,4-dichlorophenyl)-7-(1H-imidazol-1-yl)quinolin-2(1H)-one | 597.5 |
| 547 | 4-[(3R)-1-azabicyclo[2.2.2]oct-3-ylamino]-3-(1H-benzimidazol-2-yl)-6-(2-ethylphenyl)quinolin-2(1H)-one | 490.6 |
| 548 | 4-[(3R)-1-azabicyclo[2.2.2]oct-3-ylamino]-3-(1H-benzimidazol-2-yl)-6-(2-ethylphenyl)-7-fluoroquinolin-2(1H)-one | 508.6 |
| 549 | 3-[4-[(3R)-1-azabicyclo[2.2.2]oct-3-ylamino]-3-(1H-benzimidazol-2-yl)-2-oxo-1,2-dihydroquinolin-6-yl]benzoic acid | 506.6 |
| 550 | 3-amino-4-[4-[(3R)-1-azabicyclo[2.2.2]oct-3-ylamino]-3-(1H-benzimidazol-2-yl)-7-chloro-2-oxo-1,2-dihydroquinolin-6-yl]benzoic acid | 556.0 |
| 551 | 3-[4-[(3R)-1-azabicyclo[2.2.2]oct-3-ylamino]-3-(1H-benzimidazol-2-yl)-7-chloro-2-oxo-1,2-dihydroquinolin-6-yl]benzoic acid | 541.0 |
| 552 | 4-[(3R)-1-azabicyclo[2.2.2]oct-3-ylamino]-3-(1H-benzimidazol-2-yl)-6-fluoro-7-[(pyridin-2-ylmethyl)amino]quinolin-2(1H)-one | 510.6 |
| 553 | 4-[(3R)-1-azabicyclo[2.2.2]oct-3-ylamino]-3-(1H-benzimidazol-2-yl)-6-fluoro-7-[(3-pyrrolidin-1-ylpropyl)amino]quinolin-2(1H)-one | 527.6 |
| 554 | 4-[(3R)-1-azabicyclo[2.2.2]oct-3-ylamino]-3-(1H-benzimidazol-2-yl)-6-fluoro-7-[(pyridin-3-ylmethyl)amino]quinolin-2(1H)-one | 510.6 |
| 555 | 4-[(3R)-1-azabicyclo[2.2.2]oct-3-ylamino]-3-(1H-benzimidazol-2-yl)-6-fluoro-7-[(3-pyrrolidin-1-ylpropyl)amino]quinolin-2(1H)-one | 530.7 |
| 556 | 4-[(3R)-1-azabicyclo[2.2.2]oct-3-ylamino]-3-(1H-benzimidazol-2-yl)-6-fluoro-7-[(3R)-3-hydroxypyrrolidin-1-yl]quinolin-2(1H)-one | 489.6 |
| 557 | 4-[(3R)-1-azabicyclo[2.2.2]oct-3-ylamino]-3-(1H-benzimidazol-2-yl)-6-fluoro-7-{[2-(1-methylpyrrolidin-2-yl)ethyl]amino}quinolin-2(1H)-one | 530.7 |
| 558 | 4-[(3R)-1-azabicyclo[2.2.2]oct-3-ylamino]-3-(1H-benzimidazol-2-yl)-6-fluoro-7-[(pyridin-4-ylmethyl)amino]quinolin-2(1H)-one | 510.6 |
| 559 | 4-[(3R)-1-azabicyclo[2.2.2]oct-3-ylamino]-3-(1H-benzimidazol-2-yl)-6-fluoro-7-[3-(methylsulfonyl)pyrrolidin-1-yl]quinolin-2(1H)-one | 551.7 |
| 560 | 4-[(3R)-1-azabicyclo[2.2.2]oct-3-ylamino]-3-(1H-benzimidazol-2-yl)-6-fluoro-7-(3-pyridin-4-ylpyrrolidin-1-yl)quinolin-2(1H)-one | 550.7 |
| 561 | 4-[(3R)-1-azabicyclo[2.2.2]oct-3-ylamino]-3-(1H-benzimidazol-2-yl)-6-fluoro-7-[(2-morpholin-4-ylethyl)amino]quinolin-2(1H)-one | 532.6 |
| 562 | 4-[(3R)-1-azabicyclo[2.2.2]oct-3-ylamino]-3-(1H-benzimidazol-2-yl)-6-fluoro-7-[4-(pyridin-4-ylmethyl)piperazin-1-yl]quinolin-2(1H)-one | 579.7 |
| 563 | 4-[(3R)-1-azabicyclo[2.2.2]oct-3-ylamino]-3-(1H-benzimidazol-2-yl)-7-(benzylamino)-6-fluoroquinolin-2(1H)-one | 509.6 |
| 564 | 4-[(3R)-1-azabicyclo[2.2.2]oct-3-ylamino]-3-(1H-benzimidazol-2-yl)-6-fluoro-7-(2-pyridin-3-ylpyrrolidin-1-yl)quinolin-2(1H)-one | 550.7 |
| 565 | 4-[(3R)-1-azabicyclo[2.2.2]oct-3-ylamino]-3-(1H-benzimidazol-2-yl)-6-fluoro-7-[(2-pyridin-4-ylethyl)amino]quinolin-2(1H)-one | 524.6 |
| 566 | 4-[(3R)-1-azabicyclo[2.2.2]oct-3-ylamino]-3-(1H-benzimidazol-2-yl)-6-fluoro-7-[(3-morpholin-4-ylpropyl)amino]quinolin-2(1H)-one | 546.7 |
| 567 | 4-[(3R)-1-azabicyclo[2.2.2]oct-3-ylamino]-3-(1H-benzimidazol-2-yl)-6-fluoro-7-[(4-hydroxycyclohexyl)amino]quinolin-2(1H)-one | 524.6 |
| 568 | 7-{[2-(4-aminophenyl)ethyl]amino}-4-[(3R)-1-azabicyclo[2.2.2]oct-3-ylamino]-3-(1H-benzimidazol-2-yl)-6-fluoroquinolin-2(1H)-one | 538.6 |
| 569 | 4-[(3R)-1-azabicyclo[2.2.2]oct-3-ylamino]-3-(1H-benzimidazol-2-yl)-6-fluoro-7-[(4-hydroxycyclohexyl)amino]quinolin-2(1H)-one | 517.6 |
| 570 | 4-(1-azabicyclo[2.2.2]oct-3-ylamino)-3-(1H-benzimidazol-2-yl)-6-fluoro-7-[(piperidin-3-ylmethyl)amino]quinolin-2(1H)-one | 516.6 |
| 571 | 4-(1-azabicyclo[2.2.2]oct-3-ylamino)-3-(1H-benzimidazol-2-yl)-6-fluoro-7-(pyrrolidin-3-ylamino)quinolin-2(1H)-one | 488.6 |
| 572 | 4-[4-[(3R)-1-azabicyclo[2.2.2]oct-3-ylamino]-3-(1H-benzimidazol-2-yl)-7-(2-methyl-1H-imidazol-1-yl)-2-oxo-1,2-dihydroquinolin-6-yl]benzoic acid | 586.7 |
| 573 | 1-[4-[(3R)-1-azabicyclo[2.2.2]oct-3-ylamino]-3-(1H-benzimidazol-2-yl)-6-chloro-2-oxo-1,2-dihydroquinolin-7-yl]piperidine-4-carboxamide | 547.1 |
| 574 | ethyl 1-[4-[(3R)-1-azabicyclo[2.2.2]oct-3-ylamino]-3-(1H-benzimidazol-2-yl)-6-chloro-2-oxo-1,2-dihydroquinolin-7-yl]piperidine-4-carboxylate | 576.1 |
| 575 | 4-[(3R)-1-azabicyclo[2.2.2]oct-3-ylamino]-3-(1H-benzimidazol-2-yl)-7-(1H-imidazol-1-yl)quinolin-2(1H)-one | 452.5 |
| 576 | 4-[(3R)-1-azabicyclo[2.2.2]oct-3-ylamino]-3-(1H-benzimidazol-2-yl)-7-(2-methyl-1H-imidazol-1-yl)quinolin-2(1H)-one | 466.6 |
| 577 | ethyl 1-[4-[(3R)-1-azabicyclo[2.2.2]oct-3-ylamino]-3-(1H-benzimidazol-2-yl)-2-oxo-1,2-dihydroquinolin-7-yl]piperidine-4-carboxylate | 541.7 |
| 578 | 1-[4-[(3R)-1-azabicyclo[2.2.2]oct-3-ylamino]-3-(1H-benzimidazol-2-yl)-2-oxo-1,2-dihydroquinolin-7-yl]piperidine-4-carboxamide | 512.6 |
| 579 | 4-[(3R)-1-azabicyclo[2.2.2]oct-3-ylamino]-3-(1H-benzimidazol-2-yl)-6-fluoro-7-[(2-mercaptoethyl)amino]quinolin-2(1H)-one | 479.6 |
| 580 | 4-[(3R)-1-azabicyclo[2.2.2]oct-3-ylamino]-3-(1H-benzimidazol-2-yl)-6-fluoro-7-[4-(pyridin-3-ylmethyl)piperazin-1-yl]quinolin-2(1H)-one | 579.7 |
| 581 | 3-(1H-benzimidazol-2-yl)-4-[(2-hydroxyethyl)amino]-6,7-dimethoxyquinolin-2(1H)-one | 381.4 |
| 582 | 3-(1H-benzimidazol-2-yl)-4-[(3-hydroxypropyl)amino]-6,7-dimethoxyquinolin-2(1H)-one | 395.4 |
| 583 | 4-[(3R)-1-azabicyclo[2.2.2]oct-3-ylamino]-3-(1H-benzimidazol-2-yl)-6-fluoro-7-{[(1-hydroxycyclohexyl)methyl]amino}quinolin-2(1H)-one | 531.6 |
| 584 | 3-(1H-benzimidazol-2-yl)-6,7-dimethoxy-4-[(3-pyrrolidin-1-ylpropyl)amino]quinolin-2(1H)-one | 448.5 |
| 585 | 4-[(3S)-1-azabicyclo[2.2.2]oct-3-ylamino]-3-(1H-benzimidazol-2-yl)-2-oxo-1,2-dihydroquinoline-7-carbonitrile | 411.5 |
| 586 | 3-(1H-benzimidazol-2-yl)-6-chloro-4-(pyridin-3-ylamino)quinolin-2(1H)-one | 388.8 |
| 587 | 3-(1H-benzimidazol-2-yl)-4-[(1-benzylpiperidin-4-yl)amino]-6-chloroquinolin-2(1H)-one | 485.0 |
| 588 | 4-[(3S)-1-azabicyclo[2.2.2]oct-3-ylamino]-3-(1H-benzimidazol-2-yl)-7-methoxyquinolin-2(1H)-one | 416.5 |
| 589 | 4-[(3R)-1-azabicyclo[2.2.2]oct-3-ylamino]-3-(1H-benzimidazol-2-yl)-6-bromo-7-methoxyquinolin-2(1H)-one | 495.4 |
| 590 | 3-(1H-benzimidazol-2-yl)-6,7-dimethoxy-4-{[(5-methylpyrazin-2-yl)methyl]amino}quinolin-2(1H)-one | 443.5 |
| 591 | 4-[(3-amino-2-hydroxypropyl)amino]-3-(1H-benzimidazol-2-yl)-6,7-dimethoxyquinolin-2(1H)-one | 410.4 |
| 592 | 3-(1H-benzimidazol-2-yl)-6,7-dimethoxy-4-[(2-methoxyethyl)amino]quinolin-2(1H)-one | 395.4 |
| 593 | {[3-(1H-benzimidazol-2-yl)-6,7-dimethoxy-2-oxo-1,2-dihydroquinolin-4-yl]amino}acetonitrile | 376.4 |
| 594 | 3-(1H-benzimidazol-2-yl)-4-{[2-(2-hydroxyethoxy)ethyl]amino}-6,7-dimethoxyquinolin-2(1H)-one | 425.5 |
| 595 | 3-(1H-benzimidazol-2-yl)-4-[(3R)-3-hydroxypyrrolidin-1-yl]-6,7-dimethoxyquinolin-2(1H)-one | 407.4 |
| 596 | 4-[4-[(3S)-1-azabicyclo[2.2.2]oct-3-ylamino]-3-(1H-benzimidazol-2-yl)-2-oxo-1,2-dihydroquinolin-7-yl]benzonitrile | 487.6 |
| 597 | 4-[4-[(3S)-1-azabicyclo[2.2.2]oct-3-ylamino]-3-(1H-benzimidazol-2-yl)-2-oxo-1,2-dihydroquinolin-7-yl]benzoic acid | 506.6 |
| 598 | 4-[4-[(3S)-1-azabicyclo[2.2.2]oct-3-ylamino]-3-(1H-benzimidazol-2-yl)-2-oxo-1,2-dihydroquinolin-7-yl]benzamide | 505.6 |
| 599 | methyl 3-[4-[(3S)-1-azabicyclo[2.2.2]oct-3-ylamino]-3-(1H-benzimidazol-2-yl)-2-oxo-1,2-dihydroquinolin-7-yl]benzoate | 520.6 |
| 600 | 6-chloro-3-(5-morpholin-4-yl-1H-benzimidazol-2-yl)-4-({[6-(piperidin-3-yloxy)pyridin-3-yl]methyl}amino)quinolin-2(1H)-one | 587.1 |
| 601 | 6-chloro-3-(5-morpholin-4-yl-1H-benzimidazol-2-yl)-4-{[3-(2-oxopyrrolidin-1-yl)propyl]amino}quinolin-2(1H)-one | 488.0 |
| 602 | 6-chloro-3-(5-morpholin-4-yl-1H-benzimidazol-2-yl)-4-[(2-pyridin-2-ylethyl)amino]quinolin-2(1H)-one | 502.0 |
| 603 | 6-chloro-3-(5-morpholin-4-yl-1H-benzimidazol-2-yl)-4-{[3-(2-oxopyrrolidin-1-yl)propyl]amino}quinolin-2(1H)-one | 522.0 |
| 604 | 6-chloro-4-[(6-methoxypyridin-3-yl)amino]-3-(5-morpholin-4-yl-1H-benzimidazol-2-yl)quinolin-2(1H)-one | 504.0 |
| 605 | 6-chloro-3-(5-morpholin-4-yl-1H-benzimidazol-2-yl)-4-[(3-pyridin-2-ylpropyl)amino]quinolin-2(1H)-one | 516.0 |
| 606 | 6-chloro-3-(5-morpholin-4-yl-1H-benzimidazol-2-yl)-4-(pyridin-4-ylamino)quinolin-2(1H)-one | 473.9 |
| 607 | 6-chloro-3-(5-morpholin-4-yl-1H-benzimidazol-2-yl)-4-({[6-(piperidin-3-ylmethoxy)pyridin-3-yl]methyl}amino)quinolin-2(1 H)-one | 601.1 |
| 608 | 6-chloro-3-(5-morpholin-4-yl-1H-benzimidazol-2-yl)-4-(pyridin-2-ylamino)quinolin-2(1H)-one | 473.9 |
| 609 | 1-[4-[(3R)-1-azabicyclo[2.2.2]oct-3-ylamino]-3-(1H-benzimidazol-2-yl)-6-chloro-2-oxo-1,2-dihydroquinolin-7-yl]piperidine-4-carboxylic acid | 548.1 |
| 610 | 1-[4-[(3R)-1-azabicyclo[2.2.2]oct-3-ylamino]-3-(1H-benzimidazol-2-yl)-2-oxo-1,2-dihydroquinolin-7-yl]piperidine-4-carboxylic acid | 513.6 |
| 611 | 3-[4-[(3S)-1-azabicyclo[2.2.2]oct-3-ylamino]-3-(1H-benzimidazol-2-yl)-2-oxo-1,2-dihydroquinolin-7-yl]benzoic acid | 506.6 |
| 612 | 6-chloro-3-(5-morpholin-4-yl-1H-benzimidazol-2-yl)-4-({[2-(piperidin-4-yloxy)pyridin-3-yl]methyl}amino)quinolin-2(1H)-one | 430.5 |
| 613 | 4-[(3S)-1-azabicyclo[2.2.2]oct-3-ylamino]-3-(1H-benzimidazol-2-yl)-6,7-dichloroquinolin-2(1H)-one | 455.4 |
| 614 | 6-chloro-3-(5-morpholin-4-yl-1H-benzimidazol-2-yl)-4-({[2-(piperidin-4-yloxy)pyridin-3-yl]methyl}amino)quinolin-2(1H)-one | 587.1 |
| 615 | 6-chloro-3-(5-morpholin-4-yl-1H-benzimidazol-2-yl)-4-(pyrazin-2-ylamino)quinolin-2(1H)-one | 474.9 |
| 616 | 4-amino-3-(6-thiomorpholin-4-yl-1H-benzimidazol-2-yl)quinolin-2(1H)-one | 378.5 |
| 617 | 4-[(3R)-1-azabicyclo[2.2.2]oct-3-ylamino]-3-(1H-benzimidazol-2-yl)-6-fluoro-7-(3-pyridin-3-ylpyrrolidin-1-yl)quinolin-2(1H)-one | 550.7 |
| 618 | 4-[(3R)-1-azabicyclo[2.2.2]oct-3-ylamino]-3-(1H-benzimidazol-2-yl)-5-fluoro-6-[4-(methylsulfonyl)phenyl]quinolin-2(1H)-one | 558.6 |
| 619 | 6-(4-acetylphenyl)-4-[(3R)-1-azabicyclo[2.2.2]oct-3-ylamino]-3-(1H-benzimidazol-2-yl)-5-fluoroquinolin-2(1H)-one | 522.6 |
| 620 | methyl 4-[4-[(3R)-1-azabicyclo[2.2.2]oct-3-ylamino]-3-(1H-benzimidazol-2-yl)-5-fluoro-2-oxo-1,2-dihydroquinolin-6-yl]benzoate | 538.6 |
| 621 | methyl 3-amino-4-[4-[(3R)-1-azabicyclo[2.2.2]oct-3-ylamino]-3-(1H-benzimidazol-2-yl)-5-fluoro-2-oxo-1,2-dihydroquinolin-6-yl]benzoate | 553.6 |
| 622 | methyl 3-[4-[(3R)-1-azabicyclo[2.2.2]oct-3-ylamino]-3-(1H-benzimidazol-2-yl)-5-fluoro-2-oxo-1,2-dihydroquinolin-6-yl]benzoate | 538.6 |
| 623 | 4-[(3R)-1-azabicyclo[2.2.2]oct-3-ylamino]-3-(1H-benzimidazol-2-yl)-5-fluoro-6-(2-methylphenyl)quinolin-2(1H)-one | 494.6 |
| 624 | 4-[(3R)-1-azabicyclo[2.2.2]oct-3-ylamino]-3-(1H-benzimidazol-2-yl)-6-(2-ethylphenyl)-5-fluoroquinolin-2(1H)-one | 508.6 |
| 625 | 4-[(3R)-1-azabicyclo[2.2.2]oct-3-ylamino]-3-(1H-benzimidazol-2-yl)-5-fluoro-6-(2-methoxyphenyl)quinolin-2(1H)-one | 510.6 |
| 626 | 4-[(3R)-1-azabicyclo[2.2.2]oct-3-ylamino]-3-(1H-benzimidazol-2-yl)-6-(2,4-dichlorophenyl)-5-fluoroquinolin-2(1H)-one | 549.4 |
| 627 | 4-[4-[(3S)-1-azabicyclo[2.2.2]oct-3-ylamino]-3-(1H-benzimidazol-2-yl)-7-fluoro-2-oxo-1,2-dihydroquinolin-6-yl]benzoic acid | 524.6 |
| 628 | 4-[4-[(3S)-1-azabicyclo[2.2.2]oct-3-ylamino]-3-(1H-benzimidazol-2-yl)-7-fluoro-2-oxo-1,2-dihydroquinolin-6-yl]benzamide | 523.6 |
| 629 | N-{3-[4-[(3S)-1-azabicyclo[2.2.2]oct-3-ylamino]-3-(1H-benzimidazol-2-yl)-7-fluoro-2-oxo-1,2-dihydroquinolin-6-yl]phenyl}acetamide | 537.6 |
| 630 | 3-[4-[(3S)-1-azabicyclo[2.2.2]oct-3-ylamino]-3-(1H-benzimidazol-2-yl)-7-fluoro-2-oxo-1,2-dihydroquinolin-6-yl]benzoic acid | 524.6 |
| 631 | 4-[(3S)-1-azabicyclo[2.2.2]oct-3-ylamino]-3-(1H-benzimidazol-2-yl)-7-fluoro-6-(2-methylphenyl)quinolin-2(1H)-one | 494.6 |
| 632 | 4-[(3R)-1-azabicyclo[2.2.2]oct-3-ylamino]-3-(1H-benzimidazol-2-yl)-7-(2-methyl-1H-imidazol-1-yl)-6-[4-(methylsulfonyl)phenyl]quinolin-2(1H)-one | 620.7 |
| 633 | N-{3-[4-[(3R)-1-azabicyclo[2.2.2]oct-3-ylamino]-3-(1H-benzimidazol-2-yl)-7-(2-methyl-1H-imidazol-1-yl)-2-oxo-1,2-dihydroquinolin-6-yl]phenyl}acetamide | 599.7 |
| 634 | N-{3-[4-[(3R)-1-azabicyclo[2.2.2]oct-3-ylamino]-3-(1H-benzimidazol-2-yl)-2-oxo-7-piperidin-1-yl-1,2-dihydroquinolin-6-yl]phenyl}acetamide | 602.8 |
| 635 | N-{3-[7-(3-acetyl-1H-pyrrol-1-yl)-4-[(3R)-1-azabicyclo[2.2.2]oct-3-ylamino]-3-(1H-benzimidazol-2-yl)-2-oxo-1,2-dihydroquinolin-6-yl]phenyl}acetamide | 626.7 |
| 636 | N-{3-[4-[(3R)-1-azabicyclo[2.2.2]oct-3-ylamino]-3-(1H-benzimidazol-2-yl)-7-(dimethylamino)-2-oxo-1,2-dihydroquinolin-6-yl]phenyl}acetamide | 562.7 |
| 637 | N-{3-[4-[(3R)-1-azabicyclo[2.2.2]oct-3-ylamino]-3-(1H-benzimidazol-2-yl)-7-(2-ethyl-1H-imidazol-1-yl)-2-oxo-1,2-dihydroquinolin-6-yl]phenyl}acetamide | 613.7 |
| 638 | 4-[(3R)-1-azabicyclo[2.2.2]oct-3-ylamino]-3-(1H-benzimidazol-2-yl)-7-(2-ethyl-1H-imidazol-1-yl)-6-fluoroquinolin-2(1H)-one | 498.6 |
| 639 | 4-[(3R)-1-azabicyclo[2.2.2]oct-3-ylamino]-3-(1H-benzimidazol-2-yl)-6-fluoro-7-(2-isopropyl-1H-imidazol-1-yl)quinolin-2(1H)-one | 512.6 |
| 640 | 1-[4-[(3R)-1-azabicyclo[2.2.2]oct-3-ylamino]-3-(1H-benzimidazol-2-yl)-6-fluoro-2-oxo-1,2-dihydroquinolin-7-yl]-1H-pyrrole-3-carboxylic acid | 513.5 |
| 641 | 4-[(3S)-1-azabicyclo[2.2.2]oct-3-ylamino]-3-(1H-benzimidazol-2-yl)-7-chloro-6-iodoquinolin-2(1H)-one | 546.8 |
| 642 | 4-[(3R)-1-azabicyclo[2.2.2]oct-3-ylamino]-3-(1H-benzimidazol-2-yl)-5-fluoro-6-iodoquinolin-2(1H)-one | 530.4 |
| 643 | 4-[(3S)-1-azabicyclo[2.2.2]oct-3-ylamino]-3-(1H-benzimidazol-2-yl)-7-fluoro-6-iodoquinolin-2(1H)-one | 530.4 |
| 644 | 6-chloro-3-(5-morpholin-4-yl-1H-benzimidazol-2-yl)-4-[(2-pyridin-3-ylethyl)amino]quinolin-2(1H)-one | 502.0 |
| 645 | 4-{[4-(aminomethyl)benzyl]amino}-3-(1H-benzimidazol-2-yl)-7-chloroquinolin-2(1H)-one | 430.9 |
| 646 | 3-(1H-benzimidazol-2-yl)-7-chloro-4-{[2-(dimethylamino)ethyl]amino}quinolin-2(1H)-one | 382.9 |
| 647 | 3-(1H-benzimidazol-2-yl)-4-(1,4'-bipiperidin-1'-yl)-7-chloroquinolin-2(1H)-one | 463.0 |
| 648 | 3-(1H-benzimidazol-2-yl)-7-chloro-4-{[3-(4-methylpiperazin-1-yl)propyl] amino}quinolin-2(1H)-one | 452.0 |
| 649 | 3-(1H-benzimidazol-2-yl)-7-chloro-4-[(2-piperidin-1-ylethyl)amino]quinolin-2(1H)-one | 422.9 |
| 650 | 3-(1H-benzimidazol-2-yl)-7-chloro-4-{[3-(1H-imidazol-1-yl)propyl] amino}quinolin-2(1H)-one | 419.9 |
| 651 | 3-(1H-benzimidazol-2-yl)-7-chloro-4-(pyridin-3-ylamino)quinolin-2(1H)-one | 388.8 |
| 652 | 3-(1H-benzimidazol-2-yl)-7-chloro-4-(pyridin-4-ylamino)quinolin-2(1H)-one | 388.8 |
| 653 | 3-(1H-benzimidazol-2-yl)-7-chloro-4-({[6-(piperidin-3-yloxy)pyridin-3-yl]methyl}amino)quinolin-2(1H)-one | 502.0 |
| 654 | 3-(1H-benzimidazol-2-yl)-7-chloro-4-{[3-(2-oxopyrrolidin-1-yl)propyl] amino}quinolin-2(1H)-one | 436.9 |
| 655 | 4-[4-[(3R)-1-azabicyclo[2.2.2]oct-3-ylamino]-3-(1H-benzimidazol-2-yl)-7-methoxy-2-oxo-1,2-dihydroquinolin-6-yl]benzoic acid | 536.6 |
| 656 | 4-[4-[(3R)-1-azabicyclo[2.2.2]oct-3-ylamino]-3-(1H-benzimidazol-2-yl)-7-methoxy-2-oxo-1,2-dihydroquinolin-6-yl]benzamide | 535.6 |
| 657 | 6-(4-acetylphenyl)-4-[(3R)-1-azabicyclo[2.2.2]oct-3-ylamino]-3-(1H-benzimidazol-2-yl)-7-methoxyquinolin-2(1H)-one | 534.6 |
| 658 | methyl 4-[4-[(3R)-1-azabicyclo[2.2.2]oct-3-ylamino]-3-(1H-benzimidazol-2-yl)-7-methoxy-2-oxo-1,2-dihydroquinolin-6-yl]benzoate | 550.6 |
| 659 | methyl 3-amino-4-[4-[(3R)-1-azabicyclo[2.2.2]oct-3-ylamino]-3-(1H-benzimidazol-2-yl)-7-methoxy-2-oxo-1,2-dihydroquinolin-6-yl]benzoate | 565.6 |
| 660 | N-{3-[4-[(3R)-1-azabicyclo[2.2.2]oct-3-ylamino]-3-(1H-benzimidazol-2-yl)-7-methoxy-2-oxo-1,2-dihydroquinolin-6-yl]phenyl}acetamide | 549.6 |
| 661 | 6-(3-acetylphenyl)-4-[(3R)-1-azabicyclo[2.2.2]oct-3-ylamino]-3-(1H-benzimidazol-2-yl)-7-methoxyquinolin-2(1H)-one | 534.6 |
| 662 | methyl 3-[4-[(3R)-1-azabicyclo[2.2.2]oct-3-ylamino]-3-(1H-benzimidazol-2-yl)-7-methoxy-2-oxo-1,2-dihydroquinolin-6-yl]benzoate | 550.6 |
| 663 | 3-[4-[(3R)-1-azabicyclo[2.2.2]oct-3-ylamino]-3-(1H-benzimidazol-2-yl)-7-methoxy-2-oxo-1,2-dihydroquinolin-6-yl]benzoic acid | 536.6 |
| 664 | 4-[(3R)-1-azabicyclo[2.2.2]oct-3-ylamino]-3-(1H-benzimidazol-2-yl)-7-methoxy-6-(2-methylphenyl)quinolin-2(1H)-one | 506.6 |
| 665 | 4-[(3R)-1-azabicyclo[2.2.2]oct-3-ylamino]-3-(1H-benzimidazol-2-yl)-6-(2-ethylphenyl)-7-methoxyquinolin-2(1H)-one | 520.6 |
| 666 | 4-[(3R)-1-azabicyclo[2.2.2]oct-3-ylamino]-3-(1H-benzimidazol-2-yl)-7-methoxy-6-(2-methoxyphenyl)quinolin-2(1H)-one | 522.6 |
| 667 | 4-[(3R)-1-azabicyclo[2.2.2]oct-3-ylamino]-3-(1H-benzimidazol-2-yl)-6-(2,4-dichlorophenyl)-7-methoxyquinolin-2(1H)-one | 561.5 |
| 668 | 4-[(3R)-1-azabicyclo[2.2.2]oct-3-ylamino]-3-(1H-benzimidazol-2-yl)-7-[2-(dimethylamino)ethoxy]-6-fluoroquinolin-2(1H)-one | 491.6 |
| 669 | 4-[(3R)-1-azabicyclo[2.2.2]oct-3-ylamino]-3-(1H-benzimidazol-2-yl)-6-fluoro-7-[(2S)-pyrrolidin-2-ylmethoxy]quinolin-2(1H)-one | 503.6 |
| 670 | 4-[(3R)-1-azabicyclo[2.2.2]oct-3-ylamino]-3-(1H-benzimidazol-2-yl)-6-fluoro-7-[2-(2-oxopyrrolidin-1-yl)ethoxy]quinolin-2(1H)-one | 531.6 |
| 671 | 4-[(3R)-1-azabicyclo[2.2.2]oct-3-ylamino]-3-(1H-benzimidazol-2-yl)-6-fluoro-7-{[(2S)-1-(4-nitrophenyl)pyrrolidin-2-yl]methoxy}quinolin-2(1H)-one | 624.7 |
| 672 | 4-[(3R)-1-azabicyclo[2.2.2]oct-3-ylamino]-3-(1H-benzimidazol-2-yl)-6-fluoro-7-[(1-methylpiperidin-2-yl)methoxy]quinolin-2(1H)-one | 531.6 |
| 673 | 3-(1H-benzimidazol-2-yl)-6,7-dimethoxy-4-{[2-(1-methylpyrrolidin-2-yl)ethyl] amino}quinolin-2(1H)-one | 448.5 |
| 674 | 3-(1H-benzimidazol-2-yl)-6,7-dimethoxy-4-{[2-(methylsulfonyl)ethyl] amino}quinolin-2(1H)-one | 443.5 |
| 675 | 3-(1H-benzimidazol-2-yl)-6,7-dimethoxy-4-[(2-morpholin-4-yl-2-pyridin-3-ylethyl)amino]quinolin-2(1H)-one | 527.6 |
| 676 | 7-[(2-aminoethyl)amino]-4-[(3R)-1-azabicyclo[2.2.2]oct-3-ylamino]-3-(1H-benzimidazol-2-yl)-6-fluoroquinolin-2(1H)-one | 462.5 |
| 677 | 4-[(3R)-1-azabicyclo[2.2.2]oct-3-ylamino]-3-(1H-benzimidazol-2-yl)-6-fluoro-7-(3-phenylthiomorpholin-4-yl)quinolin-2(1H)-one | 581.7 |
| 678 | 4-[(3R)-1-azabicyclo[2.2.2]oct-3-ylamino]-3-(1H-benzimidazol-2-yl)-6-fluoro-7-(2-phenylthiomorpholin-4-yl)quinolin-2(1H)-one | 581.7 |
| 679 | 4-[(3R)-1-azabicyclo[2.2.2]oct-3-ylamino]-3-(1H-benzimidazol-2-yl)-6-fluoro-7-{[2-(phenylsulfonyl)ethyl]amino}quinolin-2(1H)-one | 587.7 |
| 680 | 4-[(3R)-1-azabicyclo[2.2.2]oct-3-ylamino]-3-(1H-benzimidazol-2-yl)-6-fluoro-7-{[2-(methylsulfonyl)ethyl]amino}quinolin-2(1H)-one | 525.6 |
| 681 | 7-{[(2R)-2-aminopropyl]amino}-4-[(3R)-1-azabicyclo[2.2.2]oct-3-ylamino]-3-(1H-benzimidazol-2-yl)-6-fluoroquinolin-2(1H)-one | 476.6 |
| 682 | 4-[(3R)-1-azabicyclo[2.2.2]oct-3-ylamino]-3-(1H-benzimidazol-2-yl)-6-fluoro-7-[(2-morpholin-4-yl-2-pyridin-3-ylethyl)amino]quinolin-2(1H)-one | 609.7 |
| 683 | 3-[4-[(3R)-1-azabicyclo[2.2.2]oct-3-ylamino]-3-(1H-benzimidazol-2-yl)-7-fluoro-2-oxo-1,2-dihydroquinolin-6-yl]benzoic acid | 524.6 |
| 684 | 4-[4-[(3S)-1-azabicyclo[2.2.2]oct-3-ylamino]-3-(1H-benzimidazol-2-yl)-7-(1H-imidazol-1-yl)-2-oxo-1,2-dihydroquinolin-6-yl]benzoic acid | 572.6 |
| 685 | 4-[4-[(3S)-1-azabicyclo[2.2.2]oct-3-ylamino]-3-(1H-benzimidazol-2-yl)-7-(2-methyl-1H-imidazol-1-yl)-2-oxo-1,2-dihydroquinolin-6-yl]benzoic acid | 586.7 |
| 686 | 4-[4-[(3S)-1-azabicyclo[2.2.2]oct-3-ylamino]-3-(1H-benzimidazol-2-yl)-2-oxo-7-piperidin-1-yl-1,2-dihydroquinolin-6-yl]benzoic acid | 589.7 |
| 687 | 4-[4-[(3R)-1-azabicyclo[2.2.2]oct-3-ylamino]-3-(1H-benzimidazol-2-yl)-7-(2-ethyl-1H-imidazol-1-yl)-2-oxo-1,2-dihydroquinolin-6-yl]benzoic acid | 600.7 |
| 688 | 3-[4-[(3R)-1-azabicyclo[2.2.2]oct-3-ylamino]-3-(1H-benzimidazol-2-yl)-7-(2-methyl-1H-imidazol-1-yl)-2-oxo-1,2-dihydroquinolin-6-yl]benzoic acid | 586.7 |
| 689 | 3-[4-[(3R)-1-azabicyclo[2.2.2]oct-3-ylamino]-3-(1H-benzimidazol-2-yl)-2-oxo-7-piperidin-1-yl-1,2-dihydroquinolin-6-yl]benzoic acid | 589.7 |
| 690 | 6-chloro-3-[5-(4-methylpiperazin-1-yl)-1H-benzimidazol-2-yl]-4-[(piperidin-3-ylmethyl)amino]quinolin-2(1H)-one | 507.1 |
| 691 | 3-[4-[(3S)-1-azabicyclo[2.2.2]oct-3-ylamino]-3-(1H-benzimidazol-2-yl)-7-(1H-imidazol-1-yl)-2-oxo-1,2-dihydroquinolin-6-yl]benzoic acid | 572.6 |
| 692 | 6-chloro-3-[5-(4-methylpiperazin-1-yl)-1H-benzimidazol-2-yl]-4-[(piperidin-4-ylmethyl)amino]quinolin-2(1H)-one | 507.1 |
| 693 | 3-[4-[(3S)-1-azabicyclo[2.2.2]oct-3-ylamino]-3-(1H-benzimidazol-2-yl)-7-(2-methyl-1H-imidazol-1-yl)-2-oxo-1,2-dihydroquinolin-6-yl]benzoic acid | 586.7 |
| 694 | 6-chloro-3-[5-(4-methylpiperazin-1-yl)-1H-benzimidazol-2-yl]-4-[(pyrrolidin-2-ylmethyl)amino]quinolin-2(1H)-one | 493.0 |
| 695 | 3-[4-[(3S)-1-azabicyclo[2.2.2]oct-3-ylamino]-3-(1H-benzimidazol-2-yl)-2-oxo-7-piperidin-1-yl-1,2-dihydroquinolin-6-yl]benzoic acid | 589.7 |
| 696 | 4-{[(2R)-2-aminobutyl]amino}-6-chloro-3-[5-(4-methylpiperazin-1-yl)-1H-benzimidazol-2-yl]quinolin-2(1H)-one | 481.0 |
| 697 | 4-{[(2S)-2-amino-3-methylbutyl]amino}-6-chloro-3-[5-(4-methylpiperazin-1-yl)-1H-benzimidazol-2-yl]quinolin-2(1H)-one | 495.0 |
| 698 | 4-{[(1S)-2-amino-1-benzylethyl]amino}-6-chloro-3-[5-(4-methylpiperazin-1-yl)-1H-benzimidazol-2-yl]quinolin-2(1H)-one | 543.1 |
| 699 | 4-[(3S)-1-azabicyclo[2.2.2]oct-3-ylamino]-6-chloro-3-[5-(4-methylpiperazin-1-yl)-1H-benzimidazol-2-yl]quinolin-2(1H)-one | 519.1 |
| 700 | 6-chloro-3-[5-(4-methylpiperazin-1-yl)-1H-benzimidazol-2-yl]-4-(piperidin-3-ylamino)quinolin-2(1H)-one | 493.0 |
| 701 | 6-chloro-4-{[2-(dimethylamino)ethyl]amino}-3-[5-(4-methylpiperazin-1-yl)-1H-benzimidazol-2-yl]quinolin-2(1H)-one | 481.0 |
| 702 | 7-chloro-3-(5-morpholin-4-yl-1H-benzimidazol-2-yl)-4-(piperidin-4-ylamino)quinolin-2(1H)-one | 480.0 |
| 703 | 4-{[(1R,2R)-2-aminocyclohexyl]amino}-3-(1H-benzimidazol-2-yl)-7-chloroquinolin-2(1H)-one | 408.9 |
| 704 | 3-(1H-benzimidazol-2-yl)-7-chloro-4-[(3-morpholin-4-ylpropyl)amino]quinolin-2(1H)-one | 438.9 |
| 705 | 3-(1H-benzimidazol-2-yl)-7-chloro-4-[(pyridin-3-ylmethyl)amino]quinolin-2(1H)-one | 402.9 |
| 706 | 3-(1H-benzimidazol-2-yl)-7-chloro-4-[(2-pyridin-3-ylethyl)amino]quinolin-2(1H)-one | 416.9 |
| 707 | 4-{[(1R,2R)-2-aminocyclohexyl]amino}-7-chloro-3-(5-morpholin-4-yl-1H-benzimidazol-2-yl)quinolin-2(1H)-one | 494.0 |
| 708 | 4-[(4-aminocyclohexyl)amino]-7-chloro-3-(5-morpholin-4-yl-1H-benzimidazol-2-yl)quinolin-2(1H)-one | 494.0 |
| 709 | 7-chloro-4-{[2-(methylamino)ethyl]amino}-3-(5-morpholin-4-yl-1H-benzimidazol-2-yl)quinolin-2(1H)-one | 453.9 |
| 710 | 7-chloro-3-(5-morpholin-4-yl-1H-benzimidazol-2-yl)-4-[(pyrrolidin-2-ylmethyl)amino]quinolin-2(1H)-one | 480.0 |
| 711 | 4-{[(1S)-2-amino-1-benzylethyl]amino}-7-chloro-3-(5-morpholin-4-yl-1H-benzimidazol-2-yl)quinolin-2(1H)-one | 530.0 |
| 712 | 7-chloro-3-(5-morpholin-4-yl-1H-benzimidazol-2-yl)-4-(pyrrolidin-3-ylamino)quinolin-2(1H)-one | 466.0 |
| 713 | 3-(1H-benzimidazol-2-yl)-7-chloro-4-[(2-pyrrolidin-1-ylethyl)amino]quinolin-2(1H)-one | 408.9 |
| 714 | 3-(1H-benzimidazol-2-yl)-7-chloro-4-[(2-piperidin-2-ylethyl)amino]quinolin-2(1H)-one | 422.9 |
| 715 | 3-(1H-benzimidazol-2-yl)-7-chloro-4-[(piperidin-3-ylmethyl)amino]quinolin-2(1H)-one | 408.9 |
| 716 | 3-(1H-benzimidazol-2-yl)-7-chloro-4-[(piperidin-4-ylmethyl)amino]quinolin-2(1H)-one | 408.9 |
| 717 | 3-(1H-benzimidazol-2-yl)-7-chloro-4-{[(2-methyl-1-piperidin-4-yl-1H-benzimidazol-5-yl)methyl]amino}quinolin-2(1H)-one | 539.1 |
| 718 | 4-[(4-aminocyclohexyl)amino]-3-(1H-benzimidazol-2-yl)-7-chloroquinolin-2(1H)-one | 408.9 |
| 719 | 3-(1H-benzimidazol-2-yl)-7-chloro-4-(pyrrolidin-3-ylamino)quinolin-2(1H)-one | 380.8 |
| 720 | 4-[(3S)-1-azabicyclo[2.2.2]oct-3-ylamino]-3-(1H-benzimidazol-2-yl)-6-[4-(trifluoromethyl)phenyl]quinolin-2(1H)-one | 530.6 |
| 721 | 4-[(3S)-1-azabicyclo[2.2.2]oct-3-ylamino]-3-(1H-benzimidazol-2-yl)-6-[3-(trifluoromethyl)phenyl]quinolin-2(1H)-one | 530.6 |
| 722 | 4-amino-5-fluoro-3-[6-(4-isopropylpiperazin-1-yl)-1H-benzimidazol-2-yl]quinolin-2(1H)-one | 421.5 |
| 723 | 7-chloro-3-(5-morpholin-4-yl-1H-benzimidazol-2-yl)-4-{[(2S)-pyirolidin-2-ylmethyl]amino}quinolin-2(1H)-one | 480.0 |
| 724 | 7-chloro-3-(5-morpholin-4-yl-1H-benzimidazol-2-yl)-4-{[(2R)-pyirolidin-2-ylmethyl]amino}quinolin-2(1H)-one | 480.0 |
| 725 | 7-chloro-4-({[(2S)-1-ethylpyrrolidin-2-yl]methyl}amino)-3-(5-morpholin-4-yl-1H-benzimidazol-2-yl)quinolin-2(1H)-one | 508.0 |
| 726 | 7-chloro-4-({[(2R)-1-ethylpyrrolidin-2-yl]methyl}amino)-3-(5-morpholin-4-yl-1H-benzimidazol-2-yl)quinolin-2(1H)-one | 508.0 |
| 727 | 4-[(3R)-1-azabicyclo[2.2.2]oct-3-ylamino]-7-chloro-3-(5-morpholin-4-yl-1H-benzimidazol-2-yl)quinolin-2(1H)-one | 506.0 |
| 728 | 7-chloro-3-(5-morpholin-4-yl-1H-benzimidazol-2-yl)-4-[(piperidin-3-ylmethyl)amino]quinolin-2(1H)-one | 494.0 |
| 729 | 7-chloro-3-(5-morpholin-4-yl-1H-benzimidazol-2-yl)-4-[(piperidin-4-ylmethyl)amino]quinolin-2(1H)-one | 494.0 |
| 730 | 4-{[(2S)-2-amino-3-methylbutyl]amino}-7-chloro-3-(5-morpholin-4-yl-1H-benzimidazol-2-yl)quinolin-2(1H)-one | 482.0 |
| 731 | 4-{[4-(aminomethyl)benzyl]amino}-7-chloro-3-(5-morpholin-4-yl-1H-benzimidazol-2-yl)quinolin-2(1H)-one | 516.0 |
| 732 | 4-{[(1R)-1-(aminomethyl)propyl]amino}-7-chloro-3-(5-morpholin-4-yl-1H-benzimidazol-2-yl)quinolin-2(1H)-one | 468.0 |
| 733 | 7-chloro-4-{[3-(4-methylpiperazin-1-yl)propyl]amino}-3-(5-morpholin-4-yl-1H-benzimidazol-2-yl)quinolin-2(1H)-one | 537.1 |
| 734 | 7-chloro-4-{[3-(1H-imidazol-1-yl)propyl]amino}-3-(5-morpholin-4-yl-1H-benzimidazol-2-yl)quinolin-2(1H)-one | 505.0 |
| 735 | 7-chloro-3-(5-morpholin-4-yl-1H-benzimidazol-2-yl)-4-[(2-pyrrolidin-1-ylethyl)amino]quinolin-2(1H)-one | 494.0 |
| 736 | 7-chloro-3-(5-morpholin-4-yl-1H-benzimidazol-2-yl)-4-[(piperidin-2-ylmethyl)amino]quinolin-2(1H)-one | 494.0 |
| 737 | 7-chloro-4-{[2-(dimethylamino)ethyl]amino}-3-(5-morpholin-4-yl-1H-benzimidazol-2-yl)quinolin-2(1H)-one | 468.0 |
| 738 | 7-chloro-3-(5-morpholin-4-yl-1H-benzimidazol-2-yl)-4-[(3S)-pyrrolidin-3-ylamino]quinolin-2(1H)-one | 466.0 |
| 739 | 4-[(3S)-1-azabicyclo[2.2.2]oct-3-ylamino]-3-(1H-benzimidazol-2-yl)-6-(4-hydroxyphenyl)quinolin-2(1H)-one | 478.6 |
| 740 | 4-[(3S)-1-azabicyclo[2.2.2]oct-3-ylamino]-3-(1H-benzimidazol-2-yl)-6-(3-hydroxyphenyl)quinolin-2(1H)-one | 478.6 |
| 741 | 4-[(3S)-1-azabicyclo[2.2.2]oct-3-ylamino]-3-(1H-benzimidazol-2-yl)-6-(2-hydroxyphenyl)quinolin-2(1H)-one | 478.6 |
| 742 | 3-(1H-benzimidazol-2-yl)-7-chloro-4-{[(2S)-pyrrolidin-2-ylmethyl]amino}quinolin-2(1H)-one | 394.9 |
| 743 | 3-(1H-benzimidazol-2-yl)-7-chloro-4-({[(2S)-1-ethylpyrrolidin-2-yl]methyl}amino)quinolin-2(1H)-one | 422.9 |
| 744 | 3-(1H-benzimidazol-2-yl)-7-chloro-4-({[(2R)-1-ethylpyrrolidin-2-yl]methyl}amino)quinolin-2(1H)-one | 422.9 |
| 745 | 3-(1H-benzimidazol-2-yl)-7-chloro-4-[(3S)-pyrrolidin-3-ylamino]quinolin-2(1H)-one | 380.8 |
| 746 | 3-(1H-benzimidazol-2-yl)-6-chloro-4-{[(2S)-pyrrolidin-2-ylmethyl]amino}quinolin-2(1H)-one | 394.9 |
| 747 | 3-(1H-benzimidazol-2-yl)-6-chloro-4-{[(2R)-pyrrolidin-2-ylmethyl]amino}quinolin-2(1H)-one | 394.9 |
| 748 | 3-(1H-benzimidazol-2-yl)-6-chloro-4-({[(2S)-1-ethylpyrrolidin-2-yl]methyl}amino)quinolin-2(1H)-one | 422.9 |
| 749 | 3-(1H-benzimidazol-2-yl)-6-chloro-4-({[(2R)-1-ethylpyrrolidin-2-yl]methyl}amino)quinolin-2(1H)-one | 422.9 |
| 750 | 4-amino-3-[5-(1,4'-bipiperidin-1'-ylcarbonyl)-1H-benzimidazol-2-yl]quinolin-2(1H)-one | 380.8 |
| 751 | 4-[(3R)-1-azabicyclo[2.2.2]oct-3-ylamino]-7-bromo-3-(5-morpholin-4-yl-1H-benzimidazol-2-yl)quinolin-2(1H)-one | 550.5 |
| 752 | 4-[(3R)-1-azabicyclo[2.2.2]oct-3-ylamino]-7-bromo-3-(6-methoxy-1H-benzimidazol-2-yl)quinolin-2(1H)-one | 495.4 |
| 753 | 3-{[3-(1H-benzimidazol-2-yl)-6,7-dimethoxy-2-oxo-1,2-dihydroquinolin-4-yl]amino}bicyclo[2.2.1]heptane-2-carboxamide | 474.5 |
| 754 | 4-[(3-amino-2,2-dimethylpropyl)amino]-3-(1H-benzimidazol-2-yl)-6,7-dimethoxyquinolin-2(1H)-one | 422.5 |
| 755 | 3-(1H-benzimidazol-2-yl)-4-{[3-(dimethylamino)-2,2-dimethylpropyl]amino}-6,7-dimethoxyquinolin-2(1H)-one | 450.6 |
| 756 | 3-(1H-benzimidazol-2-yl)-7-chloro-4-[(pyridin-2-ylmethyl)amino]quinolin-2(1H)-one | 402.9 |
| 757 | 3-(1H-benzimidazol-2-yl)-7-chloro-4-[(2-pyridin-2-ylethyl)amino]quinolin-2(1H)-one | 416.9 |
| 758 | 3-(1H-benzimidazol-2-yl)-7-chloro-4-{[2-(methylamino)ethyl]amino}quinolin-2(1H)-one | 368.8 |
| 759 | 3-(1H-benzimidazol-2-yl)-7-chloro-4-[(piperidin-2-ylmethyl)amino]quinolin-2(1H)-one | 408.9 |
| 760 | 3-(1H-benzimidazol-2-yl)-7-chloro-4-(piperidin-4-ylamino)quinolin-2(1H)-one | 394.9 |
| 761 | 4-amino-3-[5-(1,4'-bipiperidin-1'-ylcarbonyl)-1H-benzimidazol-2-yl]quinolin-2(1H)-one | 471.6 |
| 762 | 4-amino-3-{5-[(3S)-3-(dimethylnitroryl)pyrrolidin-1-yl]-1H-benzimidazol-2-yl}quinolin-2(1H)-one | 405.5 |
| 763 | 4-amino-3-(5-{2-[(dimethylamino)methyl]morpholin-4-yl}-1H-benzimidazol-2-yl)quinolin-2(1H)-one | 419.5 |
| 764 | methyl 4-[4-[(3R)-1-azabicyclo[2.2.2]oct-3-ylamino]-3-(1H-benzimidazol-2-yl)-5-methyl-2-oxo-1,2-dihydroquinolin-6-yl]benzoate | 534.6 |
| 765 | 3-[4-[(3R)-1-azabicyclo[2.2.2]oct-3-ylamino]-3-(1H-benzimidazol-2-yl)-5-methyl-2-oxo-1,2-dihydroquinolin-6-yl]benzoic acid | 520.6 |
| 766 | 4-[4-[(3R)-1-azabicyclo[2.2.2]oct-3-ylamino]-3-(1H-benzimidazol-2-yl)-5-methyl-2-oxo-1,2-dihydroquinolin-6-yl]benzamide | 519.6 |
| 767 | 4-[4-[(3R)-1-azabicyclo[2.2.2]oct-3-ylamino]-3-(1H-benzimidazol-2-yl)-5-methyl-2-oxo-1,2-dihydroquinolin-6-yl]benzoic acid | 520.6 |
| 768 | 4-amino-3-{5-[(2S)-2-(pyrrolidin-1-ylmethyl)pyrrolidin-1-yl]-1H-benzimidazol-2-yl}quinolin-2(1H)-one | 429.5 |
| 769 | 2-(4-amino-5-fluoro-2-oxo-1,2-dihydroquinolin-3-yl)-N-methyl-N-(1-methylpiperidin-4-yl)-1H-benzimidazole-6-carboxamide | 449.5 |
| 770 | 4-amino-3-(1H-benzimidazol-2-yl)-5-[(1-methylpiperidin-4-yl)oxy]quinolin-2(1H)-one | 390.5 |
| 771 | 4-amino-5-(1-azabicyclo[2.2.2]oct-3-yloxy)-3-(1H-benzimidazol-2-yl)quinolin-2(1 H)-one | 402.5 |
| 772 | 4-amino-5-fluoro-3-{6-[(2-piperidin-1-ylethyl)amino]-1H-benzimidazol-2-yl}quinolin-2(1H)-one | 421.5 |
| 773 | 4,6-diamino-3-[6-(4-methylpiperazin-1-yl)-1H-benzimidazol-2-yl]quinolin-2(1H)-one | 390.5 |
| 774 | 2-(4-amino-5-fluoro-2-oxo-1,2-dihydroquinolin-3-yl)-1H-benzimidazole-5-carboxylic acid | 339.3 |
| 775 | 2-(4-amino-2-oxo-1,2-dihydroquinolin-3-yl)-N-pyridin-3-yl-1H-benzimidazole-5-carboxamide | 397.4 |
| 776 | 4-amino-3-(5-{[(3R)-3-hydroxypyrrolidin-1-yl]carbonyl}-1H-benzimidazol-2-yl)quinolin-2(1H)-one | 390.4 |
| 777 | N-{4-amino-3-[6-(4-methylpiperazin-1-yl)-1H-benzimidazol-2-yl]-2-oxo-1,2-dihydroquinolin-6-yl}acetamide | 432.5 |
| 778 | 4-amino-5-fluoro-3-(6-morpholin-4-yl-1H-benzimidazol-2-yl)quinolin-2(1H)-one | 380.4 |
| 779 | 3-(5-chloro-1H-benzimidazol-2-yl)-4-{[2-(dimethylamino)ethyl]amino}-6-methylquinolin-2(1H)-one | 396.9 |
| 780 | 4-{[(1R,2R)-2-aminocyclohexyl]amino}-3-(5-chloro-1H-benzimidazol-2-yl)-6-methylquinolin-2(1H)-one | 422.9 |
| 781 | 3-(5-chloro-1H-benzimidazol-2-yl)-6-methyl-4-[(piperidin-3-ylmethyl)amino]quinolin-2(1H)-one | 422.9 |
| 782 | 3-(5-chloro-1H-benzimidazol-2-yl)-6-methyl-4-[(piperidin-4-ylmethyl)amino]quinolin-2(1H)-one | 422.9 |
| 783 | 4-[(4-aminocyclohexyl)amino]-3-(5-chloro-1H-benzimidazol-2-yl)-6-methylquinolin-2(1H)-one | 422.9 |
| 784 | 3-(5-chloro-1H-benzimidazol-2-yl)-6-methyl-4-{[2-(methylamino)ethyl]amino}quinolin-2(1H)-one | 382.9 |
| 785 | 3-(5-chloro-1H-benzimidazol-2-yl)-6-methyl-4-(pyrrolidin-3-ylamino)quinolin-2(1H)-one | 394.9 |
| 786 | 3-(5-chloro-1H-benzimidazol-2-yl)-6-methyl-4-[(piperidin-2-ylmethyl)amino]quinolin-2(1H)-one | 422.9 |
| 787 | 4-[(3S)-1-azabicyclo[2.2.2]oct-3-ylamino]-3-(5-chloro-1H-benzimidazol-2-yl)-6-methylquinolin-2(1H)-one | 434.9 |
| 788 | 4-[(3R)-1-azabicyclo[2.2.2]oct-3-ylamino]-3-(5-chloro-1H-benzimidazol-2-yl)-6-methylquinolin-2(1H)-one | 434.9 |
| 789 | 4-amino-3-(6-{(2R,5R)-2-[(dimethylamino)methyl]-5-methylmorpholin-4-yl}-1H-benzimidazol-2-yl)quinolin-2(1H)-one | 433.5 |
| 790 | 4-amino-3-(5-{[(3R)-3-hydroxypiperidin-1-yl]carbonyl}-1H-benzimidazol-2-yl)quinolin-2(1H)-one | 404.4 |
| 791 | 2-(4-amino-2-oxo-1,2-dihydroquinolin-3-yl)-N-(2-piperidin-1-ylethyl)-1H-benzimidazole-5-carboxamide | 431.5 |
| 792 | 4-amino-3-[5-(piperazin-1-ylcarbonyl)-1H-benzimidazol-2-yl]quinolin-2(1H)-one | 389.4 |
| 793 | N-{4-amino-3-[6-(4-methylpiperazin-1-yl)-1H-benzimidazol-2-yl]-2-oxo-1,2-dihydroquinolin-6-yl}-2,2-dimethylpropanamide | 474.6 |
| 794 | N-{4-amino-3-[6-(4-methylpiperazin-1-yl)-1H-benzimidazol-2-yl]-2-oxo-1,2-dihydroquinolin-6-yl}-3-phenylpropanamide | 522.6 |
| 795 | N-{4-amino-3-[6-(4-methylpiperazin-1-yl)-1H-benzimidazol-2-yl]-2-oxo-1,2-dihydroquinolin-6-yl}-2-(benzyloxy)acetamide | 538.6 |
| 796 | N-{4-amino-3-[6-(4-methylpiperazin-1-yl)-1H-benzimidazol-2-yl]-2-oxo-1,2-dihydroquinolin-6-yl}-2-thien-2-ylacetamide | 514.6 |
| 797 | N-{4-amino-3-[6-(4-methylpiperazin-1-yl)-1H-benzimidazol-2-yl]-2-oxo-1,2-dihydroquinolin-6-yl}-2-furamide | 484.5 |
| 798 | 2-(4-amino-2-oxo-1,2-dihydroquinolin-3-yl)-N-(2-pyrrolidin-1-ylethyl)-1H-benzimidazole-5-carboxamide | 417.5 |
| 799 | ethyl (4-{[2-(4-amino-2-oxo-1,2-dihydroquinolin-3-yl)-1H-benzimidazol-5-yl]carbonyl}piperazin-1-yl)acetate | 475.5 |
| 800 | N-{4-amino-3-[6-(4-methylpiperazin-1-yl)-1H-benzimidazol-2-yl]-2-oxo-1,2-dihydroquinolin-6-yl}-N'-phenylurea | 509.6 |
| 801 | N-{4-amino-3-[6-(4-methylpiperazin-1-yl)-1H-benzimidazol-2-yl]-2-oxo-1,2-dihydroquinolin-6-yl}-N'-benzylurea | 523.6 |
| 802 | N-{4-amino-3-[6-(4-methylpiperazin-1-yl)-1H-benzimidazol-2-yl]-2-oxo-1,2-dihydroquinolin-6-yl}-N'-(2-phenylethyl)urea | 537.6 |
| 803 | N-{4-amino-3-[6-(4-methylpiperazin-1-yl)-1H-benzimidazol-2-yl]-2-oxo-1,2-dihydroquinolin-6-yl}benzamide | 494.6 |
| 804 | 2-(4-amino-2-oxo-1,2-dihydroquinolin-3-yl)-N-piperidin-3-yl-1H-benzimidazole-5-carboxamide | 403.5 |
| 805 | 2-(4-amino-2-oxo-1,2-dihydroquinolin-3-yl)-N-[(3R)-1-azabicyclo[2.2.2]oct-3-yl]-1H-benzimidazole-6-carboxamide | 429.5 |
| 806 | 2-(4-amino-2-oxo-1,2-dihydroquinolin-3-yl)-N-[2-(diethylamino)ethyl]-N-ethyl-1H-benzimidazole-5-carboxamide | 447.6 |
| 807 | 4-amino-3-[6-(pyridin-4-yloxy)-1H-benzimidazol-2-yl]quinolin-2(1H)-one | 370.4 |
| 808 | 4-amino-5-fluoro-3-{6-[(4-methylpiperazin-1-yl)carbonyl]-1H-benzimidazol-2-yl}quinolin-2(1H)-one | 421.4 |
| 809 | 4-amino-5-fluoro-3-{6-[(4-isopropylpiperazin-1-yl)carbonyl]-1H-benzimidazol-2-yl}quinolin-2(1H)-one | 449.5 |
| 810 | 4-amino-3-{6-[(4-cyclohexylpiperazin-1-yl)carbonyl]-1H-benzimidazol-2-yl}-5-fluoroquinolin-2(1H)-one | 489.6 |
| 811 | 4-amino-6-(isobutylamino)-3-[6-(4-methylpiperazin-1-yl)-1H-benzimidazol-2-yl]quinolin-2(1H)-one | 446.6 |
| 812 | 2-(4-amino-5-fluoro-2-oxo-1,2-dihydroquinolin-3-yl)-N-methyl-N-(1-methylpyrrolidin-3-yl)-1H-benzimidazole-6-carboxamide | 488.6 |
| 813 | 4-amino-6-[(2-methylbutyl)amino]-3-[6-(4-methylpiperazin-1-yl)-1H-benzimidazol-2-yl]quinolin-2(1H)-one | 460.6 |
| 814 | 4-amino-6-[(cyclohexylmethyl)amino]-3-[6-(4-methylpiperazin-1-yl)-1H-benzimidazol-2-yl]quinolin-2(1H)-one | 486.6 |
| 815 | 4-amino-3-(6-{[(3S)-3-methylpiperazin-1-yl]carbonyl}-1H-benzimidazol-2-yl)quinolin-2(1H)-one | 403.5 |
| 816 | 2-(4-amino-2-oxo-1,2-dihydroquinolin-3-yl)-N-[(3S)-1-azabicyclo[2.2.2]oct-3-yl]-1H-benzimidazole-6-carboxamide | 429.5 |
| 817 | 4-amino-3-[6-(1,4'-bipiperidin-1'-ylcarbonyl)-1H-benzimidazol-2-yl]-5-fluoroquinolin-2(1H)-one | 489.6 |
| 818 | 2-(4-amino-5-fluoro-2-oxo-1,2-dihydroquinolin-3-yl)-N-methyl-N-(1-methylpyrrolidin-3-yl)-1H-benzimidazole-6-carboxamide | 435.5 |
| 819 | 4-amino-3-(1H-benzimidazol-2-yl)-5-[(4-methoxyphenyl)thio]quinolin-2(1H)-one | 415.5 |
| 820 | 4-amino-3-(1H-benzimidazol-2-yl)-5-[(4-methoxyphenyl)sulfonyl]quinolin-2(1H)-one | 447.5 |
| 821 | 4-amino-3-(1H-benzimidazol-2-yl)-5-[(2-methoxyphenyl)thio]quinolin-2(1H)-one | 415.5 |
| 822 | N-(4-{[2-(4-amino-2-oxo-1,2-dihydroquinolin-3-yl)-1H-benzimidazol-5-yl]oxy}phenyl)acetamide | 426.4 |
| 823 | 4-amino-6-(benzylamino)-3-[6-(4-methylpiperazin-1-yl)-1H-benzimidazol-2-yl]quinolin-2(1H)-one | 480.6 |
| 824 | 4-amino-3-[6-(4-methylpiperazin-1-yl)-1H-benzimidazol-2-yl]-6-{[(3-phenoxythien-2-yl)methyl]amino}quinolin-2(H)-one | 578.7 |
| 825 | 4-amino-3-[6-(4-methylpiperazin-1-yl)-1H-benzimidazol-2-yl]-6-{[(3-methylthien-2-yl)methyl]amino}quinolin-2(H)-one | 500.6 |
| 826 | 4-amino-3-[6-(4-methylpiperazin-1-yl)-1H-benzimidazol-2-yl]-6-[(1,3-thiazol-2-ylmethyl)amino]quinolin-2(1H)-one | 487.6 |
| 827 | 4-amino-3-[6-(4-methylpiperazin-1-yl)-1H-benzimidazol-2-yl]-6-[(pyrazin-2-ylmethyl)amino]quinolin-2(1H)-one | 482.6 |
| 828 | 4-amino-3-(5-{2-[(dimethylamino)methyl]-1,4-oxazepan-4-yl}-1H-benzimidazol-2-yl)-5-fluoroquinolin-2(1H)-one | 433.5 |
| 829 | 4-amino-3-(5-{2-[(dimethylamino)methyl]-1,4-oxazepan-4-yl}-1H-benzimidazol-2-yl)-5-fluoroquinolin-2(1H)-one | 451.5 |
| 830 | 6-chloro-4-{[2-(dimethylamino)-2-pyridin-3-ylethyl]amino}-3-(5-morpholin-4-yl-1H-benzimidazol-2-yl)quinolin-2(1H)-one | 545.1 |
| 831 | 6-amino-4-[(3S)-1-azabicyclo[2.2.2]oct-3-ylamino]-3-(1H-benzimidazol-2-yl)quinolin-2(1H)-one | 401.5 |
| 832 | 6-chloro-3-(5-chloro-1H-benzimidazol-2-yl)-4-{[2-(dimethylamino)ethyl]amino}quinolin-2(1H)-one | 417.3 |
| 833 | 4-{[(1R,2R)-2-aminocyclohexyl]amino}-6-chloro-3-(5-chloro-1H-benzimidazol-2-yl)quinolin-2(1H)-one | 443.3 |
| 834 | 6-chloro-3-(5-chloro-1H-benzimidazol-2-yl)-4-[(piperidin-3-ylmethyl)amino]quinolin-2(1H)-one | 443.3 |
| 835 | 6-chloro-3-(5-chloro-1H-benzimidazol-2-yl)-4-[(piperidin-4-ylmethyl)amino]quinolin-2(1H)-one | 443.3 |
| 836 | 4-[(4-aminocyclohexyl)amino]-6-chloro-3-(5-chloro-1H-benzimidazol-2-yl)quinolin-2(1H)-one | 443.3 |
| 837 | 6-chloro-3-(5-chloro-1H-benzimidazol-2-yl)-4-{[2-(methylamino)ethyl]amino}quinolin-2(1H)-one | 403.3 |
| 838 | 6-chloro-3-(5-chloro-1H-benzimidazol-2-yl)-4-(pyrrolidin-3-ylamino)quinolin-2(1H)-one | 415.3 |
| 839 | 6-chloro-3-(5-chloro-1H-benzimidazol-2-yl)-4-[(piperidin-2-ylmethyl)amino]quinolin-2(1H)-one | 443.3 |
| 840 | 4-[(3S)-1-azabicyclo[2.2.2]oct-3-ylamino]-6-chloro-3-(5-chloro-1H-benzimidazol-2-yl)quinolin-2(1H)-one | 455.4 |
| 841 | 4-[(3R)-1-azabicyclo[2.2.2]oct-3-ylamino]-6-chloro-3-(5-chloro-1H-benzimidazol-2-yl)quinolin-2(1H)-one | 455.4 |
| 842 | 4-amino-3-[6-(4-methylpiperazin-1-yl)-1H-benzimidazol-2-yl]-6-{[(2S)-pyrrolidin-2-ylmethyl]amino}quinolin-2(1H)-one | 473.6 |
| 843 | 4-amino-6-{[(5-methylisoxazol-3-yl)methyl]amino}-3-[6-(4-methylpiperazin-1-yl)-1H-benzimidazol-2-yl]quinolin-2(1H)-one | 485.6 |
| 844 | 4-amino-3-(5-{(2S,5R)-2-[(dimethylamino)methyl]-5-methylmorpholin-4-yl}-1H-benzimidazol-2-yl)quinolin-2(1H)-one | 433.5 |
| 845 | 3-(5-chloro-1H-benzimidazol-2-yl)-4-{[2-(dimethylamino)ethyl]amino}-6,7-difluoroquinolin-2(1H)-one | 418.8 |
| 846 | 4-{[(1R,2R)-2-aminocyclohexyl]amino}-3-(5-chloro-1H-benzimidazol-2-yl)-6,7-difluoroquinolin-2(1H)-one | 444.9 |
| 847 | 3-(5-chloro-1H-benzimidazol-2-yl)-6,7-difluoro-4-[(piperidin-3-ylmethyl)amino]quinolin-2(1H)-one | 444.9 |
| 848 | 3-(5-chloro-1H-benzimidazol-2-yl)-6,7-difluoro-4-[(piperidin-4-ylmethyl)amino]quinolin-2(1H)-one | 444.9 |
| 849 | 4-[(4-aminocyclohexyl)amino]-3-(5-chloro-1H-benzimidazol-2-yl)-6,7-difluoroquinolin-2(1H)-one | 444.9 |
| 850 | 3-(5-chloro-1H-benzimidazol-2-yl)-6,7-difluoro-4-{[2-(methylamino)ethyl]amino}quinolin-2(1H)-one | 404.8 |
| 851 | 3-(5-chloro-1H-benzimidazol-2-yl)-6,7-difluoro-4-(pyrrolidin-3-ylamino)quinolin-2(1H)-one | 416.8 |
| 852 | 3-(5-chloro-1H-benzimidazol-2-yl)-6,7-difluoro-4-[(piperidin-2-ylmethyl)amino]quinolin-2(1H)-one | 444.9 |
| 853 | 4-[(3S)-1-azabicyclo[2.2.2]oct-3-ylamino]-3-(5-chloro-1H-benzimidazol-2-yl)-6,7-difluoroquinolin-2(1H)-one | 456.9 |
| 854 | 4-[(3R)-1-azabicyclo[2.2.2]oct-3-ylamino]-3-(5-chloro-1H-benzimidazol-2-yl)-6,7-difluoroquinolin-2(1H)-one | 456.9 |
| 855 | 4-amino-3-(6-{[(3R)-3-methylpiperazin-1-yl]carbonyl}-1H-benzimidazol-2-yl)quinolin-2(1H)-one | 403.5 |
| 856 | 4-amino-3-(5-{[(3S)-3-hydroxypyrrolidin-1-yl]carbonyl}-1H-benzimidazol-2-yl)quinolin-2(1H)-one | 390.4 |
| 857 | 4-amino-3-(5-{[4-(2-hydroxyethyl)piperazin-1-yl]carbonyl}-1H-benzimidazol-2-yl)quinolin-2(1H)-one | 433.5 |
| 858 | 4-amino-3-[6-(4-isopropylpiperazin-1-yl)-1H-benzimidazol-2-yl]-5-methoxyquinolin-2(1H)-one | 433.5 |
| 859 | 4-amino-3-(5-{3-[(dimethylamino)methyl]pyrrolidin-1-yl}-1H-benzimidazol-2-yl)quinolin-2(1H)-one | 403.5 |
| 860 | 4-amino-3-(5-{3-[(dimethylamino)methyl]pyrrolidin-1-yl}-1H-benzimidazol-2-yl)-5-fluoroquinolin-2(1H)-one | 421.5 |
| 861 | 4-amino-3-(6-{(2R,5S)-2-[(dimethylamino)methyl]-5-methylmorpholin-4-yl}-1H-benzimidazol-2-yl)quinolin-2(1H)-one | 433.5 |
| 862 | 4-amino-3-[6-(4-methylpiperazin-1-yl)-1H-benzimidazol-2-yl]-6-(piperidin-4-ylamino)quinolin-2(1H)-one | 473.6 |
| 863 | 6-chloro-3-[5-(4-methylpiperazin-1-yl)-1H-benzimidazol-2-yl]-4-[(3S)-pyrrolidin-3-ylamino]quinolin-2(1H)-one | 479.0 |
| 864 | 4-amino-3-{5-[(3R)-3-(dimethylamino)pyrrolidin-1-yl]-1H-benzimidazol-2-yl}-5-fluoroquinolin-2(1H)-one | 407.5 |
| 865 | 4-amino-3-{5-[(3S)-3-(dimethylamino)pyrrolidin-1-yl]-1H-benzimidazol-2-yl}-5-fluoroquinolin-2(1H)-one | 407.5 |
| 866 | 4-amino-3-[6-(2,6-dimethylmorpholin-4-yl)-1H-benzimidazol-2-yl]-5-fluoroquinolin-2(1H)-one | 408.4 |
| 867 | 4-amino-3-{6-[(3-aminopyrrolidin-1-yl)carbonyl]-1H-benzimidazol-2-yl}quinolin-2(1H)-one | 389.4 |
| 868 | ethyl (3S,4R)-4-({[2-(4-amino-2-oxo-1,2-dihydroquinolin-3-yl)-1H-benzimidazol-6-yl]carbonyl}amino)-3-methoxypiperidine-1-carboxylate | 505.5 |
| 869 | 6-amino-3-(1H-benzimidazol-2-yl)-4-[(3S)-pyrrolidin-3-ylamino]quinolin-2(1H)-one | 361.4 |
| 870 | 4-amino-3-(6-{(2R,5S)-2-[(dimethylamino)methyl]-5-methylmorpholin-4-yl}-1H-benzimidazol-2-yl)-5-fluoroquinolin-2(1H)-one | 451.5 |
| 871 | N-{(3S)-1-[2-(4-amino-2-oxo-1,2-dihydroquinolin-3-yl)-1H-benzimidazol-6-yl]pyrrolidin-3-yl}-N-methylacetamide | 417.5 |
| 872 | 2-(4-amino-2-oxo-1,2-dihydroquinolin-3-yl)-N-piperidin-4-yl-1H-benzimidazole-6-carboxamide | 403.5 |
| 873 | 2-(4-amino-2-oxo-1,2-dihydroquinolin-3-yl)-N-[2-(1-methylpyrrolidin-2-yl)ethyl]-1H-benzimidazole-6-carboxamide | 431.5 |
| 874 | N-{4-amino-3-[6-(4-methylpiperazin-1-yl)-1H-benzimidazol-2-yl]-2-oxo-1,2-dihydroquinolin-6-yl}-N'-isopropylurea | 475.6 |
| 875 | N-{4-amino-3-[6-(4-methylpiperazin-1-yl)-1H-benzimidazol-2-yl]-2-oxo-1,2-dihydroquinolin-6-yl}-N'-(3,5-dimethylphenyl)urea | 537.6 |
| 876 | N-allyl-N'-{4-amino-3-[6-(4-methylpiperazin-1-yl)-1H-benzimidazol-2-yl]-2-oxo-1,2-dihydroquinolin-6-yl}urea | 473.6 |
| 877 | N-{4-amino-3-[6-(4-methylpiperazin-1-yl)-1H-benzimidazol-2-yl]-2-oxo-1,2-dihydroquinolin-6-yl}-N'-(tert-butyl)urea | 489.6 |
| 878 | N-{4-amino-3-[6-(4-methylpiperazin-1-yl)-1H-benzimidazol-2-yl]-2-oxo-1,2-dihydroquinolin-6-yl}-N'-[2-(methylthio)phenyl]urea | 555.7 |
| 879 | N-{4-amino-3-[6-(4-methylpiperazin-1-yl)-1H-benzimidazol-2-yl]-2-oxo-1,2-dihydroquinolin-6-yl}heptanamide | 502.6 |
| 880 | 4-amino-3-[6-(4-methylpiperazin-1-yl)-1H-benzimidazol-2-yl]-6-(neopentylamino)quinolin-2(1H)-one | 460.6 |
| 881 | N-{4-amino-3-[6-(4-methylpiperazin-1-yl)-1H-benzimidazol-2-yl]-2-oxo-1,2-dihydroquinolin-6-yl}-N'-(3,4-dichlorophenyl)urea | 578.5 |
| 882 | N-{4-amino-3-[6-(4-methylpiperazin-1-yl)-1H-benzimidazol-2-yl]-2-oxo-1,2-dihydroquinolin-6-yl}-N'-[3-(trifluoromethyl)phenyl]urea | 577.6 |
| 883 | N-{4-amino-3-[6-(4-methylpiperazin-1-yl)-1H-benzimidazol-2-yl]-2-oxo-1,2-dihydroquinolin-6-yl}-N'-heptylurea | 531.7 |
| 884 | N-{4-amino-3-[6-(4-methylpiperazin-1-yl)-1H-benzimidazol-2-yl]-2-oxo-1,2-dihydroquinolin-6-yl}-N'-(2-ethoxyphenyl)urea | 553.6 |
| 885 | N-{4-amino-3-[6-(4-methylpiperazin-1-yl)-1H-benzimidazol-2-yl]-2-oxo-1,2-dihydroquinolin-6-yl}-2-methylpropanamide | 460.6 |
| 886 | N-{4-amino-3-[6-(4-methylpiperazin-1-yl)-1H-benzimidazol-2-yl]-2-oxo-1,2-dihydroquinolin-6-yl}-4-ethylbenzamide | 522.6 |
| 887 | N-{4-amino-3-[6-(4-methylpiperazin-1-yl)-1H-benzimidazol-2-yl]-2-oxo-1,2-dihydroquinolin-6-yl}-4-cyanobenzamide | 519.6 |
| 888 | N-{4-amino-3-[6-(4-methylpiperazin-1-yl)-1H-benzimidazol-2-yl]-2-oxo-1,2-dihydroquinolin-6-yl}cyclohexanecarboxamide | 500.6 |
| 889 | N-{4-amino-3-[6-(4-methylpiperazin-1-yl)-1H-benzimidazol-2-yl]-2-oxo-1,2-dihydroquinolin-6-yl}pyrazine-2-carboxamide | 496.5 |
| 890 | N-{4-amino-3-[6-(4-methylpiperazinyl)benzimidazol-2-yl]-2-oxo(6-hydroquinolyl)}-2-[benzylamino]acetamide | 537.6 |
| 891 | 4-amino-6-[methyl(1-methylpiperidin-4-yl)amino]-3-[6-(4-methylpiperazin-1-yl)-1H-benzimidazol-2-yl]quinolin-2(1H)-one | 501.6 |
| 892 | 4-amino-6-[({5-[(dimethylamino)methyl]-2-furyl}methyl)amino]-3-[6-(4-methylpiperazin-1-yl)-1H-benzimidazol-2-yl]quinolin-2(1H)-one | 527.6 |
| 893 | 4-amino-6-{[(2-ethyl-5-methyl-4H-imidazol-4-yl)methyl]amino}-3-[6-(4-methylpiperazin-1-yl)-1H-benzimidazol-2-yl]quinolin-2(1H)-one | 512.6 |
| 894 | N-{4-amino-3-[6-(4-methylpiperazin-1-yl)-1H-benzimidazol-2-yl]-2-oxo-1,2-dihydroquinolin-6-yl}butanamide | 460.6 |
| 895 | 4-amino-3-(5-{[(2R)-2-(pyrrolidin-1-ylmethyl)pyrrolidin-1-yl]carbonyl}-1H-benzimidazol-2-yl)quinolin-2(1H)-one | 457.5 |
| 896 | 4-amino-3-[5-({(2R,5R)-2-[(dimethylamino)methyl]-5-methylmorpholin-4-yl}carbonyl)-1H-benzimidazol-2-yl]quinolin-2(1H)-one | 461.5 |
| 897 | 4-amino-3-[5-({(2S,5R)-2-[(dimethylamino)methyl]-5-methylmorpholin-4-yl}carbonyl)-1H-benzimidazol-2-yl]quinolin-2(1H)-one | 461.5 |
| 898 | 4-amino-5-fluoro-3-(6-{[(3S)-3-methylpiperazin-1-yl]carbonyl}-1H-benzimidazol-2-yl)quinolin-2(1H)-one | 421.4 |
| 899 | 4-amino-5-fluoro-3-(6-{[(3R)-3-methylpiperazin-1-yl]carbonyl}-1H-benzimidazol-2-yl)quinolin-2(1H)-one | 421.4 |
| 900 | 4-amino-5-fluoro-3-(5-{[(2R)-2-(pyrrolidin-1-ylmethyl)pyrrolidin-1-yl]carbonyl}-1H-benzimidazol-2-yl)quinolin-2(1H)-one | 475.5 |
| 901 | 4-amino-6-(dimethylamino)-3-[5-(4-methylpiperazin-1-yl)-1H-benzimidazol-2-yl]quinolin-2(1H)-one | 418.5 |
| 902 | 4-amino-6-(methylamino)-3-[5-(4-methylpiperazin-1-yl)-1H-benzimidazol-2-yl]quinolin-2(1H)-one | 404.5 |
| 903 | 4-amino-5-fluoro-3-[5-fluoro-6-(4-methylpiperazin-1-yl)-1H-benzimidazol-2-yl]quinolin-2(1H)-one | 411.4 |
| 904 | 4-amino-3-[6-({(2R,5S)-2-[(dimethylamino)methyl]-5-methylmorpholin-4-yl}carbonyl)-1H-benzimidazol-2-yl]quinolin-2(1H)-one | 461.5 |
| 905 | 4-amino-3-[6-({(2S,5S)-2-[(dimethylamino)methyl]-5-methylmorpholin-4-yl}carbonyl)-1H-benzimidazol-2-yl]quinolin-2(1H)-one | 461.5 |
| 906 | 4-amino-3-{6-[(3,5-dimethylpiperazin-1-yl)carbonyl]-1H-benzimidazol-2-yl}quinolin-2(1H)-one | 417.5 |
| 907 | 4-amino-3-[5-(4-ethylpiperazin-1-yl)-1H-benzimidazol-2-yl]-5-fluoroquinolin-2(1H)-one | 407.5 |
| 908 | 4-amino-3-[6-({(2R,5S)-2-[(dimethylamino)methyl]-5-methylmorpholin-4-yl}carbonyl)-1H-benzimidazol-2-yl]-5-fluoroquinolin-2(1H)-one | 479.5 |
| 909 | 4-amino-3-[6-({(2S,5S)-2-[(dimethylamino)methyl]-5-methylmorpholin-4-yl}carbonyl)-1H-benzimidazol-2-yl]-5-fluoroquinolin-2(1H)-one | 479.5 |
| 910 | 4-amino-3-[5-({(2R,5R)-2-[(dimethylamino)methyl]-5-methylmorpholin-4-yl}carbonyl)-1H-benzimidazol-2-yl]-5-fluoroquinolin-2(1H)-one | 479.5 |
| 911 | 4-amino-3-[5-({(2S,5R)-2-[(dimethylamino)methyl]-5-methylmorpholin-4-yl}carbonyl)-1H-benzimidazol-2-yl]-5-fluoroquinolin-2(1H)-one | 479.5 |
| 912 | N-[3-({4-amino-3-[6-(4-methylpiperazin-1-yl)-1H-benzimidazol-2-yl]-2-oxo-1,2-dihydroquinolin-5-yl}oxy)phenyl] acetamide | 524.6 |
| 913 | 4-amino-3-{6-[(4-ethylpiperazin-1-yl)carbonyl]-1H-benzimidazol-2-yl}quinolin-2(1H)-one | 417.5 |
| 914 | 2-(4-amino-2-oxo-1,2-dihydroquinolin-3-yl)-N,N'-dimethyl-1H-benzimidazole-6-carbohydrazide | 363.4 |
| 915 | 2-(4-amino-2-oxo-1,2-dihydroquinolin-3-yl)-N-(tetrahydrofuran-2-ylmethyl)-1H-benzimidazole-6-carboxamide | 404.4 |
| 916 | 4-amino-5-[3-(dimethylamino)phenoxy]-3-[6-(4-methylpiperazin-1-yl)-1H-benzimidazol-2-yl]quinolin-2(1H)-one | 510.6 |
| 917 | 4-amino-5-(4-aminophenoxy)-3-[6-(4-methylpiperazin-1-yl)-1H-benzimidazol-2-yl]quinolin-2(1H)-one | 482.6 |
| 918 | 6-chloro-4-{[2-(dimethylamino)ethyl]amino}-3-(6-fluoro-1H-benzimidazol-2-yl)quinolin-2(1H)-one | 400.9 |
| 919 | 4-{[(1R,2R)-2-aminocyclohexyl]amino}-6-chloro-3-(6-fluoro-1H-benzimidazol-2-yl)quinolin-2(1H)-one | 426.9 |
| 920 | 6-chloro-3-(6-fluoro-1H-benzimidazol-2-yl)-4-[(piperidin-3-ylmethyl)amino]quinolin-2(1H)-one | 426.9 |
| 921 | 6-chloro-3-(6-fluoro-1H-benzimidazol-2-yl)-4-[(piperidin-4-ylmethyl)amino]quinolin-2(1H)-one | 426.9 |
| 922 | 4-[(4-aminocyclohexyl)amino]-6-chloro-3-(6-fluoro-1H-benzimidazol-2-yl)quinolin-2(1H)-one | 426.9 |
| 923 | 6-chloro-3-(6-fluoro-1H-benzimidazol-2-yl)-4-{[2-(methylamino)ethyl]amino}quinolin-2(1H)-one | 386.8 |
| 924 | 6-chloro-3-(6-fluoro-1H-benzimidazol-2-yl)-4-[(3S)-pyrrolidin-3-ylamino]quinolin-2(1H)-one | 398.8 |
| 925 | 6-chloro-3-(6-fluoro-1H-benzimidazol-2-yl)-4-[(3R)-pyrrolidin-3-ylamino]quinolin-2(1H)-one | 398.8 |
| 926 | 6-chloro-3-(6-fluoro-1H-benzimidazol-2-yl)-4-[(piperidin-2-ylmethyl)amino]quinolin-2(1H)-one | 426.9 |
| 927 | 4-[(3S)-1-azabicyclo[2.2.2]oct-3-ylamino]-6-chloro-3-(6-fluoro-1H-benzimidazol-2-yl)quinolin-2(1H)-one | 438.9 |
| 928 | 6-bromo-4-{[2-(dimethylamino)ethyl]amino}-3-(6-fluoro-1H-benzimidazol-2-yl)quinolin-2(1H)-one | 445.3 |
| 929 | 4-{[(1R,2R)-2-aminocyclohexyl]amino}-6-bromo-3-(6-fluoro-1H-benzimidazol-2-yl)quinolin-2(1H)-one | 471.3 |
| 930 | 6-bromo-3-(6-fluoro-1H-benzimidazol-2-yl)-4-[(piperidin-3-ylmethyl)amino]quinolin-2(1H)-one | 471.3 |
| 931 | 6-bromo-3-(6-fluoro-1H-benzimidazol-2-yl)-4-[(piperidin-4-ylmethyl)amino]quinolin-2(1H)-one | 471.3 |
| 932 | 4-[(4-aminocyclohexyl)amino]-6-bromo-3-(6-fluoro-1H-benzimidazol-2-yl)quinolin-2(1H)-one | 471.3 |
| 933 | 6-bromo-3-(6-fluoro-1H-benzimidazol-2-yl)-4-{[2-(methylamino)ethyl]amino}quinolin-2(1H)-one | 431.3 |
| 934 | 6-bromo-3-(6-fluoro-1H-benzimidazol-2-yl)-4-[(3S)-pyrrolidin-3-ylamino]quinolin-2(1H)-one | 443.3 |
| 935 | 6-bromo-3-(6-fluoro-1H-benzimidazol-2-yl)-4-[(piperidin-2-ylmethyl)amino]quinolin-2(1H)-one | 471.3 |
| 936 | 4-[(3S)-1-azabicyclo[2.2.2]oct-3-ylamino]-6-bromo-3-(6-fluoro-1H-benzimidazol-2-yl)quinolin-2(1H)-one | 483.4 |
| 937 | 6-bromo-3-(6-fluoro-1H-benzimidazol-2-yl)-4-[(3R)-pyrrolidin-3-ylamino]quinolin-2(1H)-one | 443.3 |
| 938 | N-[4-({4-amino-3-[6-(4-methylpiperazin-1-yl)-1H-benzimidazol-2-yl]-2-oxo-1,2-dihydroquinolin-5-yl}oxy)phenyl]acetamide | 524.6 |
| 939 | 4-amino-3-{6-[(4-ethylpiperazin-1-yl)carbonyl]-1H-benzimidazol-2-yl}-5-fluoroquinolin-2(1H)-one | 435.5 |
| 940 | ethyl (3S,4R)-4-({[2-(4-amino-5-fluoro-2-oxo-1,2-dihydroquinolin-3-yl)-1H-benzimidazol-6-yl]carbonyl}amino)-3-methoxypiperidine-1-carboxylate | 523.5 |
| 941 | 2-(4-amino-5-fluoro-2-oxo-1,2-dihydroquinolin-3-yl)-N-[(3R)-1-azabicyclo[2.2.2]oct-3-yl]-1H-benzimidazole-6-carboxamide | 447.5 |
| 942 | 2-(4-amino-5-fluoro-2-oxo-1,2-dihydroquinolin-3-yl)-N-[(3S)-1-azabicyclo[2.2.2]oct-3-yl]-1H-benzimidazole-6-carboxamide | 447.5 |
| 943 | 4-amino-5-fluoro-3-{5-[(5-methyl-2,5-diazabicyclo[2.2.1]hept-2-yl)carbonyl]-1H-benzimidazol-2-yl}quinolin-2(1H)-one | 433.5 |
| 944 | 4-amino-3-[5-(1,4'-bipiperidin-1'-yl)-1H-benzimidazol-2-yl]-5-fluoroquinolin-2(1H)-one | 461.6 |
| 945 | 4-[(3S)-1-azabicyclo[2.2.2]oct-3-ylamino]-6-chloro-3-(7-morpholin-4-yl-1H-benzimidazol-2-yl)quinolin-2(1H)-one | 506.0 |
| 946 | 6-chloro-3-(7-morpholin-4-yl-1H-benzimidazol-2-yl)-4-(piperidin-4-ylamino)quinolin-2(1H)-one | 480.0 |
| 947 | 6-chloro-3-(7-morpholin-4-yl-1H-benzimidazol-2-yl)-4-[(3S)-pyrrolidin-3-ylamino]quinolin-2(1H)-one | 466.0 |
| 948 | 4-amino-7-fluoro-3-[5-(4-methylpiperazin-1-yl)-1H-benzimidazol-2-yl]quinolin-2(1H)-one | 393.4 |
| 949 | 4-amino-3-{6-[(2,6-dimethylpiperazin-1-yl)carbonyl]-1H-benzimidazol-2-yl}quinolin-2(1H)-one | 417.5 |
| 950 | 4-amino-3-(5-{(2S,5R)-2-[(dimethylamino)methyl]-5-methylmorpholin-4-yl}-1H-benzimidazol-2-yl)-5-fluoroquinolin-2(1H)-one | 451.5 |
| 951 | 6-chloro-3-(5-morpholin-4-yl-1H-benzimidazol-2-yl)-4-[(3S)-pyrrolidin-3-ylamino]quinolin-2(1H)-one | 466.0 |
| 952 | 4-amino-3-(5-{(2S,5S)-2-[(dimethylamino)methyl]-5-methylmorpholin-4-yl}-1H-benzimidazol-2-yl)-5-fluoroquinolin-2(1H)-one | 451.5 |
| 953 | 4-amino-3-(1H-benzimidazol-2-yl)-6-[methyl(1-methylpiperidin-4-yl)amino]quinolin-2(1H)-one | 403.5 |
| 954 | 4-amino-6-[isobutyl(methyl)amino]-3-[6-(4-methylpiperazin-1-yl)-1 H-benzimidazol-2-yl]quinolin-2(1H)-one | 460.6 |
| 955 | 4-amino-6-[(cyclohexylmethyl)(methyl)amino]-3-[6-(4-methylpiperazin-1-yl)-1H-benzimidazol-2-yl]quinolin-2(1H)-one | 500.7 |
| 956 | 4,6-diamino-3-(6,7-dimethyl-1H-benzimidazol-2-yl)quinolin-2(1H)-one | 320.4 |
| 957 | 4-amino-3-(6,7-dimethyl-1H-benzimidazol-2-yl)-6-(methylamino)quinolin-2(1H)-one | 334.4 |
| 958 | 4-amino-3-(5,6-dimethyl-1H-benzimidazol-2-yl)-6-(methylamino)quinolin-2(1H)-one | 334.4 |
| 959 | 4,6-diamino-3-(1H-benzimidazol-2-yl)quinolin-2(1H)-one | 292.3 |
| 960 | 4-amino-3-(6,7-dimethyl-1H-benzimidazol-2-yl)-6-(isobutylamino)quinolin-2(1H)-one | 376.5 |
| 961 | 4-amino-3-(5,6-dimethyl-1H-benzimidazol-2-yl)-6-(isobutylamino)quinolin-2(1H)-one | 376.5 |
| 962 | N-(3-{[2-(4-amino-2-oxo-1,2-dihydroquinolin-3-yl)-1H-benzimidazol-6-yl]oxy}phenyl)acetamide | 426.4 |
| 963 | 4-amino-3-[6-(3,4-dimethylpiperazin-1-yl)-1H-benzimidazol-2-yl]quinolin-2(1H)-one | 389.5 |
| 964 | N-[3-({4-amino-3-[6-(4-methylpiperazin-1-yl)-1H-benzimidazol-2-yl]-2-oxo-1,2-dihydroquinolin-6-yl}oxy)phenyl] acetamide | 524.6 |
| 965 | 4-amino-3-(6-{(2R,5R)-2-[(dimethylamino)methyl]-5-methylmorpholin-4-yl}-1H-benzimidazol-2-yl)-5-fluoroquinolin-2(1H)-one | 451.5 |
| 966 | 4-{[(1R,2R)-2-aminocyclohexyl]amino}-6-bromo-3-(6-chloro-5-fluoro-1H-benzimidazol-2-yl)quinolin-2(1H)-one | 505.8 |
| 967 | 6-bromo-3-(6-chloro-5-fluoro-1H-benzimidazol-2-yl)-4-[(piperidin-4-ylmethyl)amino]quinolin-2(1H)-one | 505.8 |
| 968 | 4-[(4-aminocyclohexyl)amino]-6-bromo-3-(6-chloro-5-fluoro-1H-benzimidazol-2-yl)quinolin-2(1H)-one | 505.8 |
| 969 | 6-bromo-3-(6-chloro-5-fluoro-1H-benzimidazol-2-yl)-4-{[2-(methylamino)ethyl]amino}quinolin-2(1H)-one | 465.7 |
| 970 | 6-bromo-3-(6-chloro-5-fluoro-1H-benzimidazol-2-yl)-4-(pyrrolidin-3-ylamino)quinolin-2(1H)-one | 477.7 |
| 971 | 6-bromo-3-(6-chloro-5-fluoro-1H-benzimidazol-2-yl)-4-[(3R)-pyrrolidin-3-ylamino]quinolin-2(1H)-one | 477.7 |
| 972 | 6-bromo-3-(6-chloro-5-fluoro-1H-benzimidazol-2-yl)-4-[(piperidin-2-ylmethyl)amino]quinolin-2(1H)-one | 505.8 |
| 973 | 4-[(3S)-1-azabicyclo[2.2.2]oct-3-ylamino]-6-bromo-3-(6-chloro-5-fluoro-1H-benzimidazol-2-yl)quinolin-2(1H)-one | 517.8 |
| 974 | 4-[(3R)-1-azabicyclo[2.2.2]oct-3-ylamino]-6-bromo-3-(6-chloro-5-fluoro-1H-benzimidazol-2-yl)quinolin-2(1H)-one | 517.8 |
| 975 | 4-[(3R)-1-azabicyclo[2.2.2]oct-3-ylamino]-6-bromo-3-(6-fluoro-1H-benzimidazol-2-yl)quinolin-2(1H)-one | 483.4 |
| 976 | 4-[(3R)-1-azabicyclo[2.2.2]oct-3-ylamino]-6-chloro-3-(6-fluoro-1H-benzimidazol-2-yl)quinolin-2(1H)-one | 438.9 |
| 977 | 4-amino-6-[bis(cyclohexylmethyl)amino]-3-(6,7-dimethyl-1H-benzimidazol-2-yl)quinolin-2(1H)-one | 512.7 |
| 978 | 4-amino-6-[bis(cyclohexylmethyl)amino]-3-(5,6-dimethyl-1H-benzimidazol-2-yl)quinolin-2(1H)-one | 512.7 |
| 979 | 4-amino-5-(methylamino)-3-[6-(4-methylpiperazin-1-yl)-1H-benzimidazol-2-yl]quinolin-2(1H)-one | 404.5 |
| 980 | 4-amino-6-[(cyclohexylmethyl)amino]-3-(6,7-dimethyl-1H-benzimidazol-2-yl)quinolin-2(1H)-one | 416.5 |
| 981 | 4-amino-6-[(cyclohexylmethyl)amino]-3-(5,6-dimethyl-1H-benzimidazol-2-yl)quinolin-2(1H)-one | 416.5 |
| 982 | 4-amino-6,7-difluoro-3-[5-(4-methylpiperazin-1-yl)-1H-benzimidazol-2-yl]quinolin-2(1H)-one | 411.4 |
| 983 | 4-amino-5-fluoro-3-[6-(2-methylpiperazin-1-yl)-1H-benzimidazol-2-yl]quinolin-2(1H)-one | 393.4 |
| 984 | 4-amino-7-fluoro-3-{6-[(4-isopropylpiperazin-1-yl)carbonyl]-1H-benzimidazol-2-yl}quinolin-2(1H)-one | 449.5 |
| 985 | 4-amino-3-[6-(2,4-dimethylpiperazin-1-yl)-1H-benzimidazol-2-yl]-5-fluoroquinolin-2(1H)-one | 407.5 |
| 986 | 2-(4-amino-7-fluoro-2-oxo-1,2-dihydroquinolin-3-yl)-N-methyl-N-(1-methylpiperidin-4-yl)-1H-benzimidazole-5-carboxamide | 449.5 |
| 987 | 6-chloro-3-(5-chloro-1H-benzimidazol-2-yl)-4-[(3S)-pyrrolidin-3-ylamino]quinolin-2(1H)-one | 415.3 |
| 988 | 4-amino-7-fluoro-3-(5-{[(2R)-2-(pyrrolidin-1-ylmethyl)pyrrolidin-1-yl]carbonyl}-1H-benzimidazol-2-yl)quinolin-2(1H)-one | 475.5 |
| 989 | 4-amino-3-{6-[4-(2-methoxyethyl)piperazin-1-yl]-1H-benzimidazol-2-yl}quinolin-2(1H)-one | 419.5 |
| 990 | 4-amino-3-[5-(methylamino)-1H-benzimidazol-2-yl]quinolin-2(1H)-one | 306.3 |
| 991 | 6-chloro-3-[5-(4-methylpiperazin-1-yl)-1H-benzimidazol-2-yl]-4-{[(3S)-1-methylpyrrolidin-3-yl]amino}quinolin-2(1H)-one | 493.0 |
| 992 | 6-chloro-3-(5-chloro-1H-benzimidazol-2-yl)-4-{[(3S)-1-methylpyrrolidin-3-yl]amino}quinolin-2(1H)-one | 429.3 |
| 993 | 3-(1H-benzimidazol-2-yl)-6-chloro-4-{[(3S)-1-methylpyrrolidin-3-yl]amino}quinolin-2(1H)-one | 394.9 |
| 994 | 3-(1H-benzimidazol-2-yl)-6-chloro-4-[(1-methylpiperidin-4-yl)amino]quinolin-2(1H)-one | 408.9 |
| 995 | 6-chloro-3-(5-chloro-1H-benzimidazol-2-yl)-4-[(1-methylpiperidin-4-yl)amino]quinolin-2(1H)-one | 443.3 |
| 996 | 6-chloro-3-[5-(4-methylpiperazin-1-yl)-1H-benzimidazol-2-yl]-4-[(1-methylpiperidin-4-yl)amino]quinolin-2(1H)-one | 507.1 |
| 997 | 6-chloro-3-[5-(4-methylpiperazin-1-yl)-1H-benzimidazol-2-yl]-4-{[(1-methylpiperidin-2-yl)methyl]amino}quinolin-2(1H)-one | 521.1 |
| 998 | 4-[(3S)-1-azabicyclo[2.2.2]oct-3-ylamino]-6-chloro-3-{5-[methyl(1-methylpiperidin-4-yl)amino]-1H-benzimidazol-2-yl}quinolin-2(1H)-one | 547.1 |
| 999 | 6-chloro-3-{5-[methyl(1-methylpiperidin-4-yl)amino]-1H-benzimidazol-2-yl}-4-(piperidin-4-ylamino)quinolin-2(1H)-one | 521.1 |
| 1000 | 6-chloro-3-{5-[methyl(1-methylpiperidin-4-yl)amino]-1H-benzimidazol-2-yl}-4-[(3S)-pyrrolidin-3-ylamino]quinolin-2(1H)-one | 507.1 |
| 1001 | 4- {[(2R)-2-aminobutyl]amino}-6-chloro-3-{5-[methyl(1-methylpiperidin-4-yl)amino]-1H-benzimidazol-2-yl}quinolin-2(1H)-one | 509.1 |
| 1002 | 4-amino-3-{6-[(3S)-3,4-dimethylpiperazin-1-yl]-1H-benzimidazol-2-yl}quinolin-2(1H)-one | 389.5 |
| 1003 | 4-amino-3-[5-(4-methylpiperazin-1-yl)-1H-benzimidazol-2-yl]-2-oxo-1,2-dihydroquinoline-6-carbonitrile | 400.5 |
| 1004 | 4-amino-3-[5-(4-methylpiperazin-1-yl)-1H-benzimidazol-2-yl]-2-oxo-1,2-dihydroquinoline-6-carboxylic acid | 419.5 |
| 1005 | 4-amino-5-fluoro-3-{5-[(8aS)-hexahydropyrrolo[1,2-a]pyrazin-2(1H)-yl]-1H-benzimidazol-2-yl}quinolin-2(1H)-one | 419.5 |
| 1006 | 4-amino-3-{6-[(3S)-3,4-dimethylpiperazin-1-yl]-1H-benzimidazol-2-yl}-5-fluoroquinolin-2(1H)-one | 407.5 |
| 1007 | 4-[(3S)-1-azabicyclo[2.2.2]oct-3-ylamino]-6-chloro-3-{6-[(3R)-3-(dimethylamino)pyrrolidin-1-yl]-1H-benzimidazol-2-yl}quinolin-2(1H)-one | 533.1 |
| 1008 | 6-chloro-3-{6-[(3R)-3-(dimethylamino)pyrrolidin-1-yl]-1H-benzimidazol-2-yl}-4-(piperidin-4-ylamino)quinolin-2(1H)-one | 507.1 |
| 1009 | 6-chloro-3-{6-[(3R)-3-(dimethylamino)pyrrolidin-1-yl]-1H-benzimidazol-2-yl}-4-[(3S)-pyrrolidin-3-ylamino]quinolin-2(1H)-one | 493.0 |
| 1010 | 4- {[(2R)-2-aminobutyl]amino}-6-chloro-3- {6-[(3R)-3-(dimethylamino)pyrrolidin-1-yl]-1H-benzimidazol-2-yl}quinolin-2(1H)-one | 495.0 |
| 1011 | 6-chloro-3-{6-[(3R)-3-(dimethylamino)pyrrolidin-1-yl]-1H-benzimidazol-2-yl}-4-{[(3S)-1-methylpyrrolidin-3-yl]amino}quinolin-2(1H)-one | 507.1 |
| 1012 | 6-chloro-3-{6-[(3R)-3-(dimethylamino)pyrrolidin-1-yl]-1H-benzimidazol-2-yl}-4-[(1-methylpiperidin-4-yl)amino]quinolin-2(1H)-one | 521.1 |
| 1013 | 4-amino-7-(methylamino)-3-[6-(4-methylpiperazin-1-yl)-1H-benzimidazol-2-yl]quinolin-2(1H)-one | 404.5 |
| 1014 | 3-(1H-benzimidazol-2-yl)-6-chloro-4-[(2-morpholin-4-yl-2-pyridin-3-ylethyl)amino]quinolin-2(1H)-one | 502.0 |
| 1015 | 3-(1H-benzimidazol-2-yl)-6-chloro-4-{[2-(dimethylamino)-2-pyridin-3-ylethyl] amino}quinolin-2(1H)-one | 460.0 |
| 1016 | 4-[(3S)-1-azabicyclo[2.2.2]oct-3-ylamino]-6-chloro-3-(6-{3-[(dimethylamino)methyl]pyrrolidin-1-yl}-1H-benzimidazol-2-yl)quinolin-2(1H)-one | 547.1 |
| 1017 | 6-chloro-3-(6-{3-[(dimethylamino)methyl]pyrrolidin-1-yl}-1H-benzimidazol-2-yl)-4-(piperidin-4-ylamino)quinolin-2(1H)-one | 521.1 |
| 1018 | 6-chloro-3-(6-{3-[(dimethylamino)methyl]pyrrolidin-1-yl}-1H-benzimidazol-2-yl)-4-[(3S)-pyrrolidin-3-ylamino]quinolin-2(1H)-one | 507.1 |
| 1019 | 4- {[(2R)-2-aminobutyl]amino}-6-chloro-3-(6- {3-[(dimethylamino)methyl]pyrrolidin-1-yl}-1H-benzimidazol-2-yl)quinolin-2(1H)-one | 509.1 |
| 1020 | 6-chloro-3-(6-{3-[(dimethylamino)methyl]pyrrolidin-1-yl}-1H-benzimidazol-2-yl)-4-{[(3S)-1-methylpyrrolidin-3-yl]amino}quinolin-2(1H)-one | 521.1 |
| 1021 | 6-chloro-3-(6-{3-[(dimethylamino)methyl]pyrrolidin-1-yl}-1H-benzimidazol-2-yl)-4-[(1-methylpiperidin-4-yl)amino]quinolin-2(1H)-one | 535.1 |
| 1022 | 3-(1H-benzimidazol-2-yl)-6-chloro-4-{[(3S)-piperidin-3-ylmethyl]amino}quinolin-2(1H)-one | 408.9 |
| 1023 | 3-(1H-benzimidazol-2-yl)-6-chloro-4-{[(3R)-piperidin-3-ylmethyl]amino}quinolin-2(1H)-one | 408.9 |
| 1024 | N-(3-{[4-amino-3-(1H-benzimidazol-2-yl)-2-oxo-1,2-dihydroquinolin-5-yl]oxy}phenyl)acetamide | 426.4 |
| 1025 | 4-[(3S)-1-azabicyclo[2.2.2]oct-3-ylamino]-6-chloro-3-{6-[3-(dimethylamino)pyrrolidin-1-yl]-1H-benzimidazol-2-yl}quinolin-2(1H)-one | 533.1 |
| 1026 | 6-chloro-3-{6-[3-(dimethylamino)pyrrolidin-1-yl]-1H-benzimidazol-2-yl}-4-(piperidin-4-ylamino)quinolin-2(1H)-one | 507.1 |
| 1027 | 4-{[(2R)-2-aminobutyl]amino}-6-chloro-3- {6-[3-(dimethylamino)pyrrolidin-1-yl]-1H-benzimidazol-2-yl}quinolin-2(1H)-one | 495.0 |
| 1028 | 6-chloro-3-{6-[3-(dimethylamino)pyrrolidin-1-yl]-1H-benzimidazol-2-yl}-4-[(1-methylpiperidin-4-yl)amino]quinolin-2(1H)-one | 521.1 |
| 1029 | 4-amino-7-[[2-(dimethylamino)ethyl](methyl)amino]-3-[6-(4-methylpiperazin-1-yl)-1H-benzimidazol-2-yl]quinolin-2(1H)-one | 475.6 |
| 1030 | 4-amino-5-fluoro-3-[6-(1,4-oxazepan-4-ylcarbonyl)-1H-benzimidazol-2-yl]quinolin-2(1H)-one | 422.4 |
| 1031 | methyl 4-amino-3-[5-(4-methylpiperazin-1-yl)-1H-benzimidazol-2-yl]-2-oxo-1,2-dihydroquinoline-6-carboxylate | 433.5 |
| 1032 | 4-amino-N-benzyl-3-[5-(4-methylpiperazin-1-yl)-1H-benzimidazol-2-yl]-2-oxo-1,2-dihydroquinoline-6-carboxamide | 508.6 |
| 1033 | 4-amino-3-{6-[4-(2-morpholin-4-ylethyl)piperazin-1-yl]-1H-benzimidazol-2-yl}quinolin-2(1H)-one | 474.6 |
| 1034 | 4-amino-7-fluoro-3-[6-(4-isopropylpiperazin-1-yl)-1H-benzimidazol-2-yl]quinolin-2(1H)-one | 421.5 |
| 1035 | 4-amino-3-[5-(4-ethylpiperazin-1-yl)-1H-benzimidazol-2-yl]-7-fluoroquinolin-2(1H)-one | 407.5 |
| 1036 | 4-amino-3-{6-[(2-aminoethyl)(methyl)amino]-1H-benzimidazol-2-yl}quinolin-2(1H)-one | 349.4 |
| 1037 | 4-amino-3-{6-[[(2-ethyl-4-methyl-1H-imidazol-5-yl)methyl](methyl)amino]-1H-benzimidazol-2-yl}quinolin-2(1H)-one | 428.5 |
| 1038 | 4-amino-3-[6-(hydroxymethyl)-1H-benzimidazol-2-yl]quinolin-2(1H)-one | 307.3 |
| 1039 | 4-amino-3-(6-{methyl[(2R)-pyrrolidin-2-ylmethyl]amino}-1H-benzimidazol-2-yl)quinolin-2(1H)-one | 389.5 |
| 1040 | 4-amino-3-{6-[(1H-imidazol-2-ylmethyl)(methyl)amino]-1H-benzimidazol-2-yl}quinolin-2(1H)-one | 386.4 |
| 1041 | 4-amino-3-{6-[(2-furylmethyl)(methyl)amino]-1H-benzimidazol-2-yl}quinolin-2(1H)-one | 386.4 |
| 1042 | 4-amino-3-{6-[methyl(piperidin-4-ylmethyl)amino]-1H-benzimidazol-2-yl}quinolin-2(1H)-one | 403.5 |
| 1043 | 4-amino-3-{6-[methyl(piperidin-3-ylmethyl)amino]-1H-benzimidazol-2-yl}quinolin-2(1H)-one | 403.5 |
| 1044 | 4-amino-3-(6-{methyl[2-(methylamino)ethyl]amino}-1H-benzimidazol-2-yl)quinolin-2(1H)-one | 363.4 |
| 1045 | 6-acetyl-4-amino-3-[6-(4-methylpiperazin-1-yl)-1H-benzimidazol-2-yl]quinolin-2(1H)-one | 417.5 |
| 1046 | 4-amino-5-[2-(methylamino)phenoxy]-3-[6-(4-methylpiperazin-1-yl)-1H-benzimidazol-2-yl]quinolin-2(1H)-one | 496.6 |
| 1047 | 3-(1H-benzimidazol-2-yl)-6-chloro-4-{[(2S)-piperidin-2-ylmethyl]amino}quinolin-2(1H)-one | 408.9 |
| 1048 | 4-amino-3-[6-(1,4-oxazepan-4-yl)-1H-benzimidazol-2-yl]quinolin-2(1H)-one | 376.4 |
| 1049 | 4-amino-3-[5-(4-ethylpiperazin-1-yl)-1H-benzimidazol-2-yl]-6-fluoroquinolin-2(1H)-one | 407.5 |
| 1050 | 6-chloro-3-(5-chloro-1H-benzimidazol-2-yl)-4-[(3R)-pyrrolidin-3-ylamino]quinolin-2(1H)-one | 415.3 |
| 1051 | 4-amino-6-fluoro-3-[5-(4-methylpiperazin-1-yl)-1H-benzimidazol-2-yl]-7-morpholin-4-ylquinolin-2(1H)-one | 478.5 |
| 1052 | 4-amino-6-fluoro-3-[5-(4-methylpiperazin-1-yl)-1H-benzimidazol-2-yl]-7-pyrrolidin-1-ylquinolin-2(1H)-one | 462.5 |
| 1053 | 4-amino-7-(dimethylamino)-6-fluoro-3-[5-(4-methylpiperazin-1-yl)-1 H-benzimidazol-2-yl]quinolin-2(1H)-one | 436.5 |
| 1054 | 4-amino-6-fluoro-7-(4-methylpiperazin-1-yl)-3-[5-(4-methylpiperazin-1-yl)-1H-benzimidazol-2-yl]quinolin-2(1H)-one | 491.6 |
| 1055 | 4-amino-6-fluoro-7-[(4-methoxybenzyl)amino]-3-[5-(4-methylpiperazin-1-yl)-1H-benzimidazol-2-yl]quinolin-2(1H)-one | 528.6 |
| 1056 | 4-amino-6-fluoro-3-[5-(4-methylpiperazin-1-yl)-1H-benzimidazol-2-yl]-7-[(pyridin-4-ylmethyl)amino]quinolin-2(1H)-one | 499.6 |
| 1057 | 4-amino-7-[[2-(dimethylamino)ethyl](methyl)amino]-6-fluoro-3-[5-(4-methylpiperazin-1-yl)-1H-benzimidazol-2-yl]quinolin-2(1H)-one | 493.6 |
| 1058 | 4-amino-3-[6-(4-cyclopentylpiperazin-1-yl)-1H-benzimidazol-2-yl]-5-fluoroquinolin-2(1H)-one | 447.5 |
| 1059 | 4-amino-6-[1-(methylamino)ethyl]-3-[6-(4-methylpiperazin-1-yl)-1H-benzimidazol-2-yl]quinolin-2(1H)-one | 432.5 |
| 1060 | 4-amino-5-fluoro-3-[6-(1,4-oxazepan-4-yl)-1H-benzimidazol-2-yl]quinolin-2(1H)-one | 394.4 |
| 1061 | 4-amino-3-{6-[methyl(pyridin-3-ylmethyl)amino]-1H-benzimidazol-2-yl}quinolin-2(1H)-one | 397.5 |
| 1062 | 4-amino-3-{6-[({5-[(dimethylamino)methyl]-2-furyl}methyl)(methyl)amino]-1H-benzimidazol-2-yl}quinolin-2(1H)-one | 443.5 |
| 1063 | 4-amino-3-[6-(4-oxopiperidin-1-yl)-1H-benzimidazol-2-yl]quinolin-2(1H)-one | 374.4 |
| 1064 | 4-amino-3-{6-[4-(4-methylpiperazin-1-yl)piperidin-1-yl]-1H-benzimidazol-2-yl}quinolin-2(1H)-one | 458.6 |
| 1065 | 4-amino-3-[6-(4-{[(4-benzylmorpholin-2-yl)methyl]amino}piperidin-1-yl)-1H-benzimidazol-2-yl]quinolin-2(1H)-one | 564.7 |
| 1066 | 3-(1H-benzimidazol-2-yl)-6-bromo-4-{[2-(dimethylamino)ethyl]amino}quinolin-2(1H)-one | 427.3 |
| 1067 | 4-{[(1R,2R)-2-aminocyclohexyl]amino}-3-(1H-benzimidazol-2-yl)-6-bromoquinolin-2(1H)-one | 453.4 |
| 1068 | 3-(1H-benzimidazol-2-yl)-6-bromo-4-[(piperidin-4-ylmethyl)amino]quinolin-2(1H)-one | 453.4 |
| 1069 | 4-[(4-aminocyclohexyl)amino]-3-(1H-benzimidazol-2-yl)-6-bromoquinolin-2(1H)-one | 453.4 |
| 1070 | 3-(1H-benzimidazol-2-yl)-6-bromo-4-{[2-(methylamino)ethyl]amino}quinolin-2(1H)-one | 413.3 |
| 1071 | 3-(1H-benzimidazol-2-yl)-6-bromo-4-[(3S)-pyrrolidin-3-ylamino]quinolin-2(1H)-one | 425.3 |
| 1072 | 3-(1H-benzimidazol-2-yl)-6-bromo-4-[(3R)-pyrrolidin-3-ylamino]quinolin-2(1H)-one | 425.3 |
| 1073 | 3-(1H-benzimidazol-2-yl)-6-bromo-4-[(piperidin-2-ylmethyl)amino]quinolin-2(1H)-one | 453.4 |
| 1074 | 4-amino-N-[(3S)-1-azabicyclo[2.2.2]oct-3-yl]-3-[5-(4-methylpiperazin-1-yl)-1H-benzimidazol-2-yl]-2-oxo-1,2-dihydroquinoline-6-carboxamide | 527.6 |
| 1075 | 4-amino-N-methyl-3-[5-(4-methylpiperazin-1-yl)-1H-benzimidazol-2-yl]-N-(1-methylpiperidin-4-yl)-2-oxo-1,2-dihydroquinoline-6-carboxamide | 529.7 |
| 1076 | 4-amino-3-[5-(4-methylpiperazin-1-yl)-1H-benzimidazol-2-yl]-2-oxo-N-(tetrahydrofuran-2-ylmethyl)-1,2-dihydroquinoline-6-carboxamide | 502.6 |
| 1077 | 3-(1H-benzimidazol-2-yl)-6-chloro-4-[(3R)-pyrrolidin-3-ylamino]quinolin-2(1H)-one | 380.8 |
| 1078 | 3-(1H-benzimidazol-2-yl)-6-chloro-4-{[(2R)-piperidin-2-ylmethyl]amino}quinolin-2(1H)-one | 408.9 |
| 1079 | 4-amino-3-{6-[(3R)-3,4-dimethylpiperazin-1-yl]-1H-benzimidazol-2-yl}-5-fluoroquinolin-2(1H)-one | 407.5 |
| 1080 | 6-chloro-3-(6-chloro-5-fluoro-1H-benzimidazol-2-yl)-4-{[2-(dimethylamino)ethyl]amino}quinolin-2(1H)-one | 435.3 |
| 1081 | 4-{[(1R,2R)-2-aminocyclohexyl]amino}-6-chloro-3-(6-chloro-5-fluoro-1H-benzimidazol-2-yl)quinolin-2(1H)-one | 461.3 |
| 1082 | 6-chloro-3-(6-chloro-5-fluoro-1H-benzimidazol-2-yl)-4-[(piperidin-4-ylmethyl)amino]quinolin-2(1H)-one | 461.3 |
| 1083 | 4-[(4-aminocyclohexyl)amino]-6-chloro-3-(6-chloro-5-fluoro-1H-benzimidazol-2-yl)quinolin-2(1H)-one | 461.3 |
| 1084 | 6-chloro-3-(6-chloro-5-fluoro-1H-benzimidazol-2-yl)-4-{[2-(methylamino)ethyl]amino}quinolin-2(1H)-one | 421.3 |
| 1085 | 6-chloro-3-(6-chloro-5-fluoro-1H-benzimidazol-2-yl)-4-[(3S)-pyrrolidin-3-ylamino]quinolin-2(1H)-one | 433.3 |
| 1086 | 6-chloro-3-(6-chloro-5-fluoro-1H-benzimidazol-2-yl)-4-[(3R)-pyrrolidin-3-ylamino]quinolin-2(1H)-one | 433.3 |
| 1087 | 6-chloro-3-(6-chloro-5-fluoro-1H-benzimidazol-2-yl)-4-[(piperidin-2-ylmethyl)amino]quinolin-2(1H)-one | 461.3 |
| 1088 | 4-[(3S)-1-azabicyclo[2.2.2]oct-3-ylamino]-6-chloro-3-(6-chloro-5-fluoro-1H-benzimidazol-2-yl)quinolin-2(1H)-one | 473.3 |
| 1089 | 4-[(3R)-1-azabicyclo[2.2.2]oct-3-ylamino]-6-chloro-3-(6-chloro-5-fluoro-1H-benzimidazol-2-yl)quinolin-2(1H)-one | 473.3 |
| 1090 | 4-amino-6-fluoro-3-[6-(4-methylpiperazin-1-yl)-1H-benzimidazol-2-yl]quinolin-2(1H)-one | 393.4 |
| 1091 | 4-amino-3-(1H-benzimidazol-2-yl)-5-(methylamino)quinolin-2(1H)-one | 306.3 |
| 1092 | 4-amino-3-{6-[(2S)-2,4-dimethylpiperazin-1-yl]-1H-benzimidazol-2-yl}-5-fluoroquinolin-2(1H)-one | 407.5 |
| 1093 | 4-amino-5-fluoro-3-{6-[(2S)-2-methylpiperazin-1-yl]-1H-benzimidazol-2-yl}quinolin-2(1H)-one | 393.4 |
| 1094 | 4-amino-3-{6-[(2S)-4-isopropyl-2-methylpiperazin-1-yl]-1H-benzimidazol-2-yl}quinolin-2(1H)-one | 417.5 |
| 1095 | 4-amino-5,7-difluoro-3-[5-(4-methylpiperazin-1-yl)-1H-benzimidazol-2-yl]quinolin-2(1H)-one | 411.4 |
| 1096 | 3-(1H-benzimidazol-2-yl)-6-bromo-4-{[(2S)-piperidin-2-ylmethyl]amino}quinolin-2(1H)-one | 453.4 |
| 1097 | 3-(1H-benzimidazol-2-yl)-6-bromo-4-{[(2R)-piperidin-2-ylmethyl]amino}quinolin-2(1H)-one | 453.4 |
| 1098 | 4-amino-3-{6-[methyl(1,3-thiazol-2-ylmethyl)amino]-1H-benzimidazol-2-yl}quinolin-2(1H)-one | 403.5 |
| 1099 | 4-amino-3-{6-[(1-ethylpiperidin-4-yl)(methyl)amino]-1H-benzimidazol-2-yl}quinolin-2(1H)-one | 417.5 |
| 1100 | 4-amino-3-[6-(4-morpholin-4-ylpiperidin-1-yl)-1H-benzimidazol-2-yl]quinolin-2(1H)-one | 445.5 |
| 1101 | 4-amino-3-[6-(4-isopropylpiperazin-1-yl)-1H-benzimidazol-2-yl]-5-(methylamino)quinolin-2(1H)-one | 432.5 |
| 1102 | 4-amino-3-{6-[methyl(pyridin-2-ylmethyl)amino]-1H-benzimidazol-2-yl}quinolin-2(1H)-one | 397.5 |
| 1103 | 4-amino-3-{6-[(2S)-2,4-dimethylpiperazin-1-yl]-1H-benzimidazol-2-yl}quinolin-2(1H)-one | 389.5 |
| 1104 | 4-amino-3-{6-[(2S)-2-methylpiperazin-1-yl]-1H-benzimidazol-2-yl}quinolin-2(1H)-one | 375.4 |
| 1105 | N-[2-(4-amino-2-oxo-1,2-dihydroquinolin-3-yl)-1H-benzimidazol-6-yl]-N-methylacetamide | 348.4 |
| 1106 | 4-amino-5-fluoro-3-{6-[(2S)-4-isopropyl-2-methylpiperazin-1-yl]-1H-benzimidazol-2-yl}quinolin-2(1H)-one | 435.5 |
| 1107 | 4-amino-3-{6-[(3R)-3,4-dimethylpiperazin-1-yl]-1H-benzimidazol-2-yl}quinolin-2(1H)-one | 389.5 |
| 1108 | 4-[(3S)-1-azabicyclo[2.2.2]oct-3-ylamino]-3-(1H-benzimidazol-2-yl)-6-(dimethylamino)quinolin-2(1H)-one | 429.5 |
| 1109 | 4-amino-3-{6-[(2S)-4-cyclobutyl-2-methylpiperazin-1-yl]-1H-benzimidazol-2-yl}quinolin-2(1H)-one | 429.5 |
| 1110 | 4-amino-5-fluoro-3-[6-(methylamino)-1H-benzimidazol-2-yl]quinolin-2(1H)-one | 324.3 |
| 1111 | 4-amino-3-(1H-benzimidazol-2-yl)-5-(dimethylamino)quinolin-2(1H)-one | 320.4 |
| 1112 | 4-amino-3-(1H-benzimidazol-2-yl)-5-{[2-(dimethylamino)ethyl]amino}quinolin-2(1H)-one | 363.4 |
| 1113 | 4-amino-5-fluoro-3-(5-piperazin-1-yl-1H-benzimidazol-2-yl)quinolin-2(1H)-one | 379.4 |
| 1114 | 4-amino-3-{5-[[2-(dimethylamino)ethyl](methyl)amino]-1H-benzimidazol-2-yl}-5-fluoroquinolin-2(1H)-one | 395.5 |
| 1115 | 4-amino-5-fluoro-3-{5-[methyl(piperidin-3-ylmethyl)amino]-1H-benzimidazol-2-yl}quinolin-2(1H)-one | 421.5 |
| 1116 | 4-amino-3-(1H-benzimidazol-2-yl)-5-[[2-(dimethylamino)ethyl](methyl)amino]quinolin-2(1H)-one | 377.5 |
| 1117 | 4-amino-5-fluoro-3-{5-[(2R)-4-isopropyl-2-methylpiperazin-1-yl]-1 H-benzimidazol-2-yl}quinolin-2(1H)-one | 435.5 |
| 1118 | 4-amino-3-{5-[(2S)-4-ethyl-2-methylpiperazin-1-yl]-1H-benzimidazol-2-yl}-5-fluoroquinolin-2(1H)-one | 421.5 |
| 1119 | 4-amino-3-(5-{[(1-ethylpyrrolidin-2-yl)methyl]amino}-1H-benzimidazol-2-yl)-5-fluoroquinolin-2(1H)-one | 421.5 |
| 1120 | 4-amino-3-(5-{[2-(dimethylamino)-1-methylethyl]amino}-1H-benzimidazol-2-yl)-5-fluoroquinolin-2(1H)-one | 395.5 |
| 1121 | 4-amino-3-{5-[[2-(dimethylamino)-1-methylethyl](methyl)amino]-1H-benzimidazol-2-yl}-5-fluoroquinolin-2(1H)-one | 409.5 |
| 1122 | 4-amino-3-(1H-benzimidazol-2-yl)-5-(1,2-dimethylhydrazino)quinolin-2(1H)-one | 335.4 |
| 1123 | 4-amino-5-fluoro-3-{6-[4-(2-methoxyethyl)piperazin-1-yl]-1H-benzimidazol-2-yl}quinolin-2(1H)-one | 437.5 |
| 1124 | 4-amino-5-fluoro-3-{6-[methyl(1-methylpiperidin-4-yl)amino]-1H-benzimidazol-2-yl}quinolin-2(1H)-one | 421.5 |
| 1125 | 4-amino-5-fluoro-3-(6-{[3-(4-methylpiperazin-1-yl)propyl]amino}-1H-benzimidazol-2-yl)quinolin-2(1H)-one | 450.5 |
| 1126 | 4-amino-5-fluoro-3-(6-{methyl[3-(4-methylpiperazin-1-yl)propyl]amino}-1H-benzimidazol-2-yl)quinolin-2(1H)-one | 464.6 |
| 1127 | N-[2-(4-amino-5-fluoro-2-oxo-1,2-dihydroquinolin-3-yl)-1H-benzimidazol-6-yl]-N-methylacetamide | 366.4 |
| 1128 | 4-amino-6-fluoro-3-(5-{[(2R)-2-(pyrrolidin-1-ylmethyl)pyrrolidin-1-yl]carbonyl}-1H-benzimidazol-2-yl)quinolin-2(1H)-one | 475.5 |
| 1129 | 4-amino-3-(1H-benzimidazol-2-yl)-5-(ethylamino)quinolin-2(1H)-one | 320.4 |
| 1130 | 4-amino-3-{5-[(2R)-2,4-dimethylpiperazin-1-yl]-1H-benzimidazol-2-yl}-5-fluoroquinolin-2(1H)-one | 407.5 |
| 1131 | 4-amino-5-fluoro-3-{5-[(2R)-2-methylpiperazin-1-yl]-1H-benzimidazol-2-yl}quinolin-2(1H)-one | 393.4 |
| 1132 | 4-amino-3-{5-[(2R)-4-cyclobutyl-2-methylpiperazin-1-yl]-1H-benzimidazol-2-yl}-5-fluoroquinolin-2(1H)-one | 447.5 |
| 1133 | 4-amino-5-(dimethylamino)-3-[6-(4-isopropylpiperazin-1-yl)-1H-benzimidazol-2-yl]quinolin-2(1H)-one | 446.6 |
| 1134 | 4-amino-5-{[2-(dimethylamino)ethyl]amino}-3-[6-(4-isopropylpiperazin-1-yl)-1H-benzimidazol-2-yl]quinolin-2(1H)-one | 489.6 |
| 1135 | 4-amino-5-[[2-(dimethylamino)ethyl](methyl)amino]-3-[6-(4-isopropylpiperazin-1-yl)-1H-benzimidazol-2-yl]quinolin-2(1H)-one | 503.7 |
| 1136 | 4-amino-5-(ethylamino)-3-[6-(4-isopropylpiperazin-1-yl)-1H-benzimidazol-2-yl]quinolin-2(1H)-one | 446.6 |
| 1137 | N-[2-(4-amino-2-oxo(3-hydroquinolyl))benzimidazol-6-yl]-2-(dimethylamino)-N-methylacetamide | 391.4 |
| 1138 | 4-amino-5-fluoro-3-[6-(9-isopropyl-1-oxa-4,9-diazaspiro[5.5]undec-4-yl)-1H-benzimidazol-2-yl]quinolin-2(1H)-one | 491.6 |
| 1139 | 4-amino-7-fluoro-3-[6-fluoro-5-(4-methylpiperazin-1-yl)-1H-benzimidazol-2-yl]quinolin-2(1H)-one | 411.4 |
| 1140 | 4-amino-3-(5-{(2S,5S)-2-[(dimethylamino)methyl]-5-methylmorpholin-4-yl}-6-fluoro-1H-benzimidazol-2-yl)-5-fluoroquinolin-2(1H)-one | 469.5 |
| 1141 | 4-amino-3-(5-{(2S,5S)-2-[(dimethylamino)methyl]-5-methylmorpholin-4-yl}-6-fluoro-1H-benzimidazol-2-yl)quinolin-2(1H)-one | 451.5 |
| 1142 | 4-amino-5-methyl-3-[5-(4-methylpiperazin-1-yl)-1H-benzimidazol-2-yl]quinolin-2(1H)-one | 389.5 |
| 1143 | 4-amino-3-[5-(4-methylpiperazin-1-yl)-1H-benzimidazol-2-yl]-5-(trifluoromethyl)quinolin-2(1H)-one | 443.4 |
| 1144 | 4-amino-5-fluoro-3-[6-(2-isopropyl-5-oxa-2,8-diazaspiro[3.5]non-8-yl)-1H-benzimidazol-2-yl]quinolin-2(1H)-one | 463.5 |
| 1145 | 4-amino-6-fluoro-3-[5-(4-isopropylpiperazin-1-yl)-1H-benzimidazol-2-yl]quinolin-2(1H)-one | 421.5 |
| 1146 | N-[2-(4-amino-5-fluoro-2-oxo-1,2-dihydroquinolin-3-yl)-1H-benzimidazol-6-yl]-N-methyl-2-(4-methylpiperazin-1-yl)acetamide | 464.5 |
| 1147 | N-[2-(4-amino-5-fluoro-2-oxo-1,2-dihydroquinolin-3-yl)-1H-benzimidazol-6-yl]-N-methyl-2-morpholin-4-ylacetamide | 451.5 |
| 1148 | N-[2-(4-amino-5-fluoro-2-oxo(3-hydroquinolyl))benzimidazol-6-yl]-N-methyl-2-morpholin-4-ylacetamide | 492.6 |
| 1149 | 4-amino-5-fluoro-3-(6-methyl-1H-benzimidazol-2-yl)quinolin-2(1H)-one | 309.3 |
| 1150 | 4-amino-3-[5-(4-ethylpiperazin-1-yl)-1H-benzimidazol-2-yl]-5-methylquinolin-2(1H)-one | 403.5 |
| 1151 | 4-amino-3-{6-[(4-methylpiperazin-1-yl)methyl]-1H-benzimidazol-2-yl}quinolin-2(1H)-one | 389.5 |
| 1152 | 4-amino-3-[6-(1,4-diazepan-1-yl)-1H-benzimidazol-2-yl]-5-fluoroquinolin-2(1H)-one | 393.4 |
| 1153 | 4-amino-5-fluoro-3-[6-(4-methyl-1,4-diazepan-1-yl)-1H-benzimidazol-2-yl]quinolin-2(1H)-one | 407.5 |
| 1154 | 3-[6-(4-acetylpiperazin-1-yl)-1H-benzimidazol-2-yl]-4-amino-5-fluoroquinolin-2(1H)-one | 421.4 |
| 1155 | 4-amino-3-[6-(4-ethyl-1,4-diazepan-1-yl)-1H-benzimidazol-2-yl]-5-fluoroquinolin-2(1H)-one | 421.5 |
| 1156 | 4-amino-5-fluoro-3-[6-(4-isopropyl-1,4-diazepan-1-yl)-1H-benzimidazol-2-yl]quinolin-2(1H)-one | 435.5 |

Many of the above Examples (1-1156) displayed an IC₅₀ value of less than 10 µM with respect to Flt-1, KDR, PDGF, c-KIT, FLT-3, VEGF1, VEGFR2, c-Met, CSF-1, FGFR3 and/or bFGFR. Additionally, many of the above Examples displayed an IC₅₀ value of less than 10 µM with respect to PDGFR.

### In Vitro Activity of 4-Amino-5-fluoro-3-[6-(4-methylpiperazin-1-yl)-1H-benzimidazol-2-yl]quinolin-2(1H)-one Against Various RTKs

4-Amino substituted quinolinone benzimidazolyl compounds such as 4-amino-5-fluoro-3-[6-(4-methylpiperazin-1-yl)-1H-benzimidazol-2-yl]quinolin-2(1H)-one and tautomers and salts thereof are potent inhibitors of various kinases such as VEGFR2 (KDR, Flk-1), FGFR1 and PDGFRβ with IC₅₀s ranging from 10-27 nM. See U.S. Patent No. 6,605,617, U.S. Patent Application No. 10/644,055, and U.S. Patent Application No. 10/706,328, each of which is hereby incorporated by reference in its entirety and for all purposes as if fully set forth herein, for a list of various tyrosine and serine/threonine kinases for which 4-amino-5-fluoro-3-[6-(4-methylpiperazin-1-yl)-1H-benzimidazol-2-yl]quinolin-2(1H)-one has shown activity and for assay procedures. These RTKs are important for the initiation and maintenance of new blood vessel growth as well as tumor proliferation. Systematic profiling against class III-IV RTKs as well as a subset of RTKs from other classes shows potent inhibition of CSF-R1/c-fms, c-kit, flt3 and FGFR3. FGFR3 is abnormally expressed and in some cases constitutively activated in a subset of multiple myeloma patients as a consequence of the t(4;14) translocation (about 15-20%).

The effects of 4-amino substituted quinolinone benzimidazolyl compounds such as 4-amino-5-fluoro-3-[6-(4-methylpiperazin-1-yl)-1H-benzimidazol-2-yl]quinolin-2(1H)-one on multiple myeloma cell lines with the t(4;14) translocation were investigated with respect to effects on proliferation, cell cycle, apoptosis, and FGFR3 and ERK (extracellular regulated kinase) phosphorylation. Multiple myeloma presents with detrimental bone loss mainly mediated by the large increase in IL6 production and concomitant activation of osteoclasts responsible for bone resorption. M-CSF has a role in recruitment of osteoclast precursors and may promote their survival. Blocking signaling through the CSF-1R may thus provide additional benefit to multiple myeloma patients. Inhibition of M-CSF mediated proliferation of the murine myeloid cell line M-NFS-60 correlated with inhibition of *in vitro* kinase activity against c-fms/CSF-1R.

4-Amino substituted quinolinone benzimidazolyl compounds such as 4-amino-5-fluoro-3-[6-(4-methylpiperazin-1-yl)-1H-benzimidazol-2-yl]quinolin-2(1H)-one and tautomers and salts thereof act as potent inhibitors of Class III-V RTKs. IC₅₀ values of 4-amino-5-fluoro-3-[6-(4-methylpiperazin-1-yl)-1H-benzimidazol-2-yl]quinolin-2(1H)-one are presented in the following table.

**Table 9. Activity of 4-Amino-5-fluoro-3-[6-(4-methylpiperazin-1-yl)-1H-benzimidazol-2-yl]quinolin-2(1H)-one Against Various RTKs**

| RTK | IC₅₀ (µM) |
|---|---|
| FLT3 | 0.001 |
| c-KIT | 0.002 |
| CSFR1/c-fms | 0.036 |
| FGFR1 | 0.008 |
| FGFR3 | 0.009 |
| VEGFR1/Flt1 | 0.01 |
| VEGFR2/Flk1 | 0.013 |
| VEGFR3/Flt4 | 0.008 |
| PDGFRβ | 0.027 |
| PDGFRα | 0.21 |
| EGFR1 | 2 |
| c-MET | >3 |
| EphA2 | 4 |
| TIE2 | 4 |
| IGFR1 | >10 |
| HER2 | >10 |

The *in vitro* RTK assays used to prepare the above table were run in the presence of an ATP concentration that was within three-fold or at Kₘ of enzymes used (for enzymes where the Kₘ was available). Phosphorylated peptide substrate was detected with a Europium labeled anti-phospho-tyrosine Antibody (PT66). The Europium was then detected using time resolved fluorescence. For some assays, γ-P³³ ATP was incubated with the enzyme and the radioactivity of phosphorylated peptide substrate was quantified in the presence of various concentration of 4-amino-5-fluoro-3-[6-(4-methylpiperazin-1-yl)-1H-benzimidazol-2-yl]quinolin-2(1H)-one and used to calculate the IC₅₀.

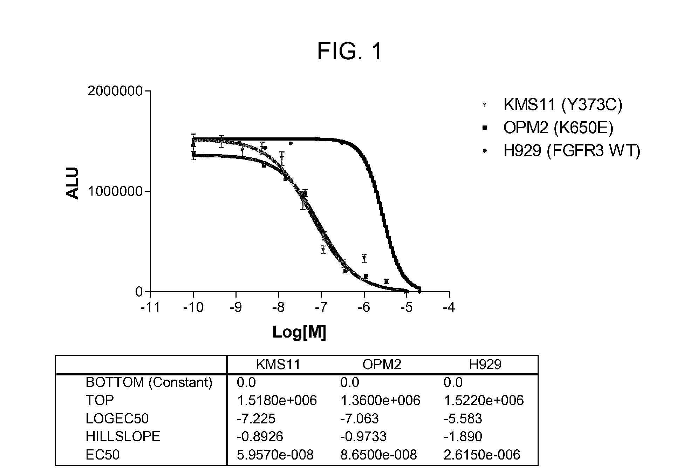

FIG. 1 shows that 4-amino-5-fluoro-3-[6-(4-methylpiperazin-1-yl)-1H-benzimidazol-2-yl]quinolin-2(1H)-one inhibits proliferation of multiple myeloma cell lines. KMS11, OPM-2, and H929 are multiple myeloma cell lines that were incubated with serial dilutions of 4-amino-5-fluoro-3-[6-(4-methylpiperazin-1-yl)-1H-benzimidazol-2-yl]quinolin-2(1H)-one. After 72 hours, the number of viable cells left was determined using the CellTiter-Glo™ Assay (Promega). KMS11 and OPM-2 have activating mutations in the FGFR3 receptor, and H929 expresses WT FGFR3. 4-Amino-5-fluoro-3-[6-(4-methylpiperazin-1-yl)-1H-benzimidazol-2-yl]quinolin-2(1H)-one inhibited FGFR3 receptor kinase (IC₅₀ = 9 nM, Table 9) and blocked proliferation of two cell lines with activating FGFR3 mutations: KMS11 (Y373C) and OPM-2 (K650E) cells with EC₅₀s of 60 nM and 87 nM, respectively (see FIG. 1). H929 cells express WT FGFR3 and mutant N-ras (13G>D), and proliferation was inhibited, but less potently, by 4-amino-5-fluoro-3-[6-(4-methylpiperazin-1-yl)-1H-benzimidazol-2-yl]quinolin-2(1H)-one in this cell line (EC₅₀ = 2.6 µM, EC₅₀ in serum reduced growth media = 0.6 µM).

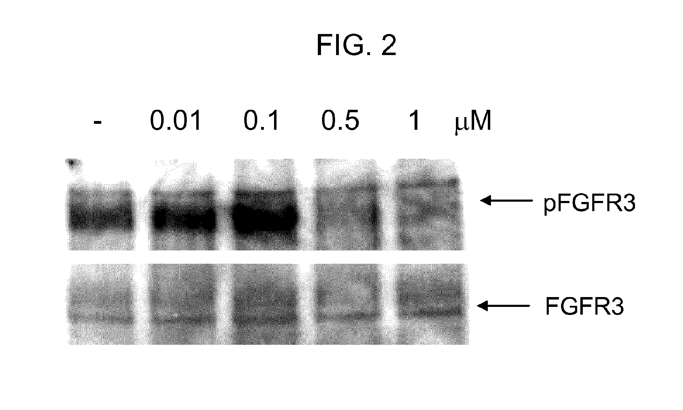

FGFR3 tyrosine phosphorylation was inhibited by 4-amino-5-fluoro-3-[6-(4-methylpiperazin-1-yl)-1H-benzimidazol-2-yl]quinolin-2(1H)-one at 0.5 µM in KMS11 cells (see FIG. 2). KMS11 cells were starved for two hours in growth media containing 1% FBS. The cells were then incubated with different concentrations of 4-amino-5-fluoro-3-[6-(4-methylpiperazin-1-yl)-1H-benzimidazol-2-yl]quinolin-2(1H)-one for two hours in growth media without FBS, washed and lysed for immunoprecipitation with FGFR3 Ab (sc123 Santa Cruz Biotech). Lysates were analyzed by western blotting and probed with anti-phosphotyrosine Antibody 4G10 (Upstate Biotech). The lower panel showed total FGFR3 after stripping the western blot and reprobing with FGFR3 Ab (See FIG. 2).

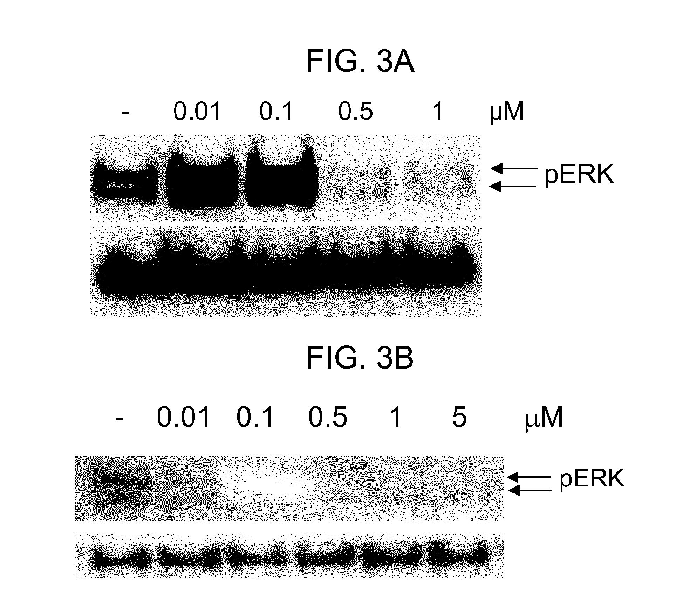

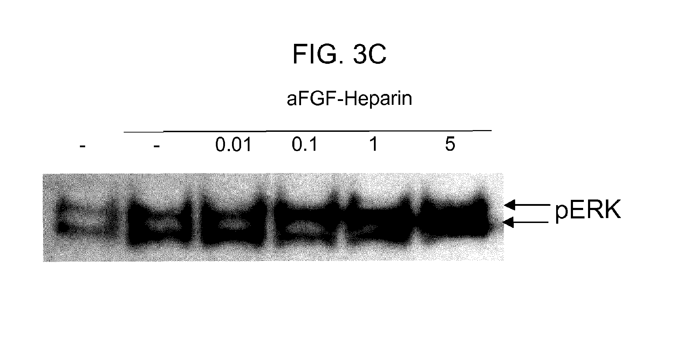

4-Amino-5-fluoro-3-[6-(4-methylpiperazin-1-yl)-1H-benzimidazol-2-yl]quinolin-2(1H)-one was found to inhibit ERK phosphorylation at 0.5 µM in KMS11 cells. KMS11 cells were starved for two hours in growth media containing 1% FBS. The cells were then incubated with different concentrations of 4-amino-5-fluoro-3-[6-(4-methylpiperazin-1-yl)-1H-benzimidazol-2-yl]quinolin-2(1H)-one for two hours in growth media without FBS, washed, lysed, and analyzed by western blotting and probed with anti phospho-ERK Antibody (Cell Signaling). The lower panel of FIG. 3A shows cyclophilin protein (Upstate Biotech) as a loading control. 4-Amino-5-fluoro-3-[6-(4-methylpiperazin-1-yl)-1H-benzimidazol-2-yl]quinolin-2(1H)-one also inhibited ERK phosphorylation at 0.1 µM in OPM-2 cells. OPM-2 cells were incubated with different concentration of 4-amino-5-fluoro-3-[6-(4-methylpiperazin-1-yl)-1H-benzimidazol-2-yl]quinolin-2(1H)-one for one hour in growth media with 1% FBS, washed, lysed, and analyzed by western blotting and probed with anti phospho-ERK Antibody (Cell Signaling). The lower panel of FIG. 3B shows 14-3-3 protein (Santa Cruz Biotech) as a loading control. ERK in the MAPK pathway is a downstream FGFR3 signaling component and phosphorylation of ERK was inhibited in both OPM-2 and KMS11 cells at 0.5 µM 4-amino-5-fluoro-3-[6-(4-methylpiperazin-1-yl)-1H-benzimidazol-2-yl]quinolin-2(1H)-one (See FIGS. 3A and 3B). In contrast, the compound had no effect on phospho-ERK levels up to 5 µM in H929 cells. H929 cells were starved for two days in growth media without FBS. The cells were then incubated with different concentrations of 4-amino-5-fluoro-3-[6-(4-methylpiperazin-1-yl)-1H-benzimidazol-2-yl]quinolin-2(1H)-one for one hour in growth media without FBS, washed, stimulated for 5 minutes with 50 ng/mL aFGF and 10 µg/mL Heparin, lysed, and analyzed by western blotting and probed with anti phospho-ERK Ab (Cell Signaling). Only a minor change in phospho-ERK in response to stimulation with aFGF after two days of serum starvation indicated that the pathway is constitutively activated due to the Ras mutation (See FIG. 3C).

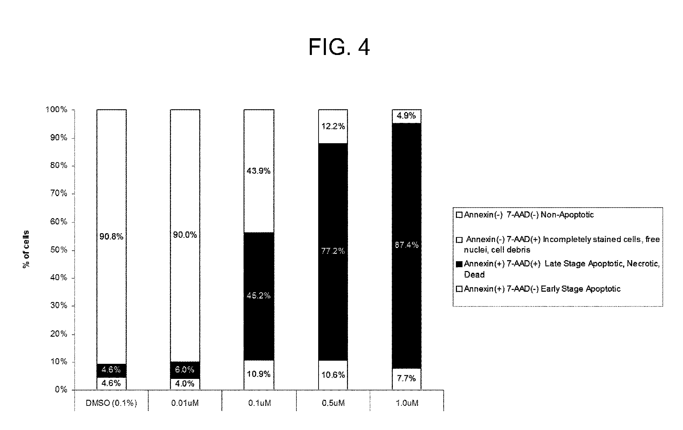

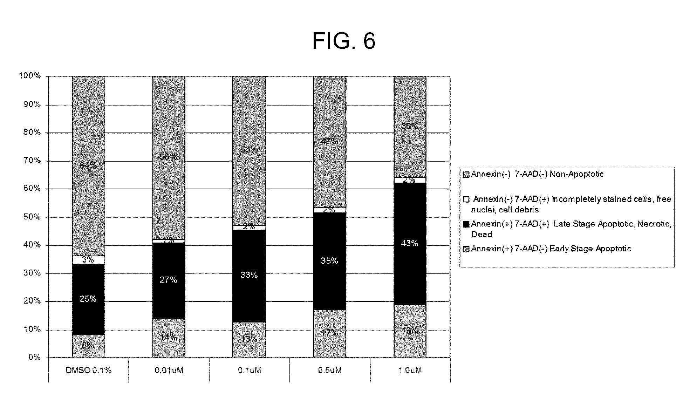

KMS11 cells were incubated with 4-amino-5-fluoro-3-[6-(4-methylpiperazin-1-yl)-1H-benzimidazol-2-yl]quinolin-2(1H)-one at various concentrations for 96 hours. The incubated KMS11 1 cells were washed and stained with Δnnexin VPE and 7AAD according to the Nexin assay protocol (Guava Technologies). Samples were run on Guava PCA^{™} instrument and percentage of cells in each category were analyzed with the Guava Nexin^{™} software. OPM-2 cells were incubated with 4-amino-5-fluoro-3-[6-(4-methylpiperazin-1-yl)-1H-benzimidazol-2-yl]quinolin-2(1H)-one at various concentrations for 72 hours. The incubated OPM-2 cells were washed and stained with Δnnexin VPE and 7AAD according to the Nexin assay protocol (Guava Technologies). Samples were run on Guava PCAT™ instrument and percentage of cells in each category were analyzed with the Guava Nexin™ software. Results of the above experiments show that 4-amino-5-fluoro-3-[6-(4-methylpiperazin-1-yl)-1H-benzimidazol-2-yl]quinolin-2(1H)-one induced apoptosis as measured by Annexin VPE staining in KMS11 and OPM-2 cells starting at concentrations of 0.1 µM and 0.5 µM respectively (FIGS. 4 and 6).

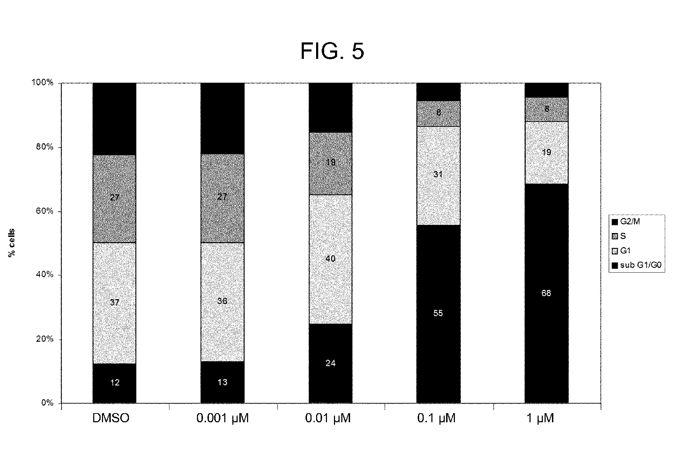

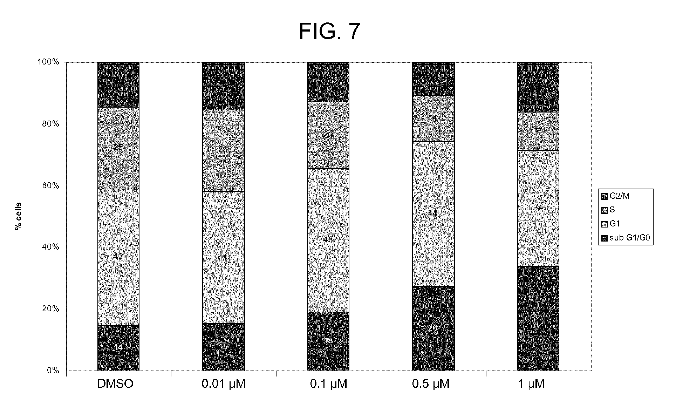

The experimental data regarding induction of apoptosis by 4-amino-5-fluoro-3-[6-(4-methylpiperazin-1-yl)-1H-benzimidazol-2-yl]quinolin-2(1H)-one in KMS11 and OPM-2 cells was confirmed by significant increases in the sub G1 population of cells in a cell cycle analysis observed at concentrations of 4-amino-5-fluoro-3-[6-(4-methylpiperazin-1-yl)-1H-benzimidazol-2-yl]quinolin-2(1H)-one of 0.1 µM and higher (FIG. 5). KMS11 cells were incubated with 4-amino-5-fluoro-3-[6-(4-methylpiperazin-1-yl)-1H-benzimidazol-2-yl]quinolin-2(1H)-one at concentrations of 0.001 µM, 0.01 µM, 0.1 µM, and 1 µM for 72 hours. Cells were then fixed and stained with propidium iodide before analyzing the samples by FACS (See FIG. 5). These results showed that the compound has minor effects on the cell cycle, but induced apoptosis in KMS11 1 cells at 0.1 µM. OPM-2 cells were also incubated with 4-amino-5-fluoro-3-[6-(4-methylpiperazin-1-yl)-1H-benzimidazol-2-yl]quinolin-2(1H)-one at concentrations of 0.001 µM, 0.01 µM, 0.1 µM, and 1 µM for 72 hours. Cells were similarly fixed and stained with propidium iodide before analyzing the samples by FACS (See FIG. 7). These results showed that the compound has minor effects on the cell cycle, but induced apoptosis in OPM-2 cells at 0.5 µM. Other effects on the cell cycle by the compound were minor e.g., there was no significant G1 arrest. Increases in the sub G1 population were less significant in the OPM-2 cell line compared to the KMS11 cells and started at 0.5 µM (FIG. 7).

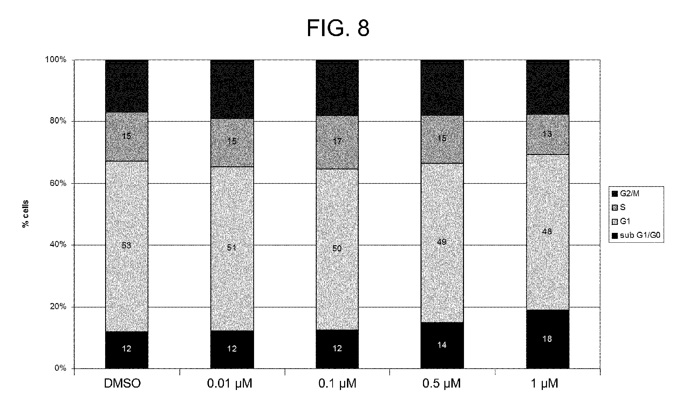

H929 cells were incubated with 4-amino-5-fluoro-3-[6-(4-methylpiperazin-1-yl)-1H-benzimidazol-2-yl]quinolin-2(1H)-one at concentrations of 0.01 µM, 0.1 µM, 0.5 µM, and 1 µM for 72 hours. Cells were then fixed and stained with propidium iodide before analyzing the samples by FACS (See FIG. 8). 4-Amino-5-fluoro-3-[6-(4-methylpiperazin-1-yl)-1H-benzimidazol-2-yl]quinolin-2(1H)-one had no effects on the cell cycle in H929 cells with concentrations up to 1 µM confirming that the FGFR3 expressing N-ras mutant cell line is less sensitive to 4-amino-5-fluoro-3-[6-(4-methylpiperazin-1-yl)-1H-benzimidazol-2-yl]quinolin-2(1H)-one (FIG. 8) than are the KMS11 and OPM-2 cells.

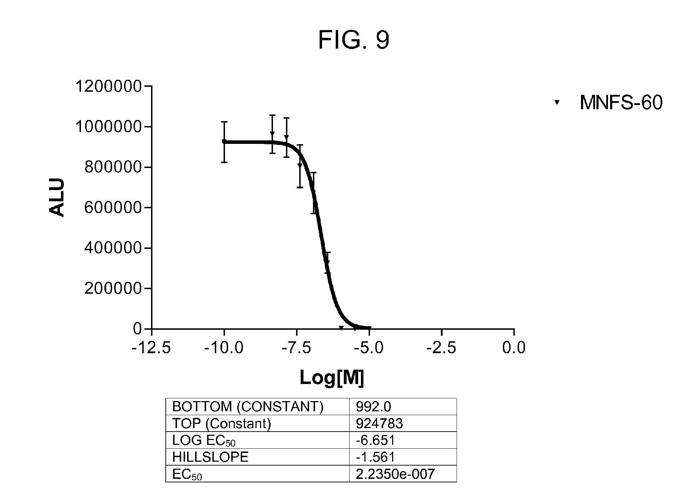

Osteolytic bone loss is one of the major complications in multiple myeloma disease. The major cytokines involved in bone resorption are IL1β and IL6. In addition, increased serum concentrations of M-CSF have been detected in patients. 4-Amino-5-fluoro-3-[6-(4-methylpiperazin-1-yl)-1H-benzimidazol-2-yl]quinolin-2(1H)-one inhibits CSF-1R activity, the only known receptor for M-CSF with an IC₅₀ of 36 nM (See Table 9). M-CSF mediated proliferation of a mouse myeloblastic cell line M-NFS-60 was inhibited with an EC₅₀ of 220 nM (FIG. 9). Murine M-NFS-60 cells were incubated with serial dilutions of 4-amino-5-fluoro-3-[6-(4-methylpiperazin-1-yl)-1H-benzimidazol-2-yl]quinolin-2(1H)-one in assay media with 10 ng/mL M-CSF and without GM-CSF. Cells in control wells were incubated with assay media only. After 72 hours incubation time, the number of viable cells left was determined using the CellTiter-Glo™ Assay (Promega). EC₅₀ values were determined using nonlinear regression (FIG. 9).

4-Amino-5-fluoro-3-[6-(4-methylpiperazin-1-yl)-1H-benzimidazol-2-yl]quinolin-2(1H)-one has significant antiproliferative activity and inhibits FGFR3 receptor phosphorylation and ERK phosphorylation in multiple myeloma cell lines with activating FGFR3 mutations. Therefore, the invention provides a method for inhibiting FGFR3 receptor phosphorylation and ERK phosphorylation in multiple myeloma cell lines with activating FGFR3 mutations which includes administering an effective amount of a 4-amino substituted quinolinone benzimidazolyl compound, a tautomer thereof, a salt of the 4-amino substituted quinolinone benzimidazolyl compound, a salt of the tautomer, a combination thereof, or a pharmaceutical formulation comprising the 4-amino substituted quinolinone benzimidazolyl compound, the tautomer thereof, the salt of the 4-amino substituted quinolinone benzimidazolyl compound, the salt of the tautomer, or the combination thereof to a subject with a multiple myeloma cell line with activating FGFR3 mutations, wherein inhibition of FGFR3 receptor phosphorylation and/or ERK phosphorylation is inhibited after administration of the compound or the pharmaceutical formulation. In some embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one. In some embodiments, the subject is a mammal such as a rodent or primate. In some such embodiments, the subject is a mouse, whereas in other embodiments the subject is a human. The invention further provides the use of a 4-amino substituted quinolinone benzimidazolyl compound, a tautomer thereof, a salt of the 4-amino substituted quinolinone benzimidazolyl compound, a salt of the tautomer, or a combination thereof, in the preparation of a medicament for inhibiting the FGFR3 receptor phosphorylation and/or ERK phosphorylation. In some such embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one.

4-Amino-5-fluoro-3-[6-(4-methylpiperazin-1-yl)-1H-benzimidazol-2yl]quinolin-2(1H)-one caused apoptosis, but had minor effects on the cell cycle in FGFR3 mutant cell lines at concentrations of < 0.5 µM. Therefore, the invention provides a method of inducing apoptosis in FGFR3 mutant cell lines which, in some embodiments, is not accompanied by a large effect on the cell cycle. The method includes administering an effective amount of an effective amount of a 4-amino substituted quinolinone benzimidazolyl compound, a tautomer thereof, a salt of the 4-amino substituted quinolinone benzimidazolyl compound, a salt of the tautomer, a combination thereof, or a pharmaceutical formulation comprising the 4-amino substituted quinolinone benzimidazolyl compound, the tautomer thereof, the salt of the 4-amino substituted quinolinone benzimidazolyl compound, the salt of the tautomer, or the combination thereof to a subject with a multiple myeloma cell line with activating FGFR3 mutations, wherein apoptosis in FGFR3 mutant cell lines is induced following administration. In some embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one. In some embodiments, the subject is a mammal such as a rodent or primate. In some such embodiments, the subject is a mouse, whereas in other embodiments the subject is a human. The invention further provides the use of a 4-amino substituted quinolinone benzimidazolyl compound, a tautomer thereof, a salt of the 4-amino substituted quinolinone benzimidazolyl compound, a salt of the tautomer, or a combination thereof, in the preparation of a medicament for inducing apoptosis in FGFR3 mutant cell lines, which in some embodiments, is not accompanied by a large effect on the cell cycle when incubated for the indicated times. In some such embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one.

Inhibition of M-CSF mediated proliferation of the murine myeloid cell line M-NFS-60 correlated with inhibition of the *in vitro* kinase activity of CSF-1R by 4-amino-5-fluoro-3-[6-(4-methylpiperazin-1-yl)-1H-benzimidazol-2-yl]quinolin-2(1H)-one. Potent activity of 4-amino-5-fluoro-3-[6-(4-methylpiperazin-1-yl)-1H-benzimidazol-2-yl]quinolin-2(1H)-one against t(4:14) multiple myeloma cell lines especially those with activating FGFR3 were observed. Furthermore, this compound and salts and tautomers thereof may be used to protect patients with multiple myeloma from osteolytic bone loss and lesions. Therefore, in some embodiments, the invention provides a method of inhibiting M-CSF mediated proliferation of myeloid cell lines and inhibiting CSF-1R activity. The method comprises administering an effective amount of an effective amount of a 4-amino substituted quinolinone benzimidazolyl compound, a tautomer thereof, a salt of the 4-amino substituted quinolinone benzimidazolyl compound, a salt of the tautomer, a combination thereof, or a pharmaceutical formulation comprising the 4-amino substituted quinolinone benzimidazolyl compound, the tautomer thereof, the salt of the 4-amino substituted quinolinone benzimidazolyl compound, the salt of the tautomer, or the combination thereof to a subject with a myeloid cell line, wherein M-CSF mediated proliferation of myeloid cell lines and/or CSF-1R activity is inhibited. In some embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one. The invention further provides the use of a 4-amino substituted quinolinone benzimidazolyl compound, a tautomer thereof, a salt of the 4-amino substituted quinolinone benzimidazolyl compound, a salt of the tautomer, or a combination thereof, in the preparation of a medicament for inhibiting M-CSF mediated proliferation of myeloid cell lines and/or CSF-1R activity. In some such embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one. The invention also provides a method of reducing osteolytic bone loss or lesions in subjects with multiple myeloma, the method comprising administering effective amount of an effective amount of a 4-amino substituted quinolinone benzimidazolyl compound, a tautomer thereof, a salt of the 4-amino substituted quinolinone benzimidazolyl compound, a salt of the tautomer, a combination thereof, or a pharmaceutical formulation comprising the 4-amino substituted quinolinone benzimidazolyl compound, the tautomer thereof, the salt of the 4-amino substituted quinolinone benzimidazolyl compound, the salt of the tautomer, or the combination thereof to a subject with multiple myeloma, wherein a reduction in osteolytic bone loss or lesions is observed in the subject after administration. In some embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one. In some embodiments, the subject is a mammal such as a rodent or primate. In some such embodiments, the subject is a mouse, whereas in other embodiments the subject is a human. The invention further provides the use of a 4-amino substituted quinolinone benzimidazolyl compound, a tautomer thereof, a salt of the 4-amino substituted quinolinone benzimidazolyl compound, a salt of the tautomer, or a combination thereof, in the preparation of a medicament for reducing osteolytic bone loss or lesions in subjects with multiple myeloma. In some such embodiments, the 4-amino substituted quinolinone benzimidazolyl compound is 4-amino-5-fluoro-3-[6-(4-methylpiperazin-1-yl)-1H-benzimidazol-2-yl]quinolin-2(1H)-one.

### TREATMENT OF MULTIPLE MYELOMA

The t(4:14) translocation that occurs uniquely in a subset (20%) of multiple myeloma (MM) patients results in the ectopic expression of the receptor tyrosine kinase (RTK), fibroblast growth factor receptor 3 (FGFR3). Inhibition of activated FGFR3 in MM cells induces apoptosis, validating FGFR3 as a therapeutic target in t(4;14) MM and encouraging the clinical development of FGFR3 inhibitors for the treatment of these poor-prognosis patients. 4-Amino substituted quinolinone benzimidazolyl compounds such as 4-amino-5-fluoro-3-[6-(4-methylpiperazin-1-yl)-1H-benzimidazol-2-yl]quinolin-2(1H)-one, act as inhibitors of FGFR3. 4-Amino-5-fluoro-3-[6-(4-methylpiperazin-1-yl)-1H-benzimidazol-2-yl]quinolin-2(1H)-one potently inhibits FGFR3 with IC₅₀ of 5 nM in *in vitro* kinase assays and selectively inhibited the growth of B9 cells and human myeloma cell lines expressing wild-type (WT) or activated mutant FGFR3. In responsive cell lines, 4-amino-5-fluoro-3-[6-(4-methylpiperazin-1-yl)-1H-benzimidazol-2-yl]quinolin-2(1H)-one induced cytostatic and cytotoxic effects. Importantly, addition of interleukin-6 (IL-6), insulin growth factor 1 (IGF-1) or co-culture on stroma did not confer resistance to 4-amino-5-fluoro-3-[6-(4-methylpiperazin-1-yl)-1H-benzimidazol-2-yl]quinolin-2(1H)-one. In primary myeloma cells from t(4;14) patients, 4-amino-5-fluoro-3-[6-(4-methylpiperazin-1-yl)-1H-benzimidazol-2-yl]quinolin-2(1H)-one inhibited downstream ERK1/2 phosphorylation with an associated cytotoxic response. Finally, therapeutic efficacy of 4-Amino substituted quinolinone benzimidazolyl compounds such as 4-amino-5-fluoro-3-[6-(4-methylpiperazin-1-yl)-1H-benzimidazol-2-yl]quinolin-2(1H)-one was demonstrated in a xenograft mouse model of FGFR3 MM. 4-Amino substituted quinolinone benzimidazolyl compounds such as 4-amino-5-fluoro-3-[6-(4-methylpiperazin-1-yl)-1H-benzimidazol-2-yl]quinolin-2(1H)-one are potent inhibitors of FGFR3-transformed hematopoietic cell lines and human multiple myeloma cell lines expressing either WT or mutant FGFR3. In addition, these compounds are potent inhibitors in a mouse model of FGFR3-mediated MM and are cytotoxic to primary myeloma cells from t(4;14) patients. Taken together, these data indicate that 4-amino substituted quinolinone benzimidazolyl compounds such as 4-amino-5-fluoro-3-[6-(4-methylpiperazin-1-yl)-1H-benzimidazol-2-yl]quinolin-2(1H)-one have significant potential in treating MM associated with FGFR3 expression.

### METHODS

### Chemical Compounds and Biological Reagents

4-Amino-5-fluoro-3-[6-(4-methylpiperazin-1-yl)-1H-benzimidazol-2-yl]quinolin-2(1H)-one was dissolved in DMSO at a stock concentration of 20 mM. For animal experiments, 4-amino-5-fluoro-3-[6-(4-methylpiperazin-1-yl)-1H-benzimidazol-2-yl]quinolin-2(1H)-one was formulated in 5 mM citrate buffer. Acidic FGF (aFGF) and heparin were purchased from R&D Systems (Minneapolis, MN) and Sigma (Ontario, Canada), respectively. FGFR3 antibodies (C15, H100 and B9) were obtained from Santa Cruz Biotechnology (Santa Cruz, CA), and 4G10 from Upstate Biotechnology (Lake Placid, NY).

### In Vitro Kinase Assays

The IC₅₀ values for the inhibition of RTKs by 4-amino-5-fluoro-3-[6-(4-methylpiperazin-1-yl)-1H-benzimidazol-2-yl]quinolin-2(1H)-one were determined in a time resolved fluorescence (TRF) or radioactive format, measuring the inhibition by 4-amino-5-fluoro-3-[6-(4-methylpiperazin-1-yl)-1H-benzimidazol-2-yl]quinolin-2(1H)-one of phosphate transfer to a substrate by the respective enzyme. Briefly, the respective RTK domain was expressed or purchased as recombinant protein and incubated with serial dilutions of 4-amino-5-fluoro-3-[6-(4-methylpiperazin-1-yl)-1H-benzimidazol-2-yl]quinolin-2(1H)-one in the presence of substrate and ATP concentrations within 2-3 times the Kₘ of the enzyme. IC₅₀ values were calculated using non-linear regression and represent the average of at least 2 experiments.

### FGFR3 Expression Vectors and B9 Cell Transfectants

B9 cells expressing WT FGFR3 (B9-WT), FGFR3-K650E (B9-K650E) and empty retrovirus (B9-MINV) have been described previously. Plowright, E. E. et al., Blood, 2000; 95:992-998. Full-length FGFR3 cDNAs, containing F384L, Y373C, or J807C (gift of Marta Chesi, Weill Medical College of Cornell, New York, NY) were cloned into an MSCV-based retroviral vector containing a green fluorescent protein (GFP) cassette. A construct carrying the G384D mutation was created from the FGFR3-WT by replacing the PmlI-BglII fragment between amino acid 290 and 413 with the same fragment obtained from the KMS18 as previously described. Ronchetti, D. et al., Oncogene, 2001; 20:3553-3562. The constructed retroviral vectors were transfected into GP-E ecotropic packaging cells. The resulting retroviruses were used to introduce FGFR3 into the IL-6 dependent murine myeloma cell line, B9. A limiting cell dilution was further performed to generate single cell clones. A high-expressing clone for each construct (B9-F384L, B9-Y373C, B9-G384D and B9-J807C) was cryopreserved.

### Cell Lines and Tissue Culture

All human MM cell lines and B9 cells were maintained in Iscove's Modified Dulbecco's Medium (IMDM) supplemented with 5% FCS, 100 g/ml penicillin and 100 g/ml streptomycin (Gibco, Invitrogen Canada, Ontario) and 1% IL-6 conditioned medium (B9 cells only). BM stroma cells (BMSCs) were derived from BM specimens obtained from MM patients. Mononuclear cells separated by Ficoll-Hipaque density sedimentation were used to establish long-term cultures, as described previously. Hideshima, T. et al., Blood, 2000; 96:2943-2950. For the purposes of viability assays BMSCs were irradiated with 20 Gy after plating on 96 well plates.

### Viability Assay

Cell viability was assessed by 3-(4,5-dimethylthiazol)-2,5-diphenyl tetrazolium (MTT) dye absorbance. Cells were seeded in 96-well plates at a density of 5,000 (B9 cells) or 20,000 (MM cell lines) cells per well in IMDM with 5% FCS. Cells were incubated with 30 ng/ml aFGF and 100 g/ml heparin or 1% IL-6 where indicated and increasing concentrations of 4-amino-5-fluoro-3-[6-(4-methylpiperazin-1-yl)-1H-benzimidazol-2-yl]quinolin-2(1H)-one. For each concentration of 4-amino-5-fluoro-3-[6-(4-methylpiperazin-1-yl)-1H-benzimidazol-2-yl]quinolin-2(1H)-one, 10 1 aliquots of drug or DMSO diluted in culture medium was added. For drug combination studies, cells were incubated with 0.5 M dexamethasone, 100 nM 4-amino-5-fluoro-3-[6-(4-methylpiperazin-1-yl)-1H-benzimidazol-2-yl]quinolin-2(1H)-one or both simultaneously where indicated. To evaluate the effect of 4-amino-5-fluoro-3-[6-(4-methylpiperazin-1-yl)-1H-benzimidazol-2-yl]quinolin-2(1H)-one on growth of MM cells adherent to BMSCs, 10,000 KMS11 cells were cultured on BMSC-coated 96-well plates, in the presence or absence of 4-amino-5-fluoro-3-[6-(4-methylpiperazin-1-yl)-1H-benzimidazol-2-yl]quinolin-2(1H)-one. Plates were incubated for 48 to 96 hours at 37°C, 5% CO₂. The MTT assay was performed according to the manufacturer's instruction (Boehringer Mannheim, Mannheim, Germany). For assessment of macrophage-colony stimulating factor (M-CSF) mediated growth, 5000 M-NFS-60 cells per well were incubated with serial dilutions of 4-amino-5-fluoro-3-[6-(4-methylpiperazin-1-yl)-1H-benzimidazol-2-yl]quinolin-2(1H)-one in media with 10 ng/ml M-CSF and without granulocytemacrophage-colony stimulating factor (GM-CSF). After 72 hours, cell viability was determined using Cell Titer-Glo™ Assay (Promega, Madison, WI). EC₅₀ values were determined using non-linear regression. Each experimental condition was performed in triplicate.

### Intracellular Phospho-Protein Staining

Determination of ERK1/2 phosphorylation by flow cytometry has been described previously. Chow, S. et al., Cytometry, 2001; 46:72-78; and Irish, J. M. et al., Cell, 2004; 118:217-228Briefly, cells were serum starved overnight and then stimulated with 30 ng/ml aFGF and 10 g/ml heparin for 10 minutes at 37°C. The cells were immediately fixed by adding 10% formaldehyde directly into the culture medium to obtain a final concentration of 2%. Cells were incubated in fixative for 10 minutes at 37°C then on ice for an additional 2 minutes. The cells were permeabilized by adding ice-cold methanol (final concentration of 90%) and incubated on ice for 30 minutes. Cells were stained with anti-ERK1/2 (Cell Signaling Technology, Beverly, MA) for 15 minutes and labeled with FITC-conjugated goat anti-rabbit and anti-CD138-PE (PharMinogen, San Diego, CA) where indicated. Malignant cells were identified as cells that express high levels of CD138. Flow cytometry was performed on a FACS Caliber flow cytometer (BD Biosciences, San Jose, CA) and analyzed using Cellquest software (Becton Dickinson).

### Apoptosis Analysis

For studies of apoptosis, cells were seeded at an initial density of 2 x 10⁵/ml medium supplemented with DMSO, 100 nM or 500 nM 4-amino-5-fluoro-3-[6-(4-methylpiperazin-1-yl)-1H-benzimidazol-2-yl]quinolin-2(1H)-one and cultured for up to 6 days. The medium and drug were replenished every 3 days, and the cell density was adjusted to 2 x 10⁵/ml. Apoptosis was determined by Annexin V staining (Boehringer Mannheim, Indianapolis, IN) and analyzed by flow cytometry.

### Primary Patient Samples

Patients identified for the study were determined to possess a t(4;14) translocation by fluorescence in situ hybridization (FISH). Expression of FGFR3 was confirmed by flow cytometry as described previously. Chesi, M. et al., Blood, 2001; 97:729-736. Briefly, erythrocytes were lysed and BM mononuclear cells were incubated on ice for 30 minutes with rabbit anti-FGFR3 (H100) or rabbit preimmune serum. The cells were stained with FITC-conjugated goat antirabbit IgG and mouse anti-CD138-PE to identify MM cells. The samples were then analyzed by flow cytometry.

All t(4;14) positive samples were further analyzed for the presence of FGFR3 or Ras mutations. Four pairs of primers were designed to amplify the regions of FGFR3-containing codons of the extracellular (EC) domain, transmembrane (TM) domain tyrosine kinase (TK) domain and stop codon (SC), known hot spots for activating mutations. Two pairs of primers were designed to amplify regions of codons 12, 13, and 61 of N-*ras* and K-*ras.* Chesi, M. et al., Blood, 2001; 97:729-736. A first PCR reaction was performed on genomic DNA extracted from CD138 purified myeloma cells and amplicons were used for DHPLC analysis. Results were confirmed by sequence analysis of the PCR products.

For cell death analysis, mononuclear cells were separated by Ficoll-Hipaque gradient sedimentation and plated at a cell density of 5 x 10⁵ cells/ml in IMDM supplemented with 20% FCS and 30 ng/ml aFGF and 10 g/ml heparin. Cells were cultured in the presence of DMSO or 500 nM 4-amino-5-fluoro-3-[6-(4-methylpiperazin-1-yl)-1H-benzimidazol-2-yl]quinolin-2(1H)-one for up to 12 days. The medium, aFGF/heparin and drug were replenished every 3 days. After 3, 7 and 12 days, cells were triple stained with anti-CD38-PE, anti-CD45-CyChrome (PharMinogen) and FITC-conjugated Annexin V as previously described. LeBlanc, R. et al., Cancer Res., 2002; 62:4996-5000. Controls included unstained cells, isotype control stained cells, and single-stained cells. Malignant cells plasma cells were defined as cells that express high levels of CD38 and no or low levels of CD45 (CD38⁺⁺/CD45⁻). Samples were analyzed by FACScan analysis using Cellquest software. BM aspirates were obtained by consent under an IRB-approved protocol.

### Xenograft Mouse Model

The xenograft mouse model was prepared as previously described. Mohammadi, M. et a.,l Embo. J., 1998; 17:5896-5904. Briefly, six to eight week old female BNX mice obtained from Frederick Cancer Research and Development Centre (Frederick, MD) were inoculated s.c. into the right flank with 3 x 10⁷ KMS11 1 cells in 150 1 of IMDM, together with 150 1 of matrigel basement membrane matrix (Becton Dickinson, Bedford, MA). Treatment was initiated when tumors reached volumes of approximately 200 mm³ at which time mice were randomized to receive 10, 30 or 60 mg/kg 4-amino-5-fluoro-3-[6-(4-methylpiperazin-1-yl)-1H-benzimidazol-2-yl]quinolin-2(1H)-one or 5 mM citrate buffer. Dosing was performed daily by gavage and continued for 21 days. Eight to 10 mice were included in each treatment group. Calliper measurements were performed twice weekly to estimate tumor volume, using the formula: 4 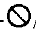 /3 x (width/2)² x (length/2). One way analysis of variance was used to compare differences between vehicle and 4-amino-5-fluoro-3-[6-(4-methylpiperazin-1-yl)-1H-benzimidazol-2-yl]quinolin-2(1H)-one treated groups.

### Immunoprecipitation and Immunoblotting

Immunoprecipitation and immunoblotting were performed as described previously. LeBlanc, R. et al., Cancer Res., 2002; 62:4996-5000. Briefly, tumors from sacrificed mice were immediately homogenized on ice and lysed in detergent buffer. Clarified cell extracts (1 mg/sample) were incubated for 6 hours with C15 FGFR3 antibody, then protein A/G agarose (Santa Cruz) was added for an additional 2 hours. Immunoblotting was performed with anti-phosphotyrosine antibody, 4G10 to assess phosphorylated FGFR3, or with anti-FGFR3 (B9) to measure total FGFR3.

### Histopathology and Immunohistochemical Analysis

Tissue samples were fixed in 10% formalin and embedded in paraffin, from which 5 m histologic sections were cut and stained with hematoxylin and eosin. Immunohistochemistry (IHC) studies were performed by indirect immunoperoxidase staining of paraffin tissue sections using a TechMate500™ BioTek automated immunostainer (Ventana Medical Systems, Inc., Tucson, AZ) and antibodies recognizing FGFR3 (C15), Ki-67 (Zymed, San Francisco, CA), and cleaved caspase 3 (Signaling Cell Technology) as previously described.

### RESULTS OF MULTIPLE MYELOMA STUDIES

### Selective Kinase Inhibition of 4-Amino-5-fluoro-3-[6-(4-methylpiperazin-1-yl)-1H-benzimidazol-2-yl]quinolin-2(1H)-one

The ability of 4-amino-5-fluoro-3-[6-(4-methylpiperazin-1-yl)-1H-benzimidazol-2-yl]quinolin-2(1H)-one to inhibit exogenous substrate phosphorylation was tested against a wide range of kinases. The concentration of 4-amino-5-fluoro-3-[6-(4-methylpiperazin-1-yl)-1H-benzimidazol-2-yl]quinolin-2(1H)-one resulting in a 50% reduction in the activity of receptor tyrosine kinases (IC₅₀) is reported in Table 9. 4-amino-5-fluoro-3-[6-(4-methylpiperazin-1-yl)-1H-benzimidazol-2-yl]quinolin-2(1H)-one inhibited members of the class III RTKs including FLT3, c-Kit, CSF-R1 and PDGFRα/ 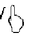 □ with IC₅₀ values of 0.001-0.21 mM as assessed by *in vitro* kinase assays. In addition, 4-amino-5-fluoro-3-[6-(4-methylpiperazin-1-yl)-1H-benzimidazol-2-yl]quinolin-2(1H)-one potently inhibited class IV (FGFR1 and 3) and class V (VEGFR1-4) RTKs with IC₅₀ values of 0.008-0.013 mM. When similar kinase assays for InsR, EGFR, c-MET, EphA2, TIE2, IGFR1 and HER2 were performed, significant inhibition was observed only at > 10-fold higher concentrations. These studies demonstrated that 4-amino-5-fluoro-3-[6-(4-methylpiperazin-1-yl)-1H-benzimidazol-2-yl]quinolin-2(1H)-one is a selective but multi-targeted inhibitor of class III, IV and V RTKs with high potency against FGFRs.

### 4-Amino-5-fluoro-3-[6-(4-methylpiperazin-1-yl)-1H-benzimidazol-2-yl]quinolin-2(1H)-one Inhibits the Growth of WT and Mutant FGFR3 Transformed Cells

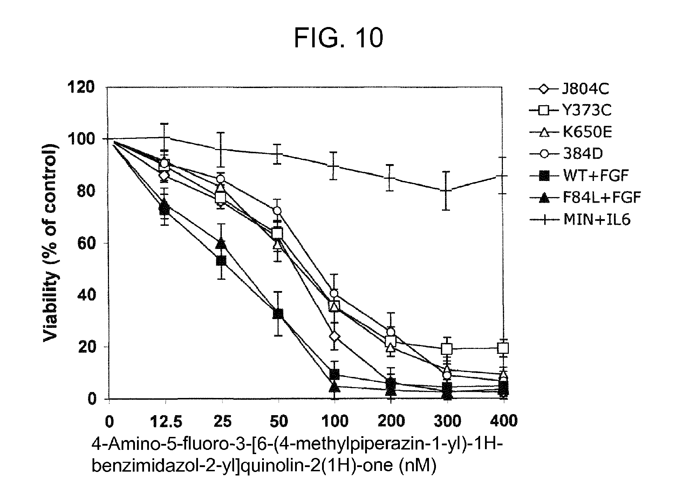

The ability of 4-amino-5-fluoro-3-[6-(4-methylpiperazin-1-yl)-1H-benzimidazol-2-yl]quinolin-2(1H)-one to inhibit constitutively activated FGFR3 mutants identified in MM patients (Y373C, G384D, K650E, J807C) was also tested. Chesi, M. et al., Blood, 2001; 97:729-736; and Ely, S. A. et al., Cancer, 2000; 89:445-452. Stable expression of these cDNAs conferred IL-6 independent growth to B9 cells, demonstrating that these mutants retain biologic activity and providing a platform for testing potential FGFR3 inhibitors against various classes of FGFR3 mutations. To determine the effect of 4-amino-5-fluoro-3-[6-(4-methylpiperazin-1-yl)-1H-benzimidazol-2-yl]quinolin-2(1H)-one on FGFR3-mediated cell growth, B9 cells expressing FGFR3-WT, FGFR3-F384L (a non-transforming polymorphism) and the FGFR3-activated mutants were grown in increasing concentrations of inhibitor for 48 hours exposure following which viability was determined by MTT assay (FIG. 10). As expected, 4-amino-5-fluoro-3-[6-(4-methylpiperazin-1-yl)-1H-benzimidazol-2-yl]quinolin-2(1H)-one potently inhibited the FGF-stimulated growth of WT and F384L-FGFR3 expressing B9 cells with IC₅₀ values of 25 nM. In addition, 4-amino-5-fluoro-3-[6-(4-methylpiperazin-1-yl)-1H-benzimidazol-2-yl]quinolin-2(1H)-one inhibited proliferation of B9 cells expressing each of the various activated mutants of FGFR3. Interestingly, there were minimal observed differences in the sensitivity of the different FGFR3 mutations to 4-amino-5-fluoro-3-[6-(4-methylpiperazin-1-yl)-1H-benzimidazol-2-yl]quinolin-2(1H)-one, with the IC₅₀ ranging from 70-90 nM for each of the various mutations. IL-6 dependent B9 cells 11 containing vector only (B9-MINV) were used to detect non-specific toxicity. B9-MINV cells were resistant to the inhibitory activity of 4-amino-5-fluoro-3-[6-(4-methylpiperazin-1-yl)-1H-benzimidazol-2-yl]quinolin-2(1H)-one at concentrations up to 1 M. These data further confirm *the in vitro* kinase data demonstrating inhibition of FGFR3 by 4-amino-5-fluoro-3-[6-(4-methylpiperazin-1-yl)-1H-benzimidazol-2-yl]quinolin-2(1H)-one and indicate that nonspecific cytotoxic effects are not observed within the effective range of drug concentration. These results also indicate that 4-amino-5-fluoro-3-[6-(4-methylpiperazin-1-yl)-1H-benzimidazol-2-yl]quinolin-2(1H)-one has potent activity against a variety of activated mutants of FGFR3 described in MM.

### 4-Amino-5-fluoro-3-[6-(4-methylpiperazin-1-yl)-1H-benzimidazol-2-yl]quinolin-2(1H)-one is Cytotoxic to FGFR3-Expressing Myeloma Cells

To assess the potential of 4-amino-5-fluoro-3-[6-(4-methylpiperazin-1-yl)-1H-benzimidazol-2-yl]quinolin-2(1H)-one as a therapeutic agent in MM, the effect of 4-amino-5-fluoro-3-[6-(4-methylpiperazin-1-yl)-1H-benzimidazol-2-yl]quinolin-2(1H)-one on the growth and survival of human myeloma cell lines was also investigated. FGFR3 positive cell lines (KMS11, KMS18, OPM2, H929) and the FGFR3 negative cell lines, U266 and 8226 were incubated with increasing concentrations of 4-amino-5-fluoro-3-[6-(4-methylpiperazin-1-yl)-1H-benzimidazol-2-yl]quinolin-2(1H)-one and cell viability was monitored (Table 10). 4-Amino-5-fluoro-3-[6-(4-methylpiperazin-1-yl)-1H-benzimidazol-2-yl]quinolin-2(1H)-one inhibited cell proliferation of KMS11 (FGFR3-Y373C) and OPM2 (FGFR3-K650E), and KMS18 (FGFR3-G384D) cells with IC₅₀ of values of 90 nM (KMS11 and OPM2) and 550 nM respectively. FGFR3 negative cell lines and H929 (FGFR3-WT), a cell line that harbors a downstream activating mutation of N-Ras (Chesi, M. et al., Blood, 2001; 97:729-736), were resistant, requiring greater than 5-fold higher concentrations to inhibit cell growth. Inhibition of cellular growth was associated with disappearance of downstream ERK1/2 phosphorylation as determined by flow cytometry. The 4-amino-5-fluoro-3-[6-(4-methylpiperazin-1-yl)-1H-benzimidazol-2-yl]quinolin-2(1H)-one sensitive cell lines (KMS11, KMS18, OPM2) all demonstrated loss of ERK1/2 phosphorylation in the presence of effective doses of 4-amino-5-fluoro-3-[6-(4-methylpiperazin-1-yl)-1H-benzimidazol-2-yl]quinolin-2(1H)-one. In contrast, H929 cells, which displayed minimal cytostatic response to 4-amino-5-fluoro-3-[6-(4-methylpiperazin-1-yl)-1H-benzimidazol-2-yl]quinolin-2(1H)-one, demonstrated high basal levels of MAP kinase activation as a result of constitutive Ras activation and showed no change in ERK1/2 phosphorylation, indicating that 4-amino-5-fluoro-3-[6-(4-methylpiperazin-1-yl)-1H-benzimidazol-2-yl]quinolin-2(1H)-one is acting upstream of Ras.

**Table 10. IC₅₀ values (in nM) of 4-Amino-5-fluoro-3-[6-(4-methylpiperazin-1-yl)-1H-benzimidazol-2-yl]quinolin-2(1H)-one Against Human Myeloma Cell Lines.**

| **Cell line** | **T(4;14)** | **FGFR3 genotype** | **IC₅₀(nM)** |
|---|---|---|---|
| KMS11 | + | Y373C | 90 |
| KMS18 | + | G384D | 550 |
| OPM2 | + | K650E | 90 |
| H929 | + | WT | >2500 |
| 8226 | - | N/D | >2500 |
| U266 | - | N/D | >2500 |

| | | | |
|---|---|---|---|
| Listed are MM cell lines and the presence (+) or absence (-) of the t(4;14) translocation and the FGFR3 mutations. WT denotes the wild-type genotype and N/D means not determined. The concentration of 4-amino-5-fluoro-3-[6-(4-methylpiperazin-1-yl)-1H-benzimidazol-2-yl]quinolin-2(1H)-one that inhibits 50% viability (IC₅₀) as compared to DMSO control (MTT assay or Cell titer Glo) after 72 hours incubation with 4-Amino-5-fluoro-3-[6-(4-methylpiperazin-1-yl)-1H-benzimidazol-2-yl]quinolin-2(1H)-one was determined. | | | |

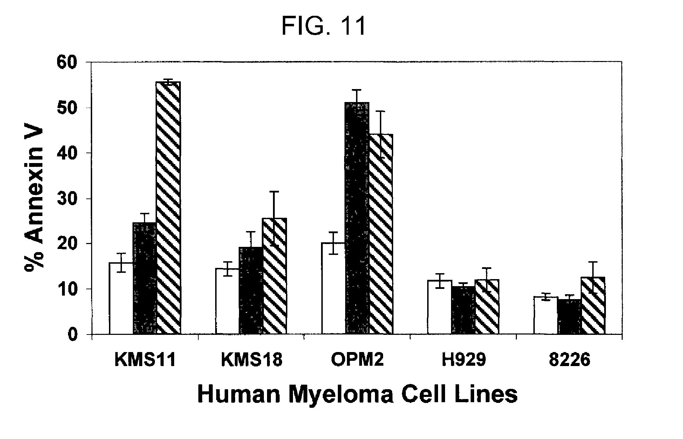

4-Amino-5-fluoro-3-[6-(4-methylpiperazin-1-yl)-1H-benzimidazol-2-yl]quinolin-2(1H)-one also induced apoptosis in responsive FGFR3 expressing cell lines. Treatment of KMS11, OPM2, and KMS18 cells with 500 nM 4-amino-5-fluoro-3-[6-(4-methylpiperazin-1-yl)-1H-benzimidazol-2-yl]quinolin-2(1H)-one for 96 hours resulted in a significant increase in the percentage of annexin-V binding cells when compared to DMSO controls (FIG. 11). The delayed induction of apoptosis observed in some myeloma cell lines is similar to that previously reported with the more selective FGFR3 inhibitor, PD173074. Trudel, S. et al., Blood, 2004; 103:3521-3528. Treatment of FGFR3-negative cells (U266 not shown) had no effect on annexin V-binding suggesting that class III and V RTKs that can potentially be inhibited by 4-amino-5-fluoro-3-[6-(4-methylpiperazin-1-yl)-1H-benzimidazol-2-yl]quinolin-2(1H)-one are not expressed or are not essential for survival of these myeloma cells.

The cytotoxic potential of 4-amino-5-fluoro-3-[6-(4-methylpiperazin-1-yl)-1H-benzimidazol-2-yl]quinolin-2(1H)-one was assessed against primary human myeloma cells. Freshly isolated BM mononuclear cells were obtained from patients previously identified by FISH as t(4;14) positive or negative. Chang, H. et al., Br. J. Haematol., 2004; 125:64-68. The presence or absence of FGFR3 expression was confirmed by flow cytometry (FIG. 12A). Of the five t(4;14) positive samples, all but one demonstrated high level expression of FGFR3 on CD138 positive myeloma cells (Table 10). In addition, these samples were screened by DHPLC for FGFR3 mutations and downstream mutations ofN and K-Ras. Results were confirmed by sequence analysis. No mutations were identified. FGF stimulation of primary cells in culture resulted in upregulation of ERK1/2 phosphorylation in CD138 positive myeloma cells demonstrating biological activity of FGFR3 in these cells (FIG. 12B). 4-amino-5-fluoro-3-[6-(4-methylpiperazin-1-yl)-1H-benzimidazol-2-yl]quinolin-2(1H)-one at 500 nM fully inhibited ERK1/2 phosphorylation in all samples. In addition, mononuclear cells were cultured with 500 nM 4-amino-5-fluoro-3-[6-(4-methylpiperazin-1-yl)-1H-benzimidazol-2-yl]quinolin-2(1H)-one or DMSO vehicle and apoptosis was determined by annexin V staining. Four of five t(4;14) myeloma samples demonstrated a cytotoxic response to 4-amino-5-fluoro-3-[6-(4-methylpiperazin-1-yl)-1H-benzimidazol-2-yl]quinolin-2(1H)-one when compared to vehicle control whereas none of the other myeloma samples were affected (FIGS. 12C and 12D and Table 11). Interestingly, the t(4;14) positive sample that demonstrated low level FGFR3 expression was 4-amino-5-fluoro-3-[6-(4-methylpiperazin-1-yl)-1H-benzimidazol-2-yl]quinolin-2(1H)-one resistant implying that only high level of WT FGFR3 expression can confer dependence. Support for this hypothesis is provided by studies of c-KIT (Rubin, B. P. et al., Cancer Res., 2001; 61:8118-8121) in gastrointestinal tumors and FLT3 (Armstrong, S. A. et al., Cancer Cell, 2003; 3:173-183) in AML where high level expression of the WT receptor, as well as receptor mutation, lead to constitutive activity and inhibitor sensitivity. Furthermore, sensitivity to Herceptin in breast cancer correlates with the level of HER2/neu expression. Vogel, C. L. et al., J. Clin. Oncol., 2002; 20:719-726. Alternatively, MM cells from this patient may have activation of additional pathways, that circumvent dependency on FGFR3 signaling.

**Table 11. Summary of Expression of FGFR3 on Primary MM Cells in Relation to Sensitivity to 4-Amino-5-fluoro-3-[6-(4-methylpiperazin-1-yl)-1H-benzimidazol-2-yl]quinolin-2(1H)-one (Compound).**

| **Patient** | **FGFR3 (flow cytometry)** | **FGFR3 genotype** | **N&K-Ras genotype** | **% Annexin V DMSO** | **% Annexin V Compound (500 nM)** | **% Increase Annexin V** |
|---|---|---|---|---|---|---|
| 1 | N/D | WT | WT | 9.0 | 21.8 | 20.9 |
| 2 | + | WT | WT | 10.4 | 8.6 | -1.8 |
| 3 | ++ | WT | WT | 9.8 | 42.1 | 32.3 |
| 4 | ++ | WT | WT | 6.8 | 25.7 | 18.9 |
| 5 | +++ | WT | WT | 10.1 | 24.5 | 14.4 |
| 6 | - | N/D | N/D | 8.8 | 10.2 | 1.4 |
| 7 | - | N/D | N/D | 15.3 | 16.0 | 0.7 |
| 8 | - | N/D | N/D | 20.9 | 20.7 | -0.2 |
| 9 | - | N/D | N/D | 12.8 | 13.4 | 0.6 |
| 10 | - | N/D | N/D | 15.0 | 17.1 | 2.1 |

| | | | | | | |
|---|---|---|---|---|---|---|
| FGFR3 expression on CD138 primary MM cells was analyzed by flow cytometry and the fluorescence was expressed as follows: +, weak; ++ intermediate; +++ strong; -, absent. CD138 selected cells were screened for the FGFR3 and N and K-Ras mutations. WT denotes wild-type status and N/D indicates not determined. | | | | | | |

### Effect of IL-6, IGF-1 and Stroma on Response of MM cells to 4-Amino-5-fluoro-3-[6-(4-methylpiperazin-1-yl)-1H-benzimidazol-2-yl]quinolin-2(1H)-one

Given the known role of IL-6 (Klein, B. et al., Blood, 1995; 85:863-872; and Anderson, K. C. et al., Semin. Hematol., 1999; 36:14-20) and more recently, IGF-1 (Ogawa, M. et al., Cancer Res., 2000; 60:4262-4269; and Mitsiades, C. S. et al., Cancer Cell, 2004; 5:221-230) in tumor cell proliferation, survival and drug resistance in MM, experiments were performed to determine whether exogenous IL-6 and IGF-1 could overcome the growth inhibitory effects produced by 4-amino-5-fluoro-3-[6-(4-methylpiperazin-1-yl)-1H-benzimidazol-2-yl]quinolin-2(1H)-one. Inhibition with 4-amino-5-fluoro-3-[6-(4-methylpiperazin-1-yl)-1H-benzimidazol-2-yl]quinolin-2(1H)-one was still observed when KMS11 cells were grown in the presence of 50 ng/ml IL-6 or 50 ng/ml IGF-1 and was comparable to that of cells cultured in the presence of aFGF (FIG. 13A). These studies highlight the critical role of FGFR3 function in the hierarchy of growth factor receptors in these cells.

Because the BM microenvironment has been shown to confer drug resistance in MM cells (Dalton, W. S. et al., Semin Hematol., 2004; 41:1-5; and Hideshima, T. et al., Semin. Oncol., 2001; 28:607-612), the effect of 4-amino-5-fluoro-3-[6-(4-methylpiperazin-1-yl)-1H-benzimidazol-2-yl]quinolin-2(1H)-one on MM cell growth was investigated in the BM milieu. The direct toxicity of 4-amino-5-fluoro-3-[6-(4-methylpiperazin-1-yl)-1H-benzimidazol-2-yl]quinolin-2(1H)-one on BMSCs was determined using the MTT assay, and no significant difference in cell viability of 4-amino-5-fluoro-3-[6-(4-methylpiperazin-1-yl)-1H-benzimidazol-2-yl]quinolin-2(1H)-one treated cells compared to DMSO controls (FIG. 13B) was observed. KMS11 1 cells were then cultured with or without BMSCs in the presence or absence of 4-amino-5-fluoro-3-[6-(4-methylpiperazin-1-yl)-1H-benzimidazol-2-yl]quinolin-2(1H)-one. BMSCs did confer a modest degree of resistance with 44.6% growth inhibition for cells treated with 500 nM 4-amino-5-fluoro-3-[6-(4-methylpiperazin-1-yl)-1H-benzimidazol-2-yl]quinolin-2(1H)-one and cultured on stroma compared to with 71.6% growth inhibition for cells grown without BMSCs. However, cell growth was still significantly inhibited by the 4-amino-5-fluoro-3-[6-(4-methylpiperazin-1-yl)-1H-benzimidazol-2-yl]quinolin-2(1H)-one despite the presence of stroma.

### 4-Amino-5-fluoro-3-[6-(4-methylpiperazin-1-yl)-1H-benzimidazol-2-yl]quinolin-2(1H)-one Augments Dexamethasone Cytotoxicity in Multiple Myeloma

FGFR3 expression results in increased STAT3 phosphorylation and higher levels of Bcl-XL expression than that observed in parental B9 cells after IL-6 withdrawas. Plowright, E. E. et al., Blood, 2000; 95:992-998; and Pollett, J. B. et al., Blood, 2002; 100:3819-3821. These findings were associated with inhibition of dexamethasone-induced apoptosis, a phenomenon that was reversed by Bcl-XL antisense oligonucleotide. Treatment of FGFR3 expressing MM cells may, thus overcome resistance to dexamethasone. As shown in Table 12, KMS11 1 cells are relatively resistant to dexamethasone; however, when combined with 4-amino-5-fluoro-3-[6-(4-methylpiperazin-1-yl)-1H-benzimidazol-2-yl]quinolin-2(1H)-one, synergistic inhibitory effects were observed. These data indicates the usefulness of combining dexamethasone with 4-amino-5-fluoro-3-[6-(4-methylpiperazin-1-yl)-1H-benzimidazol-2-yl]quinolin-2(1H)-one as a therapeutic strategy.

**Table 12. Effect of 4-Amino-5-fluoro-3-[6-(4-methylpiperazin-1-yl)-1H-benzimidazol-2-yl]quinolin-2(1H)-one (Compound) and/or Dexamethasone on KSM11 Viability.**

| **Treatment (concentration)** | **Viability (% of control) ± SD** |
|---|---|
| DMSO | 100% |
| Dexamethasone (0.5 M) | 87% ± 4.74 |
| Compound (100 nM) | 49% ± 4.64 |
| Dexamethasone (0.5 M) and Compound (100 nM) | 10% ± 6.48 |

### 4-Amino-5-fluoro-3-[6-(4-methylpiperazin-1-yl)-1H-benzimidazol-2-yl] quinolin-2(1H)-one Inhibits M-CSF Mediated Cell Growth

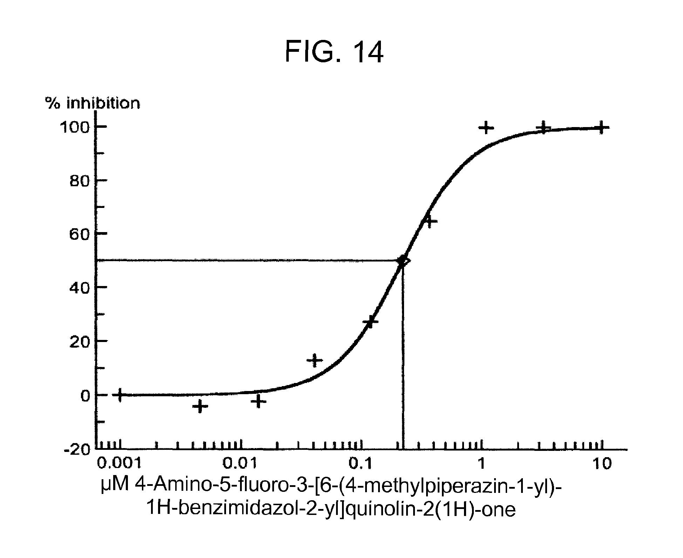

Osteolytic bone loss is one of the major complications in MM. The major osteoclast activating factors involved in bone resorption are IL-1 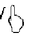 , IL-6, RANK-L and M-CSF. Croucher, P. I. et al., Br. J. Haemaatol., 1998; 103:902-910. MM cells, osteoblasts and stromal cells in the BM express M-CSF which together with RANK-L is essential for osteoclast formation. Quinn, J. M. et al., Endocrinology, 1998; 139:4424-4427. Increased serum concentrations of MCSF have been detected in MM patients. Janowska-Wieczorek, A. et al., Blood, 1991; 77:1796-1803. *In vitro* kinase assays demonstrate potent activity of 4-amino-5-fluoro-3-[6-(4-methylpiperazin-1-yl)-1H-benzimidazol-2-yl]quinolin-2(1H)-one against CSF-1R, the only known receptor for M-CSF with an IC₅₀ of 36 nM (Table 9). 4-Amino-5-fluoro-**3-[6-(4-methylpiperazin-1-yl)-1H-benzimidazol-2-yl]quinolin-2(1H)-one** inhibited proliferation of M-NFS-60, a M-CSF growth driven mouse myeloblastic cell line with an EC₅₀ of 220 nM (FIG. 14). It would appear, therefore, that in addition to inhibiting MM cell growth, 4-amino-5-fluoro-3-[6-(4-methylpiperazin-1-yl)-1H-benzimidazol-2-yl]quinolin-2(1H)-one has the advantage of potentially inhibiting tumor-associated osteolysis.

### Evaluation of 4-Amino-5-fluoro-3-[6-(4-methylpiperazin-1-yl)-1H-benzimidazol-2-yl]quinolin-2-(1H)-one in vivo in a Xenograft Mouse Model

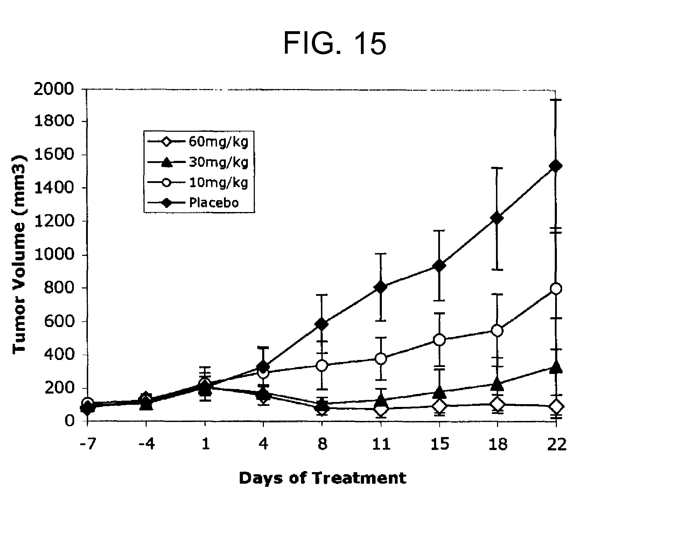

The efficacy of 4-amino-5-fluoro-3-[6-(4-methylpiperazin-1-yl)-1H-benzimidazol-2-yl]quinolin-2(1H)-one was tested in a murine model in which KMS11 cells are injected subcutaneously into BNX mice. Grad, J. M. et al., Blood, 2001; 805-813; and Lentzsch, S. et al., Leukemia, 2003; 17:41-44. A similar plasmacytoma xenograft mouse model has been used in pre-clinical studies of Bortezomib and IMiDs in MM. Each of 36 BNX mice were injected in the flank with 3 x 10⁷ KMS11 cells together with matrigel by s.c. injection. When the tumors reached approximately 200 mm³, mice were randomized (n=8-10) to receive vehicle or 4-amino-5-fluoro-3-[6-(4-methylpiperazin-1-yl)-1H-benzimidazol-2-yl]quinolin-2(1H)-one at 10 mg/kg, 30 mg/kg and 60 mg/kg, administered by oral gavage once daily for 21 days. When compared to vehicle controls, a significant (p<0.001) anti-tumor effect was observed in all three 4-amino-5-fluoro-3-[6-(4-methylpiperazin-1-yl)-1H-benzimidazol-2-yl]quinolin-2(1H)-one dose groups with a minimum effective dose of 10 mg/kg/d (FIG. 15). Specifically, 48%, 78.5% and 94% growth inhibition was calculated in the 10 mg/kg, 30 mg/kg and 60 mg/kg treatment arms, respectively, compared to the placebo treated mice. On the last day of dosing, 7 of 10 mice in the highest treatment group had achieved and maintained a partial remission with > 50% reduction in tumor volumes compared to day 1 of drug administration. Weight loss, as a marker of significant toxicity, was not observed in any of the treatment groups.

To demonstrate that the observed responses correlated with FGFR3 inhibition, mice were sacrificed 4 hours after receiving the last dose of 4-amino-5-fluoro-3-[6-(4-methylpiperazin-1-yl)-1H-benzimidazol-2-yl]quinolin-2(1H)-one and tumors were harvested for analysis of *in vivo* inhibition of FGFR3 phosphorylation. FGFR3 was immunoprecipitated from tumor cell lysates and the level of expression and phosphorylation was determined on immunoblots. *In vivo* inhibition of FGFR3 was observed, with complete inhibition of FGFR3 occurring at the 60 mg/kg dose. Inhibition of FGFR3 phosphorylation was dose dependent and correlated with the anti-tumor response.

Histopathologic examination of the tumors from representative animals further supported the interpretation of tumor reduction in the drug-treated mice compared to the placebo controls. Tumors from the drug-treated mice showed large areas of tumor necrosis. Immunohistochemistry for expression of the proliferative antigen, Ki-67, and for cleaved caspase 3, demonstrated that 4-amino-5-fluoro-3-[6-(4-methylpiperazin-1-yl)-1H-benzimidazol-2-yl]quinolin-2(1H)-one inhibited cell growth and induced apoptosis. These findings suggest that 4-amino-5-fluoro-3-[6-(4-methylpiperazin-1-yl)-1H-benzimidazol-2-yl]quinolin-2(1H)-one induces both cytostatic and cytotoxic responses *in vivo* resulting in regression of FGFR3 expressing tumors.

### DISCUSSION

The identification of recurrent cytogenetic abnormalities in MM and characterization of the translocation partners has identified novel molecular targets and presents the potential for molecular targeted therapy for this universally fatal disease. Kuehl, W. M. et al., Nat Rev Cancer, 2002; 2:175-187; and Chesi, M. et al., Nat. Genet., 1997; 16:260-265. Nearly 20% of newly diagnosed cases of MM harbor the t(4;14) translocation as detected by the presence of IgH-MMSET hybrid transcript (Santra, M. et al., Blood, 2003; 101:2374-2376), the presence of which has generally been reported to be associated with a poor-prognosis. Fonseca, R. et al., Blood, 2003; 101:4569-4575; Keats, J. J. et al., Blood, 2003; 101:1520-1529; Moreau, P. et al., Blood, 2002; 100:1579-1583.; and Chang, H. et al., Br. J. Haematol., 2004; 125:64-68. FGFR3 is expressed in approximately 70% (Keats, J. J. et al., Blood, 2003; 101:1520-1529; and Quinn, J. M. et al., Endocrinology, 1998; 139:4424-4427) of these cases and 10% (Intini, D. et al., Br. J. Haematol., 2001; 114:362-364) of patients will acquire an activating mutation of FGFR3 with disease progression.

An understanding of the genetic defects that are causally implicated in oncogenesis has led to targeted therapy for the treatment of a number of cancers. Druker, B. J. et al., N. Engl. J. Med., 2001; 344:1031-1037; Demetri, G. D. et al., N. Engl. J. Med., 2002; 347:472-480; Slamon, D. J. et al., N. Engl. J. Med. 2001; 344:783-792; and Smith, B. D. et al., Blood, 2004; 103:3669-3676. Most notably, the inhibition of BCR-ABL kinase activity by STI571 has produced major cytogenetic remissions in chronic myelogenous leukemia (CML). Druker, B. J. et al., N. Engl. J. Med., 2001; 344:1031-1037. Inhibition of activated c-Kit in gastrointestinal stromal tumors by STI571 has also been effective against this chemoresistant tumor. Demetri, G. D. et al., N. Engl. J. Med., 2002; 347:472-480. In addition, Herceptin, a monoclonal antibody targeting HER2/neu, has resulted in improved chemotherapy responses and prolonged survival of breast cancer patients. Slamon, D. J. et al., N. Engl. J. Med. 2001; 344:783-792. A similar kinase inhibitor strategy targeting FLT3 in acute myeloid leukemia (AML) is also showing promising results in Phase II clinical trial. Smith, B. D. et al., Blood, 2004; 103:3669-3676. Pre-clinical studies of FGFR3 inhibition in t(4;14) myeloma have likewise identified this RTK as a plausible candidate for targeted therapy. Two antagonists of FGFR3, PD173074 and SU5402 inhibited the growth and induced apoptosis of MM cells expressing mutant FGFR3. Trudel, S. et al., Blood, 2004; 103:3521-3528; Paterson, J. L. et al., Br. J. Haematol., 2004; 124:595-603; and Grand ,E. K. et al., Leukemia, 2004; 18:962-966. Together these studies support the clinical development of FGFR3 inhibitors for these patients. Unfortunately, PD173074 is not a candidate compound for the clinic and the IC₅₀ of SU5402, required to inhibit FGFR3 is not likely to the achieved *in vivo.*

4-Amino-5-fluoro-3-[6-(4-methylpiperazin-1-yl)-1H-benzimidazol-2-yl]quinolin-2(1H)-one is a potent inhibitor of FGFR3 and class III, IV and V RTKs including, FLT3, c-Kit, c-Fms, PDGFR and VEGFR. In this study, 4-amino-5-fluoro-3-[6-(4-methylpiperazin-1-yl)-1H-benzimidazol-2-yl]quinolin-2(1H)-one was demonstrated to be a highly active inhibitor of both WT and mutant FGFR3 17 tyrosine kinases. The activity of this inhibitor against a broad spectrum of RTKs implies that 4-amino-5-fluoro-3-[6-(4-methylpiperazin-1-yl)-1H-benzimidazol-2-yl]quinolin-2(1H)-one requires less stringent conformation requirements for binding to the kinase domain and is consistent with the retained activity of 4-amino-5-fluoro-3-[6-(4-methylpiperazin-1-yl)-1H-benzimidazol-2-yl]quinolin-2(1H)-one against many FGFR3 mutants. 4-Amino-5-fluoro-3-[6-(4-methylpiperazin-1-yl)-1H-benzimidazol-2-yl]quinolin-2(1H)-one treatment selectively induced apoptotic cell death of MM cell lines and primary patient samples that harbor FGFR3. The potential clinical application of 4-amino-5-fluoro-3-[6-(4-methylpiperazin-1-yl)-1H-benzimidazol-2-yl]quinolin-2(1H)-one for the treatment of MM was further validated using a xenograft mouse model in which 4-amino-5-fluoro-3-[6-(4-methylpiperazin-1-yl)-1H-benzimidazol-2-yl]quinolin-2(1H)-one treatment inhibited FGFR3 activity *in vivo* and produced tumor regression and significantly decrease disease progression.

Although the data suggests that FGFR3 is the primary target of 4-amino-5-fluoro-3-[6-(4-methylpiperazin-1-yl)-1H-benzimidazol-2-yl]quinolin-2(1H)-one in MM cells, it is important to note that OPM2 cells responded to this broadly active RTK inhibitor when they did not respond to the more selective FGFR3 inhibitor PD173074. Trudel, S. et al., Blood, 2004; 103:3521-3528; and Paterson, J. L. et al., Br. J. Haematol., 2004; 124:595-603. This cell line is characterized by high basal levels of AKT phosphorylation (data not shown) and biallelic PTEN deletion. Consistent with our results, Grand et al. demonstrated that the multi-targeted RTK inhibitor, SU5402 induced cytotoxic responses in OPM2 cells whereas PD173074 failed to induce apoptosis. Grand ,E. K. et al., Leukemia, 2004; 18:962-966. These findings also raise the possibility that 4-amino-5-fluoro-3-[6-(4-methylpiperazin-1-yl)-1H-benzimidazol-2-yl]quinolin-2(1H)-one is targeting other, as yet to be defined, targets important for myeloma cell viability, a fact that is of further relevance given the demonstration that FGFR3 is sometimes lost during disease progression and may, therefore, be supplanted by other downstream signaling mediators.

With the latter point in mind, it is important to note that the clinical relevance of FGFR3 in t(4;14) myeloma has been questioned by observations that the der(14) chromosome is lost in some myeloma patients suggesting that FGFR3 is dispensible and that MMSET is the true causal target of t(4;14) in MM. Keats, J. J. et al., Blood, 2003; 101:1520-1529; and Intini, D. et al., Br. J. Haematol., 2001; 114:362-364. Moreover, studies in model systems indicate that WT FGFR3 is not dominantly transforming, requiring additional cooperating oncogenic events to complement transformation. Chesi, M. et al., Blood, 2001; 97:729-736; and Li, Z. et al., Blood, 2001; 97:2413-2419. The data presented above, however, indicates that primary MM cells that definitively express FGFR3 remain dependent on this pathway for survival despite the presence of additional genetic events. It is likely, therefore, that FGFR3 acts in concert with TACC3 and MMSET providing survival signals through the stimulation by FGF ligands expressed in the BM microenvironment. Along these lines, FLT3 mutations and high level expression of FLT3 have been described in acute lymphoblastic leukemia where MLL, a gene similar to MMSET, is also expressed. Armstrong, S. A. et al., Cancer Cell, 2003; 3:173-183. These observations suggest a possible mechanism of complementation between tyrosine kinases and trithorax genes.

Studies of FGFR3 inhibition in MM cell lines indicated that only cell lines expressing the constitutively active receptor responded to FGFR3 inhibition. Trudel, S. et al., Blood, 2004; 103:3521-3528; and Paterson, J. L. et al., Br. J. Haematol., 2004; 124:595-603. This highlights the limitation of using MM cell lines that grow independently of BM microenvironment and, thus, are no longer reliant on FGF produced by the stroma for growth and survival. Studies using primary patient material are therefore critical. The cytotoxic effect demonstrated by primary MM cells exposed to 4-amino-5-fluoro-3-[6-(4-methylpiperazin-1-yl)-1H-benzimidazol-2-yl]quinolin-2(1H)-one indicates that this drug will be an effective therapy in patients expressing either WT or mutant FGFR3. Nevertheless, the only modest and delayed cytotoxic response to 4-amino-5-fluoro-3-[6-(4-methylpiperazin-1-yl)-1H-benzimidazol-2-yl]quinolin-2(1H)-one observed in primary MM cells may imply that inhibition of WT FGFR3 does not itself introduce proapoptotic signal, but more likely results in the withdrawal of strong anti-apoptotic signals. One would predict, therefore, the most effective use of 4-amino-5-fluoro-3-[6-(4-methylpiperazin-1-yl)-1H-benzimidazol-2-yl]quinolin-2(1H)-one may be in combination with chemotherapeutic agents such as dexamethasone as demonstrated in KMS 11 cells.

The importance of the BM microenvironment in supporting tumor growth is becoming increasingly clear. Mitsiades, C. S. et al., Cancer Cell, 2004; 5:221-230; and Dalton, W. S. et al., Semin Hematol., 2004; 41:1-5. In particular, cytokines such as IL-6 and IGF-1 and direct interaction with BMSCs have been shown to confer drug resistance. The *in vitro* experiments demonstrate that these paracrine factors failed to overcome the anti-tumor effects of 4-amino-5-fluoro-3-[6-(4-methylpiperazin-l-yl)-1H-benzimidazol-2-yl]quinolin-2(1H)-one. Given its target profile, 4-amino-5-fluoro-3-[6-(4-methylpiperazin-1-yl)-1H-benzimidazol-2-yl]quinolin-2(1H)-one may also impact host-derived tumor-associated cells within the BM that have implications in supporting tumor growth. 4-Amino-5-fluoro-3-[6-(4-methylpiperazin-1-yl)-1H-benzimidazol-2-yl]quinolin-2(1H)-one exhibits potent antiangiogenic activity in several angiogenesis assays including endothelial cell migration and tube formation on fibrin gels as well as in the ex vivo rat aortic ring assay. Wiesmann, M. et al., Proc AACR, 2003; 44:934a. In agreement, tumors from 4-amino-5-fluoro-3-[6-(4-methylpiperazin-1-yl)-1H-benzimidazol-2-yl]quinolin-2(1H)-one treated mice were less vascular when compared to controls (data not shown). It has been demonstrated that 4-amino-5-fluoro-3-[6-(4-methylpiperazin-1-yl)-1H-benzimidazol-2-yl]quinolin-2(1H)-one also inhibits CSF-1R activity, the receptor for M-CSF, an osteoclast activating factor that may contribute to pathogenesis of bone disease in MM. Taken together, the data suggests that 4-amino-5-fluoro-3-[6-(4-methylpiperazin-1-yl)-1H-benzimidazol-2-yl]quinolin-2(1H)-one can potentially target both the MM cell within the BM milieu and the BM microenvironment directly.

In summary, 4-amino-5-fluoro-3-[6-(4-methylpiperazin-1-yl)-1H-benzimidazol-2-yl]quinolin-2(1H)-one represents a novel and potent small molecule inhibitor of FGFR3 for the treatment of t(4;14) myeloma. The cytotoxic effects of 4-amino-5-fluoro-3-[6-(4-methylpiperazin-1-yl)-1H-benzimidazol-2-yl]quinolin-2(1H)-one on MM cell lines and primary patient samples, and a target profile that suggests the potential to favorably modulate the BM milieu, lead to the prediction that this will be an effective therapy in this poor-prognosis group, particularly in combination therapies. The ultimate success of this therapeutic strategy now awaits the outcome of clinical trials of that are soon to be underway to evaluate the efficacy of 4-amino-5-fluoro-3-[6-(4-methylpiperazin-1-yl)-1H-benzimidazol-2-yl]quinolin-2(1H)-one of the treatment of t(4;14) MM.

The preparation of numerous 4-amino substituted quinolinone benzimidazolyl compounds, pharmaceutical formulations thereof, and descriptions of salts and tautomers thereof are set forth in the following U.S. Patent, U.S. Patent Applications, and U.S. Provisional Applications each of which is hereby incorporated by reference in its entirety and for all purposes as if fully set forth herein: U.S. Patent No. 6,605,617; U.S. Patent Application No. 10/644,055; U.S. Patent Application No. 10/706,328; U.S. Provisional Application No. 60/526,426; U.S. Provisional Application No. 60/517,915, and U.S. Provisional Application No. 60/546,017.

It should be understood that the organic compounds according to the invention may exhibit the phenomenon of tautomerism. As the chemical structures within this specification can only represent one of the possible tautomeric forms at a time, it should be understood that the invention encompasses any tautomeric form of the drawn structure. For example, the compound of formula IIIB is shown below with one tautomer, Tautomer IIIBa:

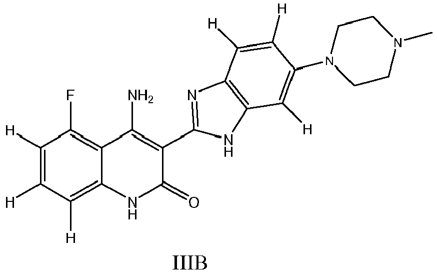

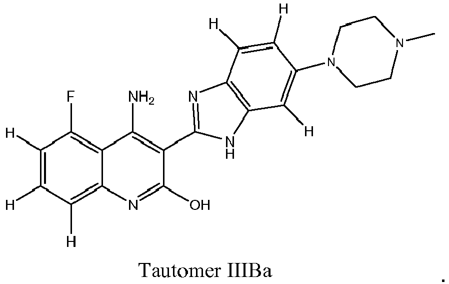

Other tautomers of the compound of formula IIIB, Tautomer IIIIBb and Tautomer IIIIBc, are shown below:

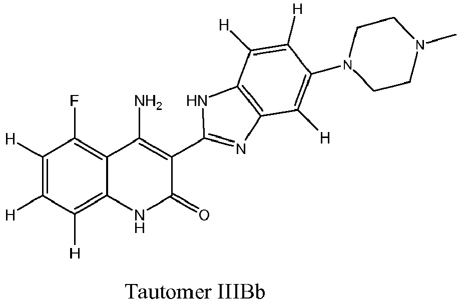

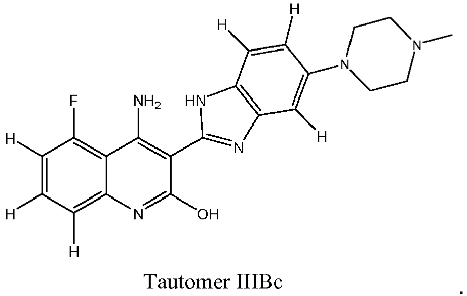

All documents or references cited herein are hereby incorporated by reference in their entireties and for all purposes as if fully set forth herein.

It is understood that the invention is not limited to the embodiments set forth herein for illustration, but embraces all such forms thereof as come within the scope of the claims.

## Claims

1. A salt of 4-amino-5-fluoro-3-[6-(4-methylpiperazin-1-yl)-1H-benzimidazol-2-yl]quinolin-2(1H)-one or a tautomer thereof, wherein the salt is a malate, mesylate, bismesylate, tartrate, bishydrochloride, citrate, acetate, or bis-acetate salt.

2. The salt according to claim 1, wherein the salt is the malate salt.

3. The salt according to claim 1, wherein the salt is the mesylate salt.

4. The salt according to claim 1, wherein the salt is the bismesylate salt.

5. Use of the lactate salt according to any one of claims 1 to 4 in the preparation of a medicament for:
i) inhibiting the proliferation of capillaries; or
ii) treating cancer.

6. The salt according to any one of claims 1 to 4 for use in a method of inhibiting the proliferation of capillaries.

7. The salt according to any one of claims 1 to 4 for use in a method of treating cancer.
